# EUROPEAN PATENT APPLICATION

(11) **EP 4 524 654 A1**
(43) Date of publication of application: **19.03.2025**
(21) Application number: 24200165.9
(22) Date of filing: 13.09.2024
(51) Int. Cl.: G03F 7/004, G03F 7/039

(54) **ONIUM SALT, CHEMICALLY AMPLIFIED POSITIVE RESIST COMPOSITION, AND RESIST PATTERN FORMING PROCESS**

(30) Priority: 13.09.2023 JP 2023148154
(71) Applicant: SHIN-ETSU CHEMICAL CO., LTD., Tokyo 1000005 (JP)
(72) Inventor: Fukushima, Masahiro, NIIGATA-KEN (JP); Watanabe, Satoshi, NIIGATA-KEN (JP); Kotake, Masaaki, NIIGATA-KEN (JP); Matsuzawa, Yuta, NIIGATA-KEN (JP); Masunaga, Keiichi, NIIGATA-KEN (JP)
(74) Representative: Ter Meer Steinmeister & Partner

(57) **Abstract**

An onium salt containing an aromatic sulfonic acid anion having an alkyl or fluoroalkyl group, iodized aromatic ring, and fluorinated substituent group generates an acid with controlled diffusion. A chemically amplified positive resist composition comprising the onium salt is also provided.

## Description

### TECHNICAL FIELD

This invention relates to an onium salt, a chemically amplified positive resist composition, and a resist pattern forming process.

### BACKGROUND ART

Pattern formation to a smaller feature size is required to meet the recent demand for higher integration in integrated circuits. Acid-catalyzed chemically amplified resist compositions are most often used in forming resist patterns with a feature size of 0.2 µm or less. High-energy radiation such as UV, deep-UV or EB is used as the energy source for exposure of these resist compositions. In particular, the EB lithography, which is utilized as the ultra-fine microfabrication technique, is also indispensable in processing a photomask blank into a photomask for use in the fabrication of semiconductor devices. Resist compositions for use in the EB lithography include positive ones wherein exposed regions are dissolved away to form a pattern and negative ones wherein exposed regions are retained to form a pattern. Either one which is easier to use is chosen in accordance with the morphology of the necessary resist pattern.

In general, the EB lithography is by writing an image with EB, without using a mask. In the case of positive resist, those regions of a resist film other than the regions to be retained are successively irradiated with EB having a minute area. In the case of negative resist, those regions of a resist film to be retained are successively irradiated with EB. The operation of successively scanning all finely divided regions on the work surface takes a long time as compared with full wafer exposure through a photomask. To prevent any throughput decline, a resist film having a high sensitivity is required. Because of a long image writing time, it is likely that a difference arises between an initially imaged portion and a lately imaged portion. The stability with time of the exposed portion in vacuum is one of the important performance factors. One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate.

Attempts were made to ameliorate resist sensitivity and pattern profile in a controlled way by properly selecting and combining components used in resist compositions and adjusting processing conditions. One outstanding problem is the diffusion of acid, which has a significant impact on the resolution of a chemically amplified resist film. In the processing of photomasks, it is required that the profile of the resist pattern resulting from exposure does not change depending on the time taken until PEB. The major cause for time-dependent changes is the diffusion of acid generated upon exposure. Since the problem of acid diffusion has large impacts on sensitivity and resolution not only in the photomask processing, but also in general resist compositions, many studies are made thereon.

Patent Documents 1 and 2 describe acid generators capable of generating bulky acids upon exposure, for thereby controlling acid diffusion and reducing roughness. Since these acid generators are still insufficient to control acid diffusion, it is desired to have an acid generator with more controlled diffusion.

Patent Document 3 discloses a resist composition comprising a base polymer having bound thereto an acid generator capable of generating a sulfonic acid upon light exposure whereby acid diffusion is controlled. This approach of controlling acid diffusion by binding repeat units capable of generating acid upon exposure to a base polymer is effective in forming a pattern with reduced LER. However, the base polymer having bound therein repeat units capable of generating acid upon exposure encounters a problem with respect to its solubility in organic solvent, depending on the structure and proportion of the relevant units.

Polymers comprising a major proportion of aromatic structure having an acidic side chain, for example, polyhydroxystyrene are useful in resist materials for the KrF lithography. These polymers are not used in resist materials for the ArF lithography since they exhibit strong absorption at a wavelength of around 200 nm. These polymers, however, are expected to form useful resist materials for the EB and EUV lithography for forming patterns of smaller size than the processing limit of ArF lithography because they offer high etching resistance.

Often used as the base polymer in positive resist compositions for EB and EUV lithography is a polymer having an acidic functional group on phenol side chain masked with an acid labile group (or acid-decomposable protective group). Upon exposure to high-energy radiation, the acid labile group is deprotected by the catalysis of an acid generated from a photoacid generator so that the polymer may turn soluble in alkaline developer. Typical of the acid labile group are tertiary alkyl, tert-butoxycarbonyl, and acetal groups. The use of protective groups (e.g., acetal groups) requiring a relatively low level of activation energy for deprotection offers the advantage that a resist film having a high sensitivity is obtainable. However, if the diffusion of generated acid is not fully controlled, deprotection reaction can occur even in the unexposed region of the resist film, giving rise to problems like a degradation of LER and a lowering of critical dimension uniformity (CDU) of pattern line width.

Patent Documents 4 and 5 describe photoacid generators capable of generating a non-fluorinated aromatic sulfonic acid having a plurality of bulky alkyl substituents. Since a plurality of alkyl substituents are introduced, the generated acid has a higher molecular weight, which is effective for suppressing acid diffusion. The control of acid diffusion is insufficient for the purpose of forming small-size patterns. There remains room for further improvement.

In forming small-size patterns by the EB lithography, there is the problem that the profile of a resist pattern is inversely tapered by the influence of back scattering resulting from reflection of EB to the substrate. The inversely tapered profile may permit the resist pattern to collapse during development. For meeting the demand for further miniaturization, it is desired to solve the outstanding problem and to have a photoacid generator capable of suppressing acid diffusion while correcting the pattern profile.

### Citation List

| | |
|---|---|
| Patent Document 1: | JP-A 2009-053518 |
| Patent Document 2: | JP-A 2010-100604 |
| Patent Document 3: | JP-A 2011-022564 |
| Patent Document 4: | JP 6248882 |
| Patent Document 5: | JP-A 2019-202974 |

### DISCLOSURE OF INVENTION

Resist compositions are recently demanded which are capable of forming not only line-and-space (LS), isolated line (IL) and isolated space (IS) patterns of satisfactory profile, but also hole patterns of satisfactory profile. The acid generator described in Patent Document 4 generates a bulky acid, indicating that acid diffusion is controlled to some extent. This acid generator, however, gives rise to a problem that when a small-size pattern is formed, the pattern takes an inversely tapered profile and collapses during development in alkaline developer.

An object of the invention is to provide an onium salt capable of generating an acid with controlled diffusion, a chemically amplified positive resist composition comprising the onium salt, and a resist pattern forming process using the composition.

The inventors have found that an onium salt containing an aromatic sulfonic acid anion having an alkyl or fluoroalkyl group, iodized aromatic ring, and fluorinated substituent group is effective as an acid generator. When a resist film is formed from a resist composition comprising the onium salt and processed by lithography, the onium salt segregates in an upper layer of the resist film due to the effect of the alkyl or fluoroalkyl group to exert a pattern profile-correcting effect. By virtue of the pattern profile-correcting effect, a pattern with satisfactory resolution, reduced LER and improved CDU is obtained. By virtue of proper dissolution inhibition, a pattern of rectangular profile is obtained.

Accordingly, the invention provides an onium salt, chemically amplified positive resist composition, and resist pattern forming process, as defined below.
1. An onium salt having the formula (A): 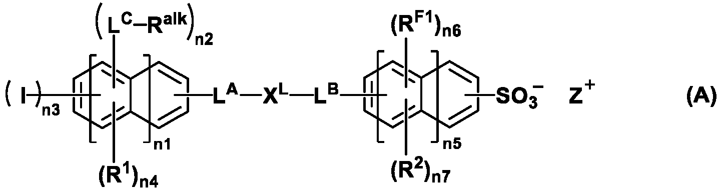 wherein n1 is 0 or 1, n2 is 1, 2 or 3, n3 is an integer of 0 to 4, n4 is an integer of 0 to 4, with the proviso that n2+n3+n4 is from 1 to 5 in case of n1=0, and n2+n3+n4 is from 1 to 7 in case of n1=1,
   n5 is 0, 1 or 2, n6 is an integer of 0 to 4 in case of n5=0, an integer of 0 to 6 in case of n5=1, and an integer of 0 to 8 in case of n5=2, n7 is an integer of 0 to 4 in case of n5=0, an integer of 0 to 6 in case of n5=1, and an integer of 0 to 8 in case of n5=2, with the proviso that n6+n7 is from 0 to 4 in case of n5=0, from 0 to 6 in case of n5=1, and from 0 to 8 in case of n5=2,
   R^{alk} is a C₆-C₁₈ alkyl group or C₄-C₁₈ fluorinated alkyl group, with the proviso that when R^{alk} is a C₆-C₁₈ alkyl group, the alkyl group has at least one straight chain structure of 6 or more carbon atoms, and when R^{alk} is a C₄-C₁₈ fluorinated alkyl group, the fluorinated alkyl group has at least two groups selected from -CF₂- and -CF₃, some -CH₂-in the alkyl or fluorinated alkyl group may be replaced by an ether bond or carbonyl moiety, and the alkyl or fluorinated alkyl group may contain a cyclic structure selected from cyclopentane, cyclohexane, adamantane, norbornane and benzene rings at the end or in a carbon-carbon bond thereof,
   R¹ is a halogen exclusive of iodine or a C₁-C₂₀ hydrocarbyl group which may contain a heteroatom,
   R² is a C₁-C₂₀ hydrocarbyl group which may contain a heteroatom, a plurality of R² may bond together to form a ring with the carbon atom to which they are attached when n7 is 2 or more,
   R^{F1} is fluorine, a C₁-C₆ fluorinated alkyl group, C₁-C₆ fluorinated alkoxy group, or C₁-C₆ fluorinated thioalkoxy group, a plurality of R^{F1} may be identical or different when n6 is 2 or more,
   L^{A}, L^{B}, and L^{C} are each independently a single bond, ether bond, ester bond, sulfonate ester bond, carbonate bond, or carbamate bond,
   X^{L} is a single bond or a C₁-C₄₀ hydrocarbylene group which may contain a heteroatom, and
   Z⁺ is an onium cation.
2. The onium salt of 1, having the formula (A1): 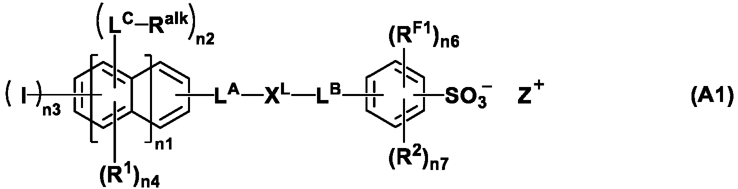 wherein n1 to n4, n6, n7, R^{alk}, R¹, R² , R^{F1}, L^{A}, L^{B}, L^{C}, X^{L}, and Z⁺ are as defined above.
3. The onium salt of 2, having the formula (A2): 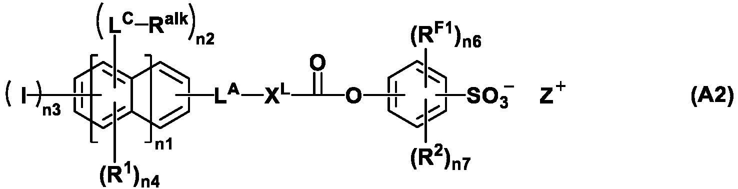 wherein n1 to n4, n6, n7, R^{alk}, R¹, R², R^{F1}, L^{A}, L^{C}, X^{L}, and Z⁺ are as defined above.
4. The onium salt of any one of 1 to 3 wherein Z⁺ is a sulfonium cation having the formula (cation-1) or iodonium cation having the formula (cation-2): 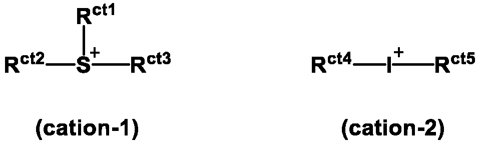 wherein R^{ct1} to R^{ct5} are each independently halogen or a C₁-C₃₀ hydrocarbyl group which may contain a heteroatom, and R^{ct1} and R^{ct2} may bond together to form a ring with the sulfur atom to which they are attached.
5. A photoacid generator comprising the onium salt of any one of 1 to 4.
6. A chemically amplified positive resist composition comprising the photoacid generator of 5.
7. The chemically amplified positive resist composition of 6, further comprising a base polymer containing a polymer which is decomposed under the action of acid to increase its solubility in alkaline developer.
8. The chemically amplified positive resist composition of 7 wherein the polymer comprises repeat units having the formula (B1): 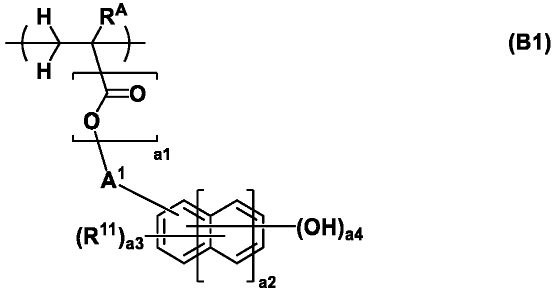 wherein a1 is 0 or 1, a2 is an integer of 0 to 2, a3 is an integer satisfying 0 ≤ a3 ≤ 5+2(a2)-a4, a4 is an integer of 1 to 3,
   R^{A} is hydrogen, fluorine, methyl or trifluoromethyl,
   R¹¹ is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group, and
   A¹ is a single bond or C₁-C₁₀ saturated hydrocarbylene group in which some -CH₂-may be replaced by -O-.
9. The chemically amplified positive resist composition of 7 or 8 wherein the polymer further comprises repeat units having the formula (B2-1): 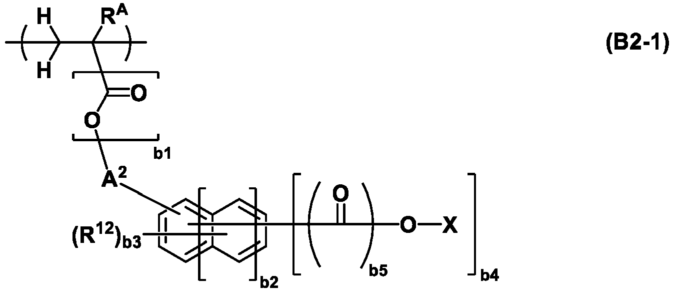 wherein b1 is 0 or 1, b2 is an integer of 0 to 2, b3 is an integer satisfying 0 ≤ b3 ≤ 5+2(b2)-b4, b4 is an integer of 1 to 3, b5 is 0 or 1,
   R^{A} is hydrogen, fluorine, methyl or trifluoromethyl,
   R¹² is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group,
   A² is a single bond or C₁-C₁₀ saturated hydrocarbylene group in which some -CH₂-may be replaced by -O-,
   X is an acid labile group when b4 is 1, and X is each independently hydrogen or an acid labile group, at least one being an acid labile group, when b4 is 2 or 3.
10. The chemically amplified positive resist composition of any one of 7 to 9 wherein the polymer further comprises repeat units having the formula (B2-2): 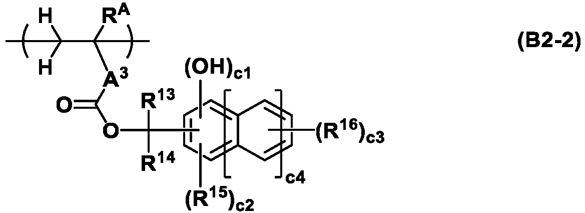 wherein c1 is an integer of 0 to 2, c2 is an integer of 0 to 2, c3 is an integer of 0 to 5, c4 is an integer of 0 to 2,
   R^{A} is hydrogen, fluorine, methyl or trifluoromethyl,
   R¹³ and R¹⁴ are each independently a C₁-C₁₀ hydrocarbyl group which may contain a heteroatom, R¹³ and R¹⁴ may bond together to form a ring with the carbon atom to which they are attached,
   R¹⁵ is each independently fluorine, C₁-C₅ fluorinated alkyl group or C₁-C₅ fluorinated alkoxy group,
   R¹⁶ is each independently a C₁-C₁₀ hydrocarbyl group which may contain a heteroatom, and
   A³ is a single bond, phenylene group, naphthylene group, or *-C(=O)-O-A³¹-, wherein A³¹ is a C₁-C₂₀ aliphatic hydrocarbylene group which may contain hydroxy, ether bond, ester bond or lactone ring, or a phenylene or naphthylene group, * designates a point of attachment to the carbon atom in the backbone.
11. The chemically amplified positive resist composition of any one of 7 to 10 wherein the polymer further comprises repeat units of at least one type selected from repeat units having the formula (B3), repeat units having the formula (B4), and repeat units having the formula (B5): 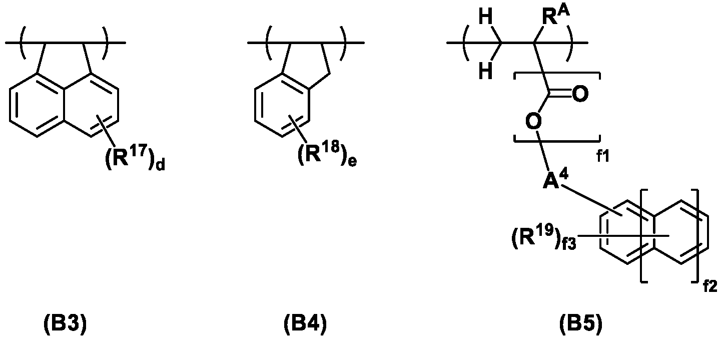 wherein d is an integer of 0 to 6, e is an integer of 0 to 4, f1 is 0 or 1, f2 is an integer of 0 to 2, and f3 is an integer of 0 to 5,
   R^{A} is hydrogen, fluorine, methyl or trifluoromethyl,
   R¹⁷ and R¹⁸ are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
   R¹⁹ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group, or cyano group, R¹⁹ may be a hydroxy group when f2 is 1 or 2, and
   A⁴ is a single bond or C₁-C₁₀ saturated hydrocarbylene group in which some -CH₂-may be replaced by -O-.
12. The chemically amplified positive resist composition of any one of 7 to 11 wherein the polymer further comprises repeat units of at least one type selected from repeat units having the formulae (B6) to (B9): 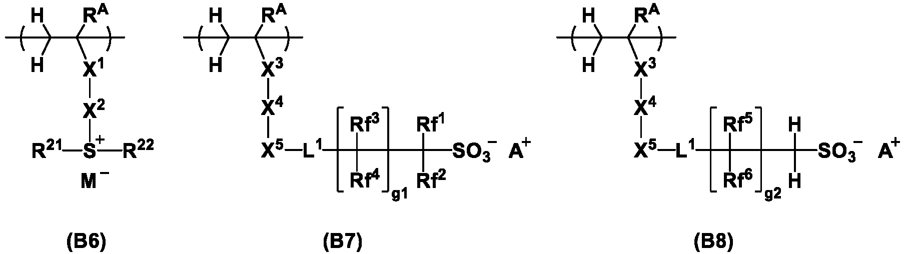 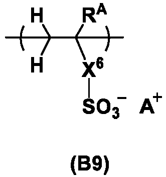 wherein R^{A} is each independently hydrogen, fluorine, methyl or trifluoromethyl,
   X¹ is a single bond or phenylene group,
   X² is *¹-C(=O)-O-X²¹-, *¹-C(=O)-NH-X²¹- or *¹-O-X²¹-, X²¹ is a C₁-C₆ aliphatic hydrocarbylene group, phenylene group, or divalent group obtained by combining the foregoing, which may contain a carbonyl moiety, ester bond, ether bond or hydroxy moiety, *¹ designates a point of attachment to X¹,
   X³ is each independently a single bond, phenylene group, naphthylene group or *²-C(=O)-O-X³¹-, X³¹ is a C₁-C₁₀ aliphatic hydrocarbylene group, phenylene group, or naphthylene group, the aliphatic hydrocarbylene group may contain a hydroxy moiety, ether bond, ester bond or lactone ring, *² designates a point of attachment to the carbon atom in the backbone,
   X⁴ is each independently a single bond, *³-X⁴¹-C(=O)-O-, *³-C(=O)-NH-X⁴¹- or *³-O-X⁴¹-, X⁴¹ is a C₁-C₂₀ hydrocarbylene group which may contain a heteroatom, *³ designates a point of attachment to X³,
   X⁵ is each independently a single bond, *⁴-X⁵¹-C(=O)-O-, *⁴-C(=O)-NH-X⁵¹- or *⁴-O-X⁵¹-, X⁵¹ is a C₁-C₂₀ hydrocarbylene group which may contain a heteroatom, *⁴ designates a point of attachment to X⁴,
   X⁶ is a single bond, methylene, ethylene, phenylene, fluorinated phenylene, trifluoromethyl-substituted phenylene, *²-C(=O)-O-X⁶¹-, *²-C(=O)-N(H)-X⁶¹-, or *²-O-X⁶¹-, X⁶¹ is a C₁-C₆ aliphatic hydrocarbylene group, phenylene group, fluorinated phenylene group, or trifluoromethyl-substituted phenylene group, which may contain a carbonyl moiety, ester bond, ether bond or hydroxy moiety, *² designates a point of attachment to the carbon atom in the backbone,
   R²¹ and R²² are each independently a C₁-C₂₀ hydrocarbyl group which may contain a heteroatom, R²¹ and R²² may bond together to form a ring with the sulfur atom to which they are attached,
   L¹ is a single bond, ether bond, ester bond, carbonyl group, sulfonate ester bond, carbonate bond or carbamate bond,
   Rf¹ and Rf² are each independently fluorine or a C₁-C₆ fluorinated saturated hydrocarbyl group,
   Rf³ and Rf⁴ are each independently hydrogen, fluorine or a C₁-C₆ fluorinated saturated hydrocarbyl group,
   Rf⁵ and Rf⁶ are each independently hydrogen, fluorine or a C₁-C₆ fluorinated saturated hydrocarbyl group, excluding that all Rf⁵ and Rf⁶ are hydrogen at the same time,
   M⁻ is a non-nucleophilic counter ion,
   A⁺ is an onium cation,
   g1 and g2 are each independently an integer of 0 to 3.
13. The chemically amplified positive resist composition of any one of 6 to 12, further comprising an organic solvent.
14. The chemically amplified positive resist composition of any one of 6 to 13, further comprising a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 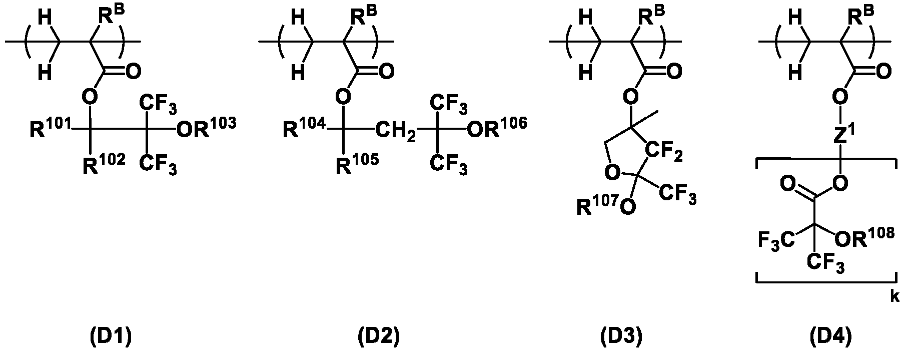 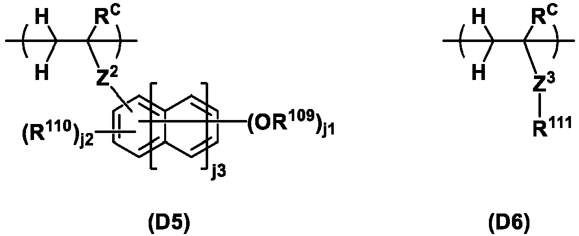 wherein j 1 is an integer of 1 to 3, j2 is an integer satisfying 0 ≤ j2 ≤ 5+2(j3)j1, j3 is 0 or 1, k is an integer of 1 to 3,
   R^{B} is each independently hydrogen, fluorine, methyl or trifluoromethyl,
   R^{C} is each independently hydrogen or methyl,
   R¹⁰¹, R¹⁰², R¹⁰⁴ and R¹⁰⁵ are each independently hydrogen or a C₁-C₁₀ saturated hydrocarbyl group,
   R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
   R¹⁰⁹ is hydrogen or a C₁-C₅ straight or branched hydrocarbyl group in which a heteroatom-containing moiety may intervene in a carbon-carbon bond,
   R¹¹⁰ is a C₁-C₅ straight or branched hydrocarbyl group in which a heteroatom-containing moiety may intervene in a carbon-carbon bond,
   R¹¹¹ is a C₁-C₂₀ saturated hydrocarbyl group in which at least one hydrogen is substituted by fluorine, and in which some -CH₂- may be replaced by an ester bond or ether bond,
   Z¹ is a C₁-C₂₀ (k+1)-valent hydrocarbon group or C₁-C₂₀ (k+1)-valent fluorinated hydrocarbon group,
   Z² is a single bond, *-C(=O)-O- or *-C(=O)-NH-, * designates a point of attachment to the carbon atom in the backbone,
   Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.
15. The chemically amplified positive resist composition of any one of 6 to 14, further comprising a quencher.
16. The chemically amplified positive resist composition of any one of 6 to 15, further comprising a photoacid generator other than the photoacid generator of 5.
17. A resist pattern forming process comprising the steps of:
   applying the chemically amplified positive resist composition of any one of 6 to 16 onto a substrate to form a resist film thereon,
   exposing the resist film to high-energy radiation, and
   developing the exposed resist film in an alkaline developer.
18. The process of 17 wherein the high-energy radiation is EUV or EB.
19. The process of 17 or 18 wherein the substrate has the outermost surface of a chromium-containing material.
20. The process of any one of 17 to 19 wherein the substrate is a photomask blank.

### ADVANTAGEOUS EFFECTS OF INVENTION

When processed by the microfabrication technology, especially EB and EUV lithography processes, a chemically amplified positive resist composition comprising an onium salt within the scope of the invention as a photoacid generator can form a resist pattern having a very high resolution and reduced LER. A pattern of rectangular profile is obtainable by virtue of properly inhibited dissolution.

### BRIEF DESCRIPTION OF DRAWINGS

FIG. 1 is a diagram showing the ¹H-NMR/DMSO-d₆ spectrum of onium salt PAG-1 synthesized in Example 1-1.
FIG. 2 is a diagram showing the ¹H-NMR/DMSO-d₆ spectrum of onium salt PAG-2 synthesized in Example 1-2.
FIG. 3 is a diagram showing the ¹H-NMR/DMSO-d₆ spectrum of onium salt PAG-3 synthesized in Example 1-3.

### DESCRIPTION OF PREFERRED EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, the broken line (---) and asterisk (*) each designate a point of attachment, namely valence bond. Me stands for methyl and Ac for acetyl. As used herein, the term "fluorinated" refers to a fluorine-substituted or fluorine-containing compound or group, and "iodized" refers to an iodine-substituted or iodine-containing compound or group. The terms "group" and "moiety" are interchangeable.

The abbreviations and acronyms have the following meaning.
PAG: photoacid generator
Mw: weight average molecular weight
Mn: number average molecular weight
Mw/Mn: molecular weight distribution or dispersity
GPC: gel permeation chromatography
PEB: post-exposure baking
LER: line edge roughness
CDU: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Onium salt

One embodiment of the invention is an onium salt having the formula (A).

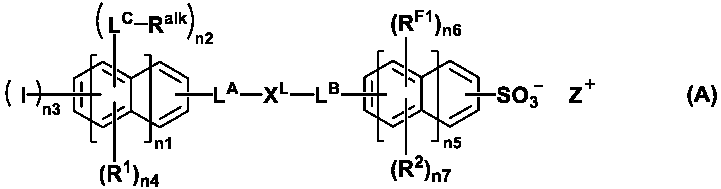

In formula (A), n1 is 0 or 1. The relevant structure represents a benzene ring in case of n1=0 and a naphthalene ring in case of n1=1. From the aspect of solvent solubility, a benzene ring corresponding to n1=0 is preferred. The subscript n2 is 1, 2 or 3, preferably 1 or 2. The subscript n3 is an integer of 0 to 4, preferably 1, 2 or 3 from the aspects of solvent solubility and lithography performance. The subscript n4 is an integer of 0 to 4, preferably 0 or 1. It is noted that n2+n3+n4 is from 1 to 5 in case of n1=0, and n2+n3+n4 is from 1 to 7 in case of n1=1.

In formula (A), n5 is 0, 1 or 2. The relevant structure represents a benzene ring in case of n5=0, a naphthalene ring in case of n5=1, and an anthracene ring in case of n5=2. From the aspects of solvent solubility and lithography performance, a benzene ring corresponding to n5=0 and a naphthalene ring corresponding to n5=1 are preferred. The subscript n6 is an integer of 0 to 4 in case of n5=0, an integer of 0 to 6 in case of n5=1, and an integer of 0 to 8 in case of n5=2. The subscript n7 is an integer of 0 to 4 in case of n5=0, an integer of 0 to 6 in case ofn5=1, and an integer of 0 to 8 in case of n5=2. It is noted that n6+n7 is from 0 to 4 in case of n5=0, from 0 to 6 in case of n5=1, and from 0 to 8 in case of n5=2.

In formula (A), R^{alk} is a C₆-C₁₈ alkyl group or C₄-C₁₈ fluorinated alkyl group. When R^{alk} is a C₆-C₁₈ alkyl group, the alkyl group has at least one straight chain structure of 6 or more carbon atoms. Examples of the C₆-C₁₈ alkyl group include 1-hexyl, 1-heptyl, 1-octyl, 1-nonyl, 1-decyl, 1-undecyl, 1-dodecyl, 1-tridecyl, 1-tetradecyl, 1-hexadecyl, 1-octadecyl, octan-2-yl, decan-2-yl, decan-4-yl, octadecane-8-yl, 7,7-dimethyloctyl, 7,7-diethylnonyl, and 4-butyldodecyl. In the alkyl group, some constituent -CH₂- may be replaced by an ether bond or carbonyl moiety. The alkyl group may contain a cyclic structure at the end or in a carbon-carbon bond thereof as a partial structure. Exemplary cyclic structures include cyclopentane, cyclohexane, adamantane, norbornane and benzene rings. Preferably, R^{alk} is a straight alkyl group or straight glyme chain. In case of n2=2 or 3, a plurality of R^{alk} may be identical or different.

When R^{alk} is a C₄-C₁₈ fluorinated alkyl group, the fluorinated alkyl group has at least two groups selected from -CF₂- and -CF₃. Examples of the C₄-C₁₈ fluorinated alkyl group include 1-butyl, 1-pentyl, 1-hexyl, 1-heptyl, 1-octyl, 1-nonyl, 1-decyl, 1-undecyl, 1-dodecyl, 1-tridecyl, 1-tetradecyl, 1-hexadecyl, 1-octadecyl, octan-2-yl, decan-2-yl, decan-4-yl, octadecane-8-yl, 7,7-dimethyloctyl, 7,7-diethylnonyl, and 4-butyldodecyl in which some or all hydrogen atoms are substituted by fluorine. In the fluorinated alkyl groups, some constituent -CH₂- may be replaced by an ether bond or carbonyl moiety. The fluorinated alkyl group may contain a cyclic structure at the end or in a carbon-carbon bond thereof. Exemplary cyclic structures include cyclopentane, cyclohexane, adamantane, norbornane and benzene rings.

Preferred examples of R^{alk} are shown below, but not limited thereto.

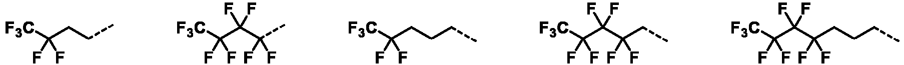

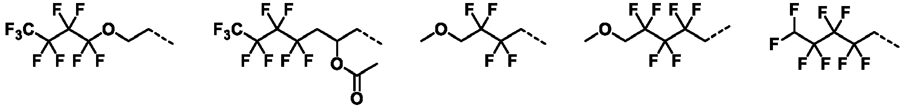

In formula (A), R¹ is a halogen exclusive of iodine or a C₁-C₂₀ hydrocarbyl group which may contain a heteroatom. Suitable halogen atoms include fluorine, chlorine and bromine, with fluorine being preferred.

The hydrocarbyl group R¹ may be saturated or unsaturated. Examples thereof include, but are not limited to, C₃-C₂₀ branched alkyl groups such as isopropyl, sec-butyl, tert-butyl, tert-pentyl, and 2-ethylhexyl; C₃-C₂₀ cyclic aliphatic hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, oxanorbornyl, tricyclo[5.2.1.0^{2,6}]decyl, and adamantyl; C₆-C₂₀ aryl groups such as phenyl, naphthyl and anthracenyl, and combinations thereof. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonate ester bond, carbonate bond, lactone ring, sultone ring, carboxylic anhydride (-C(=O)-O-C(=O)-) or haloalkyl moiety.

When n4 is 2 or more, two R¹ may bond together to form a ring with the carbon atoms to which they are attached. Examples of the ring include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy moiety, cyano moiety, fluorine, chlorine, bromine, iodine, carbonyl moiety, ether bond, ester bond, sulfonate ester bond, carbonate bond, lactone ring, sultone ring, carboxylic anhydride (-C(=O)-O-C(=O)-) or haloalkyl moiety.

In formula (A), R² is a C₁-C₂₀ hydrocarbyl group which may contain a heteroatom. The hydrocarbyl group may be saturated or unsaturated and straight, branched or cyclic. Examples thereof are as exemplified above for the hydrocarbyl group R¹. When n7 is 2 or more, a plurality of R² may be identical or different.

When n7 is 2 or more, a plurality of R² may bond together to form a ring with the carbon atoms to which they are attached. The ring is preferably 5 to 8-membered.

In formula (A), R^{F1} is fluorine, a C₁-C₆ fluorinated alkyl group, C₁-C₆ fluorinated alkoxy group, or C₁-C₆ fluorinated thioalkoxy group. R^{F1} is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. Owing to fluorine included, the generated acid has a high acid strength enough to ensure smooth progress of deprotection reaction. When n6 is 2 or more, a plurality of R^{F1} may be identical or different.

In formula (A), L^{A}, L^{B}, and L^{C} are each independently a single bond, ether bond, ester bond, sulfonate ester bond, carbonate bond, or carbamate bond. Inter alia, L^{A} and L^{B} are preferably a single bond, ether bond, ester bond or sulfonate ester bond, more preferably a single bond, ether bond or ester bond. L^{C} is preferably a single bond, ether bond or ester bond, more preferably a single bond or ether bond.

In formula (A), X^{L} is a single bond or a C₁-C₄₀ hydrocarbylene group which may contain a heteroatom. The hydrocarbylene group may be straight, branched or cyclic. Examples thereof include alkanediyl groups and cyclic saturated hydrocarbylene groups. Suitable heteroatoms include oxygen, nitrogen, and sulfur.

Examples of the optionally heteroatom-containing C₁-C₄₀ hydrocarbylene group X^{L} are shown below, but not limited thereto. Herein * designates a point of attachment to L^{A} or L^{B}.

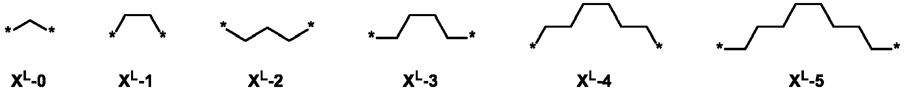

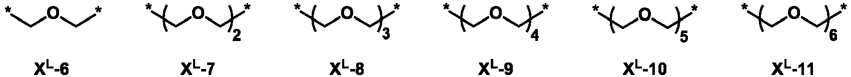

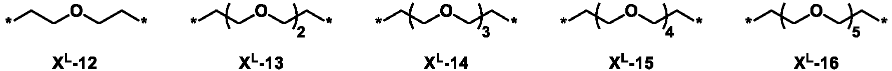

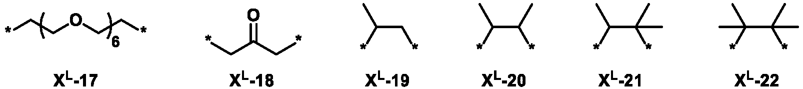

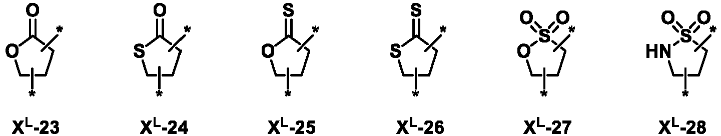

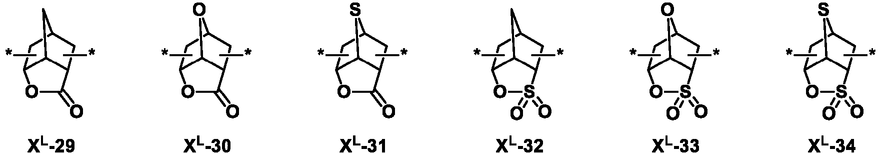

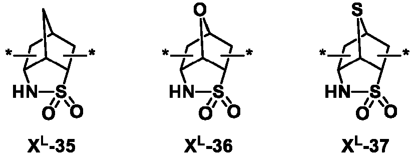

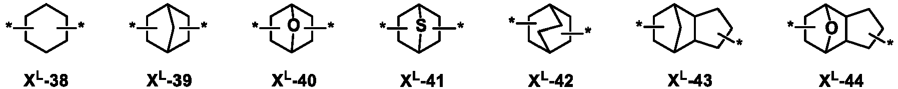

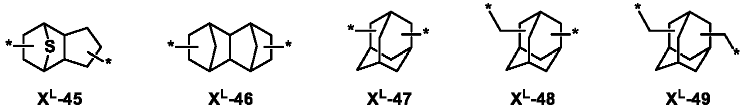

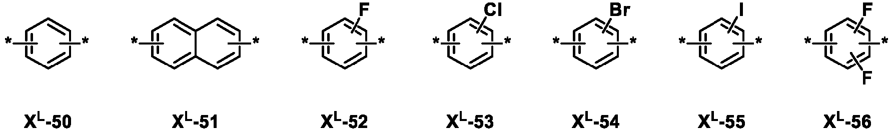

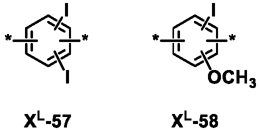

Of these, X^{L}-0 to X^{L}-22 and X^{L}-47 to X^{L}-58 are preferred.

Preferably, the onium salt of formula (A) has the formula (A1). 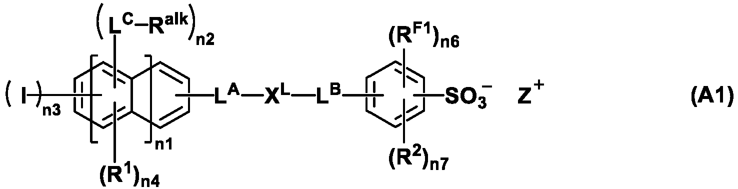 Herein n1 to n4, n6, n7, R^{alk}, R¹, R², R^{F1}, L^{A}, L^{B}, L^{C}, X^{L}, and Z⁺ are as defined above.

More preferably, the onium salt of formula (A1) has the formula (A2). 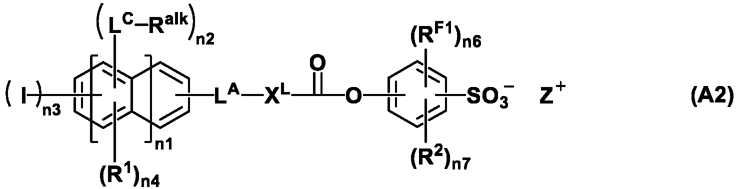 Herein n1 to n4, n6, n7, R^{alk}, R¹, R², R^{F1}, L^{A}, L^{C}, X^{L}, and Z⁺ are as defined above.

Preferred examples of the anion in the onium salt having formula (A) are shown below, but not limited thereto.

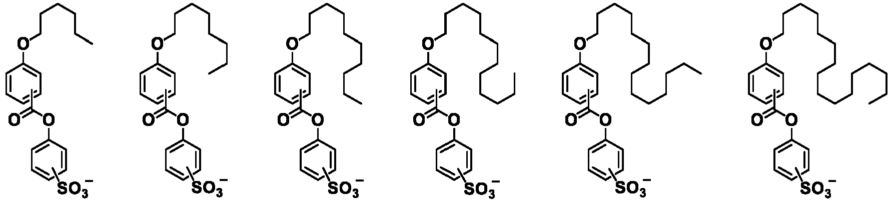

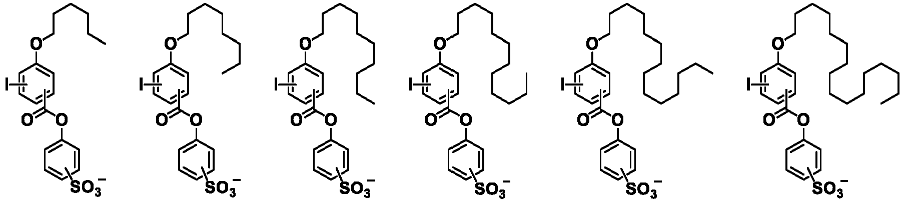

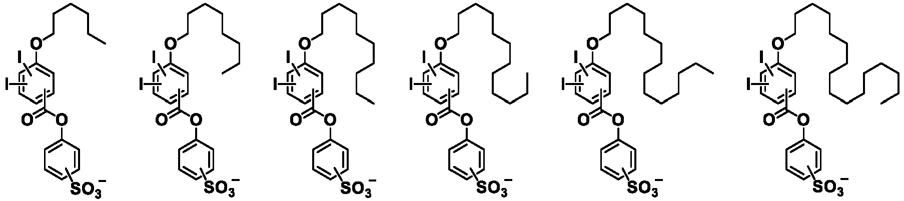

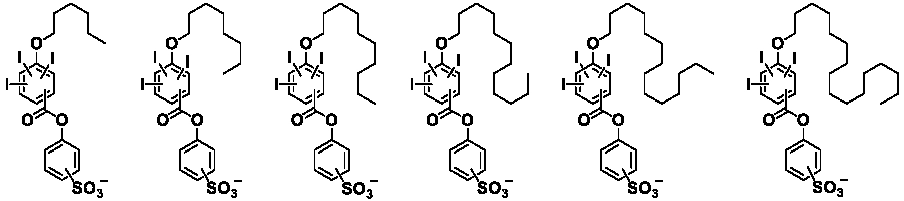

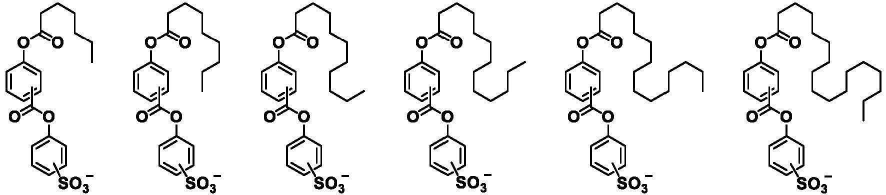

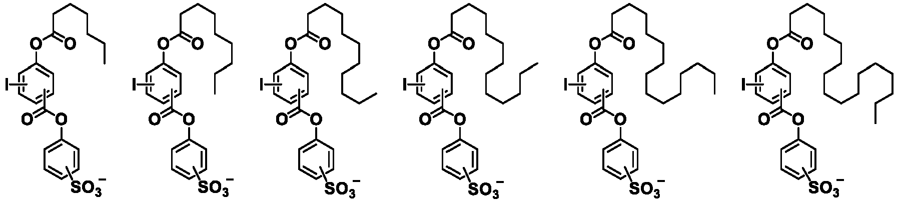

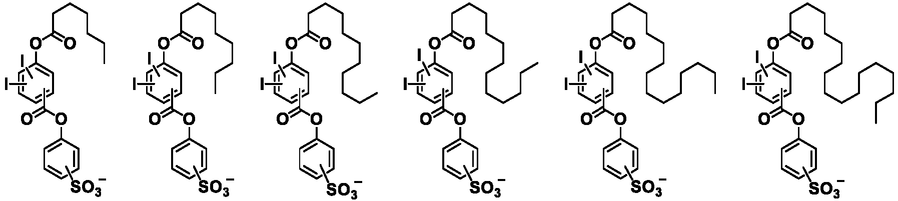

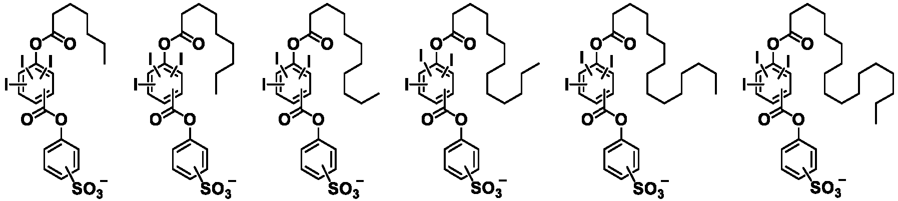

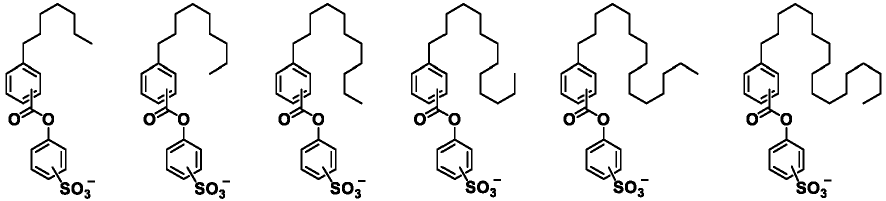

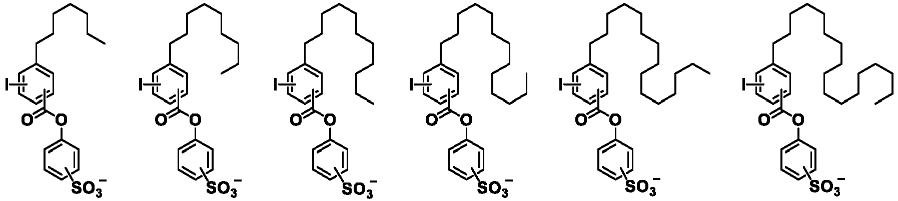

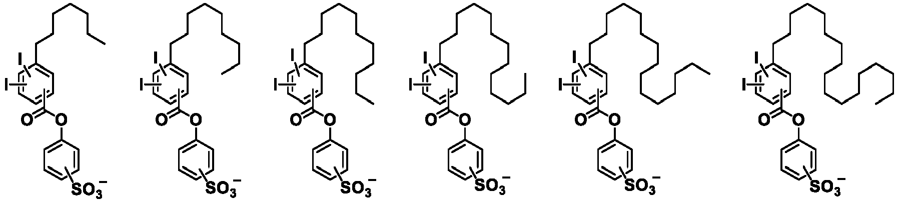

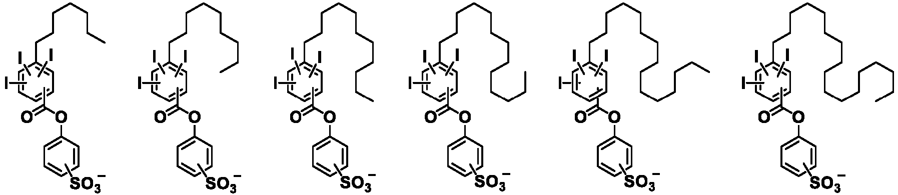

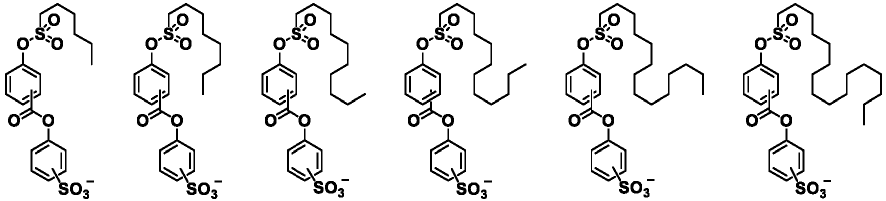

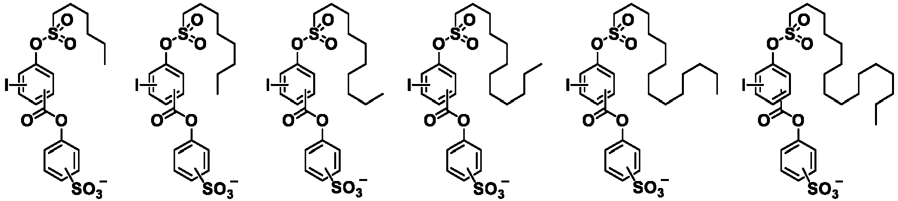

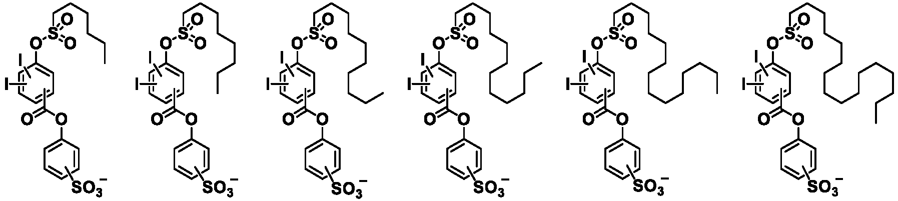

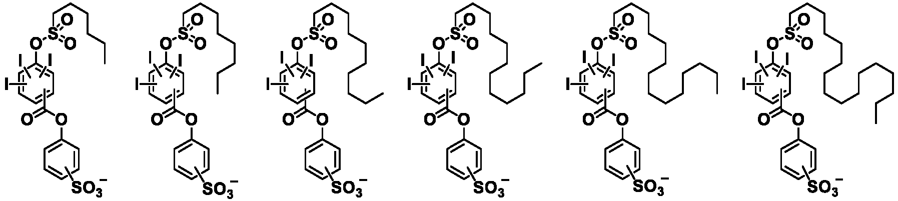

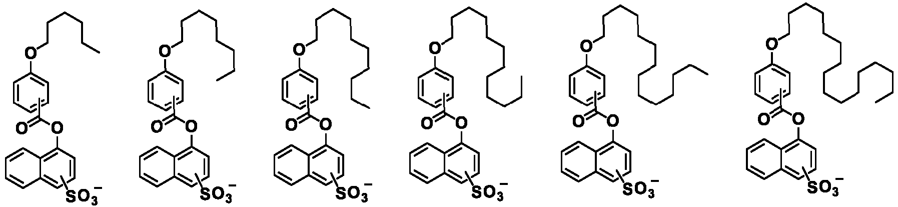

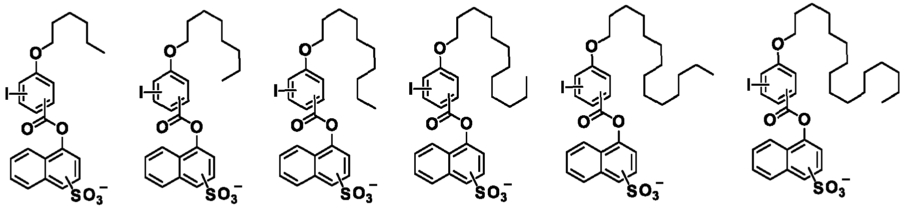

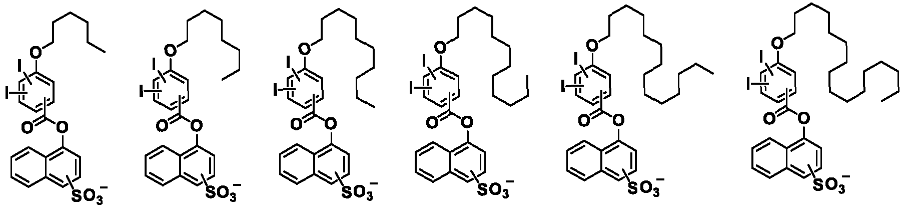

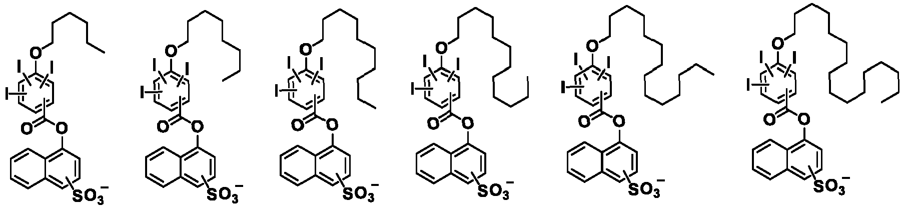

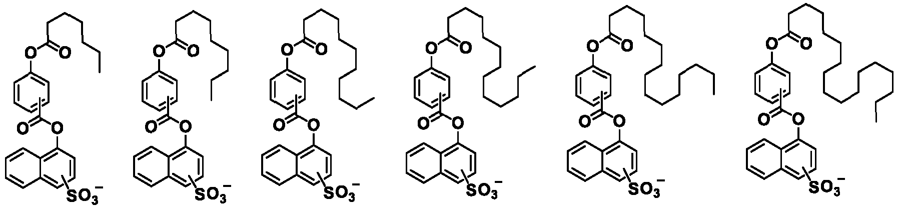

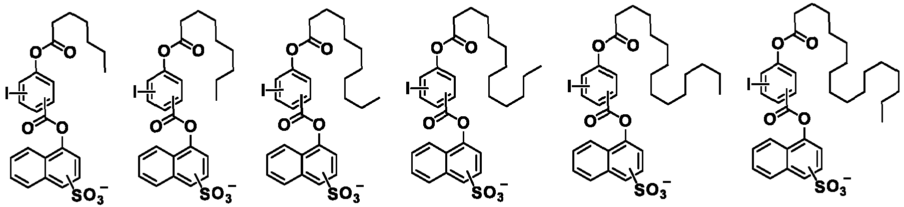

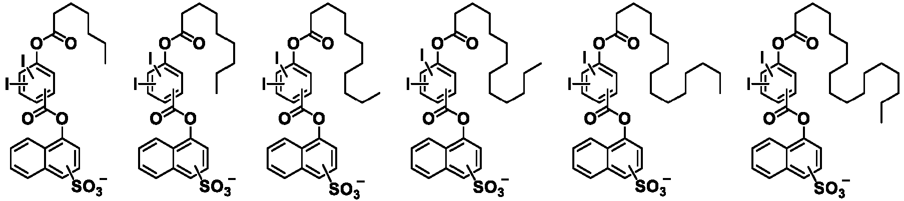

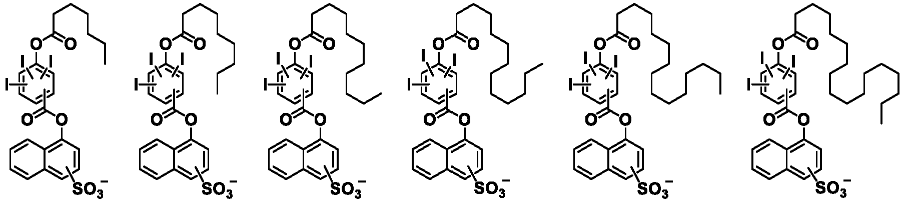

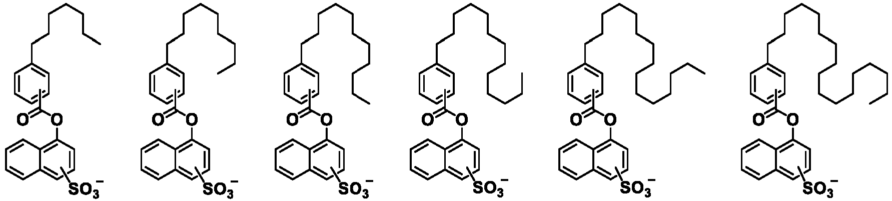

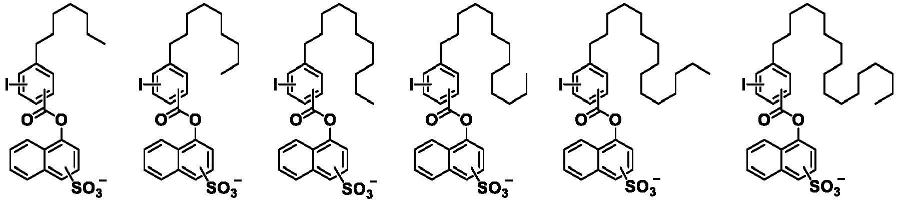

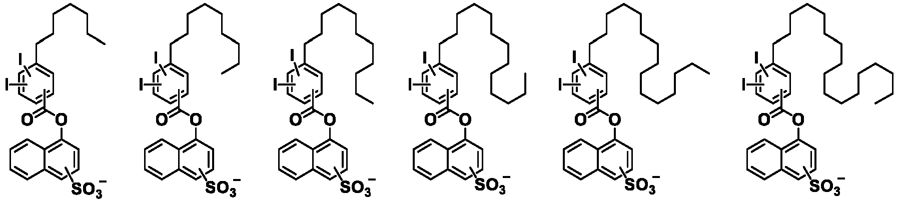

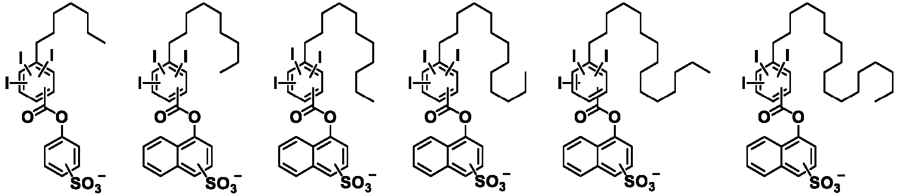

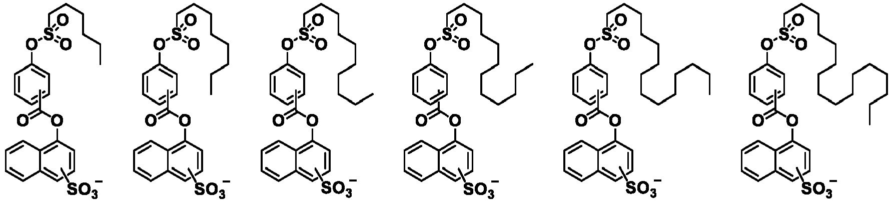

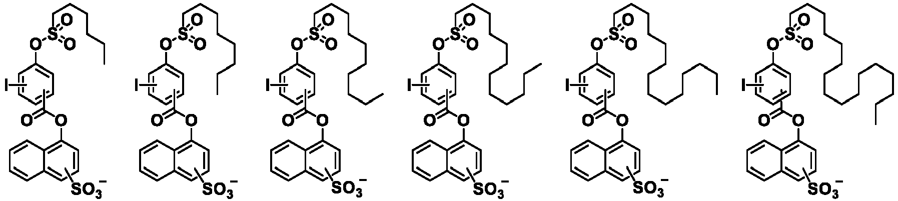

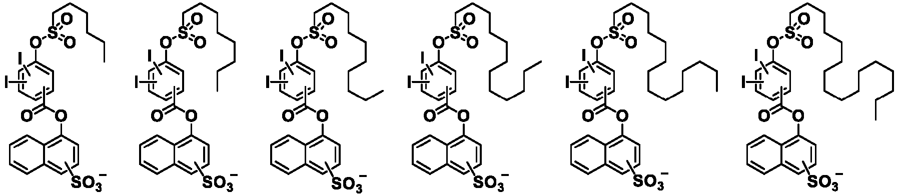

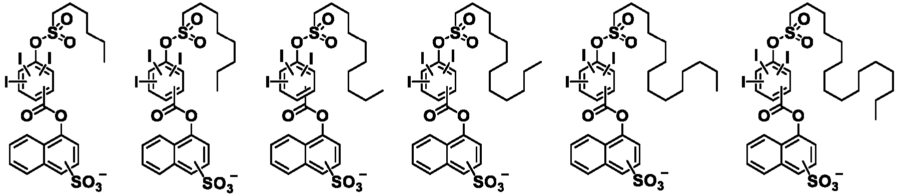

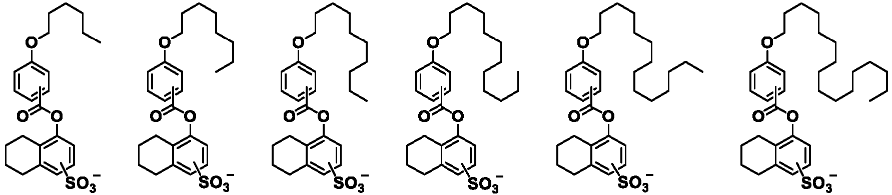

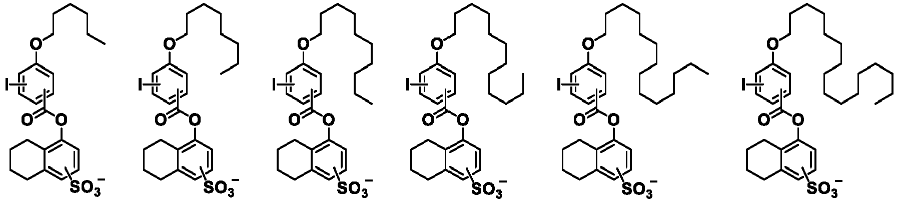

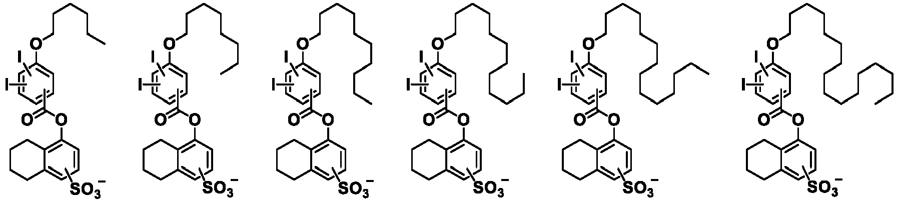

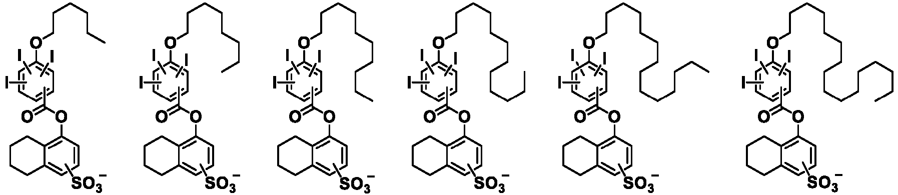

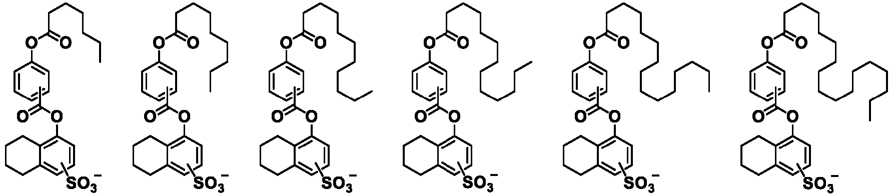

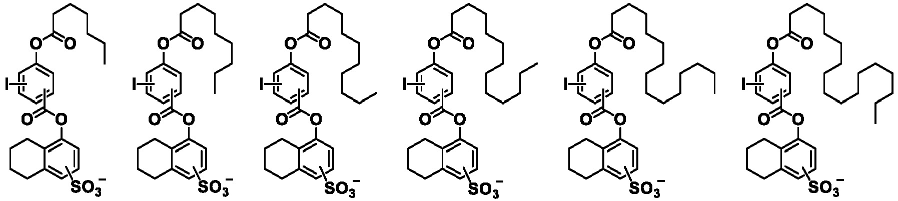

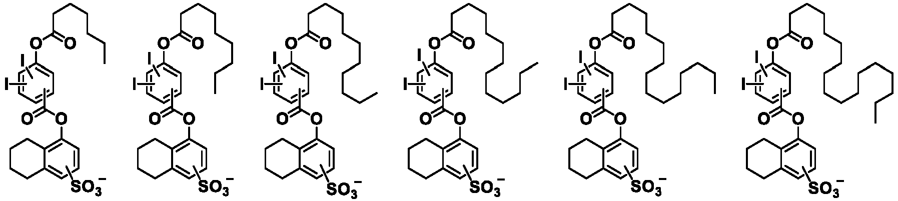

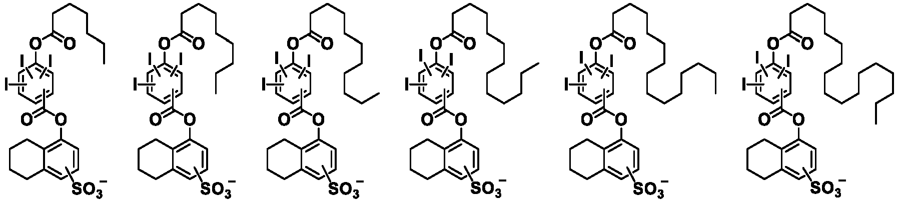

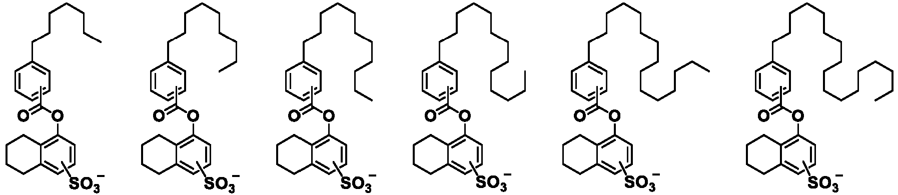

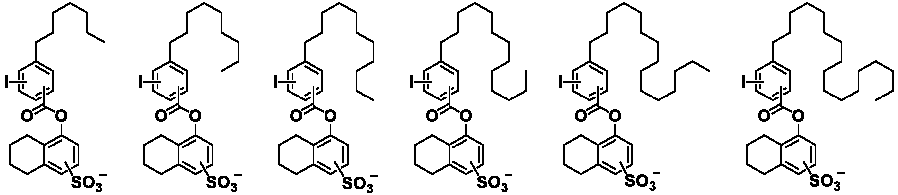

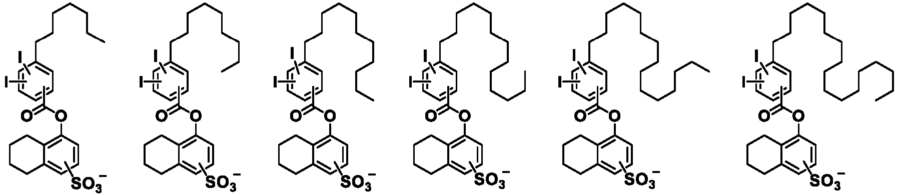

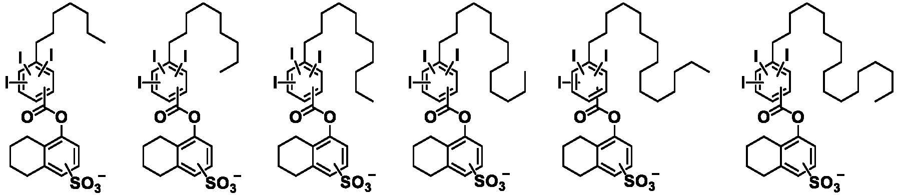

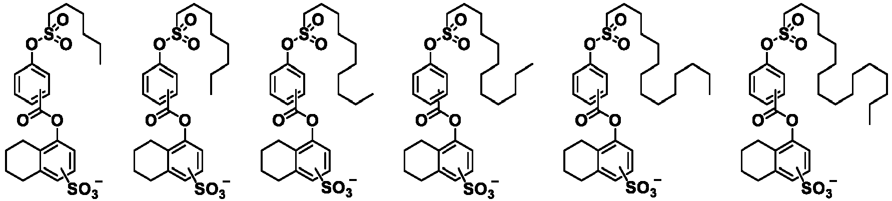

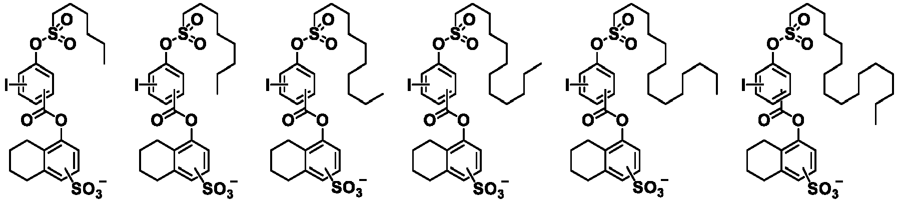

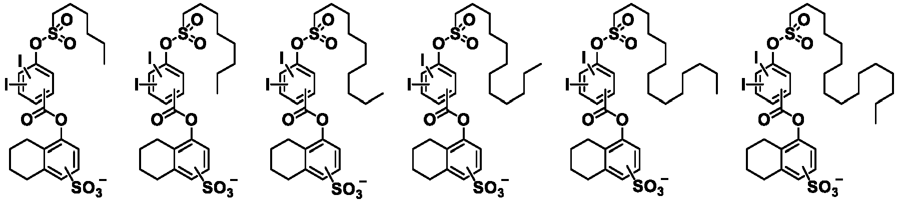

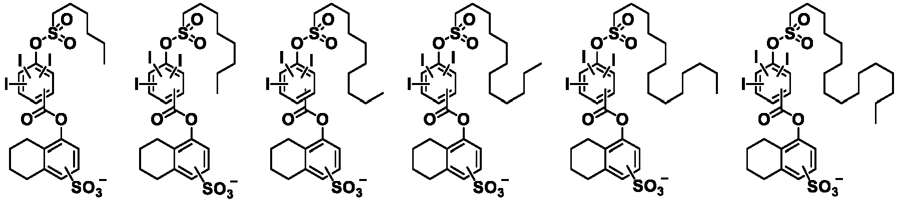

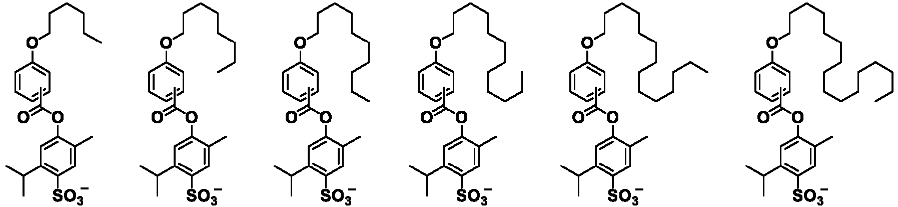

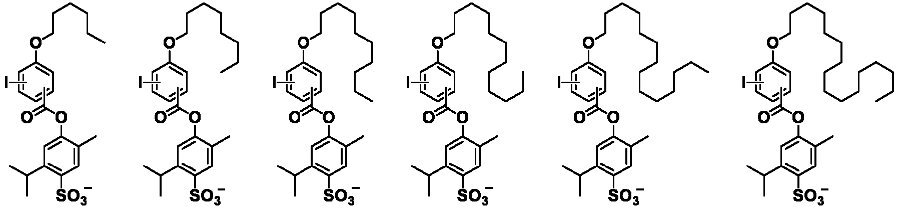

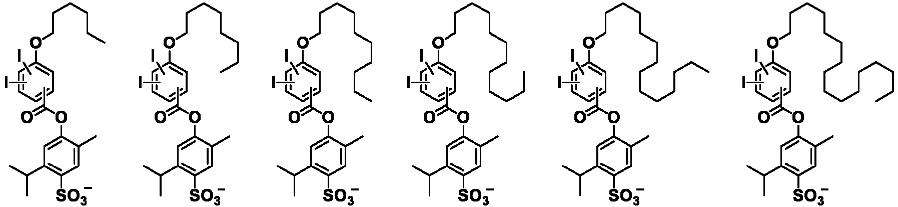

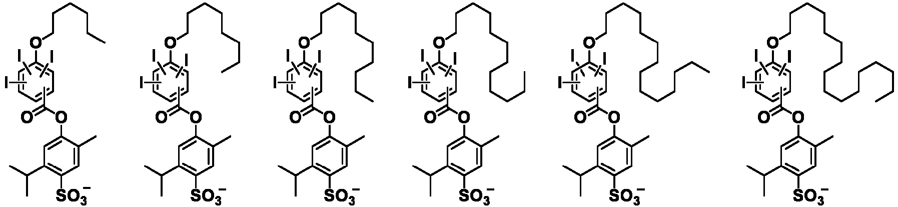

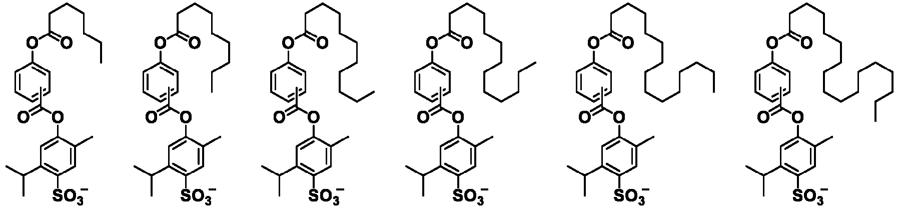

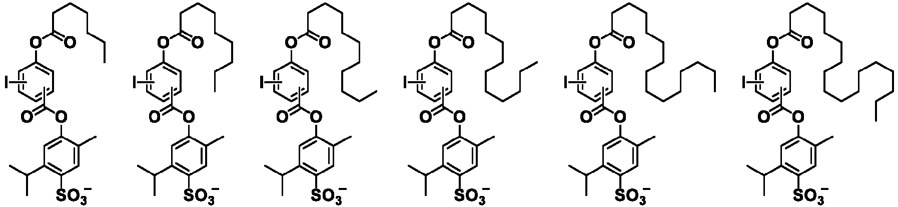

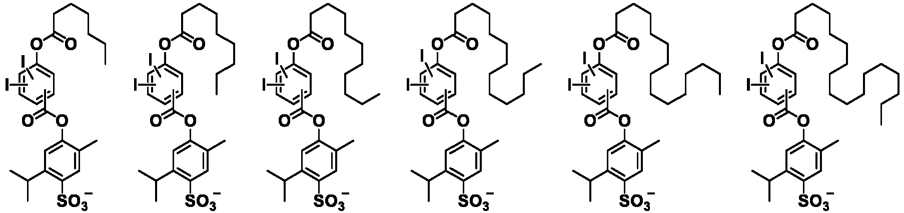

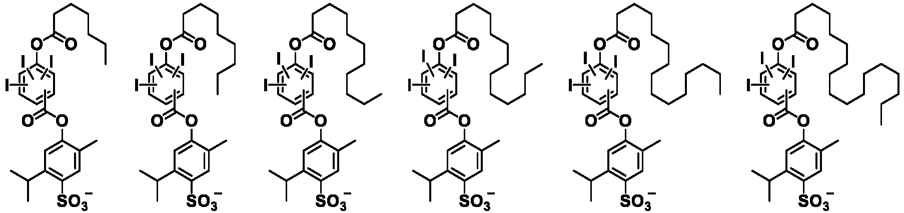

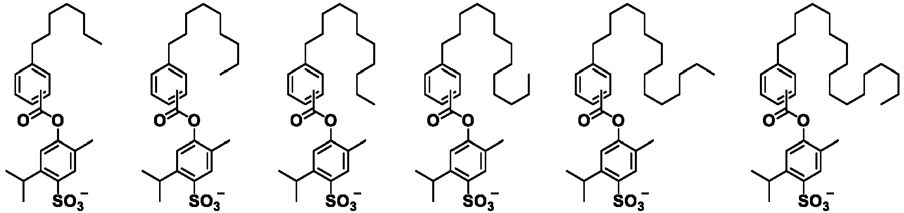

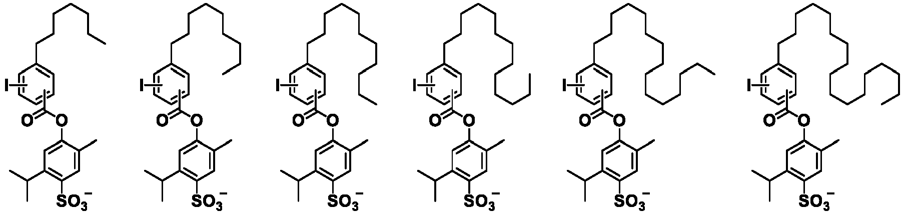

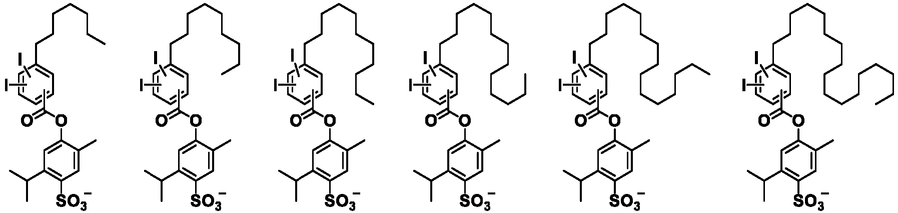

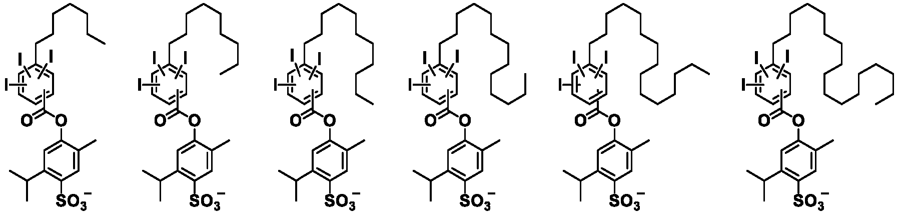

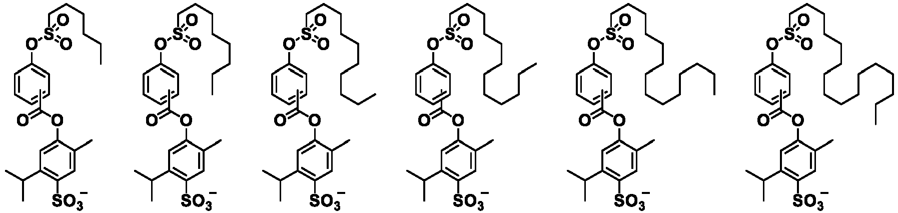

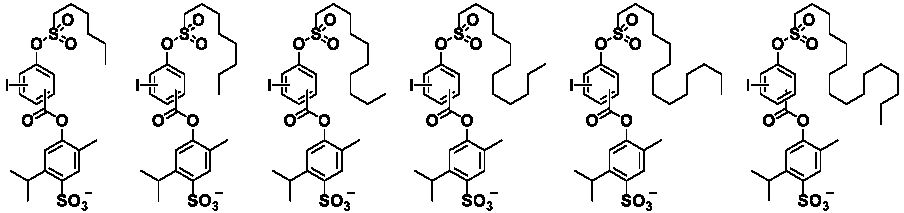

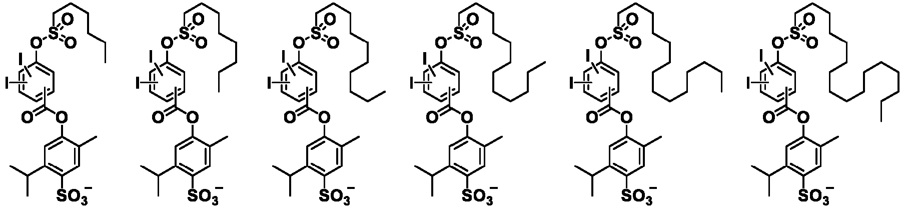

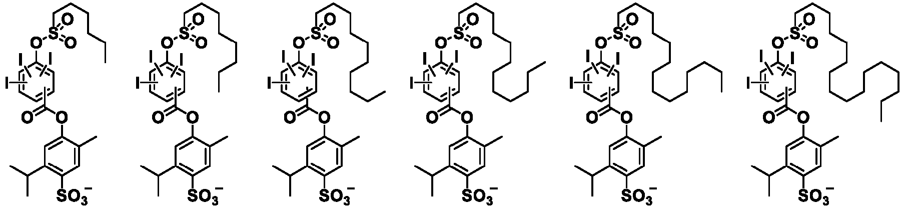

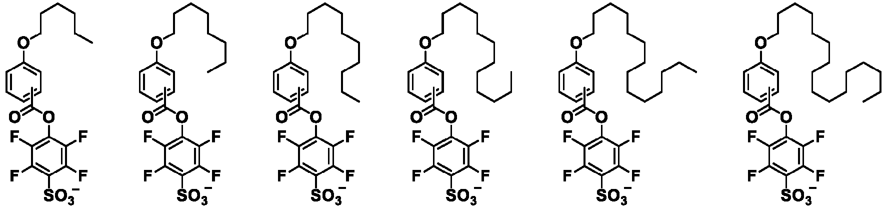

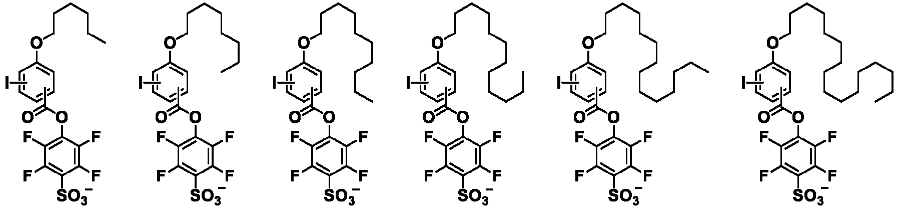

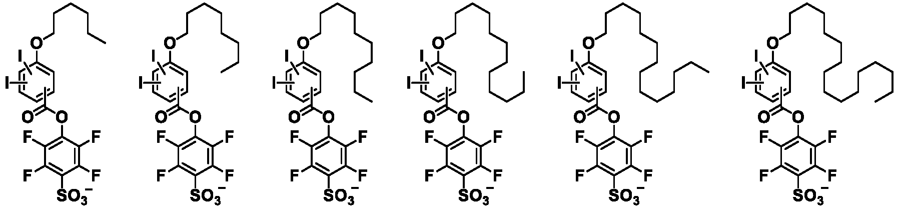

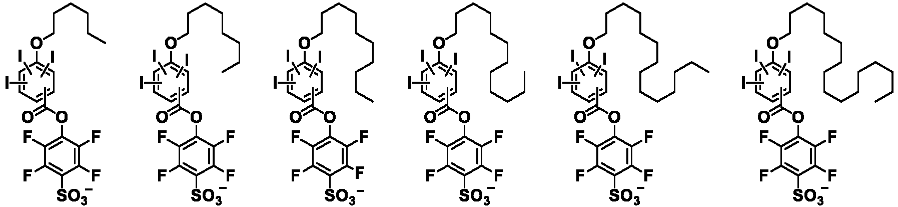

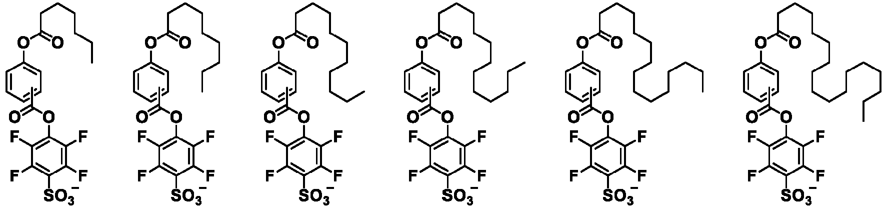

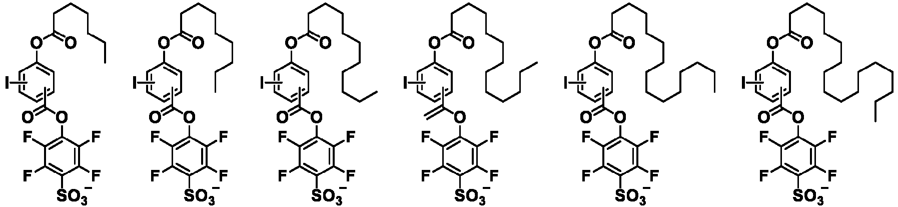

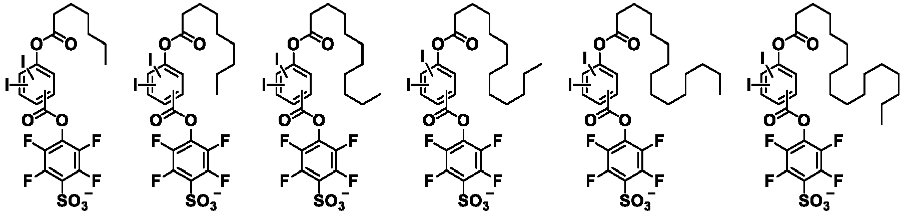

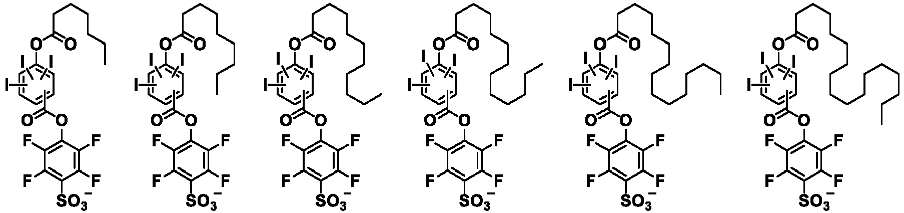

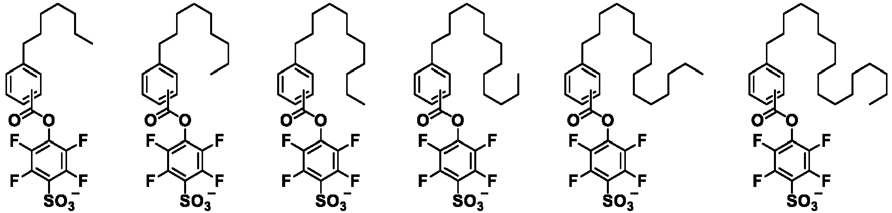

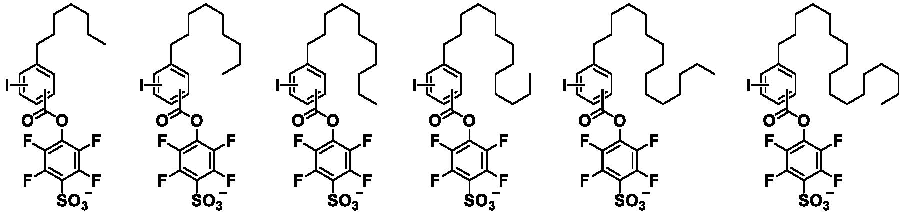

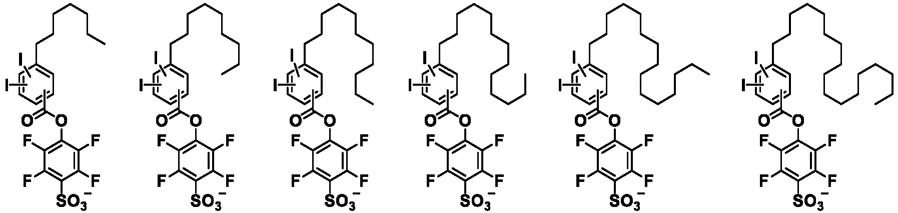

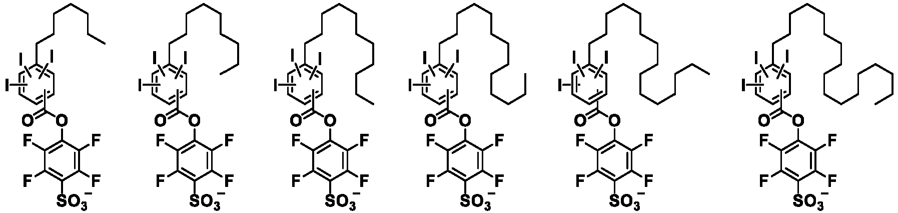

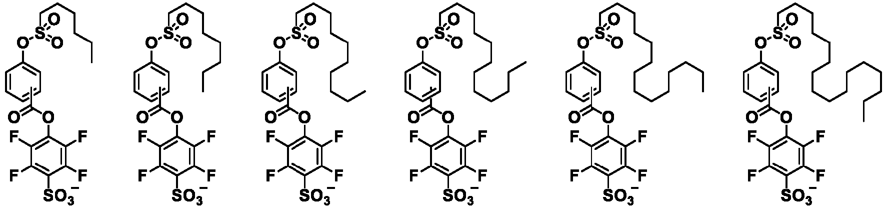

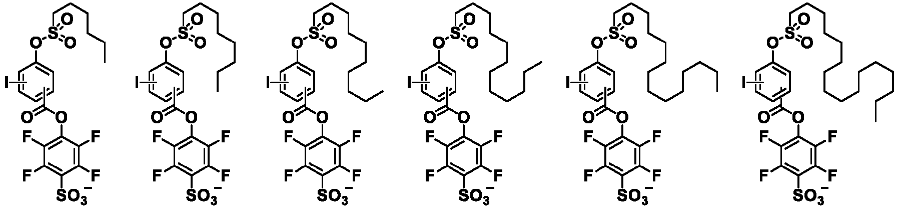

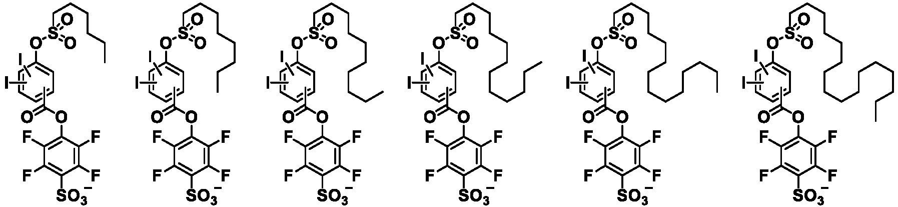

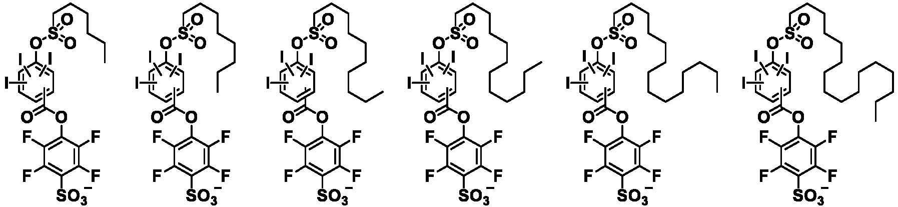

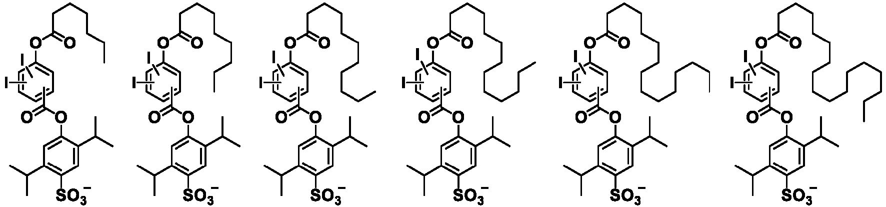

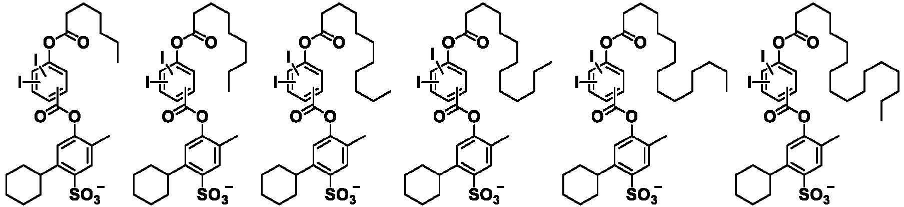

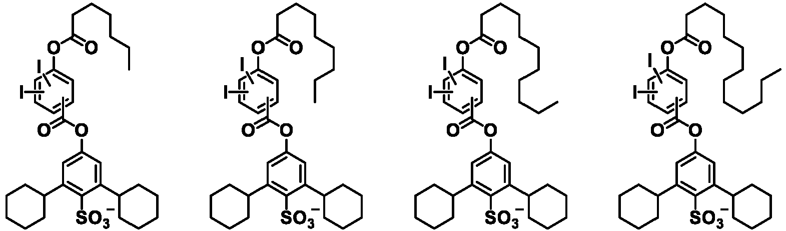

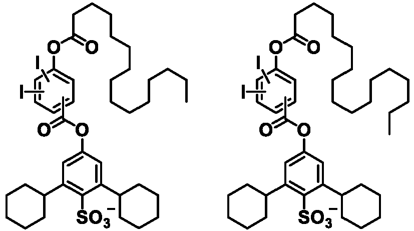

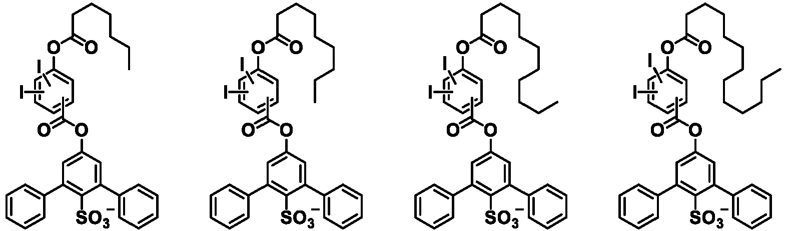

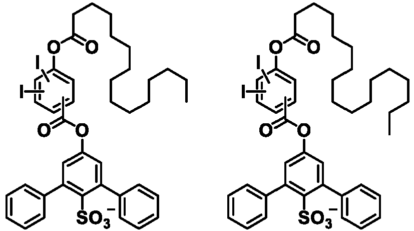

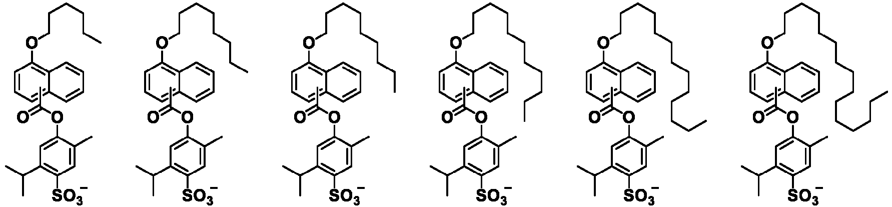

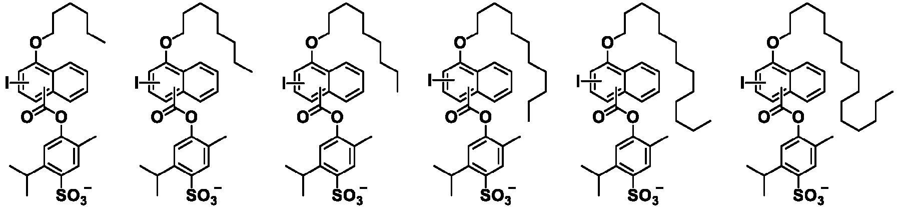

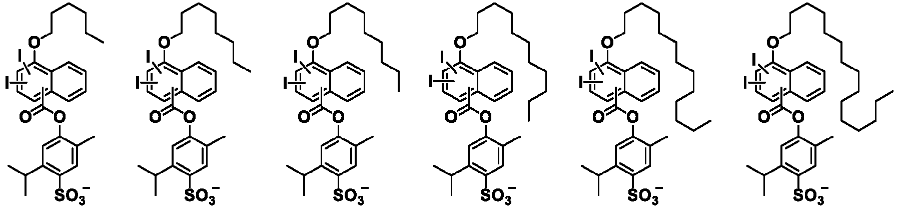

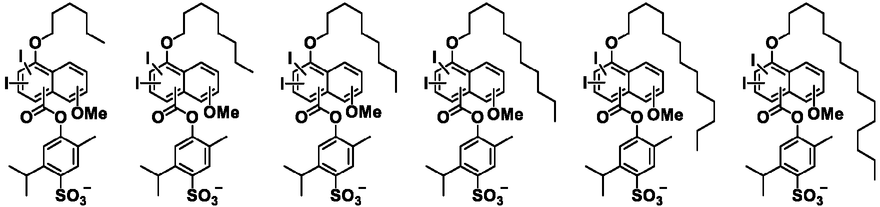

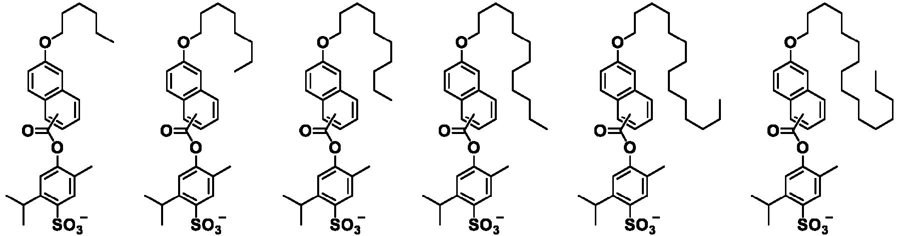

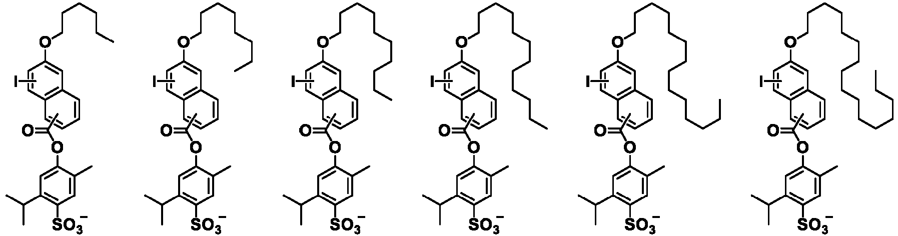

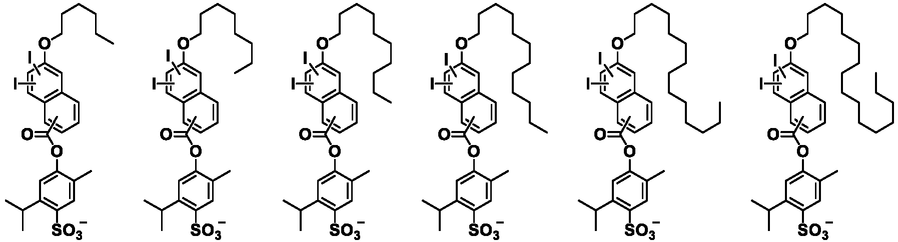

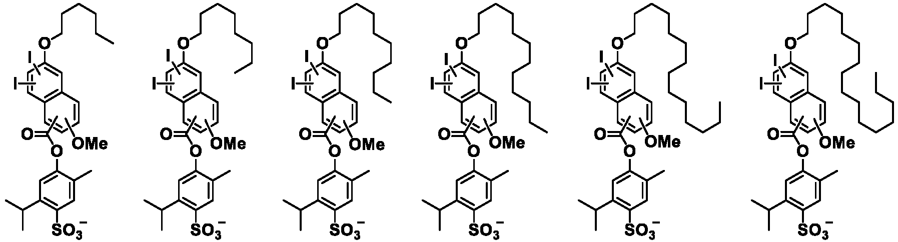

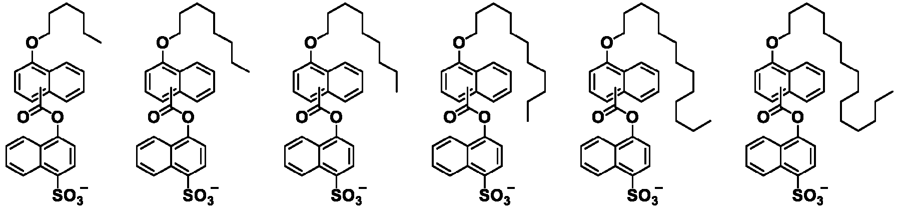

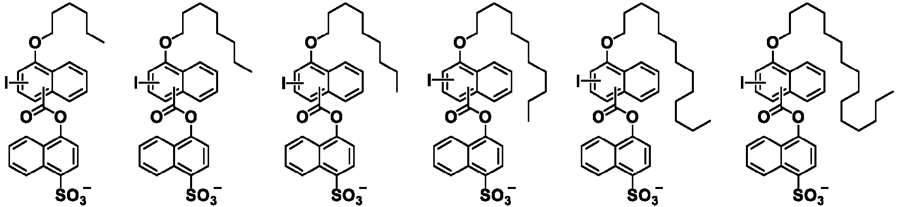

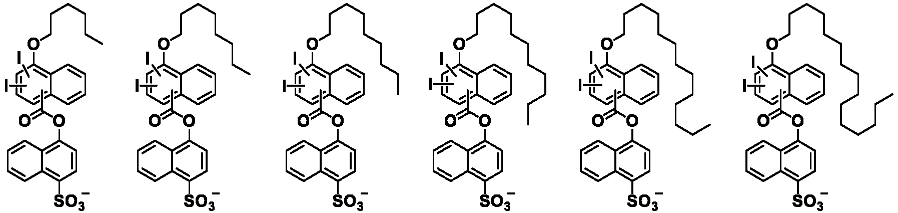

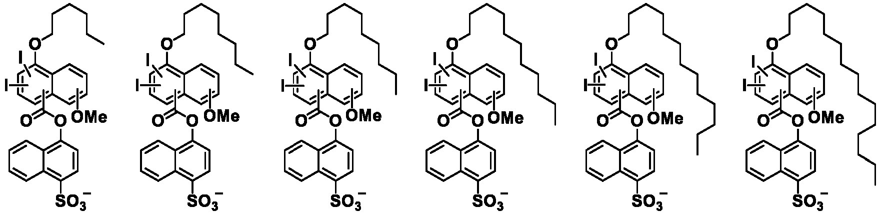

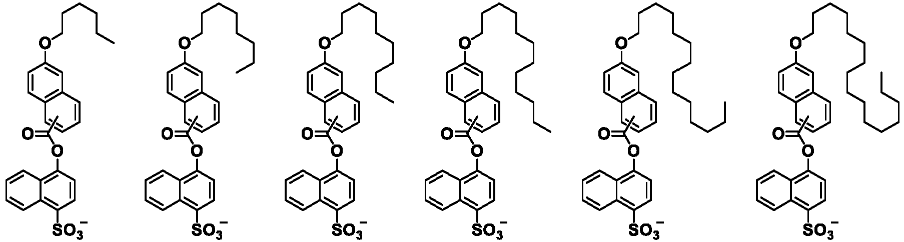

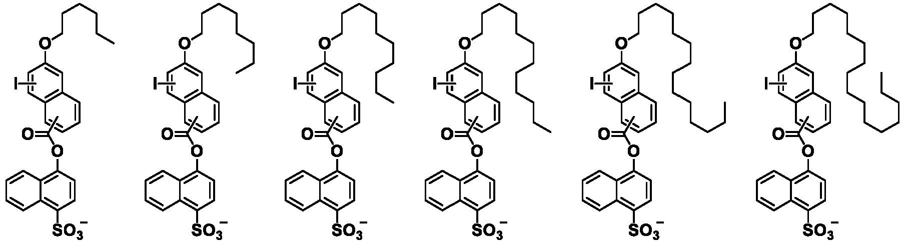

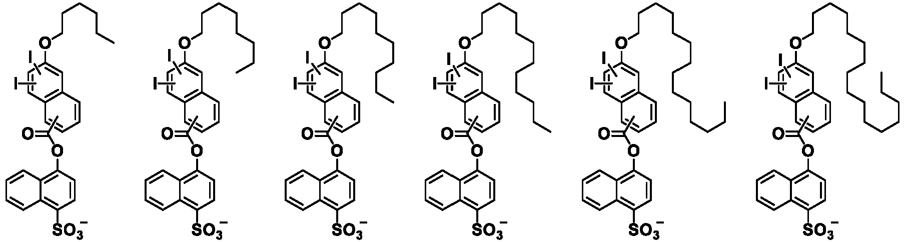

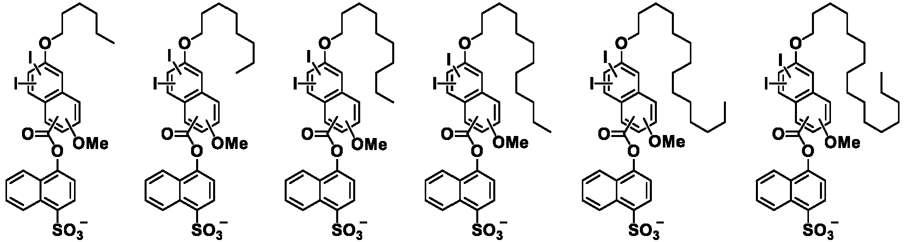

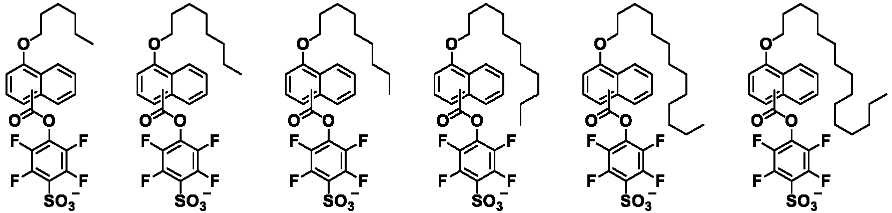

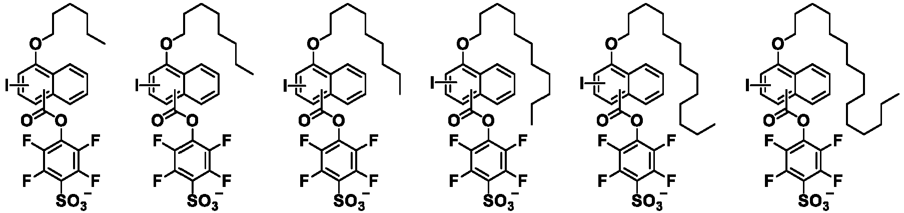

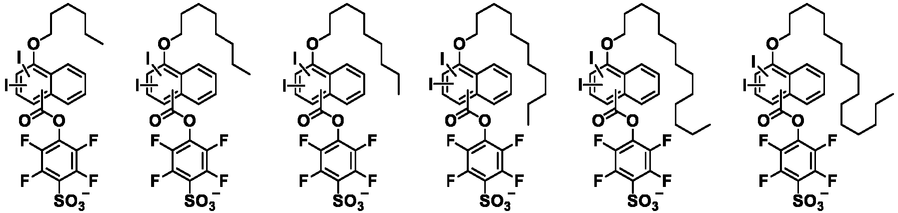

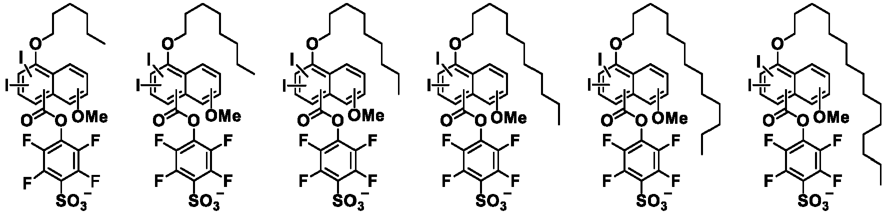

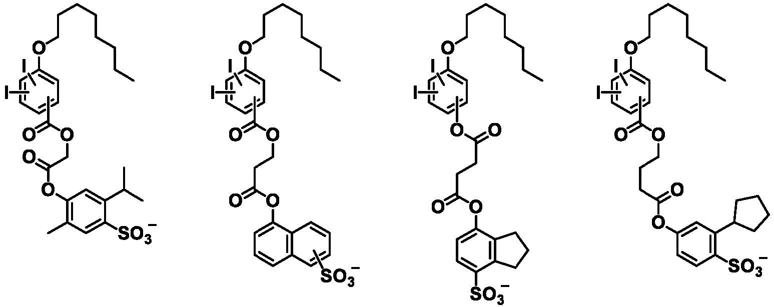

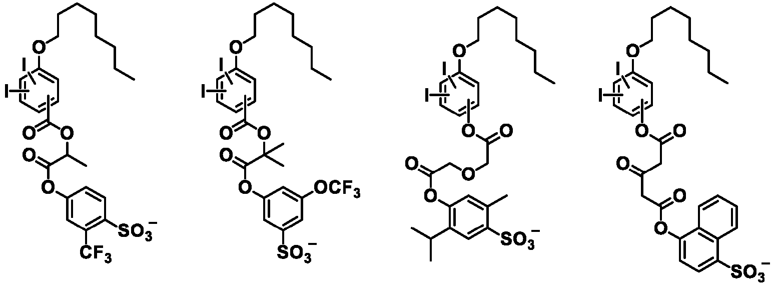

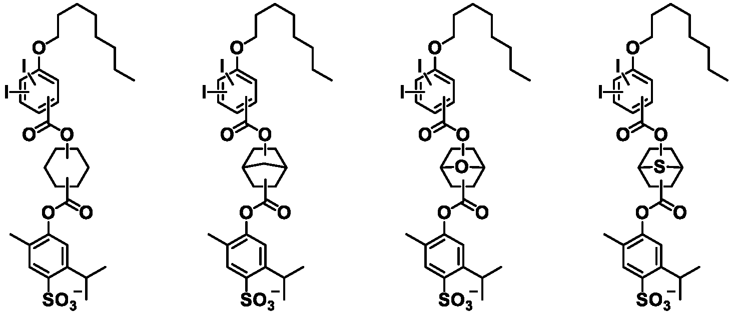

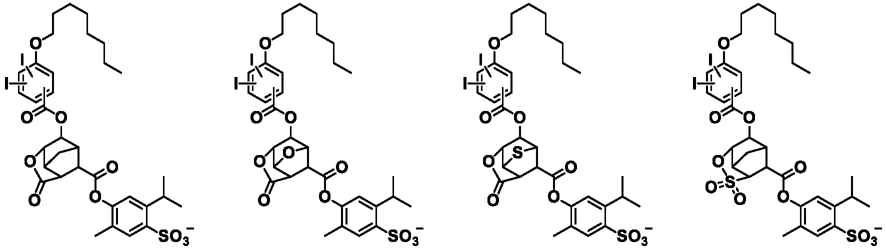

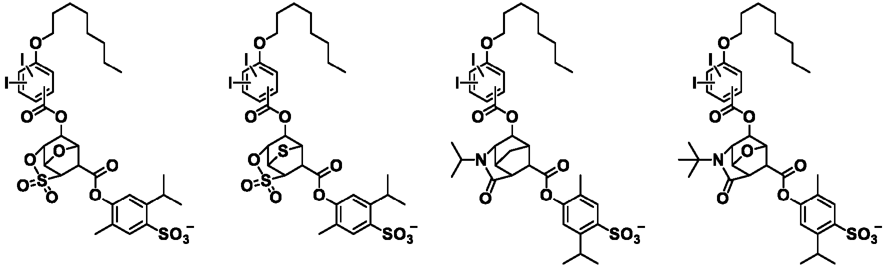

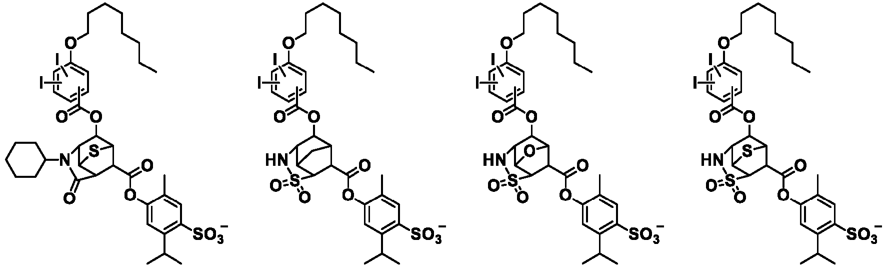

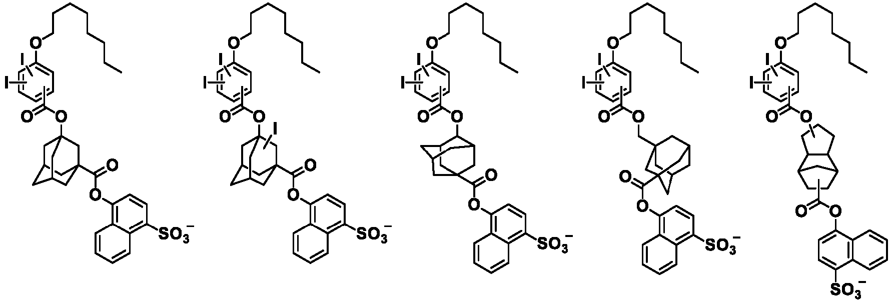

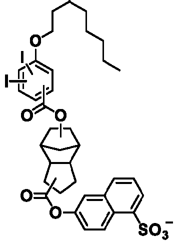

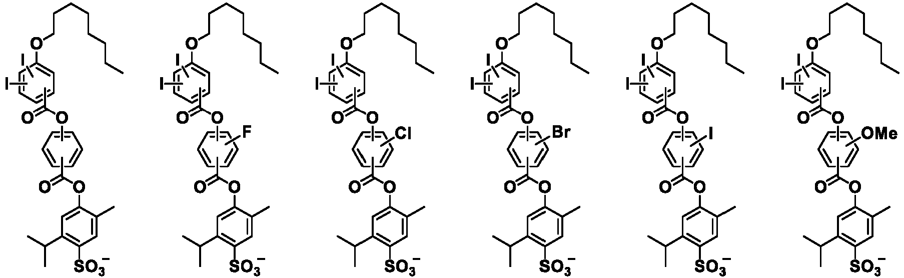

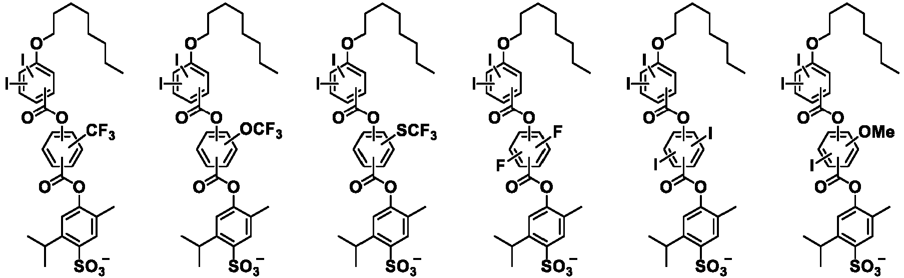

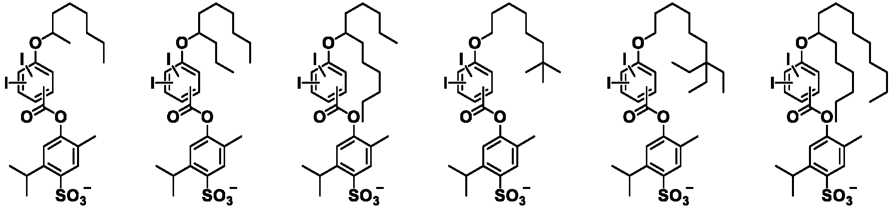

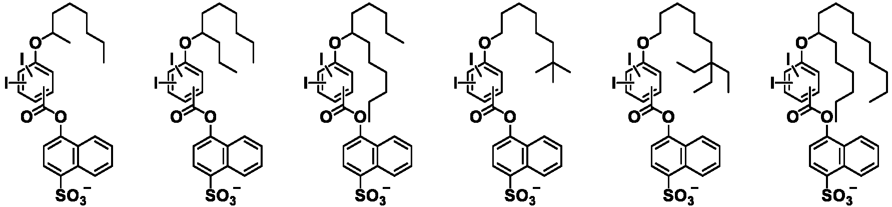

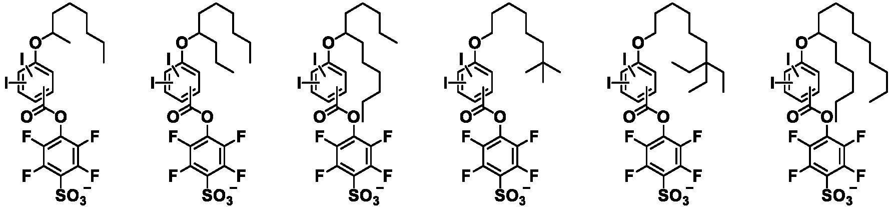

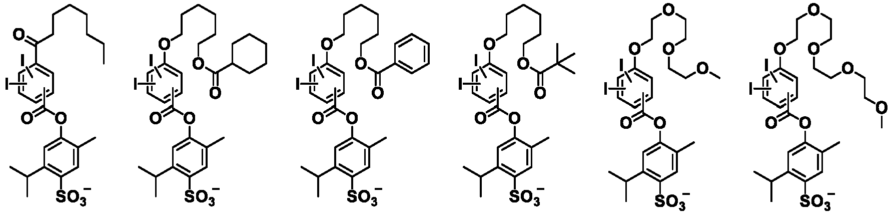

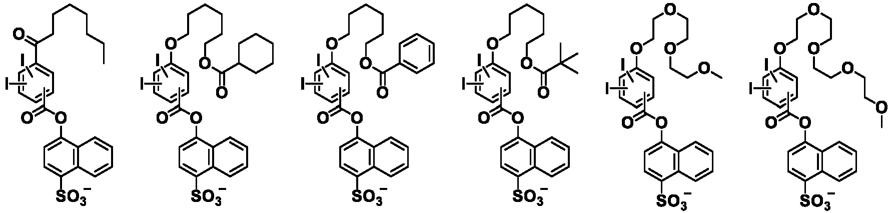

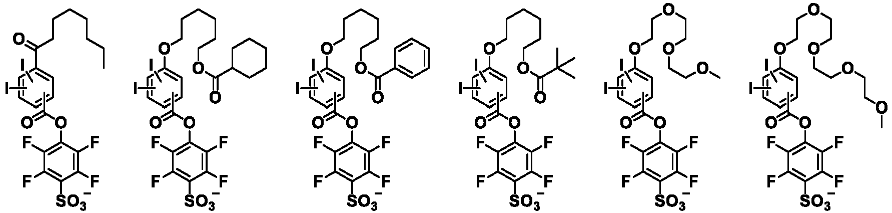

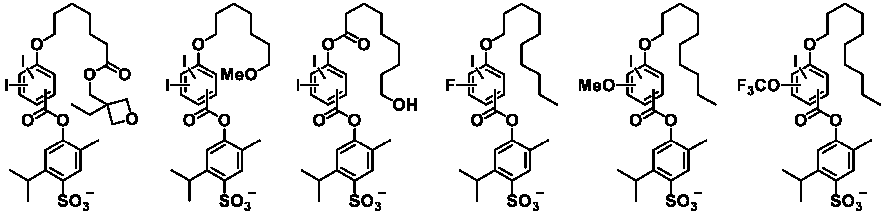

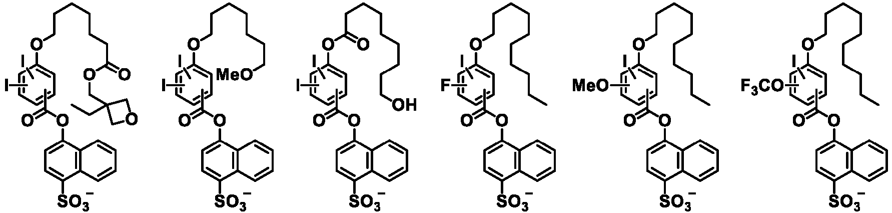

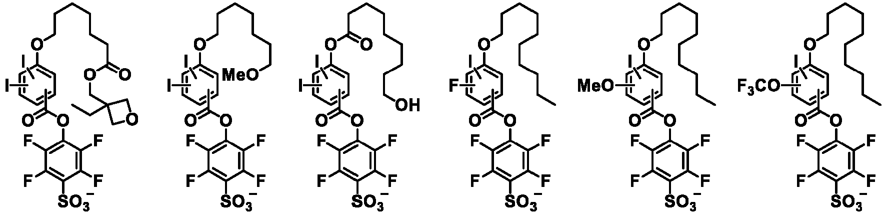

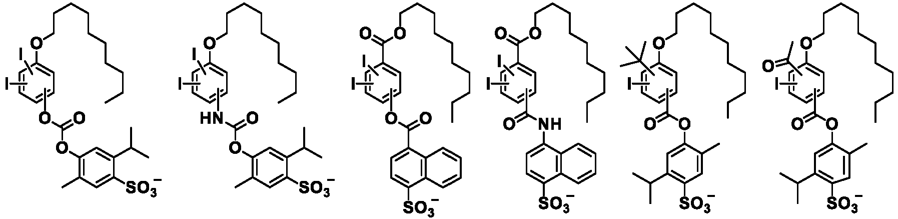

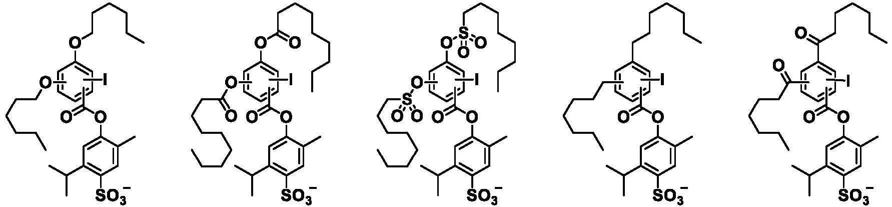

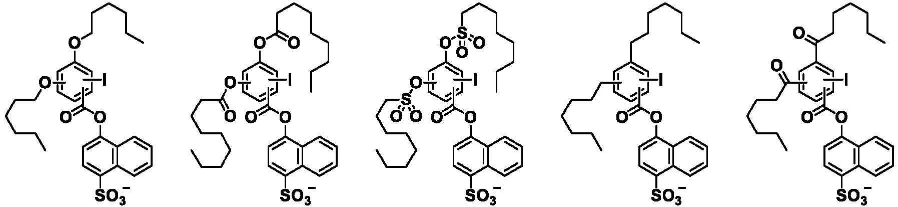

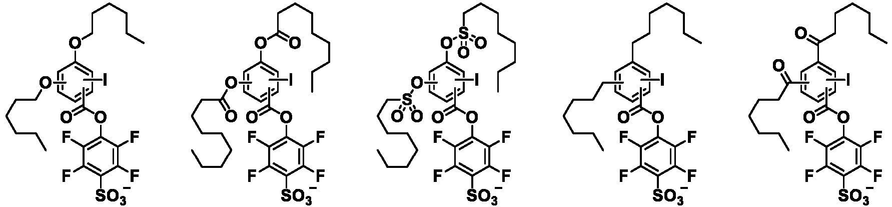

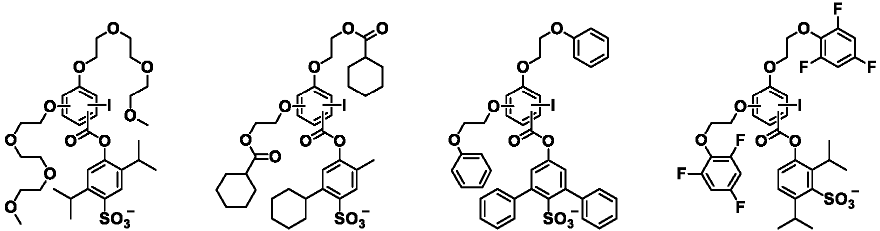

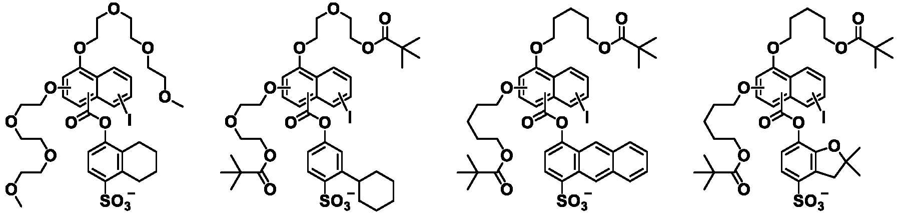

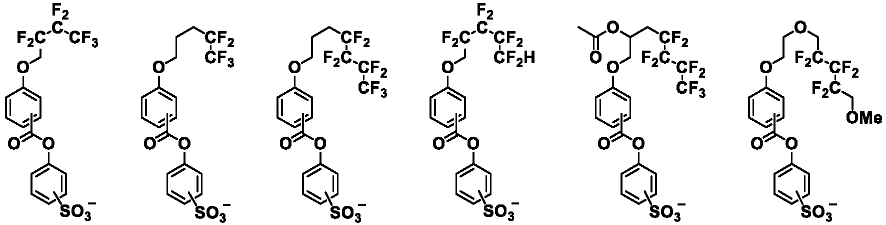

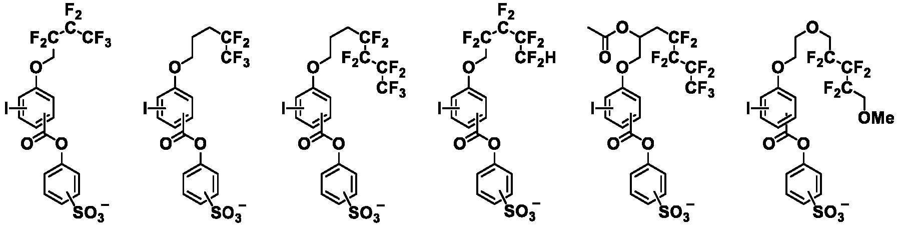

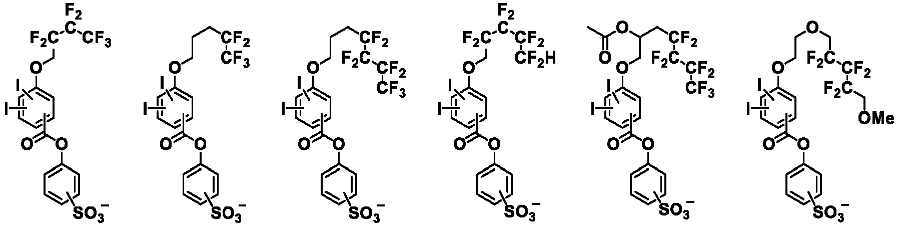

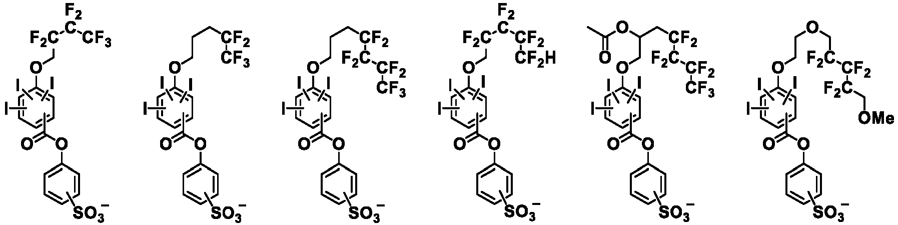

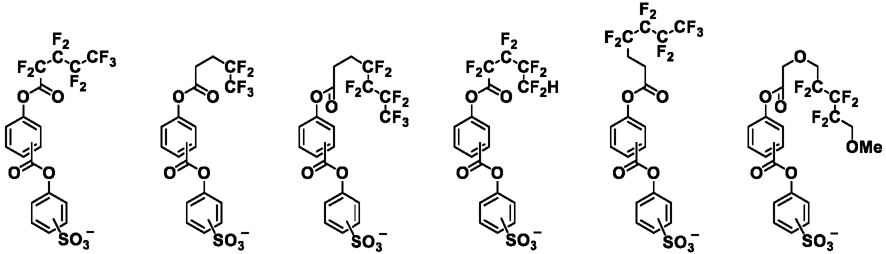

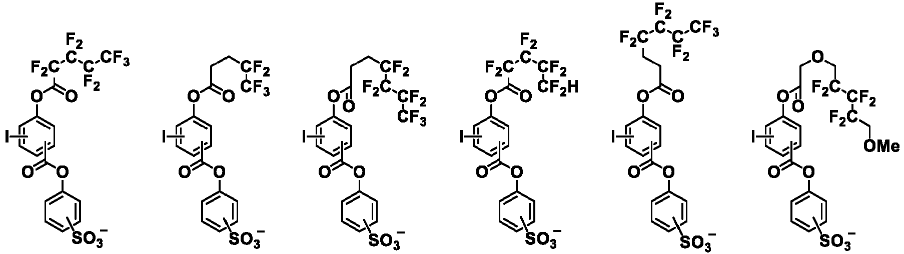

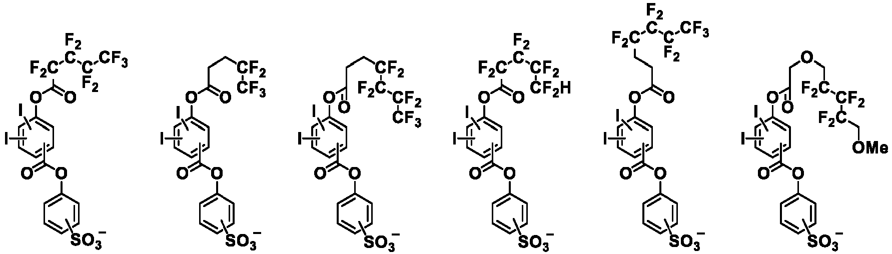

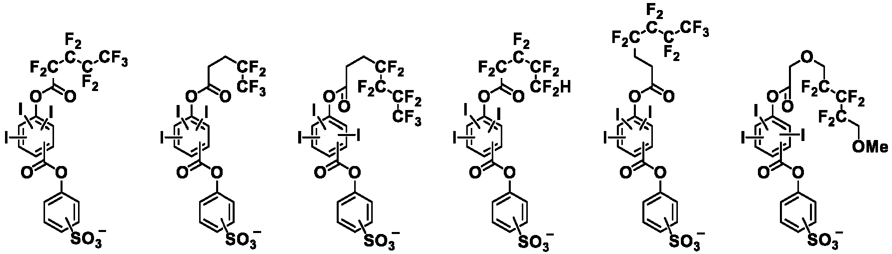

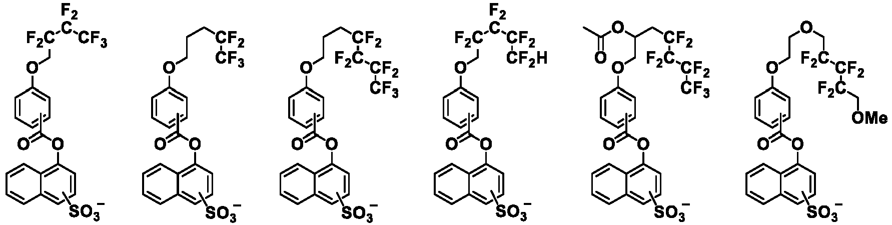

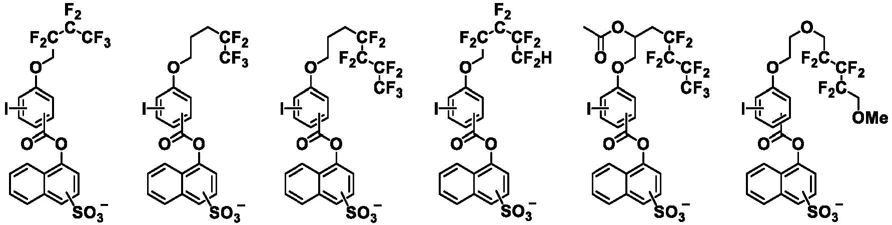

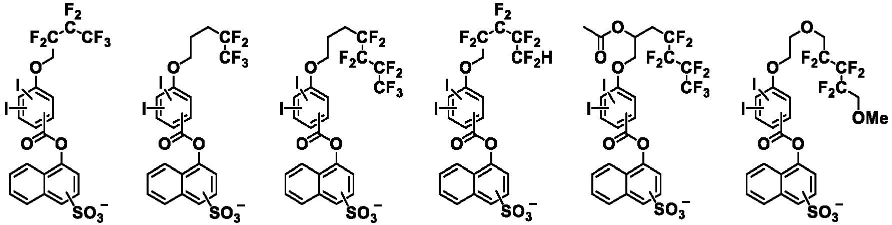

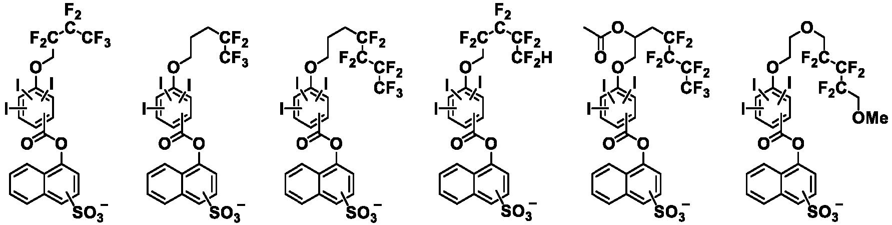

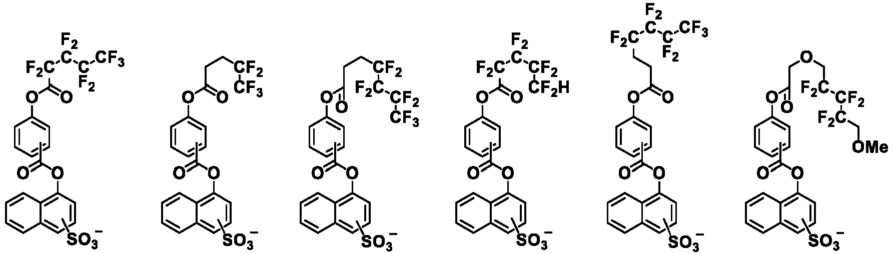

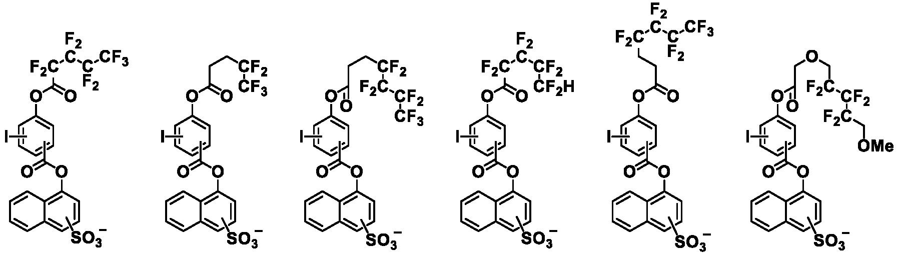

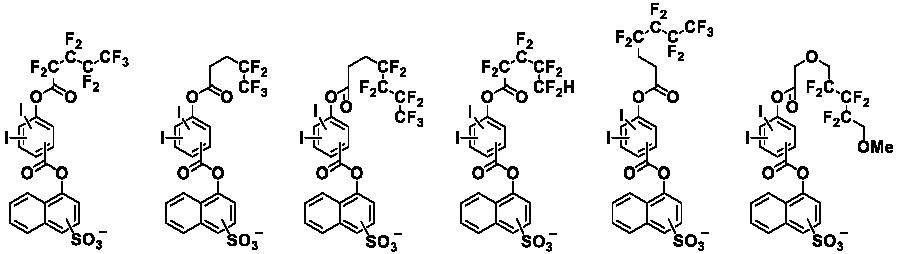

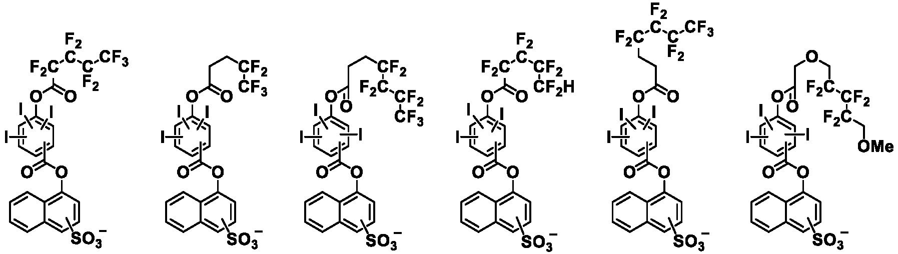

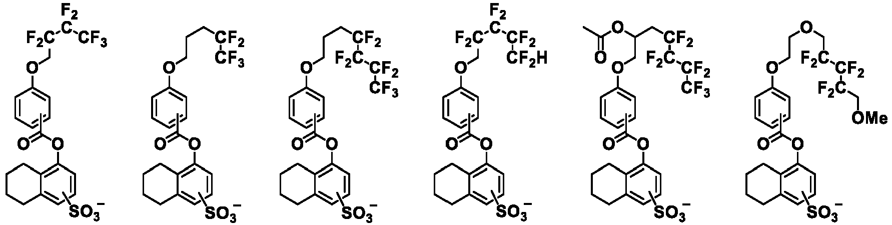

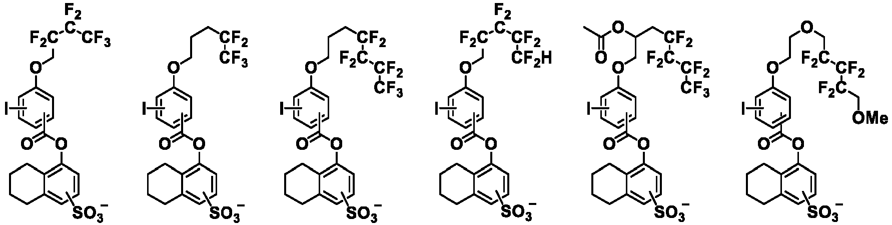

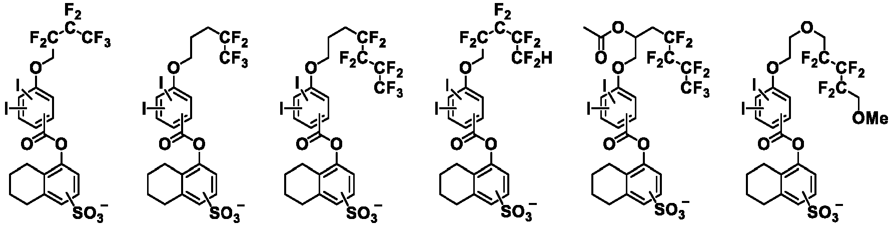

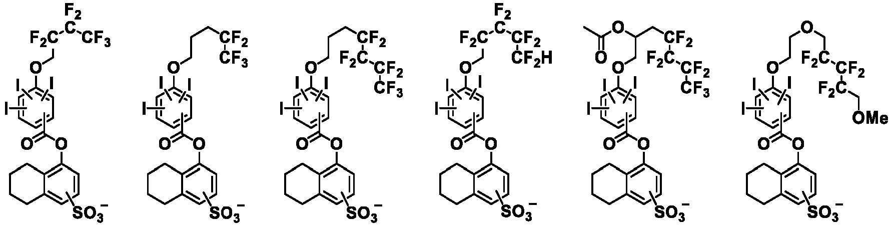

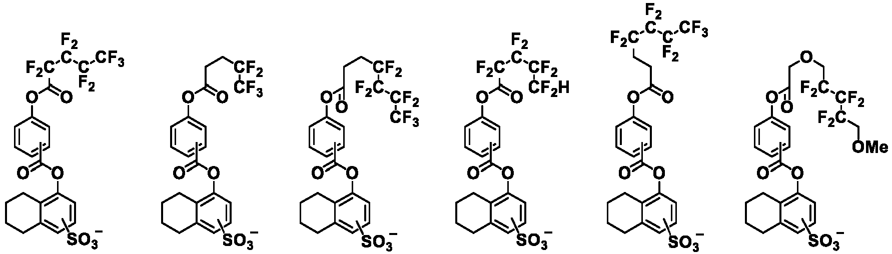

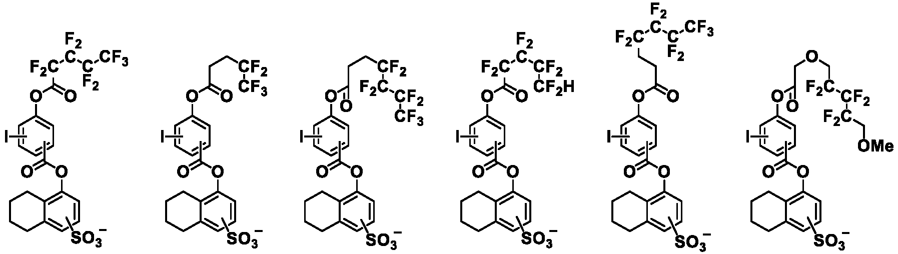

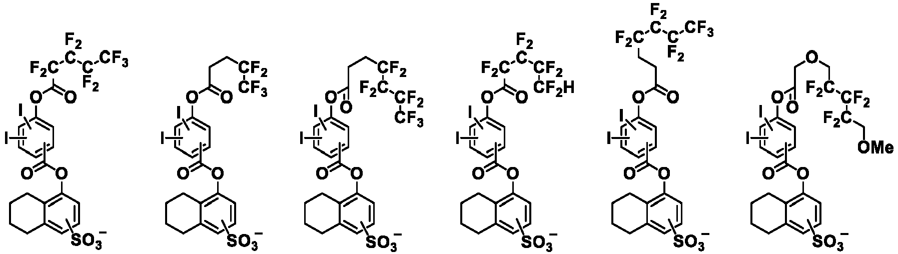

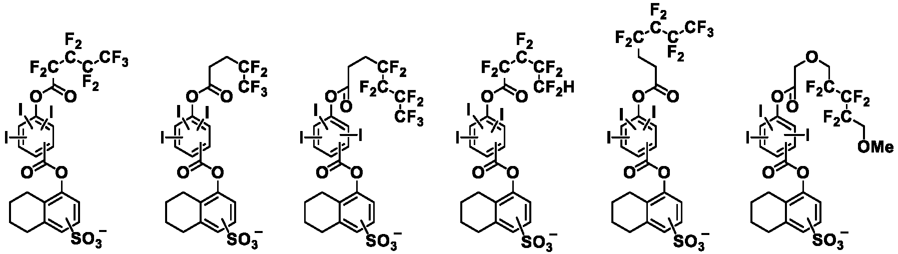

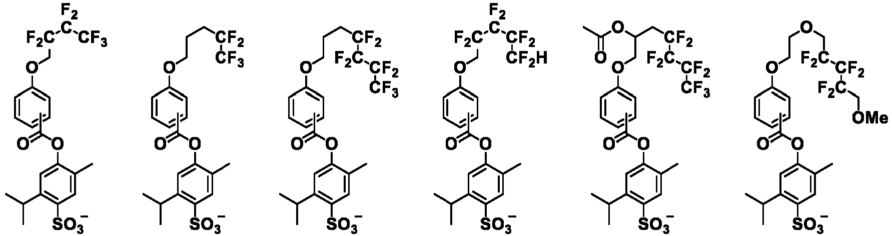

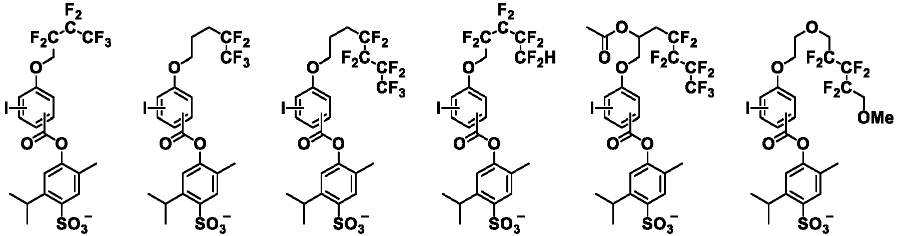

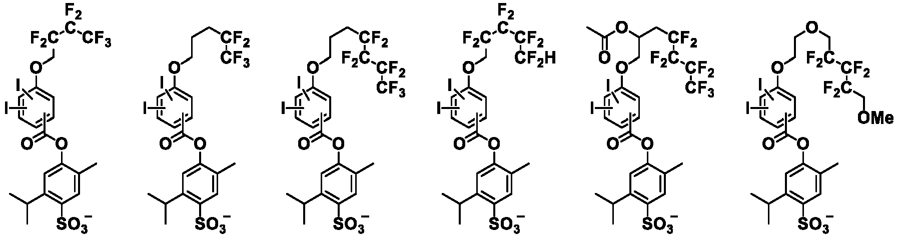

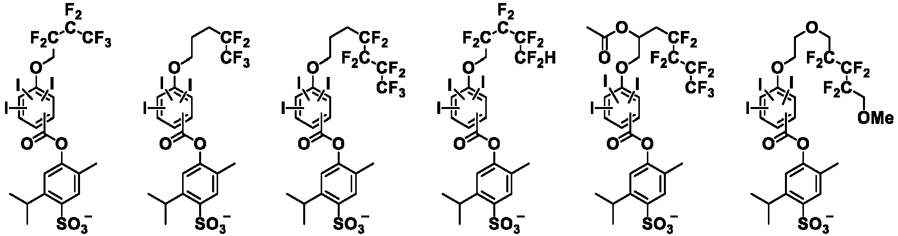

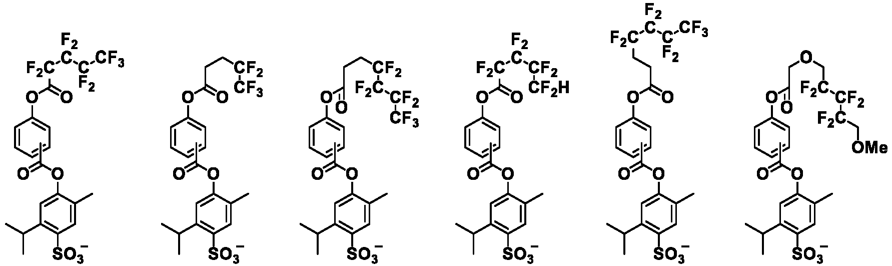

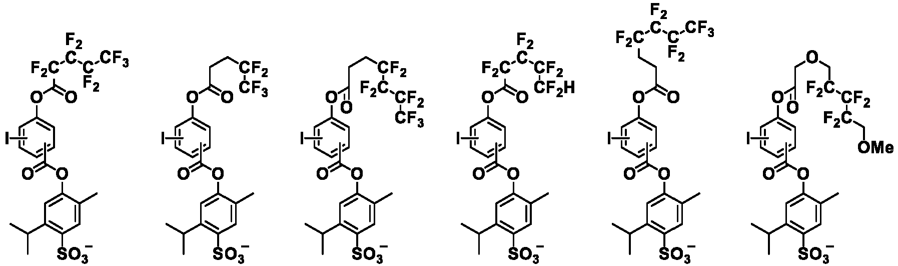

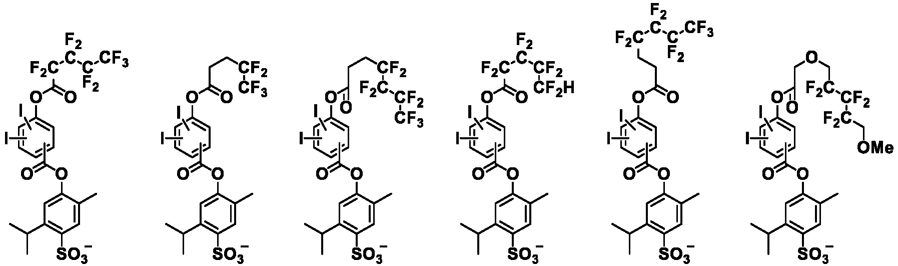

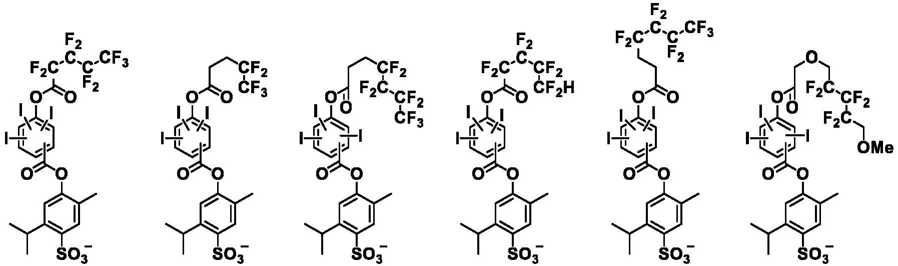

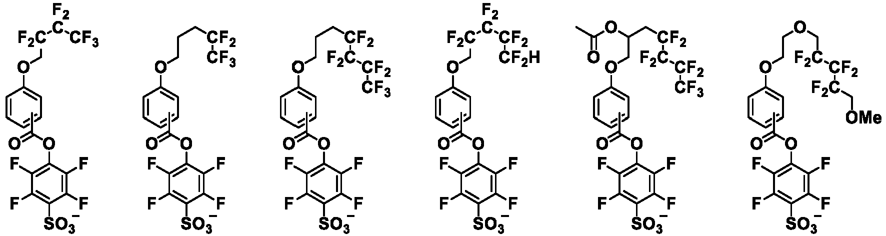

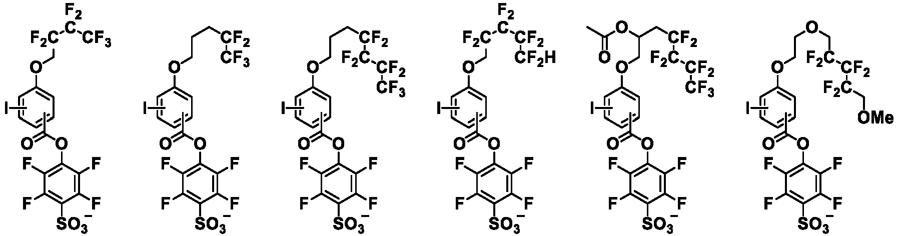

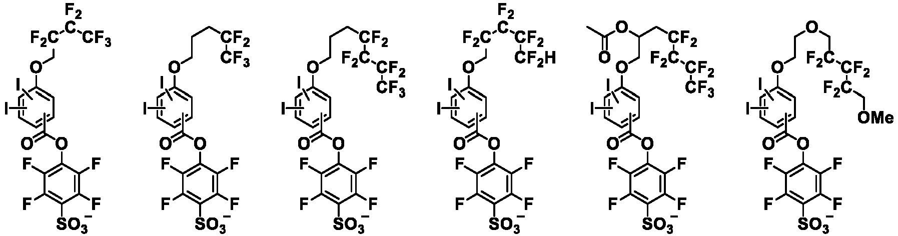

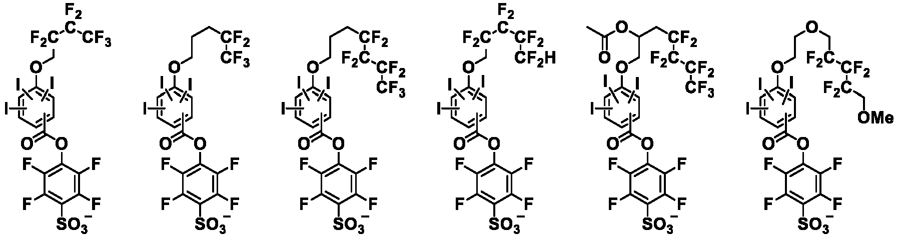

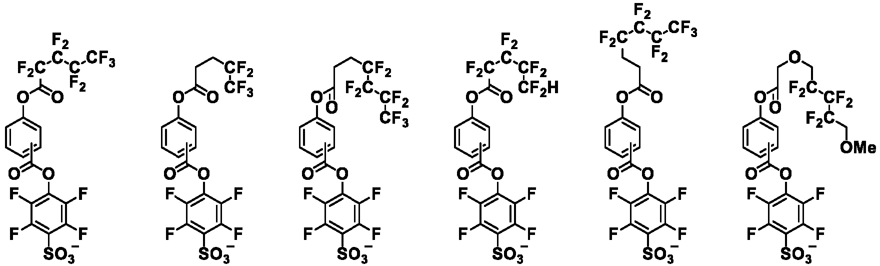

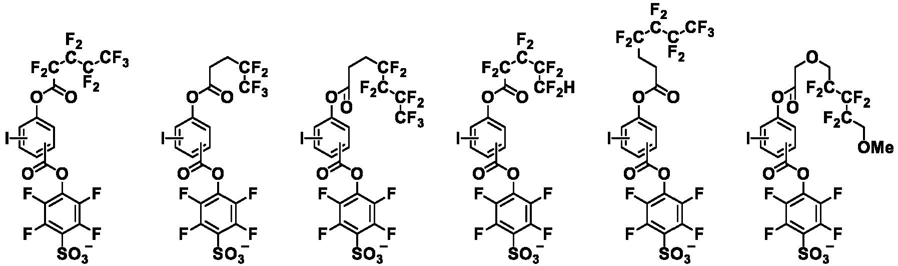

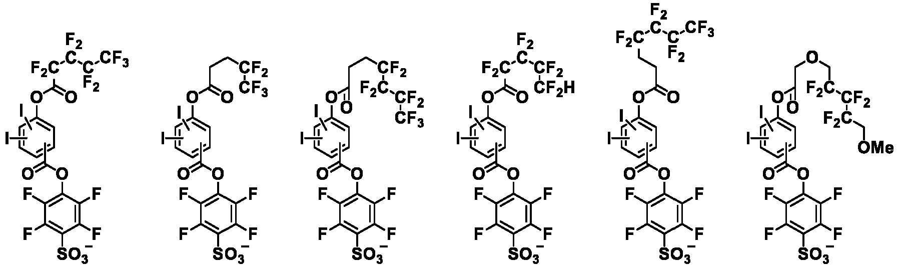

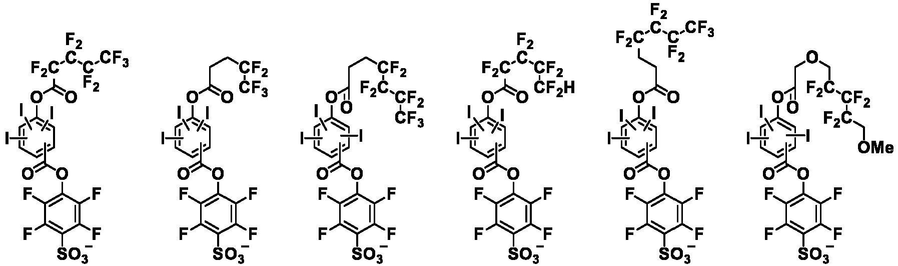

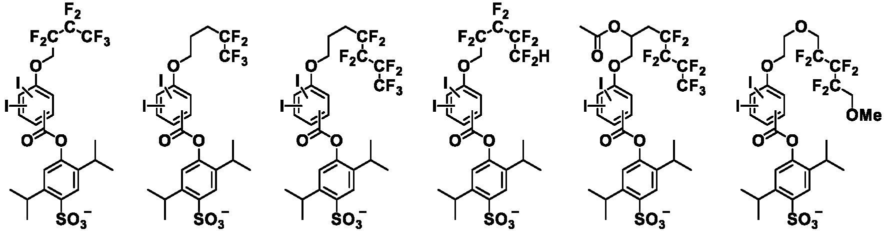

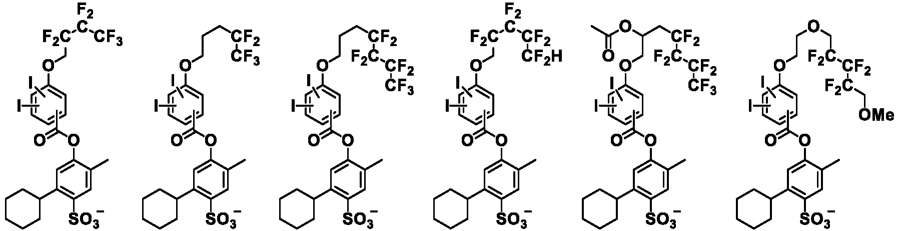

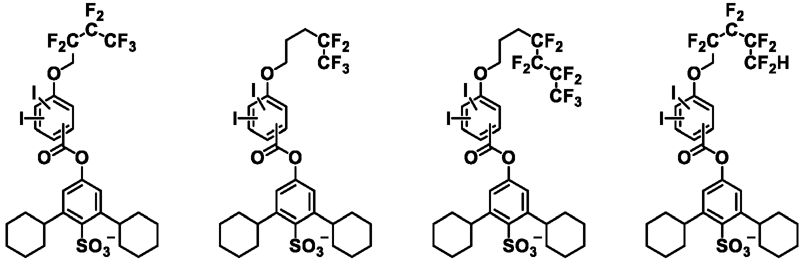

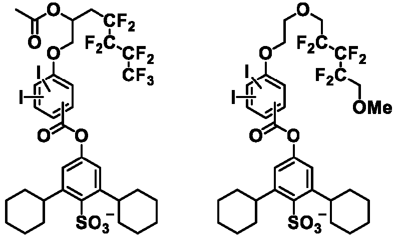

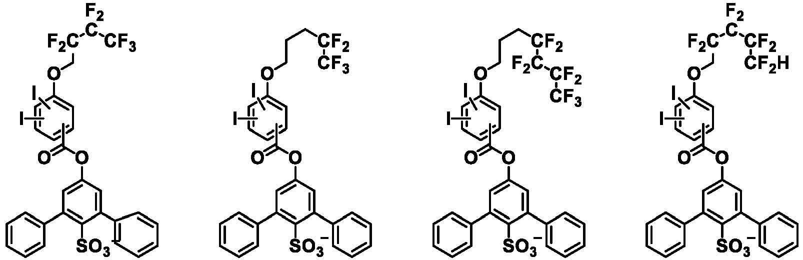

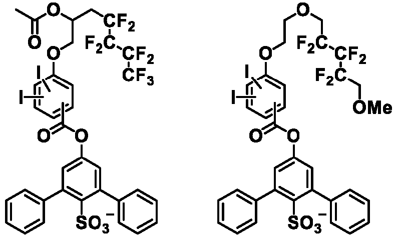

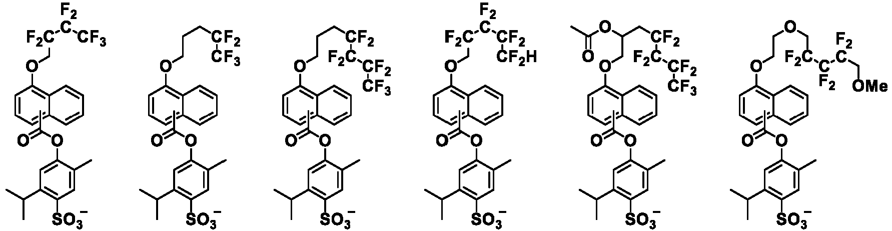

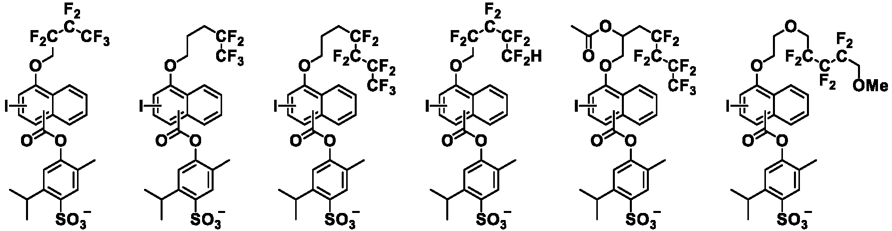

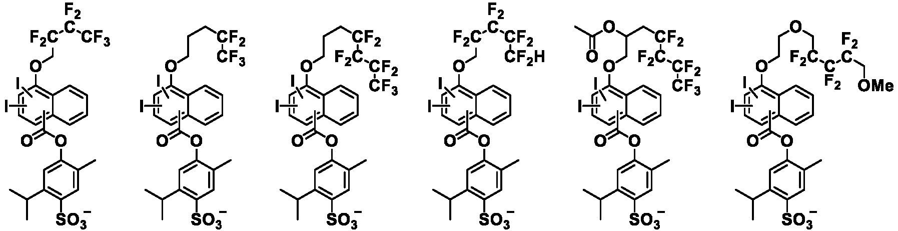

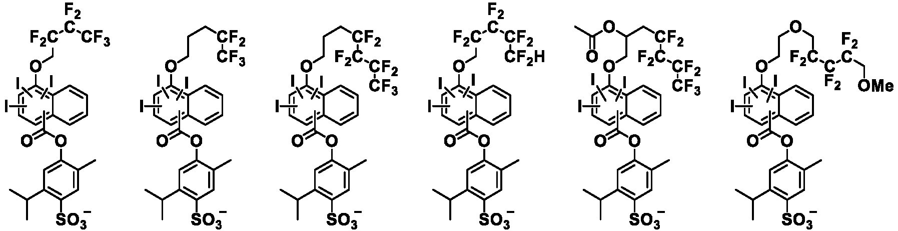

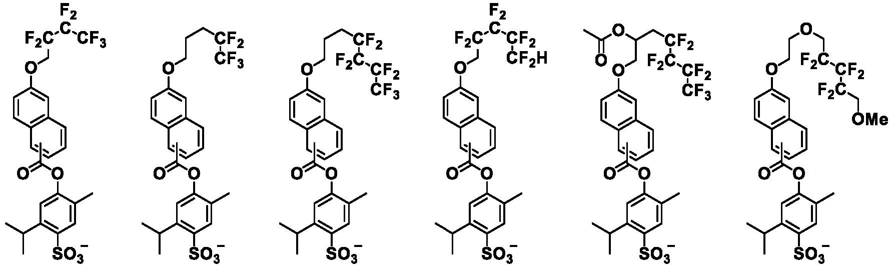

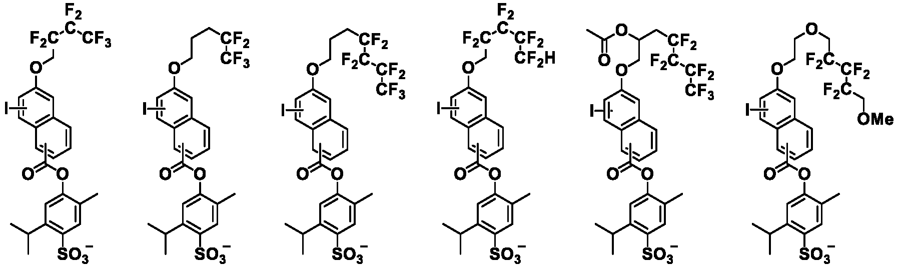

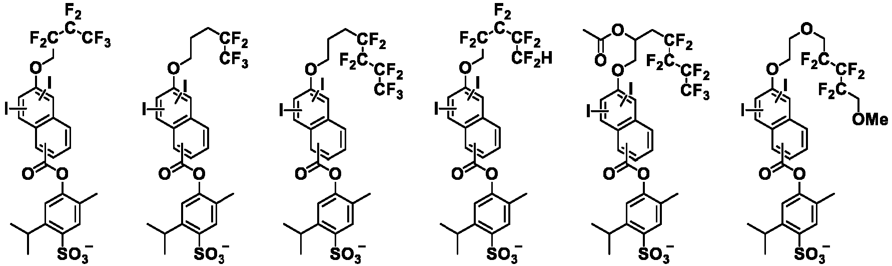

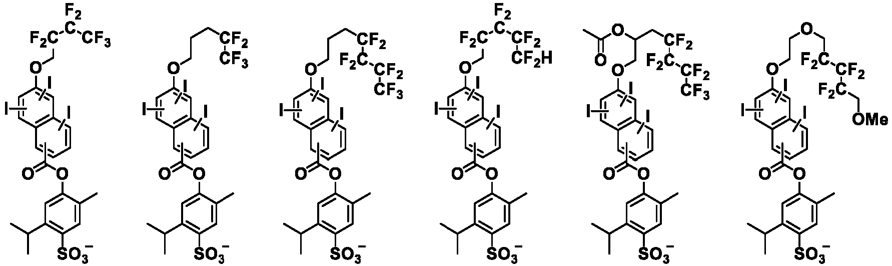

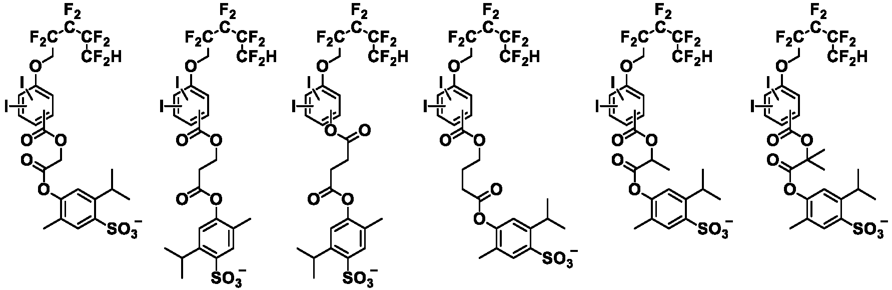

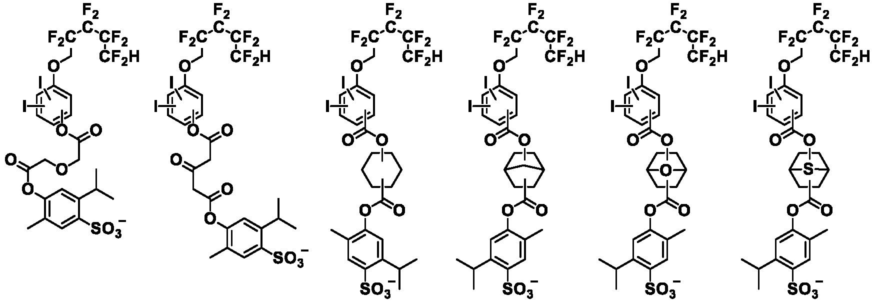

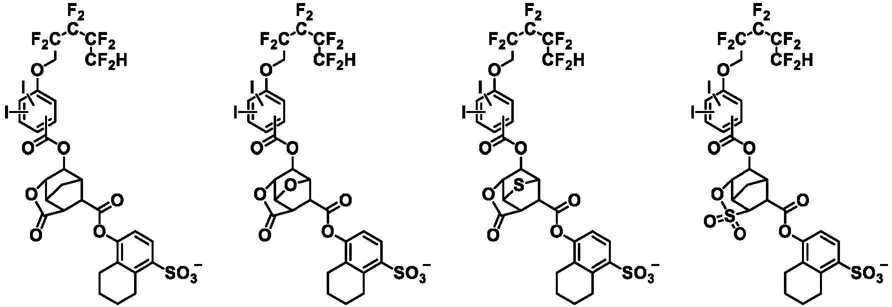

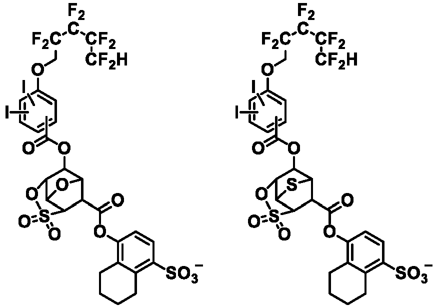

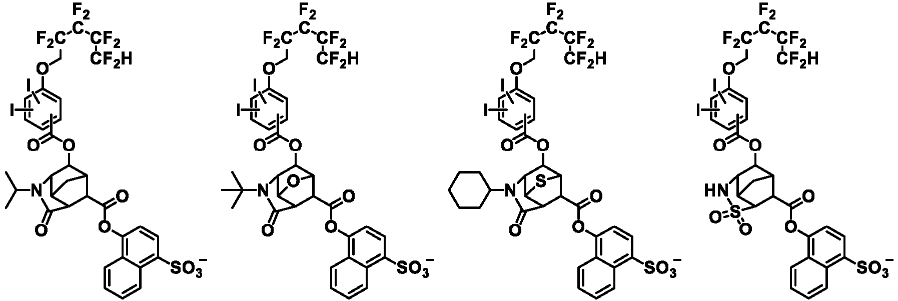

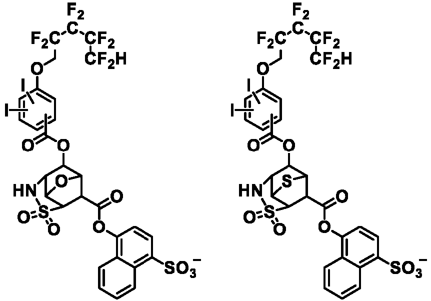

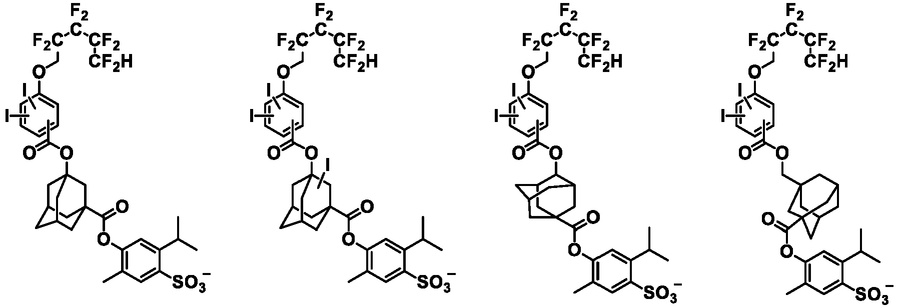

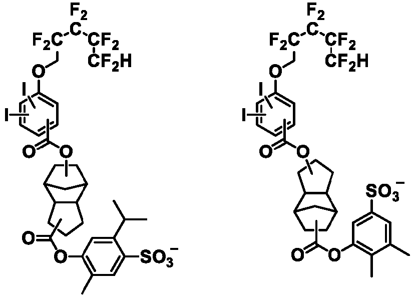

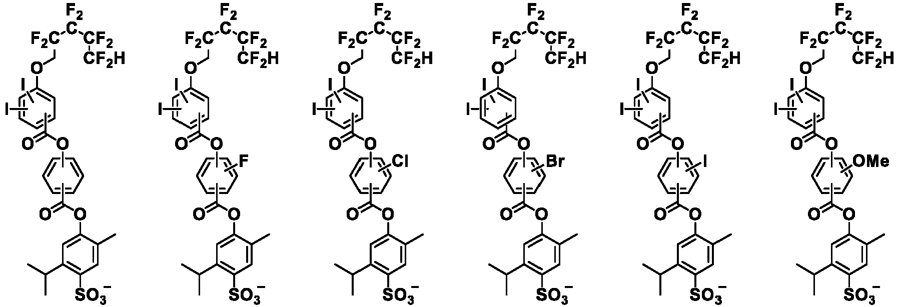

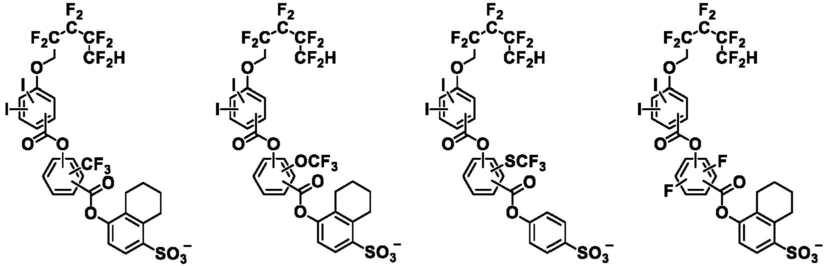

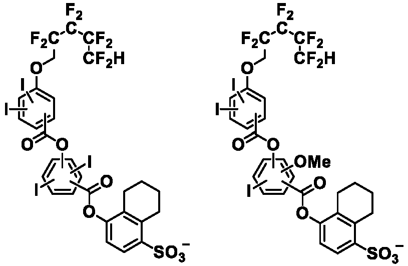

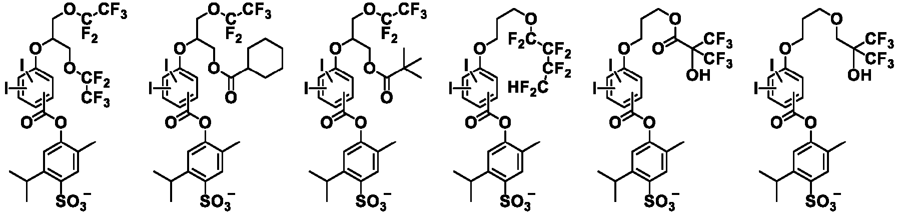

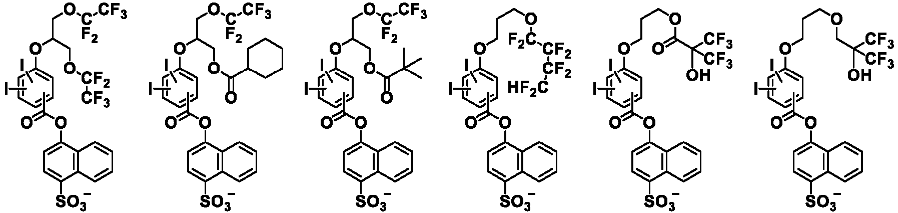

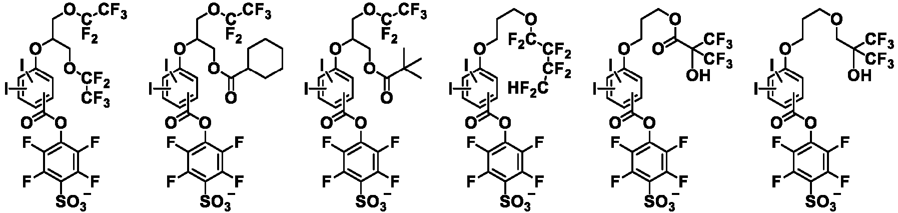

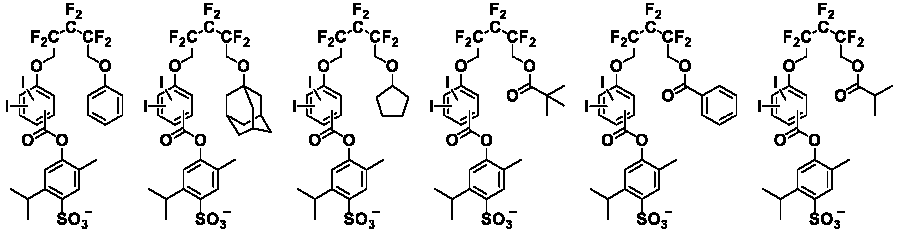

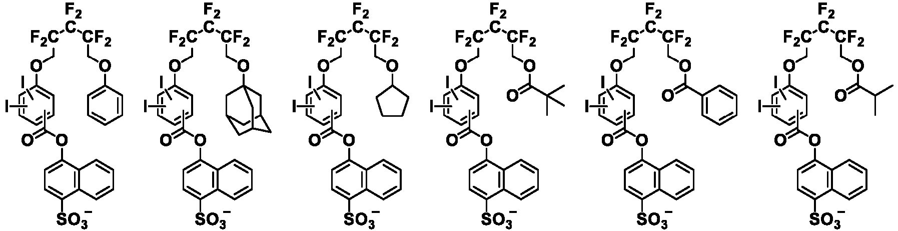

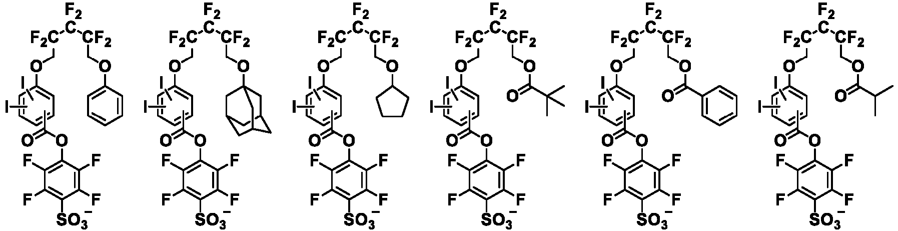

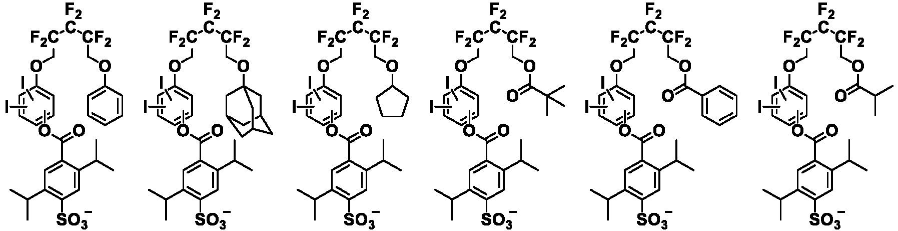

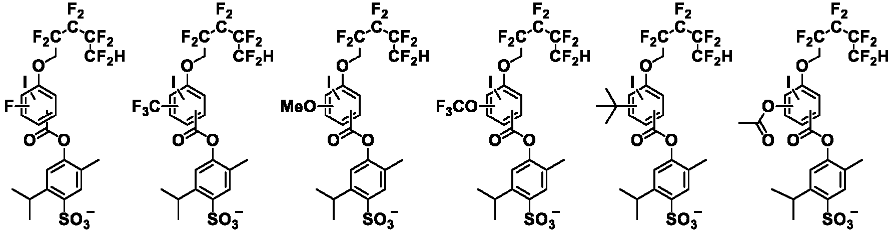

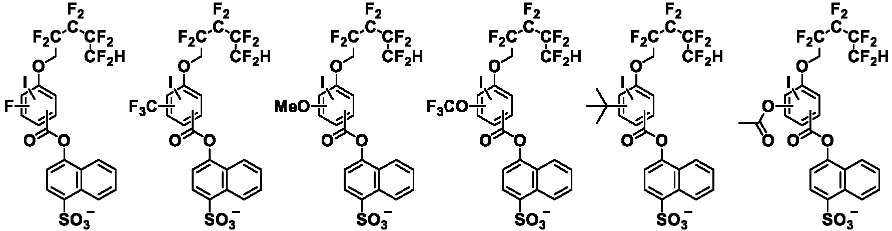

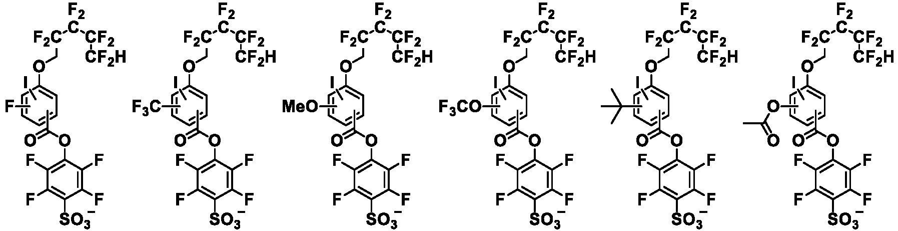

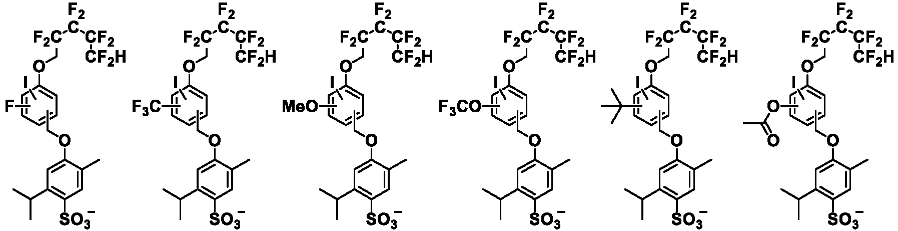

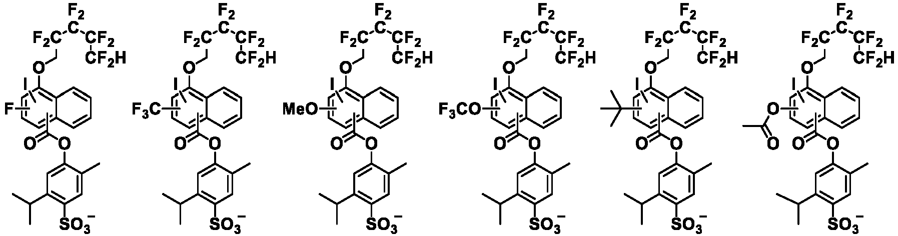

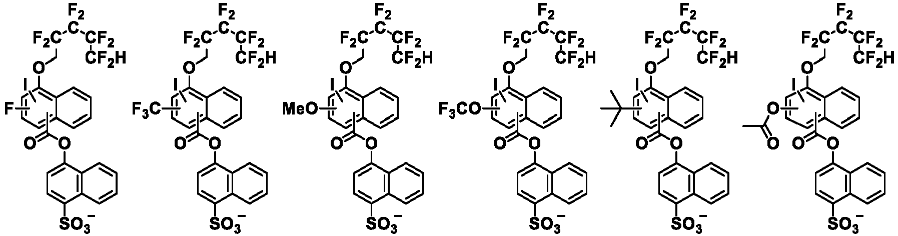

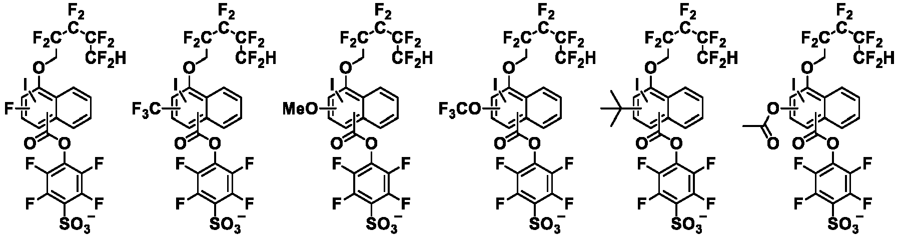

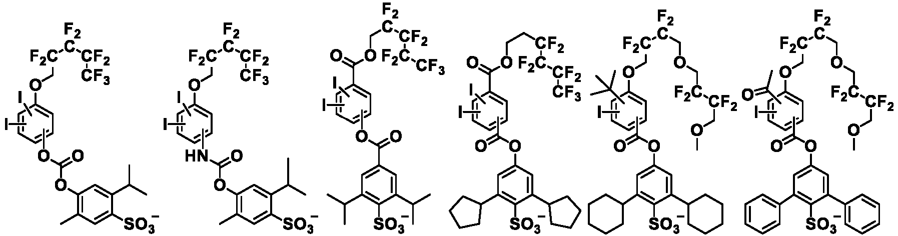

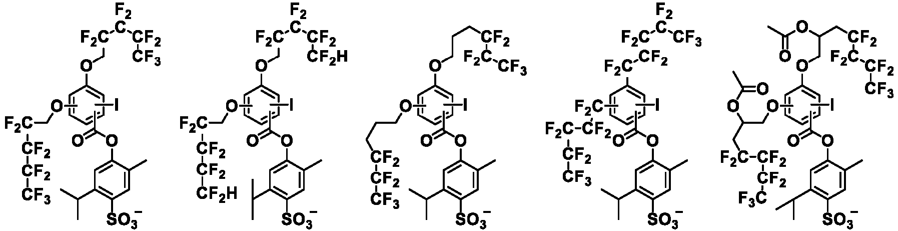

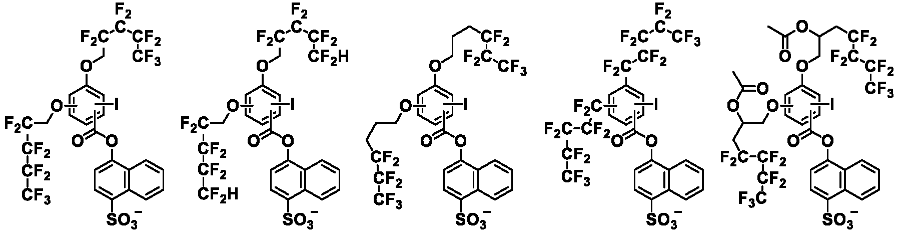

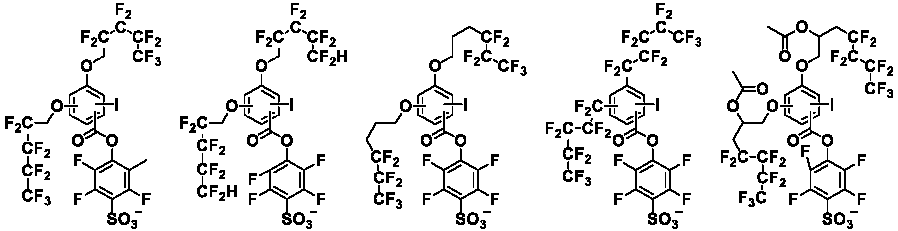

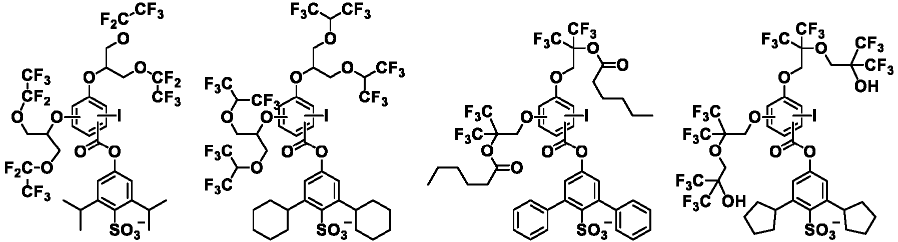

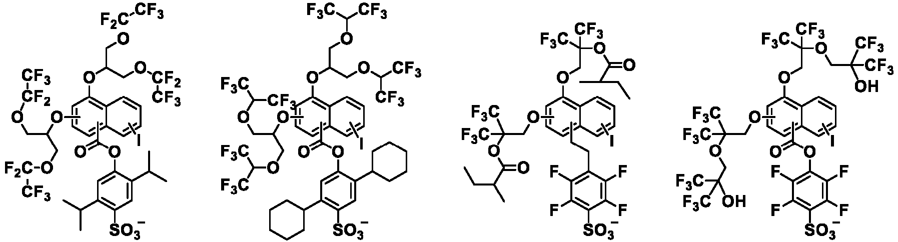

In formula (A), Z⁺ is an onium cation. The preferred onium cation is a sulfonium cation having the formula (cation-1) or iodonium cation having the formula (cation-2).

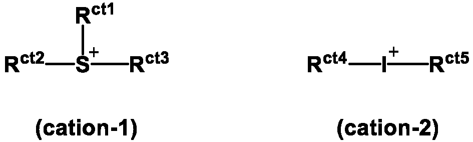

In formulae (cation-1) and (cation-2), R^{ct1} to R^{ct5} are each independently halogen or a C₁-C₃₀ hydrocarbyl group which may contain a heteroatom.

Suitable halogen atoms include fluorine, chlorine, bromine and iodine.

The hydrocarbyl group may be saturated or unsaturated and straight, branched or cyclic. Examples thereof include C₁-C₃₀ alkyl groups such as methyl, ethyl, n-propyl, isopropyl, n-butyl, isobutyl, sec-butyl, and tert-butyl; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₃₀ alkenyl groups such as vinyl, allyl, propenyl, butenyl, and hexenyl; C₃-C₃₀ cyclic unsaturated hydrocarbyl groups such as cyclohexenyl; C₆-C₃₀ aryl groups such as phenyl, naphthyl and thienyl; C₇-C₃₀ aralkyl groups such as benzyl, 1-phenylethyl and 2-phenylethyl, and combinations thereof. Inter alia, aryl groups are preferred. In the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, cyano, nitro, fluorine, chlorine, bromine, iodine, carbonyl, ether bond, ester bond, sulfonate ester bond, carbonate bond, lactone ring, sultone ring, carboxylic anhydride (-C(=O)-O-C(=O)-), or haloalkyl moiety.

Also, R^{ct1} and R^{ct2} may bond together to form a ring with the sulfur atom to which they are attached. Examples of the ring are shown below.

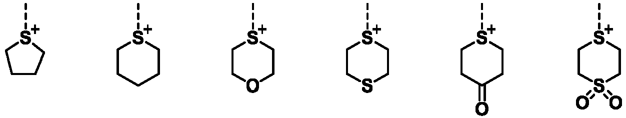

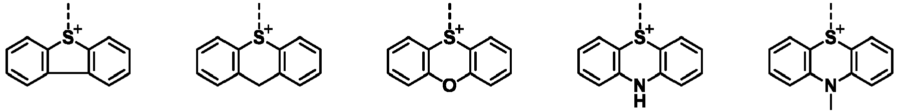

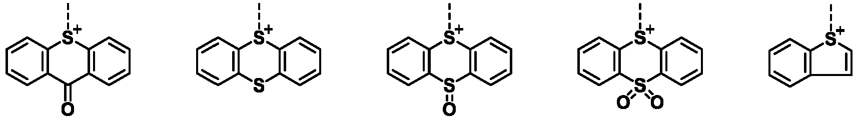

Herein the broken line designates a point of attachment to R^{ct3}.

Examples of the sulfonium cation having formula (cation-1) are shown below, but not limited thereto.

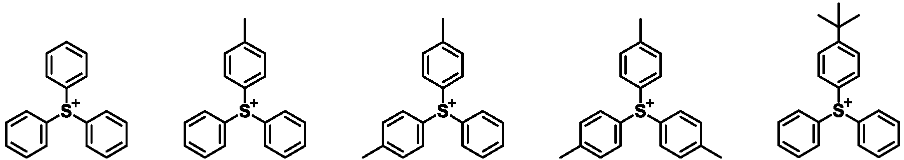

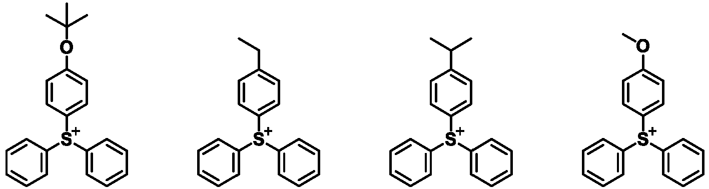

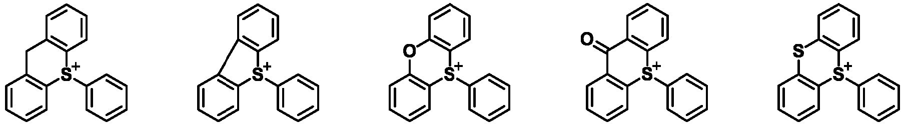

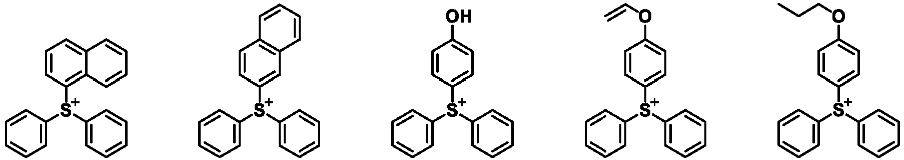

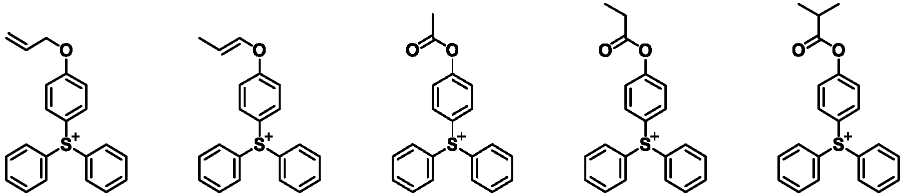

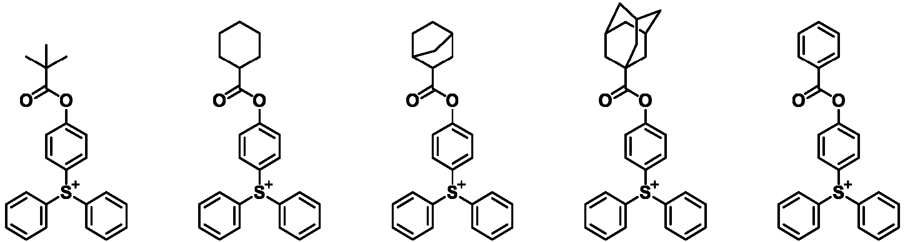

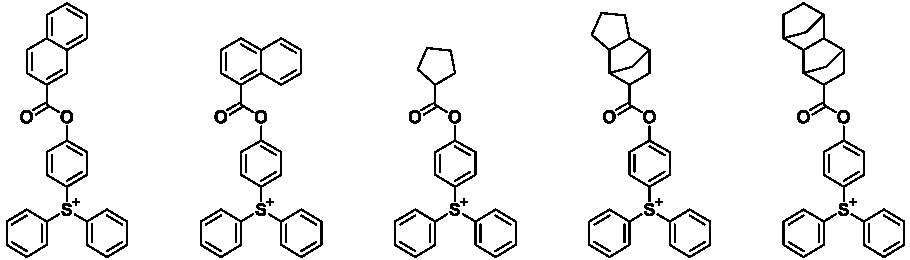

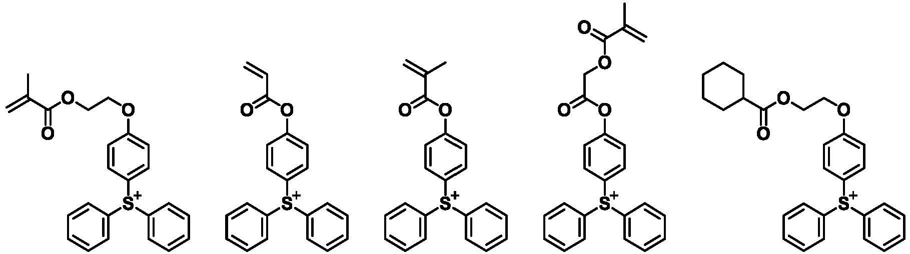

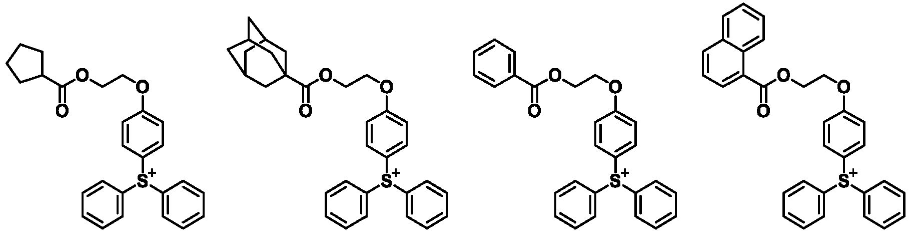

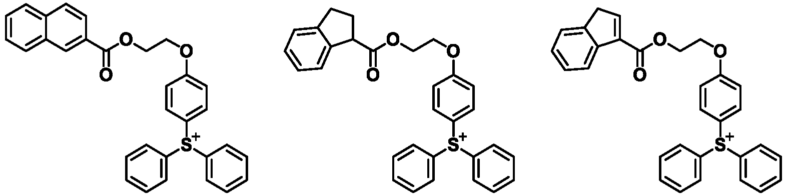

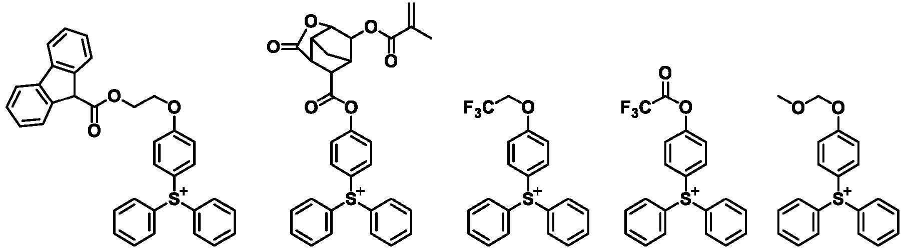

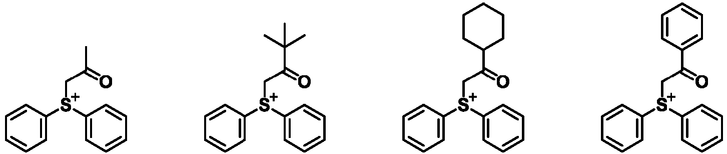

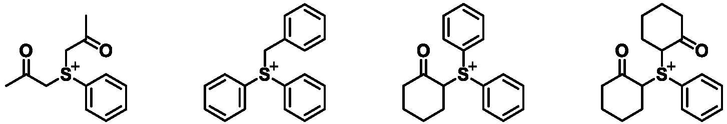

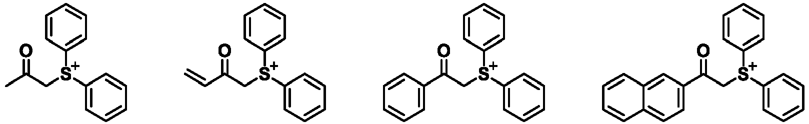

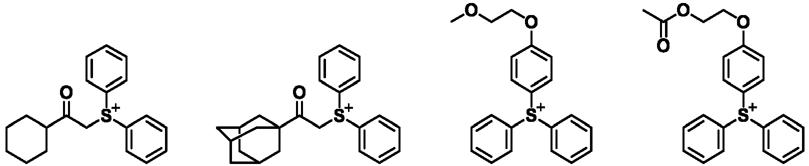

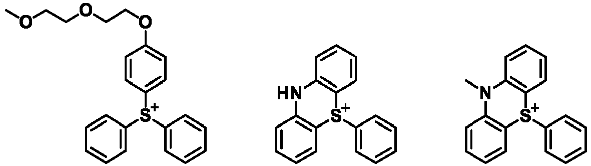

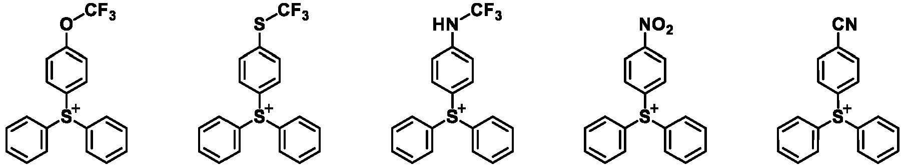

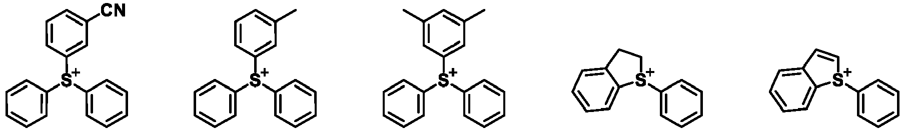

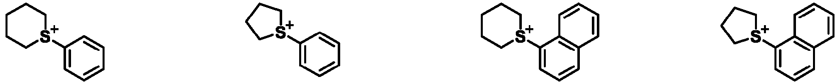

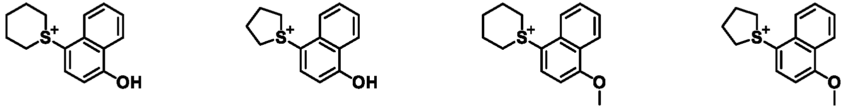

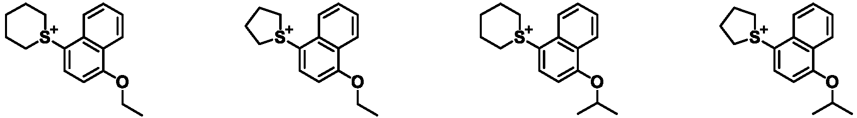

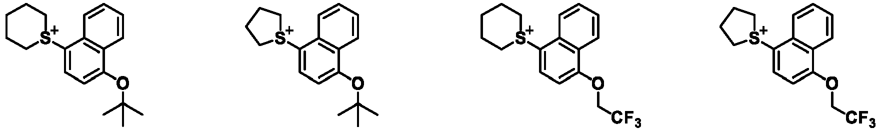

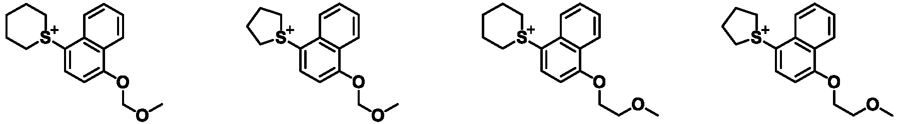

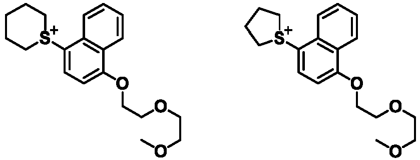

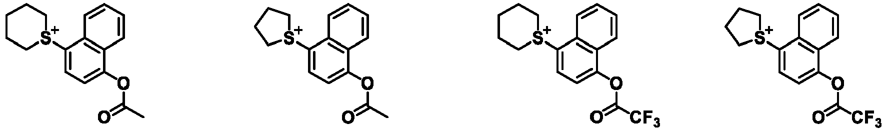

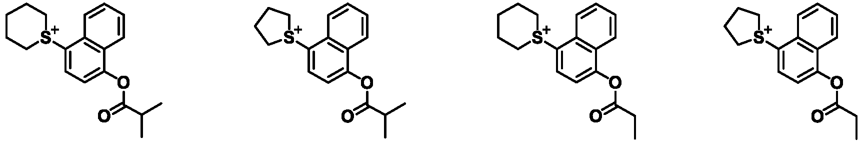

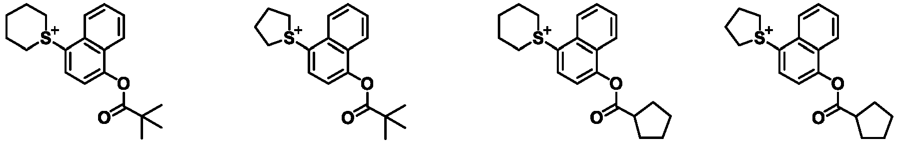

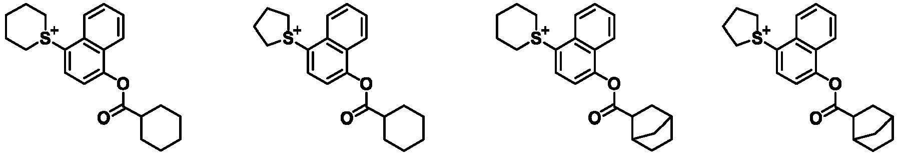

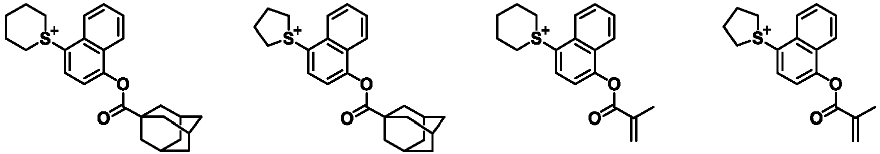

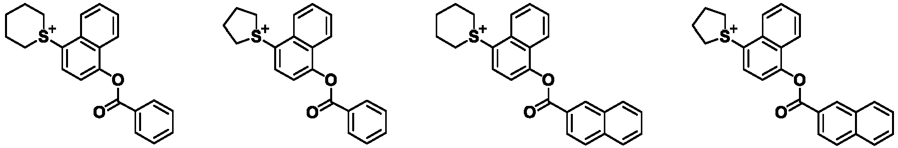

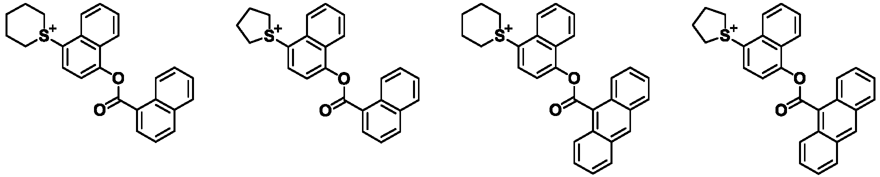

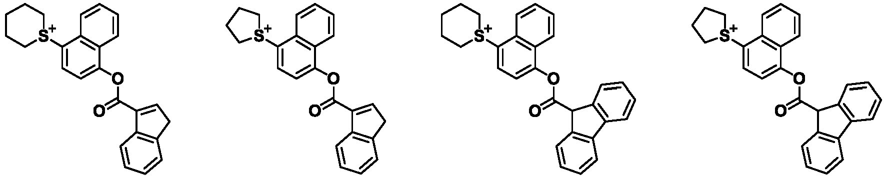

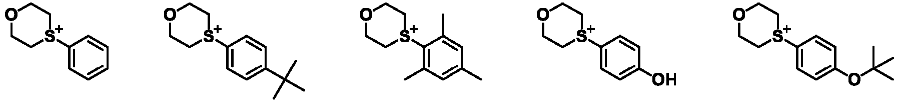

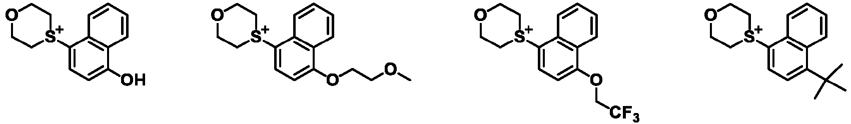

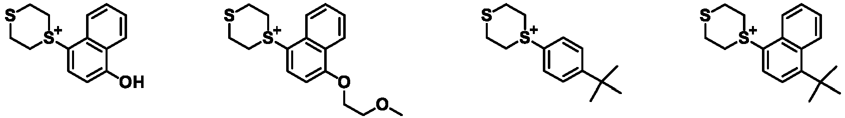

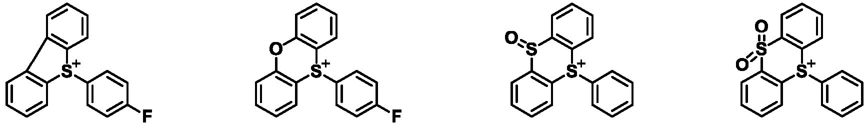

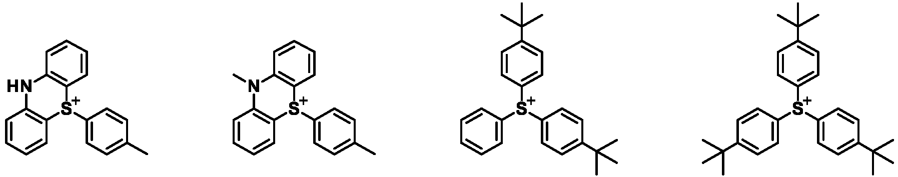

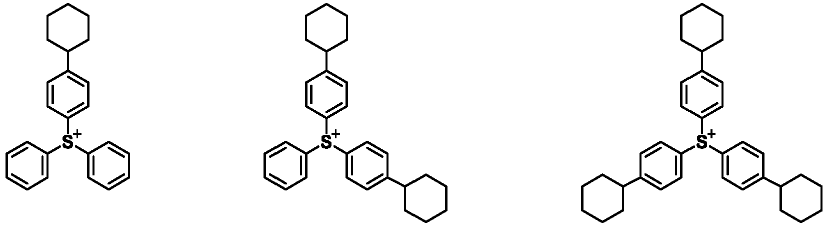

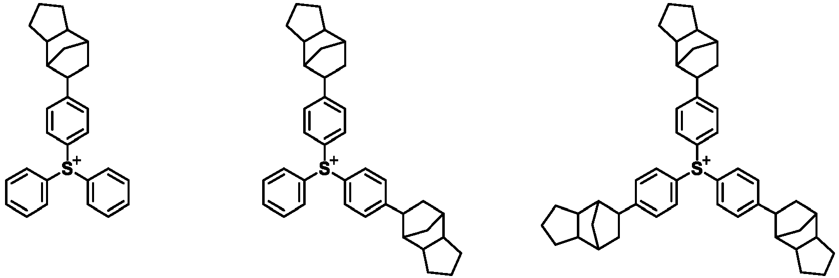

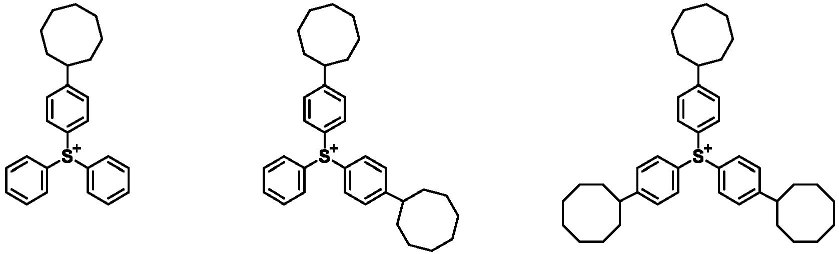

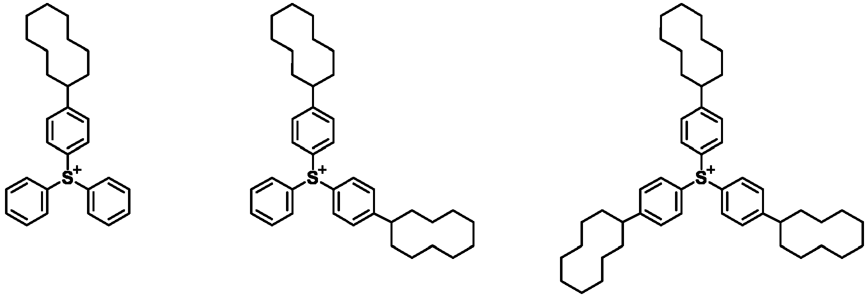

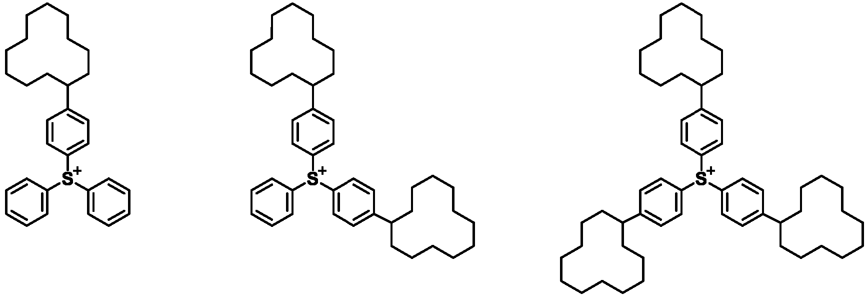

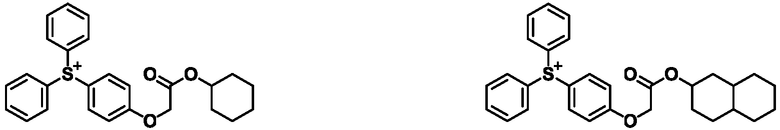

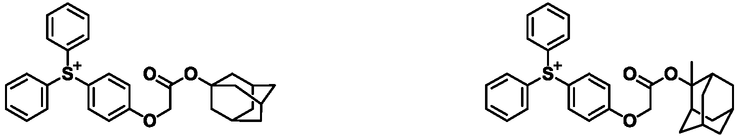

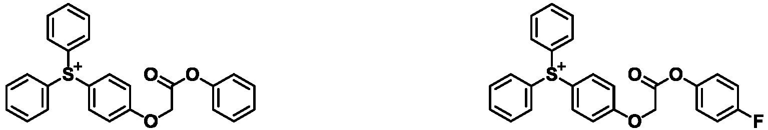

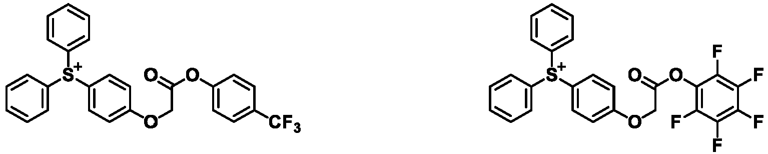

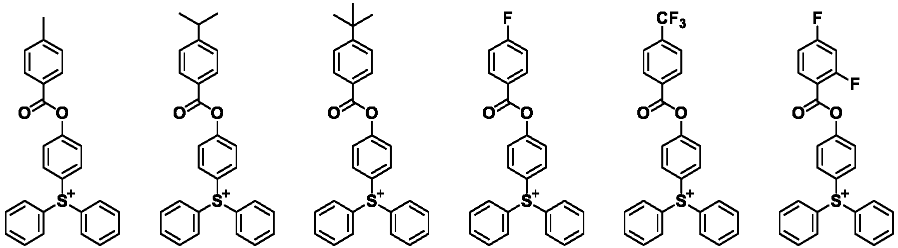

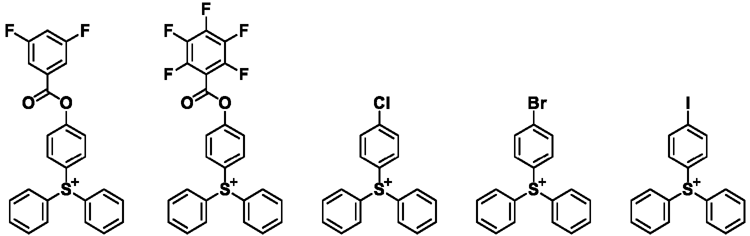

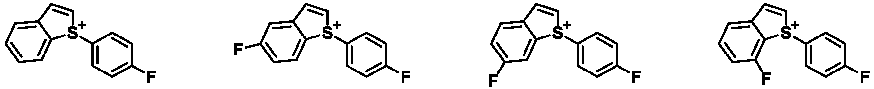

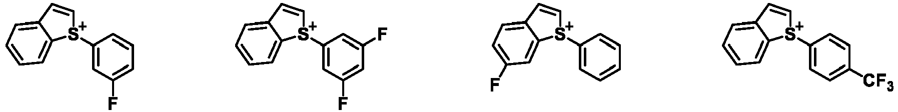

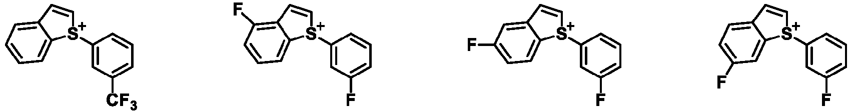

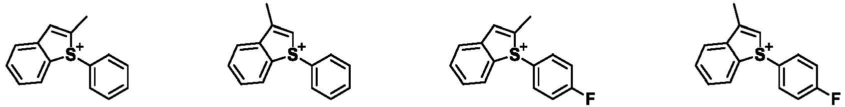

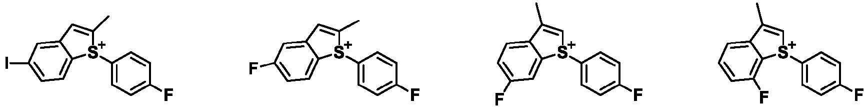

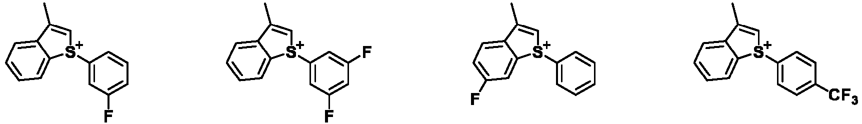

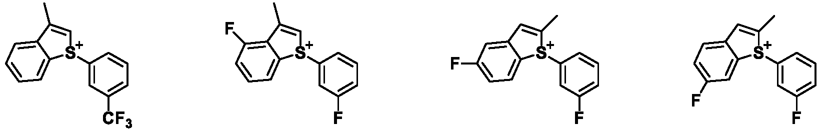

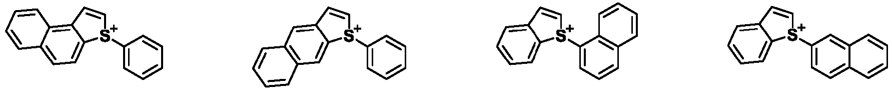

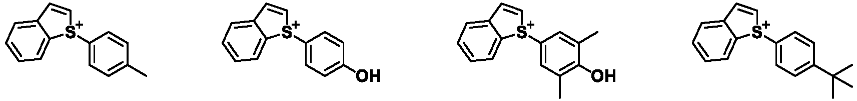

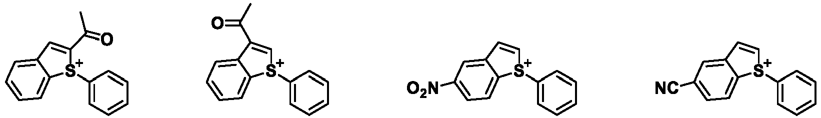

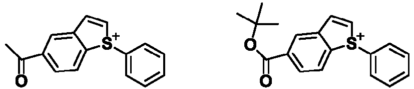

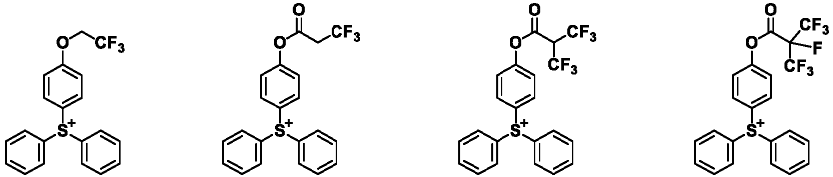

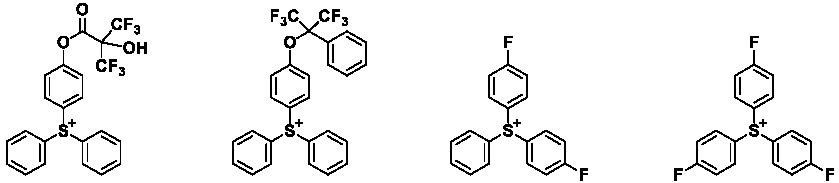

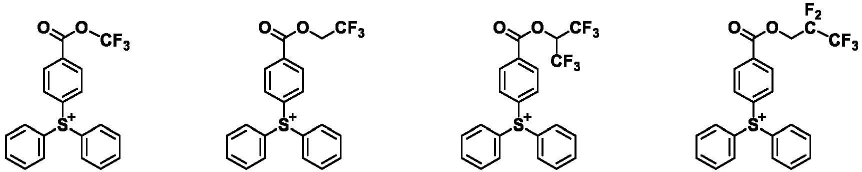

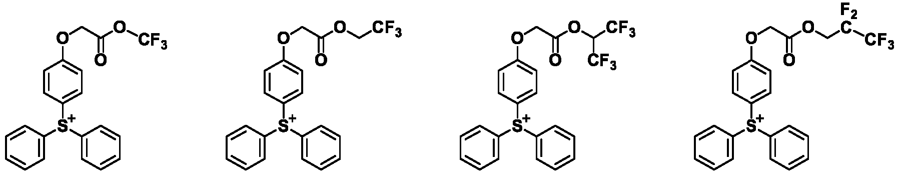

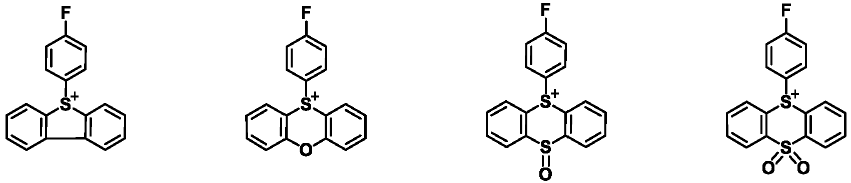

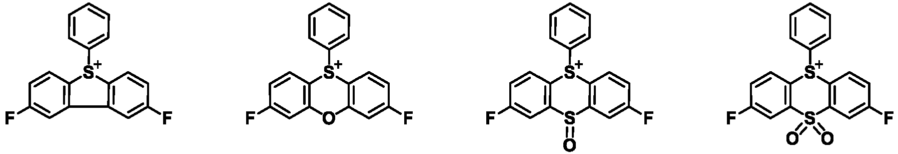

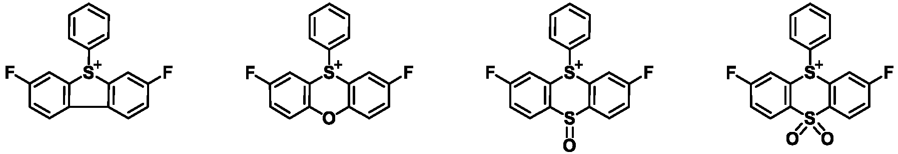

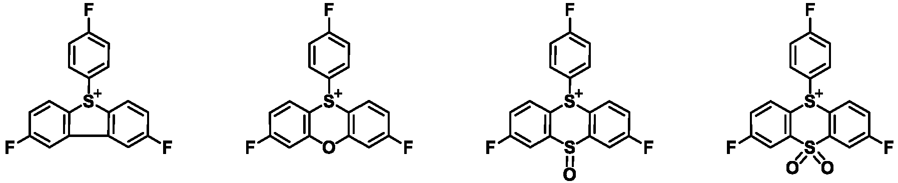

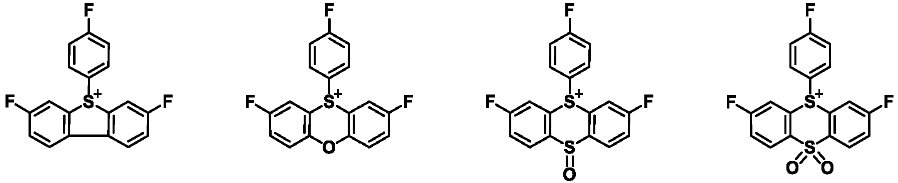

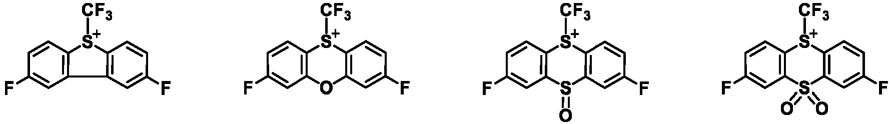

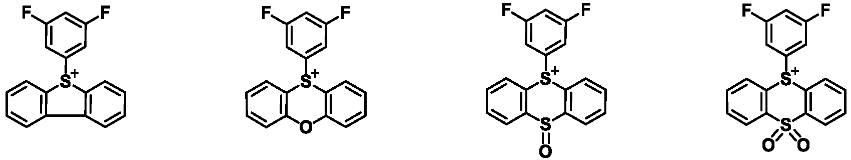

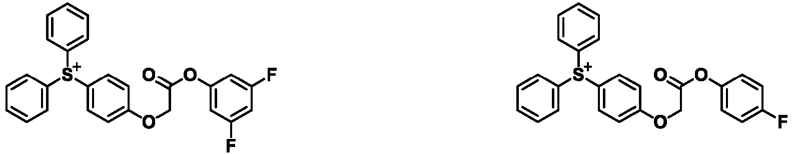

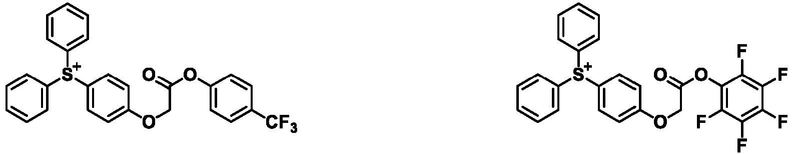

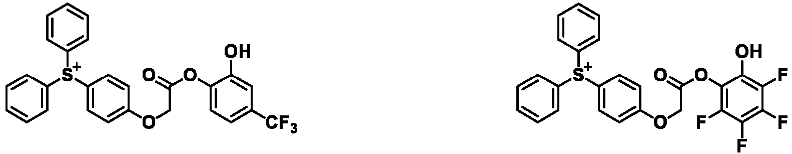

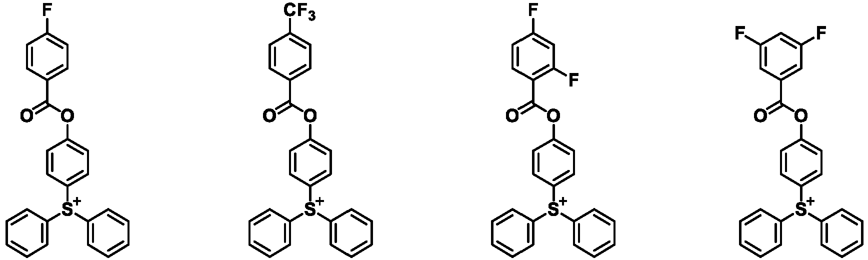

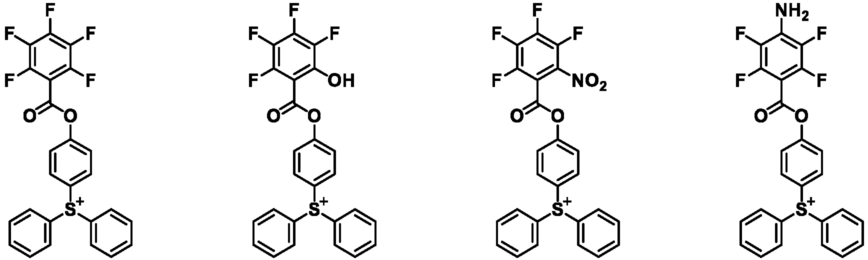

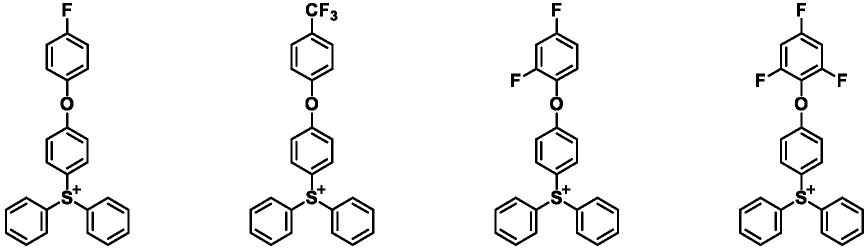

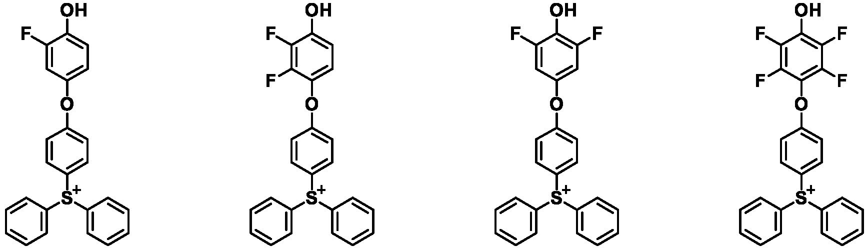

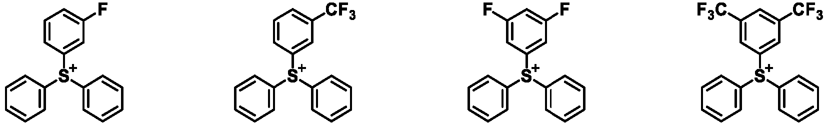

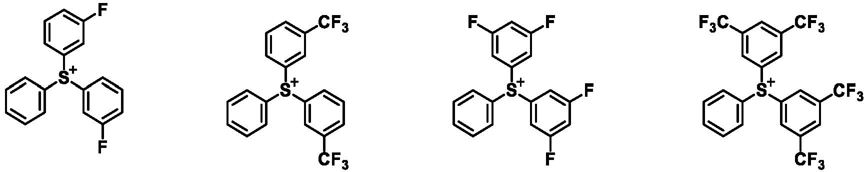

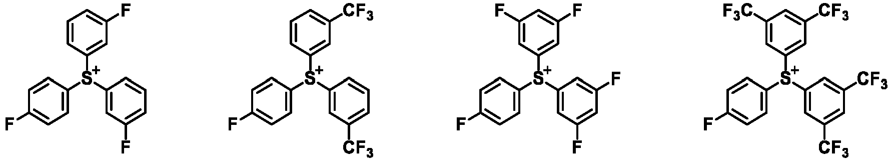

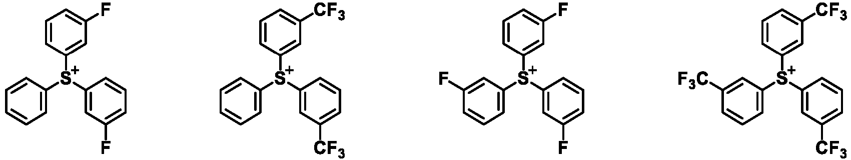

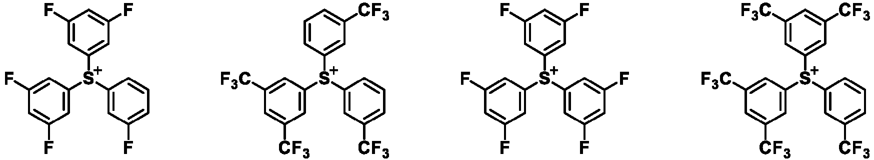

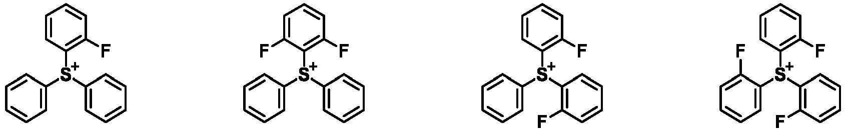

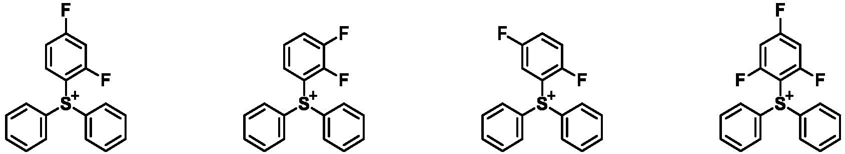

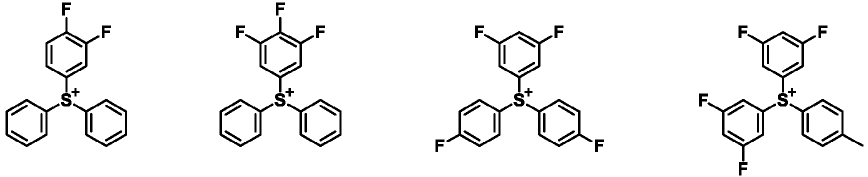

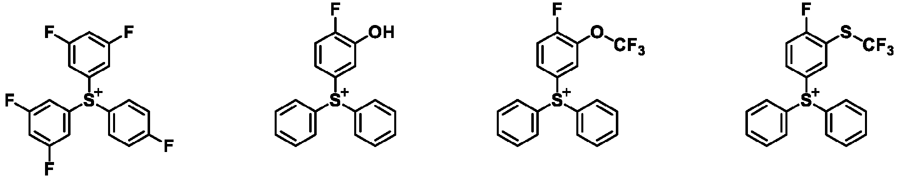

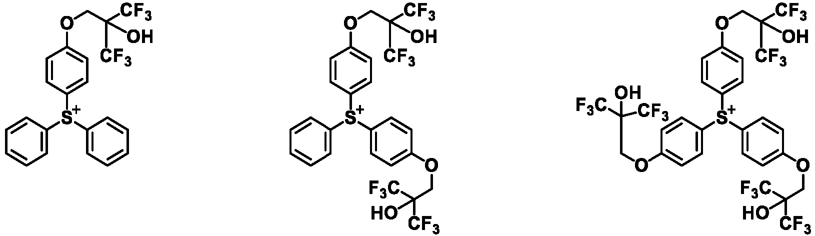

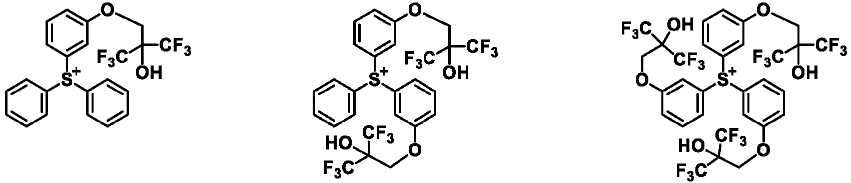

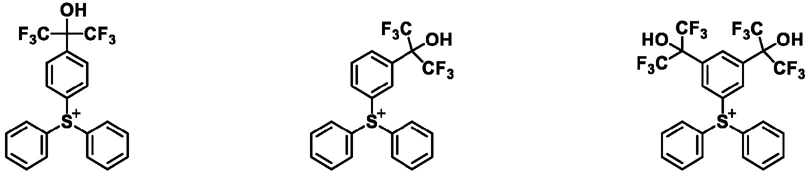

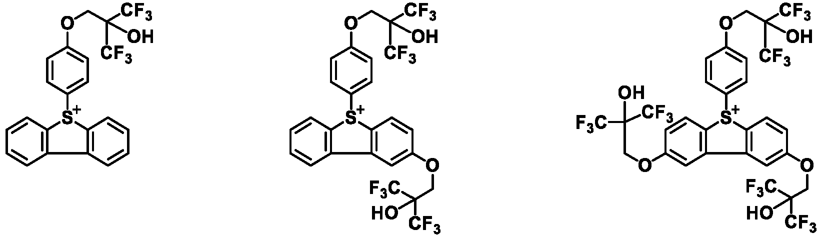

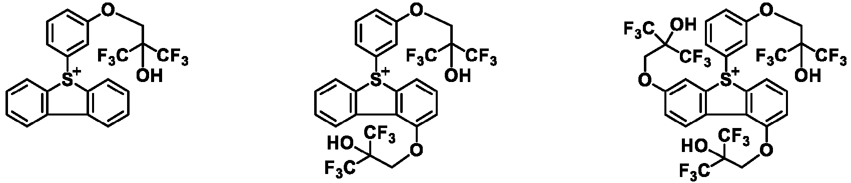

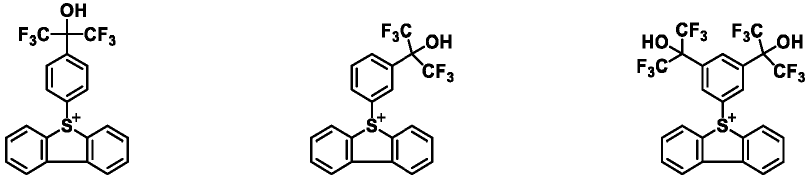

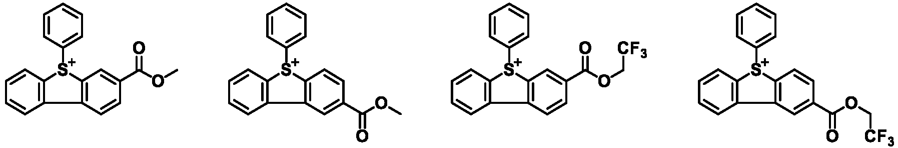

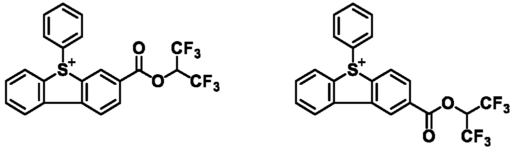

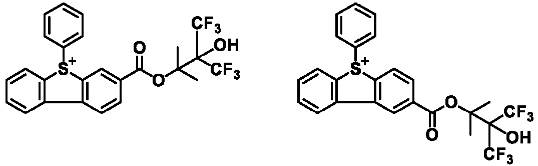

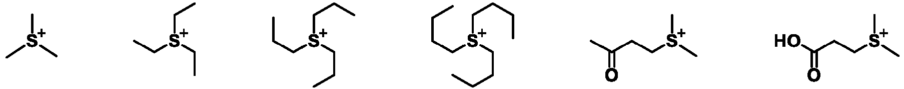

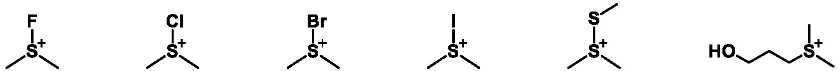

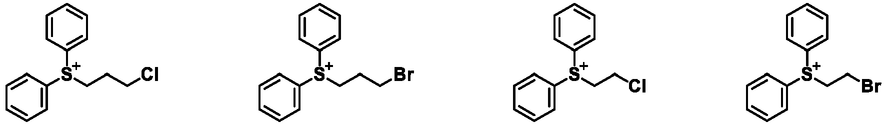

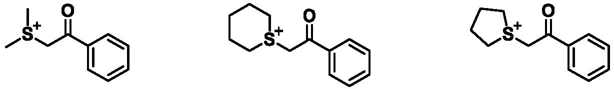

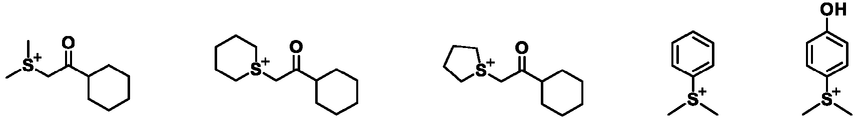

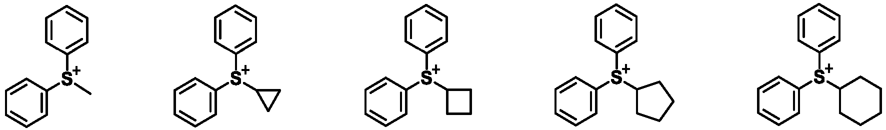

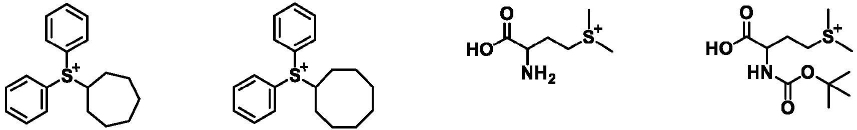

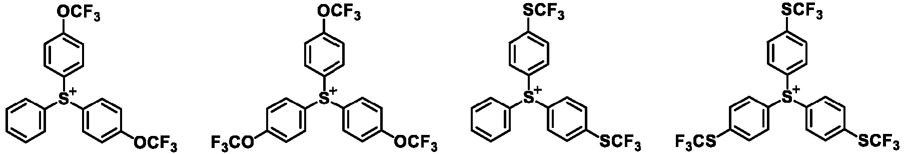

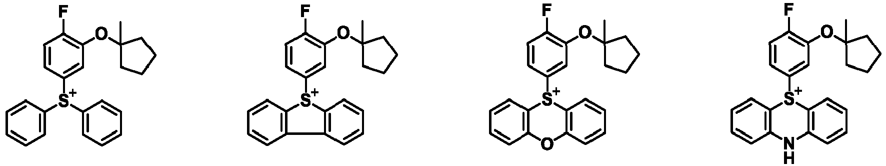

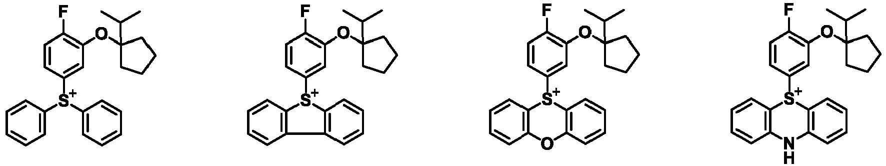

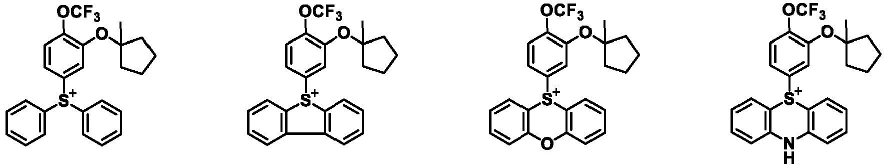

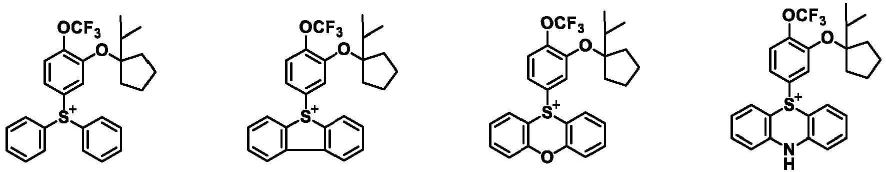

Examples of the iodonium cation having formula (cation-2) are shown below, but not limited thereto.

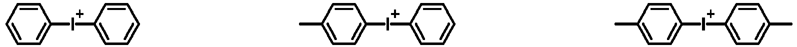

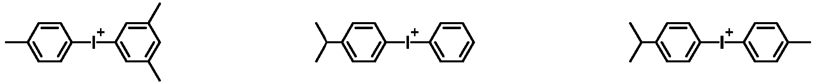

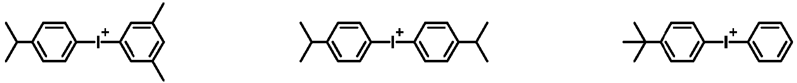

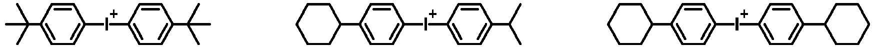

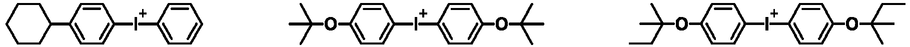

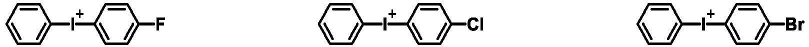

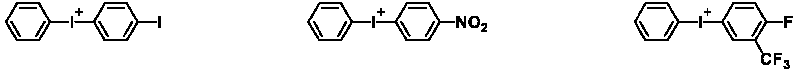

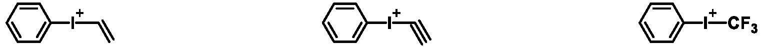

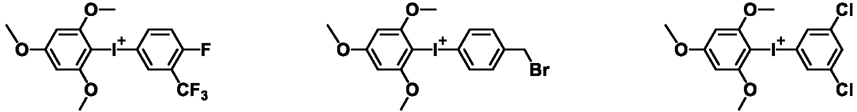

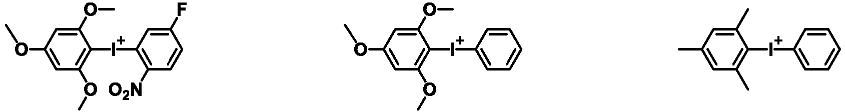

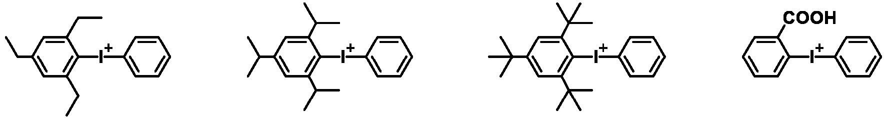

Specific structures of the onium salt include arbitrary combinations of the anion with the cation, both as exemplified above.

The onium salt (A) is synthesized by any well-known methods. Reference is now made to the preparation of an onium salt having the following formula (PAG-1-ex), for example. 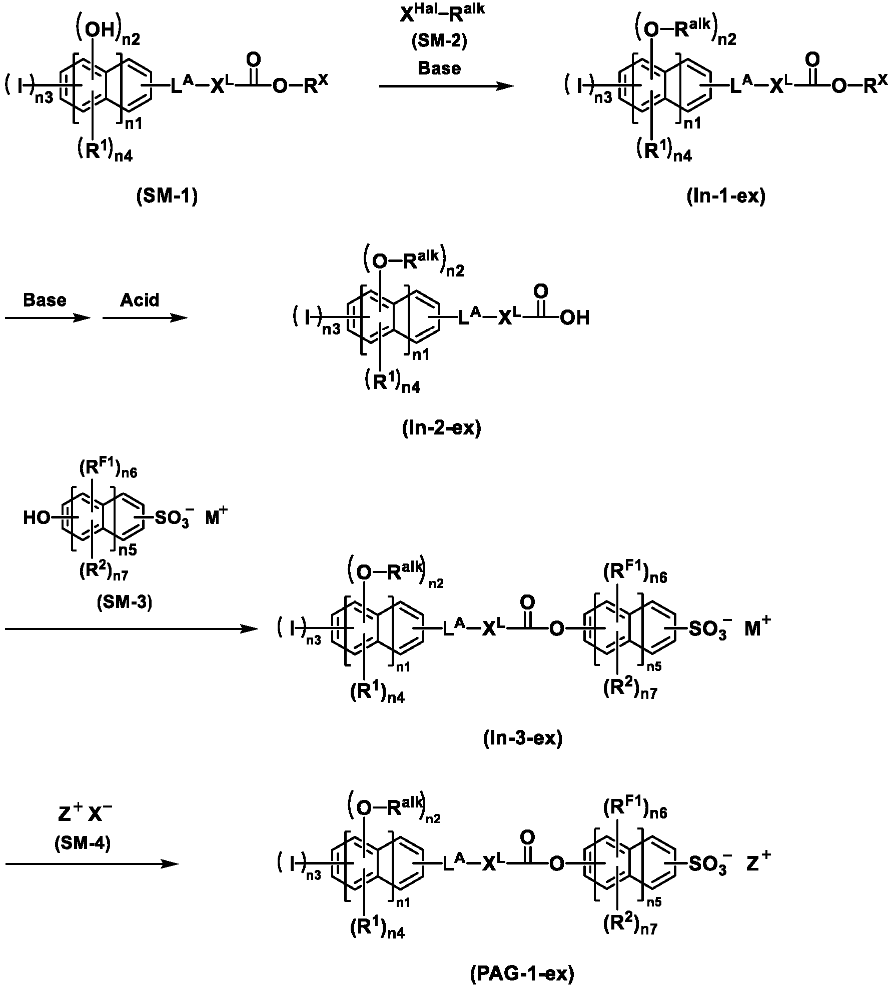 Herein n1 to n7, R^{alk}, R¹, R², R^{F1}, L^{A}, X^{L}, and Z⁺ are as defined above. X^{Hal} is chlorine, bromine or iodine, R^{X} is a group which forms a primary or secondary ester with the adjacent -CO₂-, M⁺ is a counter cation, and X⁻ is a counter anion.

The first step is to react phenol compound (SM-1), which is commercially available or can be synthesized by a well-known method, with haloalkyl compound (SM-2) to form Intermediate (In-1-ex). The reaction may be performed by a well-known organic synthesis method. Specifically, the reaction is performed by dissolving reactant SM-1 in an aprotic polar solvent, adding a base thereto, and adding dropwise reactant SM-2. Examples of the aprotic polar solvent include acetone, acetonitrile, dimethyl sulfoxide (DMSO), and N,N-dimethylformamide (DMF). Examples of the base include hydroxides such as sodium hydroxide, potassium hydroxide, and tetramethylammonium hydroxide, and carbonate salts such as potassium carbonate and sodium hydrogencarbonate. The bases may be used alone or in admixture. The reaction may be performed while heating if necessary. The reaction temperature may range from room temperature to nearly the boiling point of the solvent although an elevated temperature is preferably selected to facilitate the reaction. The reaction may be accelerated by adding an alkali metal iodide as a catalyst. Sodium iodide and potassium iodide are exemplary. While it is desirable in view of yield that the reaction time is determined by monitoring the progress of reaction by silica gel thin layer chromatography (TLC) to drive the reaction to completion, the reaction time is typically about 4 to 12 hours. Thereafter, water is added to quench the reaction. The desired compound is extracted from the reaction mixture and subj ected to ordinary aqueous workup, obtaining Intermediate In-1-ex. Intermediate In-1-ex may be purified by conventional means such as chromatography or recrystallization, if necessary.

The second step is hydrolysis reaction of Intermediate In-1-ex with a base, specifically alkaline hydrolysis of primary or secondary ester bond: -CO₂-R^{x} in Intermediate In-1-ex, to form Intermediate In-2-ex. The reaction may be performed by a well-known organic synthesis method. Specifically, the hydrolysis reaction is performed by suspending Intermediate In-1-ex in water or an ether solvent such as tetrahydrofuran (THF) and adding a base. Preferred examples of the base used include alkali metal hydroxides such as sodium hydroxide and potassium hydroxide. The reaction temperature may range from room temperature to nearly the boiling point of the solvent although an elevated temperature is preferably selected to facilitate the reaction. While it is desirable in view of yield that the reaction time is determined by monitoring the progress of reaction by TLC to drive the reaction to completion, the reaction time is typically about 4 to 12 hours. Thereafter, dilute hydrochloric acid or the like is added to quench the reaction and to render the pH acidic. The desired compound is extracted from the reaction mixture and subjected to ordinary aqueous workup, obtaining Intermediate In-2-ex. Intermediate In-2-ex may be purified by conventional means such as chromatography or recrystallization, if necessary.

The third step is to react Intermediate In-2-ex with reactant SM-3 to form Intermediate In-3-ex. Any condensing agents may be used when an ester bond is formed directly from the carboxy group in Intermediate In-2-ex and the hydroxy group in reactant SM-3. Suitable condensing agents include N,N'-dicyclohexylcarbodiimide, N,N'-diisopropylcarbodiimide, 1-[3-(dimethylamino)propyl]-3-ethylcarbodiimide, and 1-ethyl-3-(3-dimethylaminopropyl)carbodiimide hydrochloride. From the aspect of easy removal of a urea compound formed as the by-product after the reaction, it is preferred to use 1-ethyl-3-(3-dimethylaminopropyl)carbodiimide hydrochloride. The reaction is performed by dissolving Intermediate In-2-ex and reactant SM-3 in a halide solvent such as methylene chloride, and adding a condensing agent thereto. The reaction rate may be accelerated by adding 4-dimethylaminopyridine (DMAP) as a catalyst. The reaction time is determined as appropriate by monitoring the reaction process by TLC because it is desirable from the yield aspect to drive the reaction to completion. The reaction time is usually about 12 to 24 hours. After the reaction is stopped, the by-product, urea compound is removed by filtration or water washing if necessary. The reaction solution is subjected to ordinary aqueous work-up, obtaining Intermediate In-3-ex. Alternatively, Intermediate In-3-ex may be obtained by treating Intermediate In-2-ex with oxalyl chloride or thionyl chloride to form an acid chloride and reacting the acid chloride with reactant SM-3. In the reaction, a base is preferably used, for example, triethylamine, diisopropylethylamine, pyridine or 2,6-lutidine. The reaction rate may be accelerated by adding DMAP as a catalyst. The resulting Intermediate In-3-ex may be purified by a standard technique such as chromatography or recrystallization if necessary.

The fourth step is a salt exchange between Intermediate In-3-ex and onium salt: Z⁺X⁻ to form the desired onium salt PAG-1-ex. It is preferred that X⁻ be a chloride ion, bromide ion, iodide ion or methylsulfate anion, which allows the exchange reaction to run in a quantitative manner. It is preferred from the yield aspect to monitor the progress of reaction by TLC. By ordinary aqueous work-up, the onium salt PAG-1-ex is recovered from the reaction mixture. The onium salt may be purified by a standard technique such as chromatography or recrystallization if necessary.

In the above reaction scheme, the ion exchange in the fourth step may be readily performed by any well-known technique, for example, with reference to JP-A 2007-145797.

The above-mentioned preparation method is merely exemplary and the method of preparing the inventive onium salt is not limited thereto.

The inventive onium salt is structurally characterized by containing an alkyl or fluoroalkyl group on an aromatic ring in its anion. It is believed that the onium salt segregates in a surface layer of a resist film because the alkyl or fluoroalkyl group acts as a hydrophobic group. Particularly in alkaline development of a positive resist composition by EB lithography, the back scattering phenomenon between EB and the substrate causes the pattern profile to be inversely tapered to incur pattern collapse after development. The onium salt allows deprotection reaction on the base polymer to take place even in the upper layer of the resist film or pattern and is thus successful in correcting the pattern profile so as to be rectangular. Also, the onium salt preferably contains iodine in its anion. The iodine on the aromatic ring serves to enhance the sensitivity in the EB lithography and has an adequate dissolution inhibitory effect. By virtue of the synergy of these effects, the resist composition comprising the inventive onium salt forms a resist pattern having improved properties including satisfactory resolution, high contrast, reduced LER, and improved CDU. By virtue of adequate dissolution inhibition, a pattern of rectangular profile is obtained. Since the onium salt is fully lipophilic, it is easy to prepare and handle.

The onium salt is advantageously used as a PAG.

### Chemically amplified positive resist composition

### (A) Photoacid generator

Another embodiment of the invention is a chemically amplified positive resist composition essentially comprising (A) the onium salt having formula (A) as a photoacid generator.

In the resist composition, the PAG (A) is preferably present in an amount of 0.1 to 20 parts by weight, more preferably 1 to 10 parts by weight per 80 parts by weight of a base polymer (B) to be described later. The range of the PAG ensures that it generates an acid in a necessary amount for the deprotection of acid labile groups (to eventually form a pattern of satisfactory profile) and provides for storage stability. The PAG may be used alone or in admixture.

### (B) Base polymer

In a preferred embodiment, the resist composition further comprises (B) a base polymer containing a polymer which is decomposed under the action of acid to increase its solubility in alkaline developer.

The polymer preferably contains repeat units having the formula (B1), which are also referred to as repeat units B1.

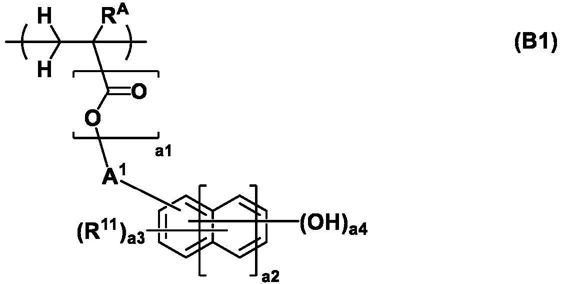

In formula (B 1), a1 is 0 or 1. The subscript a2 is an integer of 0 to 2. The relevant structure is a benzene ring in case of a2=0, a naphthalene ring in case of a2=1, and an anthracene ring in case of a2=2. The subscript a3 is an integer satisfying 0 ≤ a3 ≤ 5+2(a2)-a4, and a4 is an integer of 1 to 3. When a2 is 0, preferably a3 is an integer of 0 to 3 and a4 is an integer of 1 to 3. When a2 is 1 or 2, preferably a3 is an integer of 0 to 4 and a4 is an integer of 1 to 3.

In formula (B 1), R^{A} is hydrogen, fluorine, methyl or trifluoromethyl.

In formula (B 1), R¹¹ is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group. The saturated hydrocarbyl group and saturated hydrocarbyl moiety in the saturated hydrocarbylcarbonyloxy and saturated hydrocarbyloxy groups may be straight, branched or cyclic. Examples thereof include alkyl groups such as methyl, ethyl, n-propyl, isopropyl, butyl, pentyl, hexyl, and structural isomers thereof; cycloalkyl groups such as cyclopropyl, cyclobutyl, cyclopentyl and cyclohexyl; and combinations thereof. A carbon count within the upper limit ensures a satisfactory solubility in alkaline developer. A plurality of R¹¹ may be identical or different when a3 is 2 or more.

In formula (B 1), A¹ is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of a1=1 in formula (B 1), the ether bond may be incorporated at any position excluding the position between the α- and β-carbons relative to the ester oxygen. In case of a1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units B 1 wherein a1=0 and A¹ is a single bond (meaning that the aromatic ring is directly bonded to the backbone of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Exemplary units are shown below, but not limited thereto.

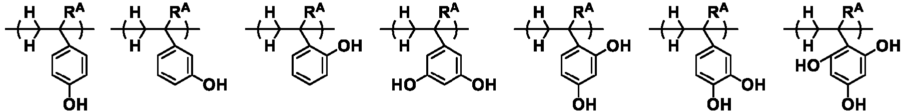

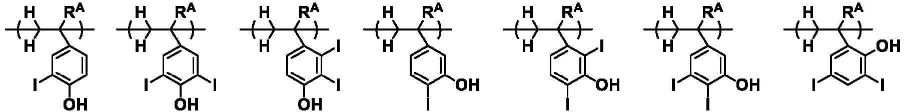

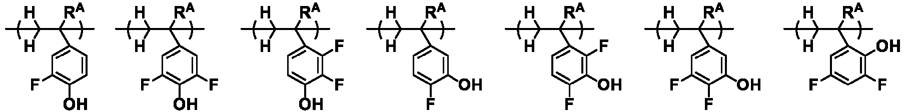

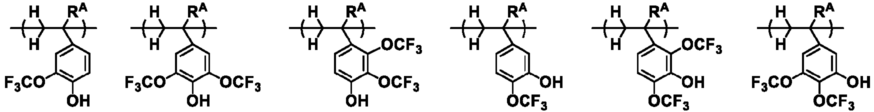

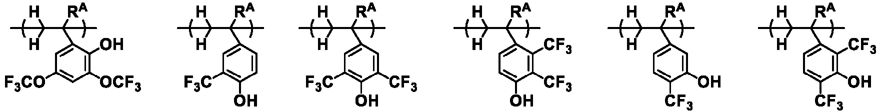

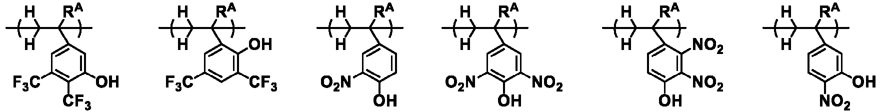

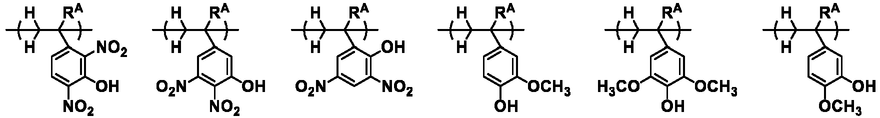

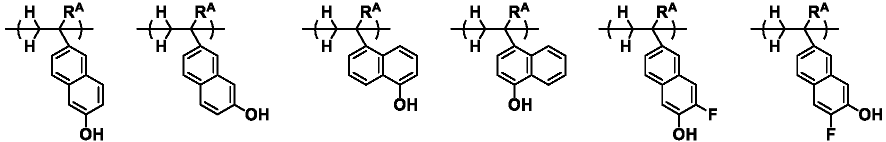

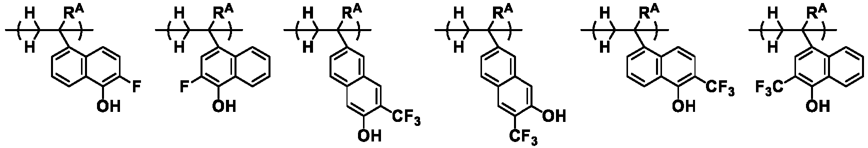

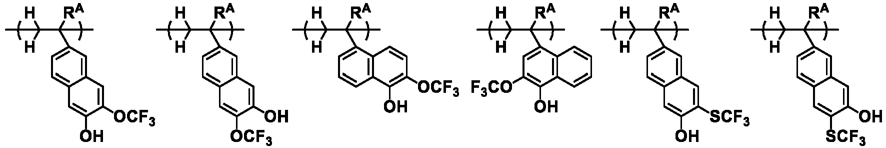

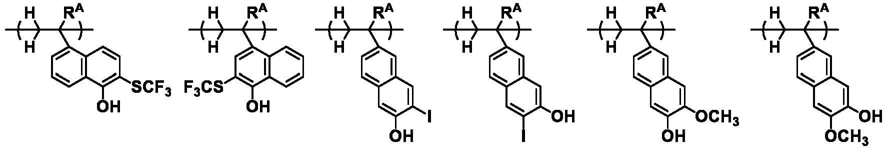

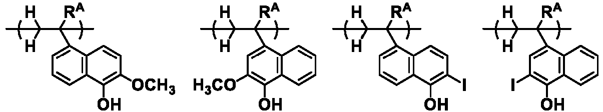

Preferred examples of the repeat units B 1 wherein a 1 = 1, that is, having a linker: -C(=O)-O-A¹- are shown below, but not limited thereto. Herein R^{A} is as defined above.

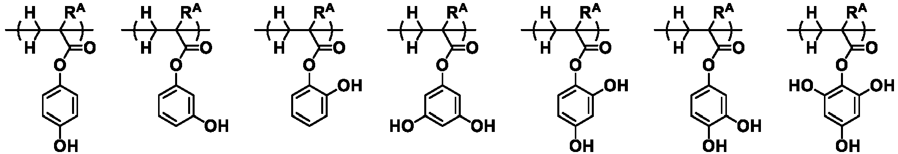

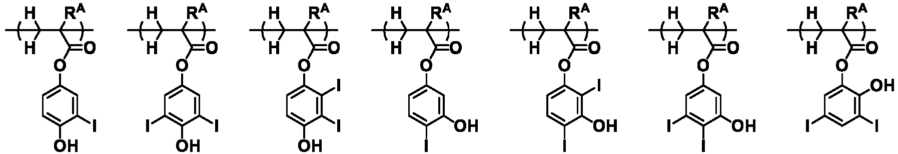

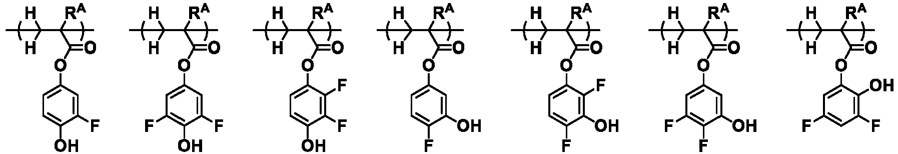

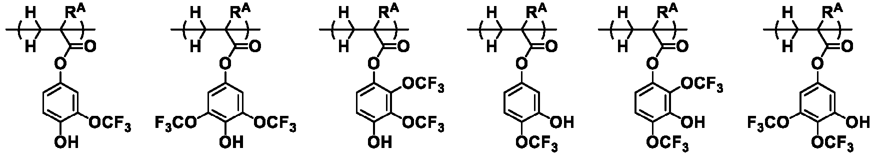

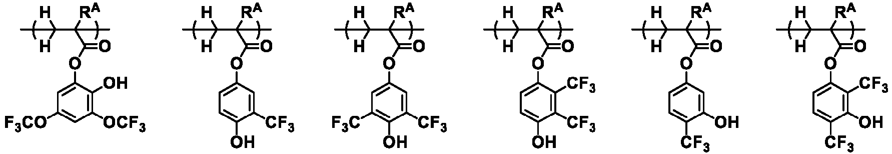

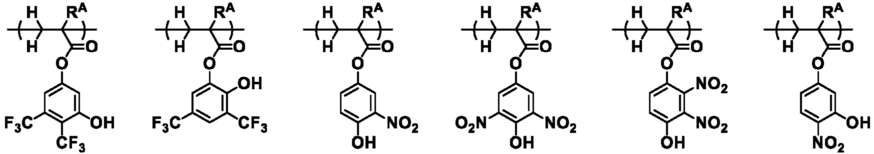

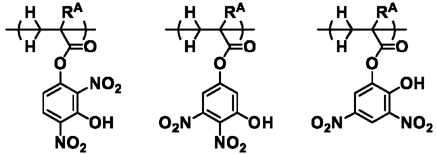

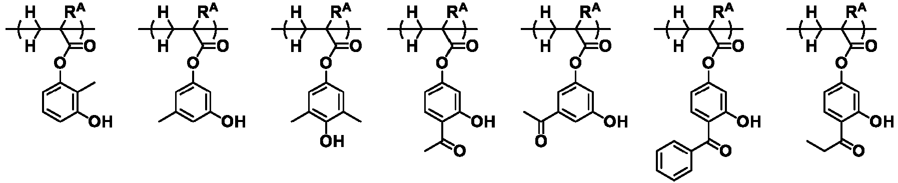

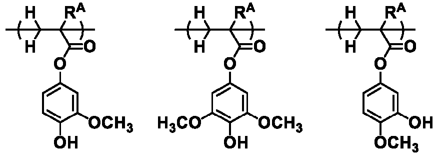

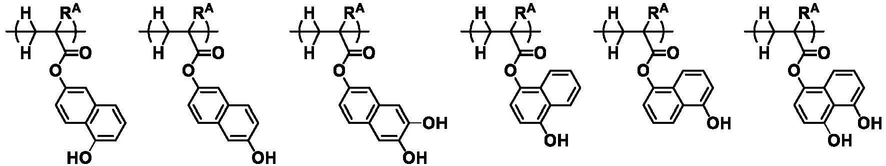

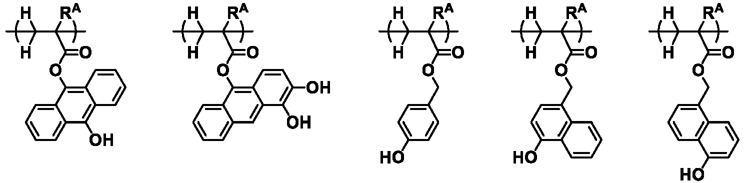

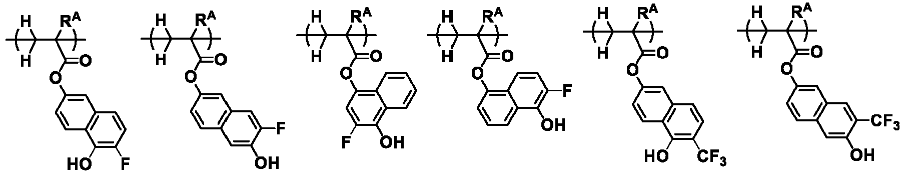

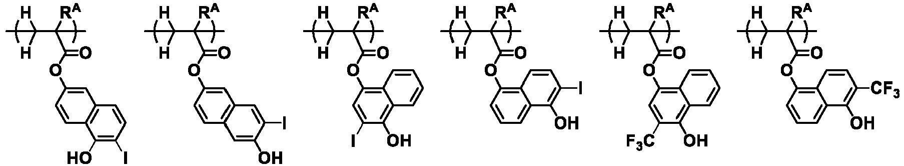

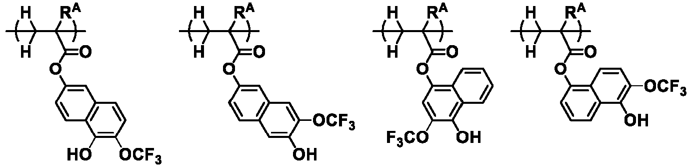

The content of repeat units B 1 is preferably 15 to 90 mol%, more preferably 15 to 80 mol% of the overall units of the polymer. When the polymer further comprises repeat units of at least one type selected from repeat units having formula (B3) and repeat units having formula (B4), which provide the polymer with higher etch resistance, the repeat units containing a phenolic hydroxy group as a substituent, the total content of repeat units B1 and repeat units B3 and/or B4 should preferably fall in the range. The repeat units B 1 may be of one type or a combination of plural types.

In a preferred embodiment, the polymer further contains repeat units B2 having an acidic functional group protected with an acid labile group (i.e., repeat units protected with an acid labile group and adapted to turn alkali soluble under the action of acid) in order that the positive resist composition in an exposed region turn soluble in alkaline developer.

Typical of the repeat unit B2 is a unit having the formula (B2-1), also referred to as repeat unit B2-1.

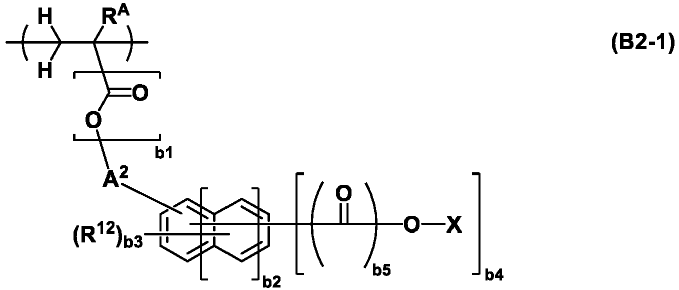

In formula (B2-1), b1 is 0 or 1. The subscript b2 is an integer of 0 to 2. The structure represents a benzene skeleton when b2=0, a naphthalene skeleton when b2=1, and an anthracene skeleton when b2=2. The subscript b3 is an integer meeting 0 ≤ b3 ≤ 5+2(b2)-b4. The subscript b4 is an integer of 1 to 3, and b5 is 0 or 1. When b2=0, preferably b3 is an integer of 0 to 3 and b4 is an integer of 1 to 3. When b2=1 or 2, preferably b3 is an integer of 0 to 4 and b4 is an integer of 1 to 3.

In formula (B2-1), R^{A} is hydrogen, fluorine, methyl or trifluoromethyl.

In formula (B2-1), R¹² is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R¹² may be identical or different when b3 is 2 or more.

In formula (B2-1), A² is a single bond or a C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (B2-1), the ether bond may be incorporated at any position excluding the position between the α-carbon and β-carbon relative to the ester oxygen. In case of b1=0, the atom that bonds with the backbone becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

In formula (B2-1), X is an acid labile group when b4=1, and hydrogen or an acid labile group, at least one X being an acid labile group, when b4=2 or 3. That is, repeat units B2-1 have phenolic hydroxy groups bonded to an aromatic ring, at least one of which is protected with an acid labile group, or repeat units B2-1 have a carboxy group bonded to an aromatic ring, which is protected with an acid labile group. The acid labile group used herein is not particularly limited as long as it is commonly used in a number of well-known chemically amplified resist compositions and eliminated under the action of acid to release an acidic group.

Typical of the acid labile group is a tertiary saturated hydrocarbyl group. The tertiary saturated hydrocarbyl group is preferably of 4 to 18 carbon atoms because a monomer for use in polymerization is recoverable by distillation.

The saturated hydrocarbyl group bonded to the tertiary carbon atom in the tertiary saturated hydrocarbyl group is preferably of 1 to 15 carbon atoms. The C₁-C₁₅ saturated hydrocarbyl group may be straight, branched or cyclic and contain an oxygen-containing functional group such as an ether bond or carbonyl group in its carbon-carbon bond. The saturated hydrocarbyl groups bonded to the tertiary carbon atom may bond together to form a ring with the tertiary carbon atom to which they are attached.

Examples of the alkyl substituent include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, tetracyclo[4.4.0.1^{2,5}.1^{7,10}]dodecyl, and 3-oxo-1-cyclohexyl.

Examples of the tertiary saturated hydrocarbyl group include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyll)ethyl, 1-methyl-1-(tetrahydrofuran-2-yl)ethyl, 1-methyl-1-(7-oxanorbornan-2-yl)ethyl, 1-methylcyclopentyl, 1-ethylcyclopentyl, 1-propylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, 3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl, and 5-hydroxy-2-ethyl-2-adamantyl.

A group having the following formula (B2-1-1) is also suitable as the acid labile group. The group having formula (B2-1-1) is often used as the acid labile group. It is a good choice of the acid labile group that ensures to form a pattern having a relatively rectangular pattern-substrate interface in a consistent manner. An acetal structure is formed when X is a group having formula (B2-1-1).

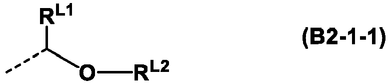

In formula (B2-1-1), R^{L1} is hydrogen or a C₁-C₁₀ saturated hydrocarbyl group. The saturated hydrocarbyl group may be straight, branched or cyclic.

A choice of R^{L1} may depend on the designed sensitivity of labile group to acid. For example, hydrogen or a group in which the carbon atom bonded to acetal carbon is tertiary is selected when the acid labile group is designed to ensure relatively high stability and to be decomposed with strong acid. Examples of R^{L1} bonded to acetal carbon via tertiary carbon include tert-butyl, tert-pentyl, and 1-adamantyl, but are not limited thereto. A straight alkyl group is selected when the acid labile group is designed to have relatively high reactivity and high sensitivity to pH changes. Although the choice varies with a particular combination of acid generator and quencher in the resist composition, R^{L1} is preferably a group in which the carbon in bond with acetal carbon is secondary, when R^{L2} is a relatively large alkyl group substituted at the end and the acid labile group is designed to undergo a substantial change of solubility by decomposition. Examples of R^{L1} bonded to acetal carbon via secondary carbon include isopropyl, sec-butyl, cyclopentyl, and cyclohexyl, but are not limited thereto.

In formula (B2-1-1), R^{L2} is a C₁-C₃₀ hydrocarbyl group. The hydrocarbyl group may be saturated or unsaturated and straight, branched or cyclic. Some constituent -CH₂-in the hydrocarbyl group may be replaced by a heteroatom such as oxygen or sulfur so that the group may contain an ether bond or sulfide bond. Illustrative are C₁-C₃₀ saturated hydrocarbyl groups and C₆-C₃₀ aryl groups. R^{L2} is preferably a C₁-C₆ hydrocarbyl group for acquiring a higher resolution in forming small-size patterns. When R^{L2} is a C₁-C₆ hydrocarbyl group, the alcohol created after a progress of acid-aided deprotection reaction is water soluble. Then, when a positive pattern is formed using an alkaline developer, the alcohol is dissolved in the developer so that defects remaining in the exposed region are minimized.

Preferred examples of the group having formula (B2-1-1) are given below, but not limited thereto. Herein R^{L1} is as defined above.

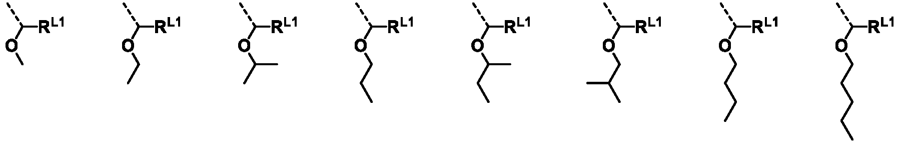

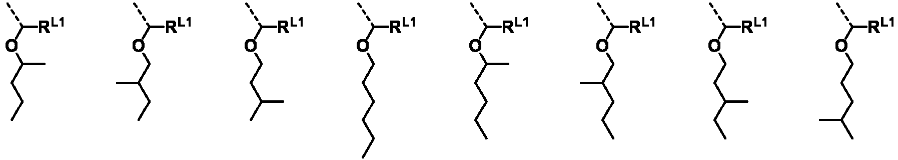

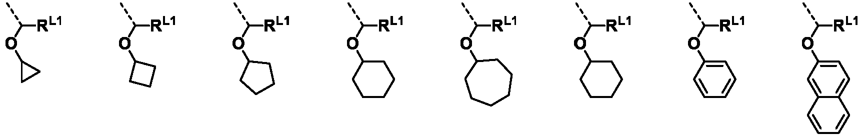

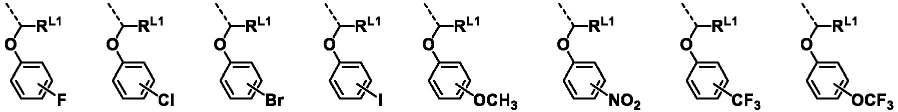

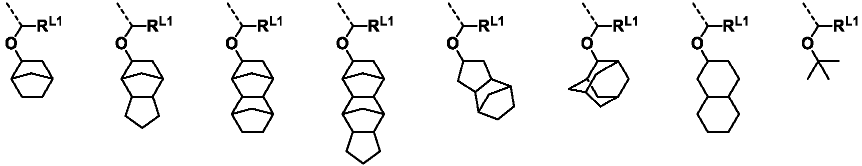

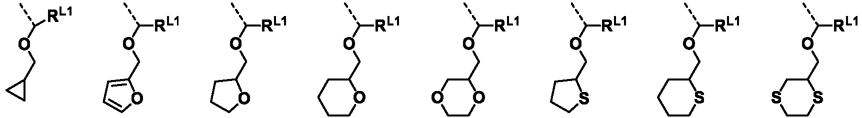

Another acid labile group which can be used herein is a phenolic hydroxy group whose hydrogen is substituted by -CH₂COO-(tertiary saturated hydrocarbyl group). The tertiary saturated hydrocarbyl group may be the same as the foregoing tertiary saturated hydrocarbyl group used for the protection of a phenolic hydroxy group.

Another example of repeat unit B2 is a repeat unit having the following formula (B2-2), referred to as repeat unit B2-2. The repeat unit B2-2 which enables to increase the dissolution rate in the exposed region is a useful choice of the acid labile group-containing unit which affords satisfactory performance against line width variations during develop loading.

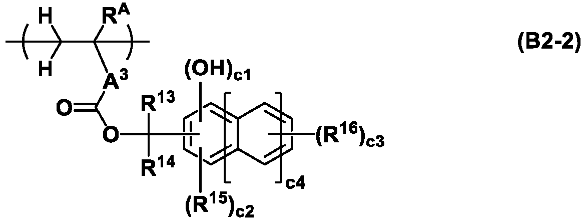

In formula (B2-2), c1 is an integer of 0 to 2, c2 is an integer of 0 to 2, c3 is an integer of 0 to 5, and c4 is an integer of 0 to 2.

In formula (B2-2), R^{A} is hydrogen, fluorine, methyl or trifluoromethyl.

In formula (B2-2), R¹³ and R¹⁴ are each independently a C₁-C₁₀ hydrocarbyl group which may contain a heteroatom. R¹³ and R¹⁴ may bond together to form a ring with the carbon atom to which they are attached.

In formula (B2-2), R¹⁵ is each independently fluorine, C₁-C₅ fluorinated alkyl group or C₁-C₅ fluorinated alkoxy group.

In formula (B2-2), R¹⁶ is each independently a C₁-C₁₀ hydrocarbyl group which may contain a heteroatom.

In formula (B2-2), A³ is a single bond, phenylene group, naphthylene group, or *-C(=O)-O-A³¹-. A³¹ is a C₁-C₂₀ aliphatic hydrocarbylene group which may contain hydroxy, ether bond, ester bond or lactone ring, or phenylene or naphthylene group, and * is a point of attachment to the carbon atom in the backbone.

Preferred examples of the repeat unit B2-2 are shown below, but not limited thereto. Herein R^{A} is as defined above.

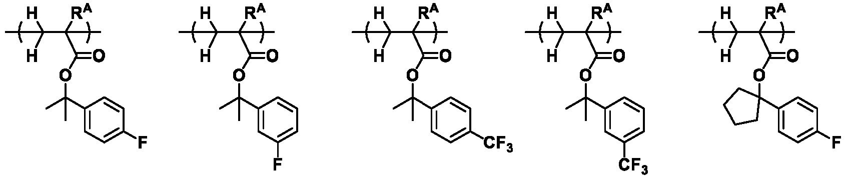

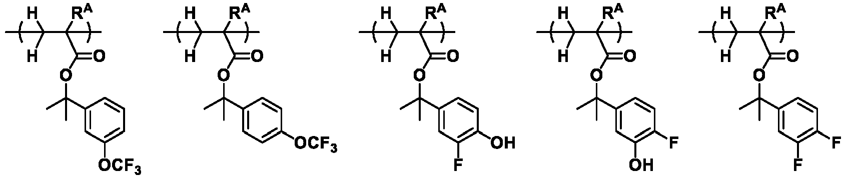

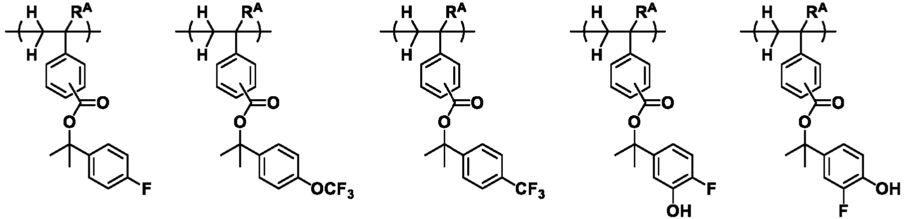

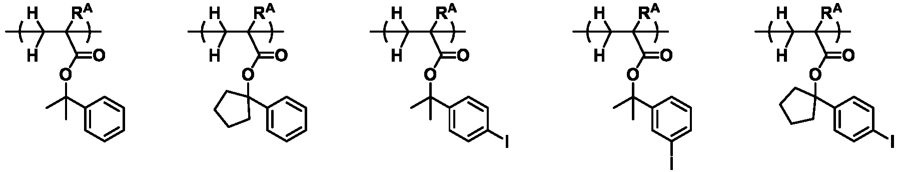

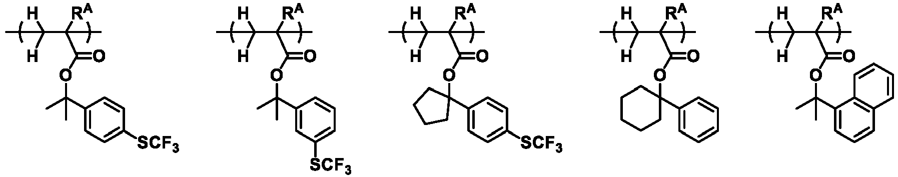

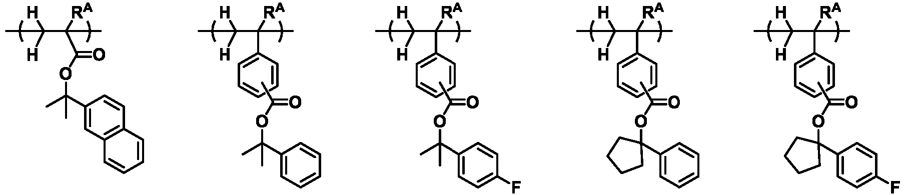

The content of repeat units B2 is preferably 5 to 95 mol%, more preferably 20 to 80 mol% based on the overall repeat units of the polymer. Each of repeat units B2 may be of one type or a mixture of two or more types.

In a preferred embodiment, the polymer further comprises repeat units of at least one type selected from units having the formulae (B3), (B4) and (B5). These repeat units are simply referred to as repeat units B3, B4 and B5, respectively.

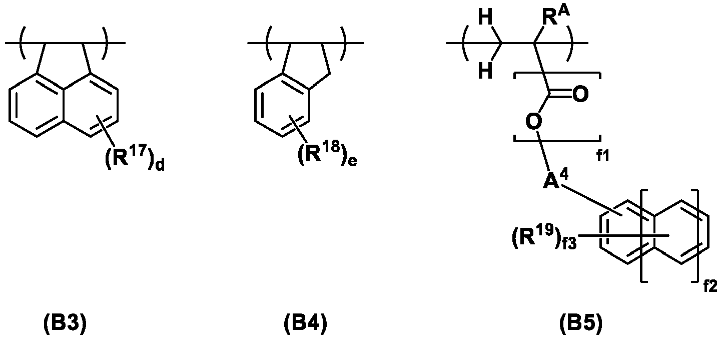

In formulae (B3) and (B4), d is an integer of 0 to 6 and e is an integer of 0 to 4.

In formulae (B3) and (B4), R¹⁷ and R¹⁸ are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When d is 2 or more, a plurality of R¹⁷ may be identical or different. When e is 2 or more, a plurality of R¹⁸ may be identical or different.

In formula (B5), f1 is 0 or 1. The subscript f2 is an integer of 0 to 2, and the corresponding structure represents a benzene skeleton when f2=0, a naphthalene skeleton when f2=1, and an anthracene skeleton when f2=2. The subscript f3 is an integer of 0 to 5. In case of f2=0, preferably f3 is an integer of 0 to 3. In case of f2=1 or 2, preferably f3 is an integer of 0 to 4.

In formula (B5), R^{A} is hydrogen, fluorine, methyl or trifluoromethyl.

In formula (B5), R¹⁹ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen atom, nitro group, or cyano group. In case of f2=1 or 2, R¹⁹ may also be hydroxy. The saturated hydrocarbyl group, saturated hydrocarbyloxy group, saturated hydrocarbylcarbonyloxy group, saturated hydrocarbyloxyhydrocarbyl group, and saturated hydrocarbylthiohydrocarbyl group may be straight, branched or cyclic. When f3 is 2 or more, a plurality of R¹⁹ may be identical or different.

In formula (B5), A⁴ is a single bond or a C₁-C₁₀ saturated hydrocarbylene group in which some constituent -CH₂- may be replaced by -O-. The saturated hydrocarbylene group may be straight, branched or cyclic. Examples thereof are as exemplified above for A¹ in formula (B 1).

When repeat units of at least one type selected from repeat units B3 to B5 are incorporated, better performance is obtained because not only the aromatic ring possesses etch resistance, but the cyclic structure incorporated into the backbone also exerts the effect of improving etch resistance and resistance to EB irradiation during pattern inspection step.

The content of repeat units B3 to B5 is preferably at least 5 mol% based on the overall repeat units of the polymer for obtaining the effect of improving etch resistance. Also, the content of repeat units B3 to B5 is preferably up to 25 mol%, more preferably up to 20 mol% based on the overall repeat units of the polymer. When the relevant units are free of functional groups or have a functional group other than hydroxy, their content of up to 25 mol% is preferred because the risk of forming development defects is eliminated. Each of the repeat units B3 to B5 may be of one type or a combination of plural types.

It is preferred that the polymer comprise repeat units B 1, repeat units B2, and repeat units of at least one type selected from repeat units B3 to B5, because both etch resistance and high resolution are achievable. The total content of these repeat units is preferably at least 60 mol%, more preferably at least 70 mol%, even more preferably at least 80 mol%, most preferably at least 90 mol% based on the overall repeat units of the polymer.

In another preferred embodiment, the polymer further comprises repeat units of at least one type selected from repeat units having the formula (B6), repeat units having the formula (B7), repeat units having the formula (B8), and repeat units having the formula (B9), shown below. Notably these repeat units are also referred to as repeat units B6 to B9.

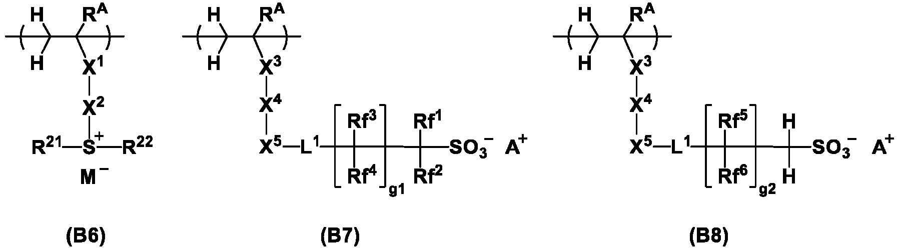

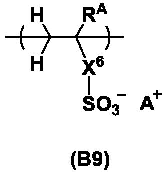

In formulae (B6) to (B9), R^{A} is each independently hydrogen, fluorine, methyl or trifluoromethyl. X¹ is a single bond or phenylene group. X² is *¹-C(=O)-O-X²¹-, *¹-C(=O)-NH-X²¹-, or *¹-O-X²¹-. X²¹ is a C₁-C₆ aliphatic hydrocarbylene group, phenylene group, or divalent group obtained by combining the foregoing, which may contain a carbonyl moiety, ester bond, ether bond or hydroxy moiety, and *¹ designates a point of attachment to X¹. X³ is each independently a single bond, phenylene group, naphthylene group or *²-C(=O)-O-X³¹-. X³¹ is a C₁-C₁₀ aliphatic hydrocarbylene group, phenylene group, or naphthylene group, the aliphatic hydrocarbylene group may contain a hydroxy moiety, ether bond, ester bond or lactone ring, and *² designates a point of attachment to the carbon atom in the backbone. X⁴ is each independently a single bond, *³-X⁴¹-C(=O)-O-, *³-C(=O)-NH-X⁴¹- or *³-O-X⁴¹-. X⁴¹ is a C₁-C₂₀ hydrocarbylene group which may contain a heteroatom, and *³ designates a point of attachment to X³. X⁵ is each independently a single bond, *⁴-X⁵¹-C(=O)-O-, *⁴-C(=O)-NH-X⁵¹- or *⁴-O-X⁵¹-. X⁵¹ is a C₁-C₂₀ hydrocarbylene group which may contain a heteroatom, and *⁴ designates a point of attachment to X⁴. X⁶ is a single bond, methylene, ethylene, phenylene, fluorinated phenylene, trifluoromethyl-substituted phenylene, *²-C(=O)-O-X⁶¹-, *²-C(=O)-N(H)-X⁶¹-, or *²-O-X⁶¹-. X⁶¹ is a C₁-C₆ aliphatic hydrocarbylene group, phenylene group, fluorinated phenylene group, or trifluoromethyl-substituted phenylene group, which may contain a carbonyl moiety, ester bond, ether bond or hydroxy moiety, and *² designates a point of attachment to the carbon atom in the backbone.

The aliphatic hydrocarbylene group represented by X²¹, X³¹ and X⁶¹ may be straight, branched or cyclic. Examples thereof include alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, and hexane-1,6-diyl; cycloalkanediyl groups such as cyclopropanediyl, cyclobutanediyl, cyclopentanediyl, and cyclohexanediyl; and combinations thereof.

The hydrocarbylene group represented by X⁴¹ and X⁵¹ may be saturated or unsaturated and straight, branched or cyclic. Examples thereof are shown below, but not limited thereto.

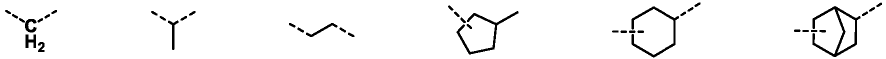

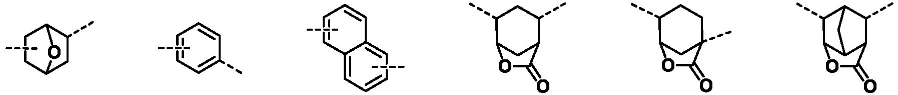

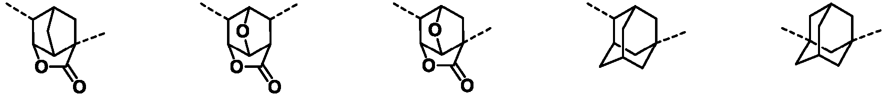

In formula (B6), R²¹ and R²² are each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, and tert-butyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₂₀ alkenyl groups such as vinyl, allyl, propenyl, butenyl, and hexenyl; C₃-C₂₀ cyclic unsaturated hydrocarbyl groups such as cyclohexenyl; C₆-C₂₀ aryl groups such as phenyl, naphthyl and thienyl; C₇-C₂₀ aralkyl groups such as benzyl, 1-phenylethyl and 2-phenylethyl, and combinations thereof. Inter alia, aryl groups are preferred. In the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-), or haloalkyl moiety.

R²¹ and R²² may bond together to form a ring with the sulfur atom to which they are attached. Examples of the ring are as exemplified above for the ring that R^{ct1} and R^{ct2} in formula (cation-1) form with the sulfur atom to which they are attached.

Examples of the cation in repeat units B6 are shown below, but not limited thereto. Herein R^{A} is as defined above.

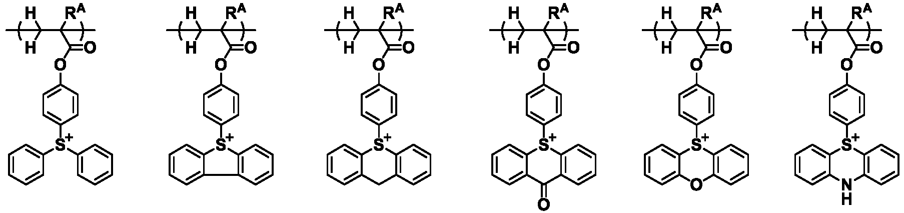

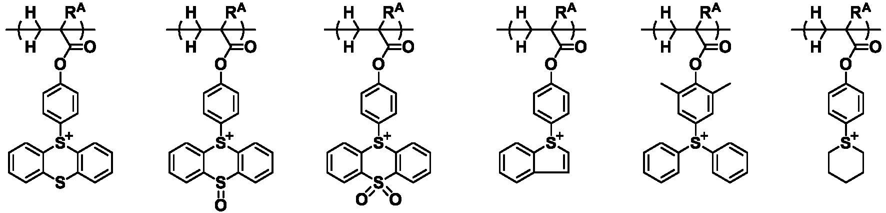

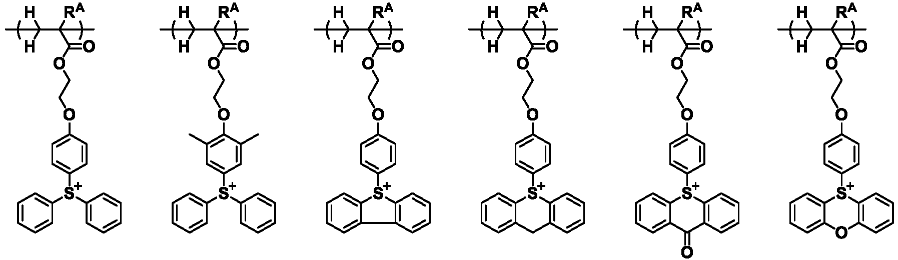

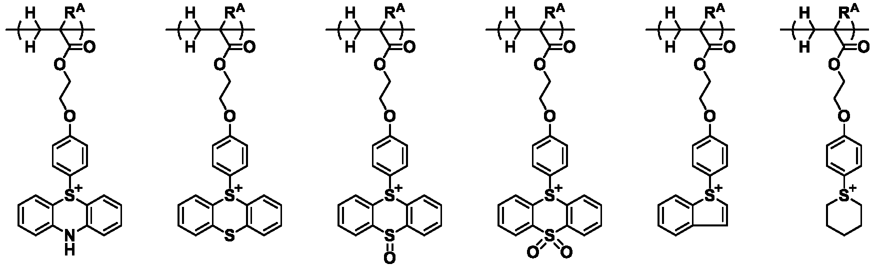

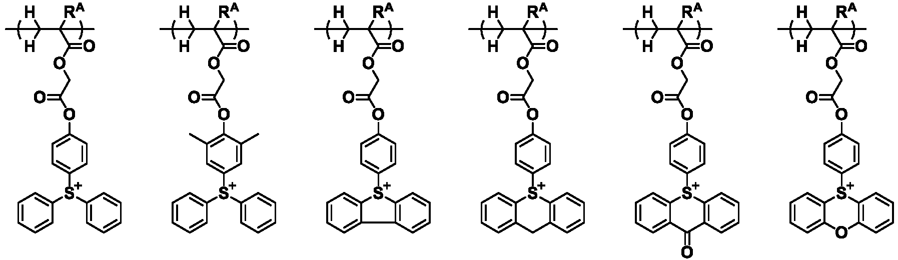

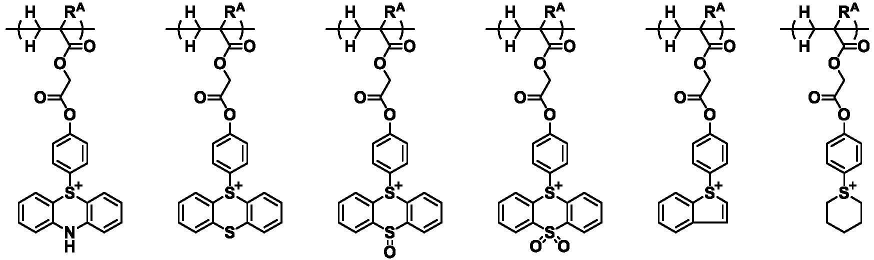

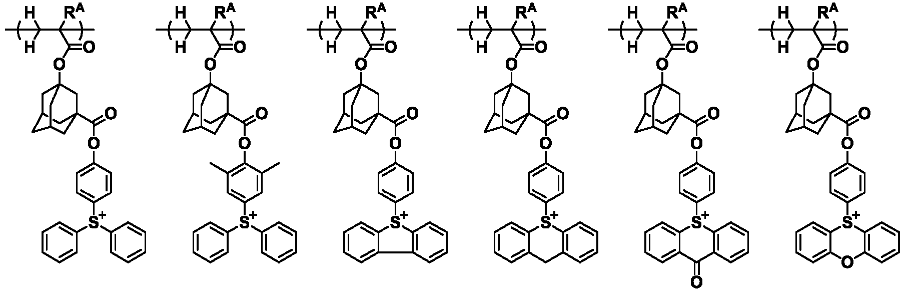

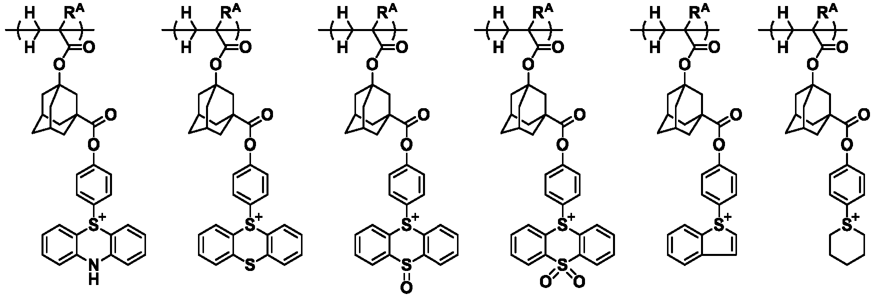

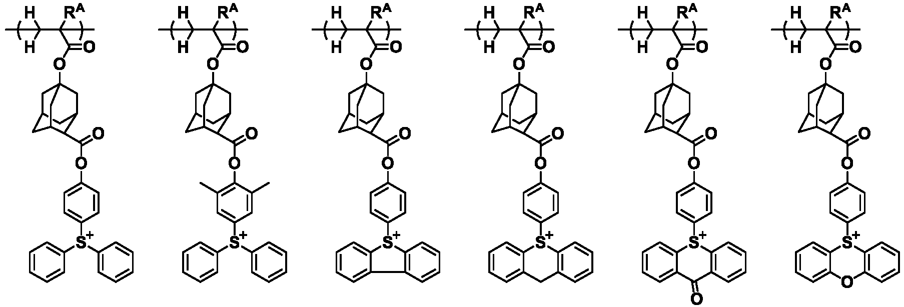

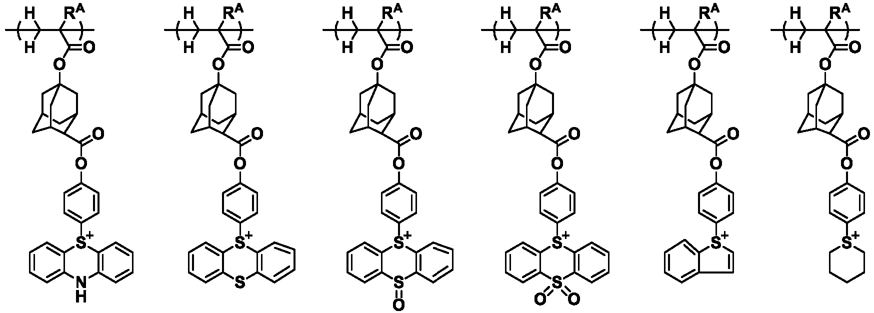

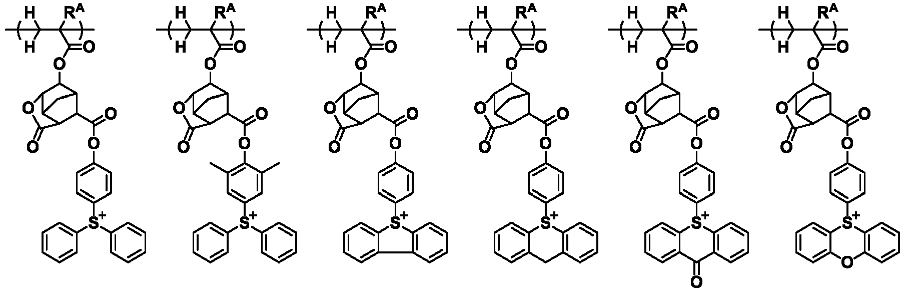

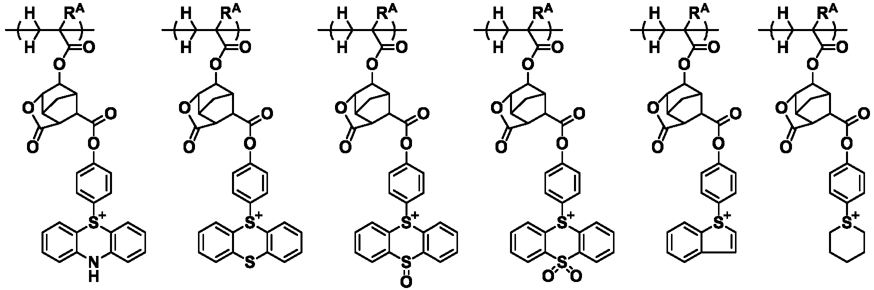

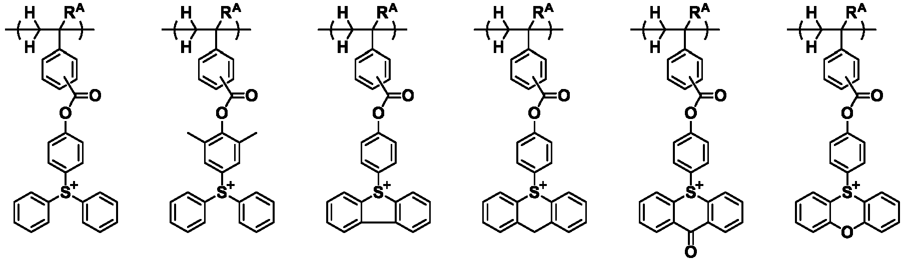

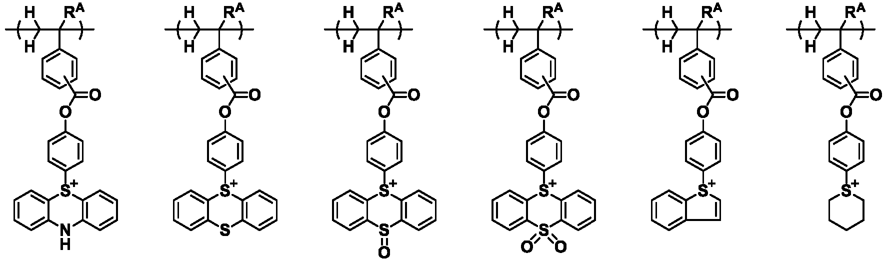

In formula (B6), M⁻ is a non-nucleophilic counter ion. Halide ions, sulfonate anions, imide anions, and methide anions are preferred. Examples of the non-nucleophilic counter ion include halide ions such as chloride and bromide ions; sulfonate anions, specifically fluoroalkylsulfonate ions such as triflate, 1,1,1-trifluoroethanesulfonate, and nonafluorobutanesulfonate, arylsulfonate ions such as tosylate, benzenesulfonate, 4-fluorobenzenesulfonate, and 1,2,3,4,5-pentafluorobenzenesulfonate, alkylsulfonate ions such as mesylate and butanesulfonate; imide ions such as bis(trifluoromethylsulfonyl)imide, bis(perfluoroethylulfonyl)imide and bis(perfluorobutylsulfonyl)imide; and methide ions such as tris(trifluoromethylsulfonyl)methide and tris(perfluoroethylsulfonyl)methide.

Anions having the following formulae (B6-1) to (B6-4) are also useful as the non-nucleophilic counter ion.

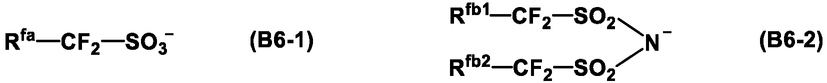

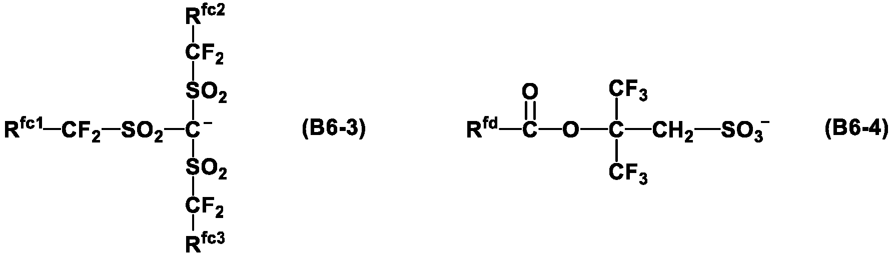

In formula (B6-1), R^{fa} is fluorine or a C₁-C₄₀ hydrocarbyl group which may contain a heteroatom. The hydrocarbyl group may be saturated or unsaturated and straight, branched or cyclic. Examples thereof are as will be exemplified below for the hydrocarbyl group R^{fa1} in formula (B6-1-1).

Of the anions of formula (B6-1), an anion having the formula (B6-1-1) is preferred.

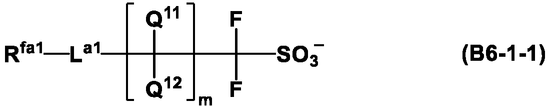

In formula (B6-1-1), Q¹¹ and Q¹² are each independently hydrogen, fluorine or a C₁-C₆ fluorinated saturated hydrocarbyl group. It is preferred for solvent solubility that at least one of Q¹¹ and Q¹² be trifluoromethyl. The subscript m is an integer of 0 to 4, preferably 1.

R^{fa1} is a C₁-C₃₅ hydrocarbyl group which may contain a heteroatom. As the heteroatom, oxygen, nitrogen, sulfur and halogen atoms are preferred, with oxygen being most preferred. Of the hydrocarbyl groups, those groups of 6 to 30 carbon atoms are preferred from the aspect of achieving a high resolution in forming patterns of small feature size. The hydrocarbyl group may be saturated or unsaturated and straight, branched or cyclic. Examples thereof include C₁-C₃₅ alkyl groups such as methyl, ethyl, n-propyl, isopropyl, n-butyl, isobutyl, sec-butyl, tert-butyl, pentyl, neopentyl, hexyl, heptyl, 2-ethylhexyl, nonyl, undecyl, tridecyl, pentadecyl, heptadecyl, and icosyl; C₃-C₃₅ cyclic saturated hydrocarbyl groups such as cyclopentyl, cyclohexyl, 1-adamantyl, 2-adamantyl, 1-adamantylmethyl, norbornyl, norbornylmethyl, tricyclodecyl, tetracyclododecyl, tetracyclododecylmethyl, and dicyclohexylmethyl; C₂-C₃₅ unsaturated aliphatic hydrocarbyl groups such as allyl and 3-cyclohexenyl; C₆-C₃₅ aryl groups such as phenyl, 1-naphthyl, 2-naphthyl and 9-fluorenyl; and C₇-C₃₅ aralkyl groups such as benzyl and diphenylmethyl, and combinations thereof.

In the foregoing hydrocarbyl groups, some or all hydrogen may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety. Examples of the heteroatom-containing hydrocarbyl group include tetrahydrofuryl, methoxymethyl, ethoxymethyl, methylthiomethyl, acetamidomethyl, trifluoroethyl, (2-methoxyethoxy)methyl, acetoxymethyl, 2-carboxy-1-cyclohexyl, 2-oxopropyl, 4-oxo-1-adamantyl, and 3-oxocyclohexyl.

In formula (B6-1-1), L^{a1} is a single bond, ether bond, ester bond, sulfonate ester bond, carbonate bond or carbamate bond. From the aspect of synthesis, an ether bond or ester bond is preferred, with the ester bond being more preferred.

Examples of the anion having formula (B6-1) are shown below, but not limited thereto. Herein Q¹¹ is as defined above.

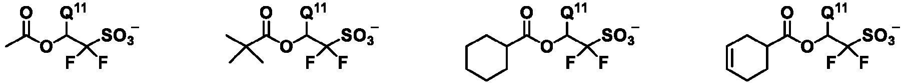

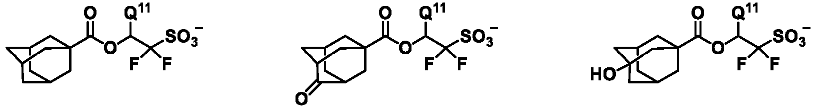

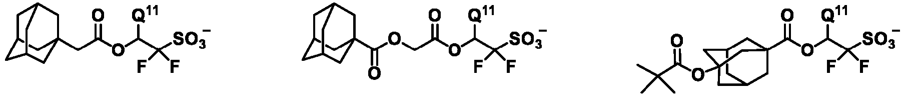

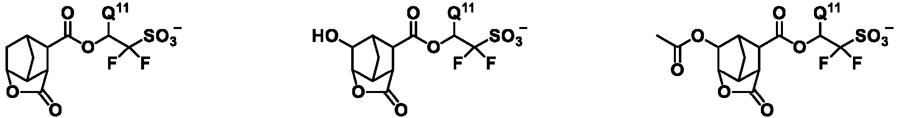

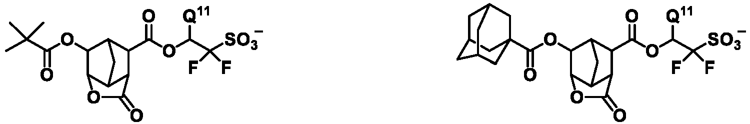

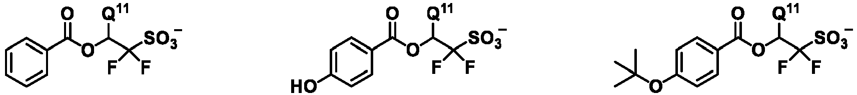

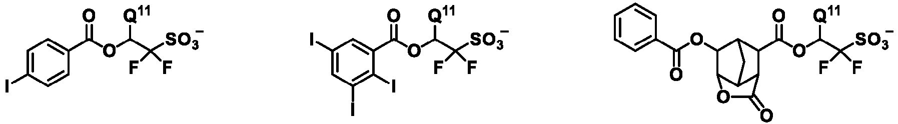

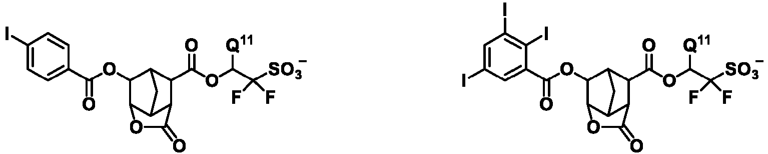

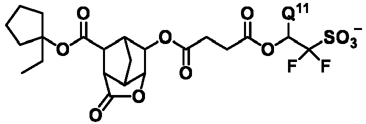

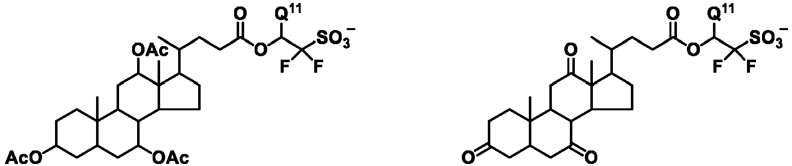

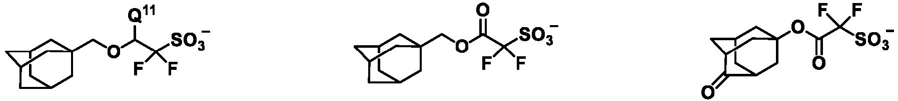

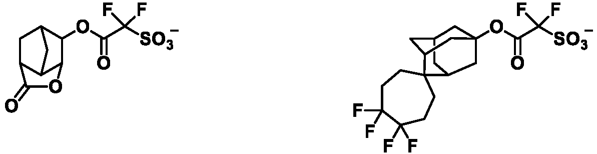

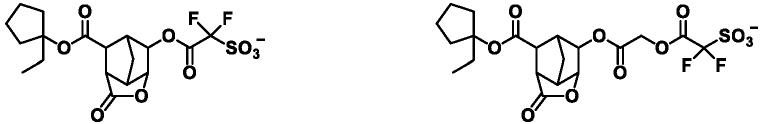

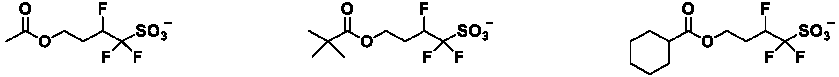

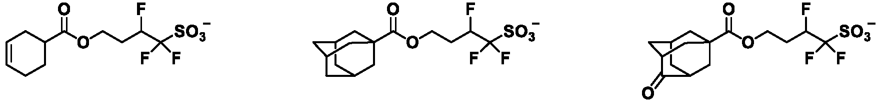

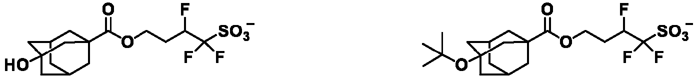

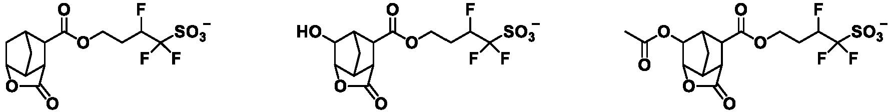

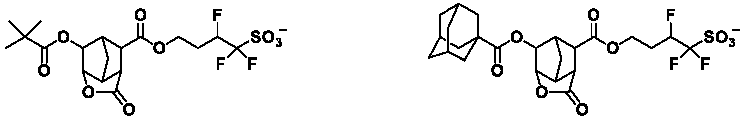

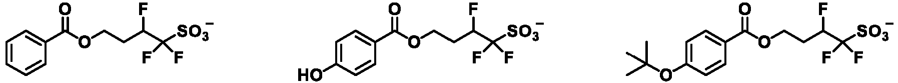

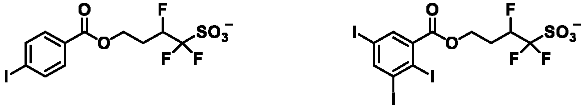

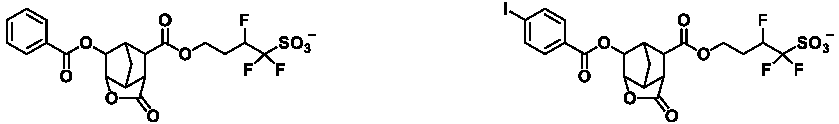

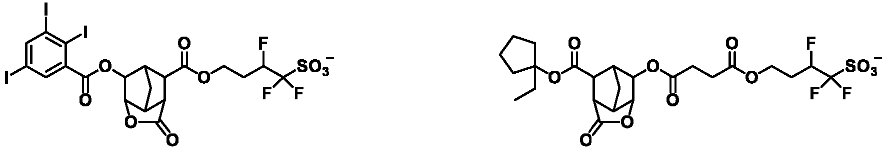

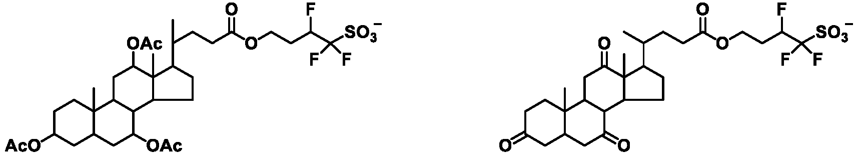

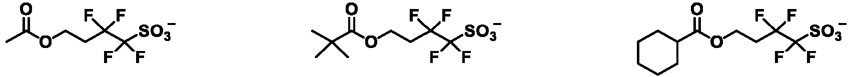

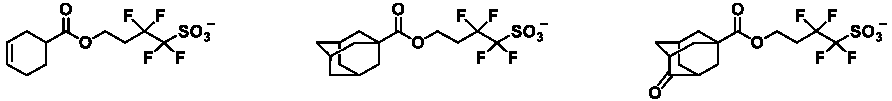

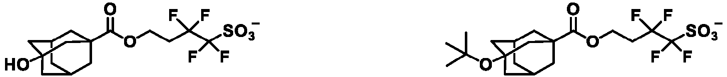

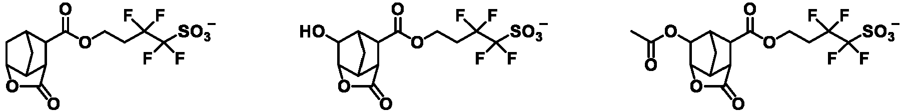

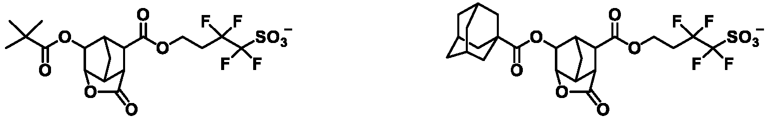

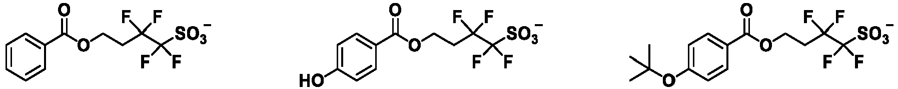

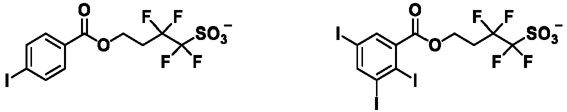

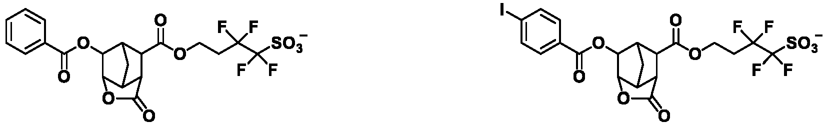

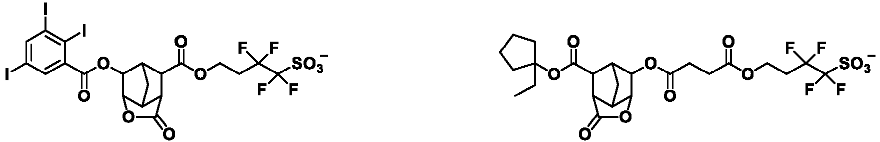

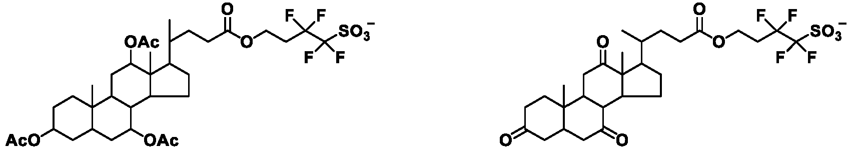

In formula (B6-2), R^{fb1} and R^{fb2} are each independently fluorine or a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1). Preferably R^{fb1} and R^{fb2} are fluorine or C₁-C₄ straight fluorinated alkyl groups. Also, R^{fb1} and R^{fb2} may bond together to form a ring with the linkage: -CF₂-SO₂-N⁻-SO₂-CF₂- to which they are attached. It is preferred that a combination of R^{fb1} and R^{fb2} be a fluorinated ethylene or fluorinated propylene group.

In formula (B6-3), R^{fc1}, R^{fc2} and R^{fc3} are each independently fluorine or a C₁-C₄₀ hydrocarbyl group which may contain a heteroatom. The hydrocarbyl group may be saturated or unsaturated and straight, branched or cyclic. Examples thereof are as exemplified for R^{fa1} in formula (B6-1-1). Preferably R^{fc1}, R^{fc2} and R^{fc3} are fluorine or C₁-C₄ straight fluorinated alkyl groups. Also, R^{fc1} and R^{fc2} may bond together to form a ring with the linkage: -CF₂-SO₂-C-SO₂-CF₂- to which they are attached. It is preferred that a combination of R^{fc1} and R^{fc2} be a fluorinated ethylene or fluorinated propylene group.

In formula (B6-4), R^{fd} is a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1).

Examples of the anion having formula (B6-4) are shown below, but not limited thereto.

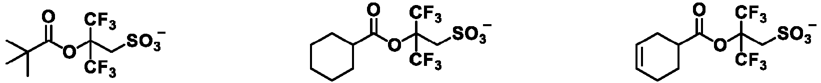

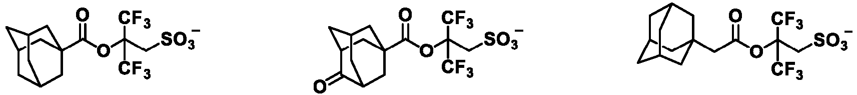

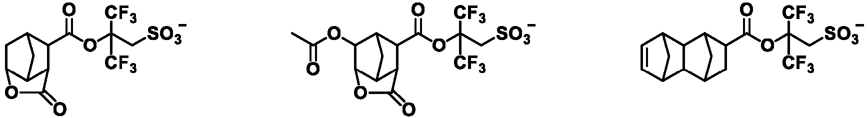

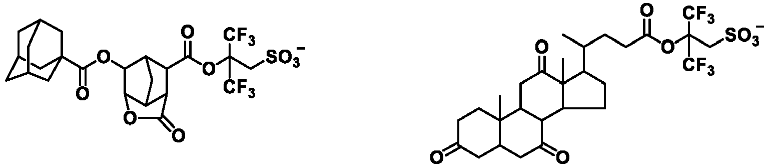

Anions having an iodized or brominated aromatic ring are also useful as the non-nucleophilic counter ion. These anions have the formula (B6-5).

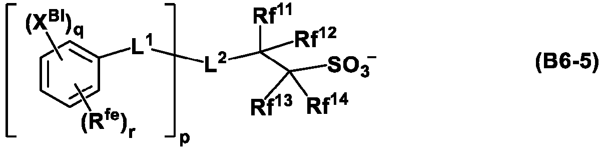

In formula (B6-5), p is an integer of 1 to 3, q is an integer of 1 to 5, r is an integer of 0 to 3, and q+r is from 1 to 5. Preferably, q is 1, 2 or 3, more preferably 2 or 3, and r is 0, 1 or 2.

In formula (B6-5), X^{BI} is iodine or bromine. A plurality of X^{BI} may be identical or different when p and/or q is 2 or more.

In formula (B6-5), L¹ is a single bond, ether bond, ester bond, or a C₁-C₆ saturated hydrocarbylene group which may contain an ether bond or ester bond. The saturated hydrocarbylene group may be straight, branched or cyclic.

In formula (B6-5), L² is a single bond or a C₁-C₂₀ divalent linking group when p=1, or a C₁-C₂₀ (p+1)-valent linking group when p=2 or 3. The linking group may contain an oxygen, sulfur or nitrogen atom.

In formula (B6-5), R^{fe} is hydroxy, carboxy, fluorine, chlorine, bromine, amino group, or a C₁-C₂₀ hydrocarbyl, C₁-C₂₀ hydrocarbyloxy, C₂-C₂₀ hydrocarbylcarbonyl, C₂-C₂₀ hydrocarbyloxycarbonyl, C₂-C₂₀ hydrocarbylcarbonyloxy, or C₁-C₂₀ hydrocarbylsulfonyloxy group, which may contain fluorine, chlorine, bromine, hydroxy, amino or ether bond, or -N(R^{feA})(R^{feB}), -N(R^{feC})-C(=O)-R^{feD} or -N(R^{feC})-C(=O)-O-R^{feD}. R^{feA} and R^{feB} are each independently hydrogen or a C₁-C₆ saturated hydrocarbyl group. R^{feC} is hydrogen, or a C₁-C₆ saturated hydrocarbyl group which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. R^{feD} is a C₁-C₁₆ aliphatic hydrocarbyl group, C₆-C₁₂ aryl group or C₇-C₁₅ aralkyl group, which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. The aliphatic hydrocarbyl group may be saturated or unsaturated and straight, branched or cyclic. The hydrocarbyl, hydrocarbyloxy, hydrocarbylcarbonyl, hydrocarbyloxycarbonyl, hydrocarbylcarbonyloxy, and hydrocarbylsulfonyloxy groups may be straight, branched or cyclic. A plurality of R^{fe} may be identical or different when p and/or r is 2 or more.

Of these, R^{fe} is preferably hydroxy, -N(R^{feC})-C(=O)-R^{feD}, -N(R^{feC})-C(=O)-O-R^{feD}, fluorine, chlorine, bromine, methyl or methoxy.

In formula (B6-5), Rf¹¹ to Rf¹⁴ are each independently hydrogen, fluorine or trifluoromethyl, at least one of Rf¹¹ to Rf¹⁴ is fluorine or trifluoromethyl. Rf¹¹ and Rf¹², taken together, may form a carbonyl group. More preferably, both Rf¹³ and Rf¹⁴ are fluorine.

Examples of the anion having formula (B6-5) are shown below, but not limited thereto. X^{BI} is as defined above.

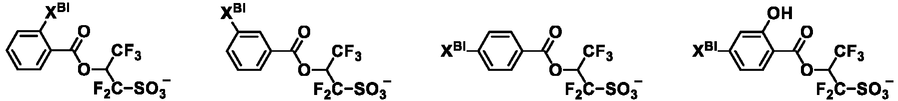

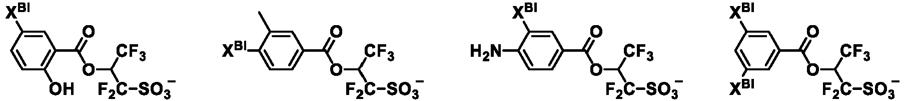

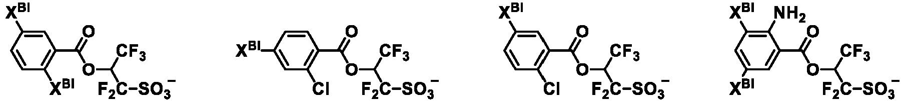

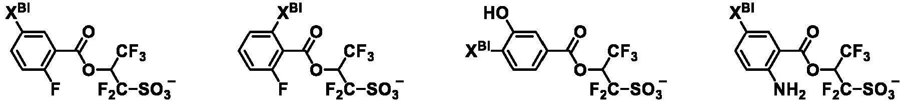

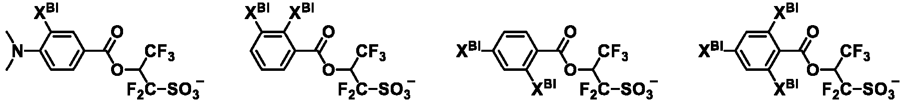

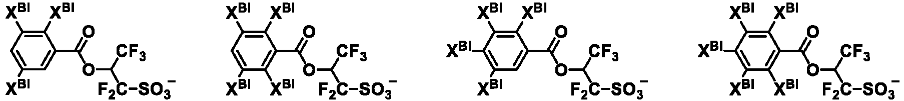

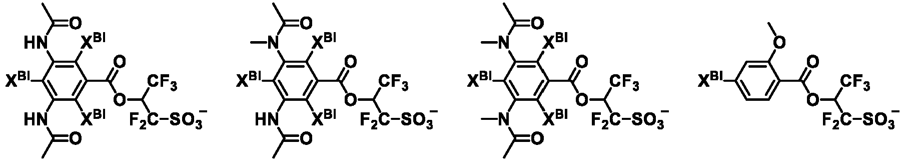

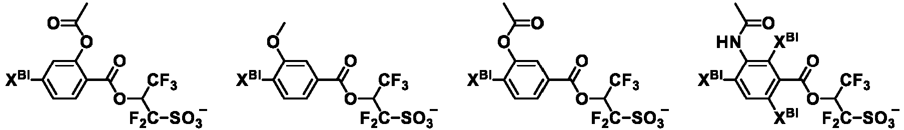

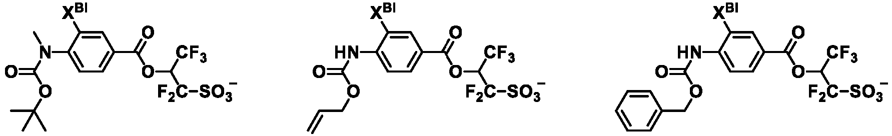

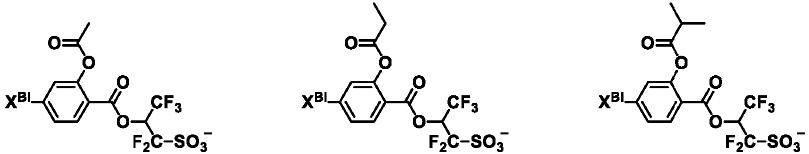

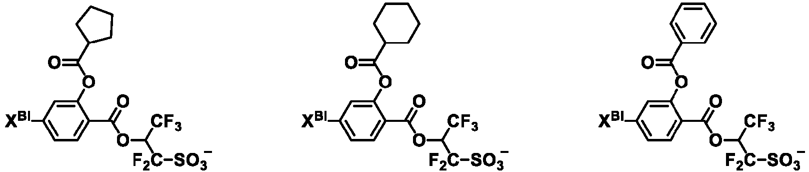

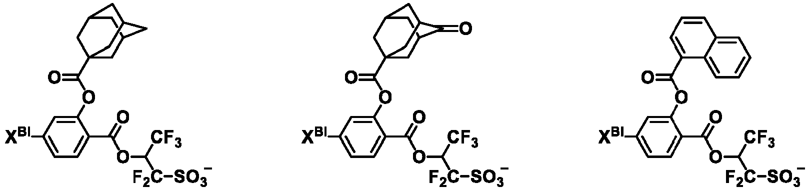

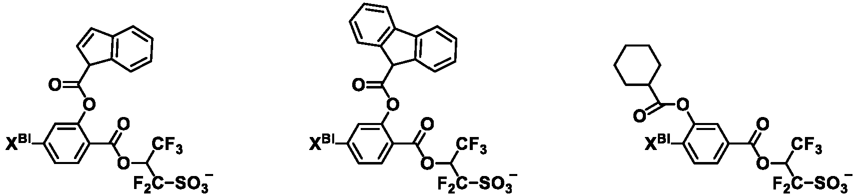

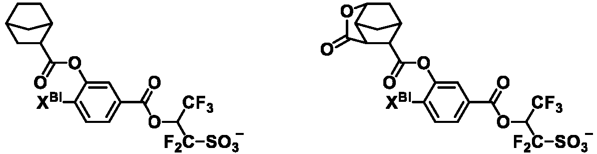

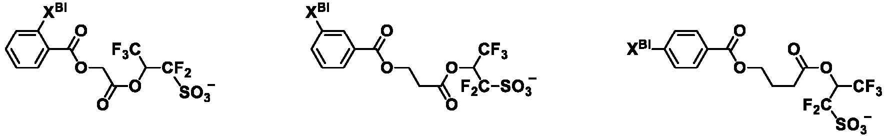

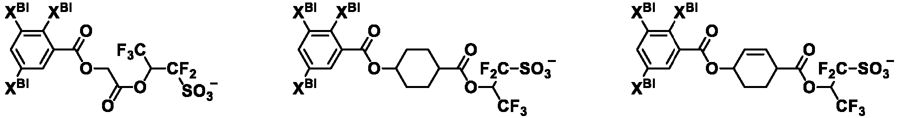

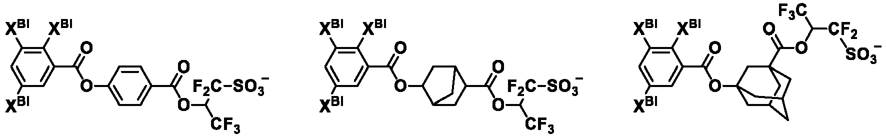

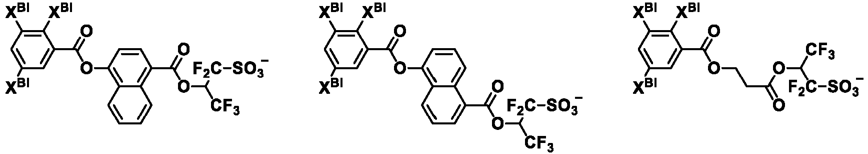

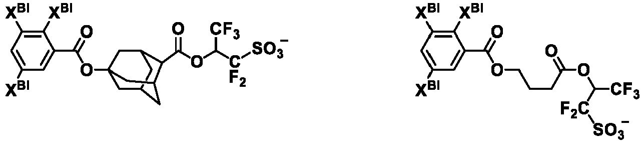

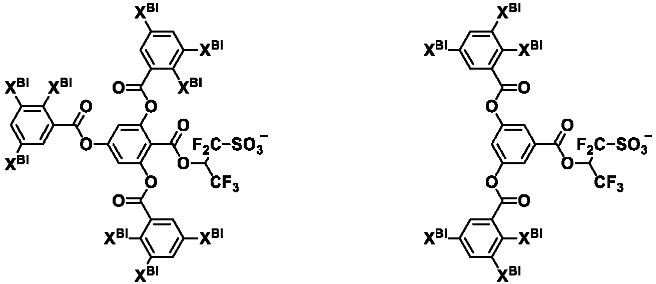

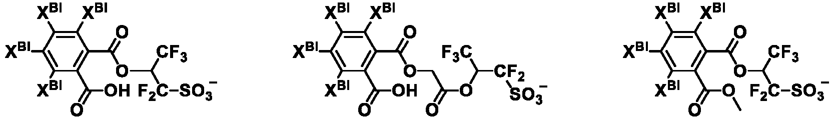

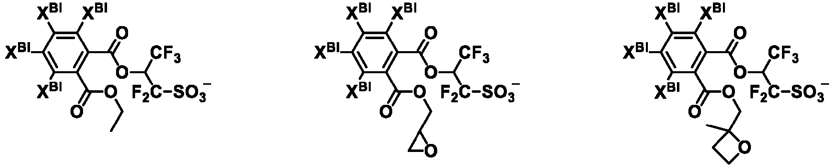

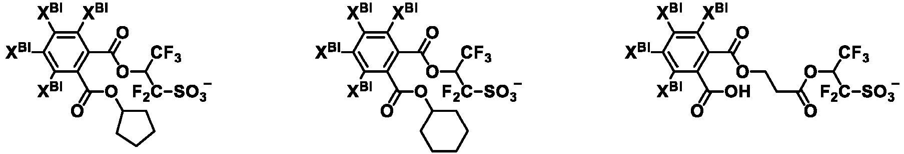

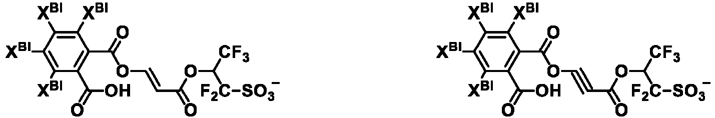

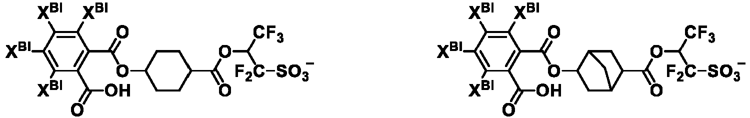

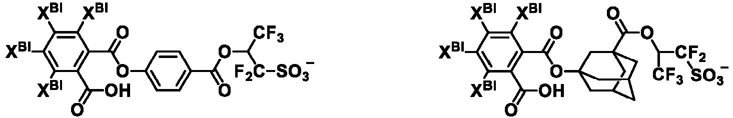

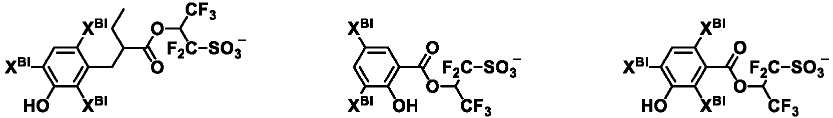

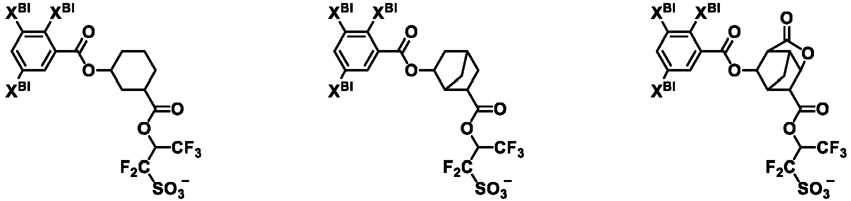

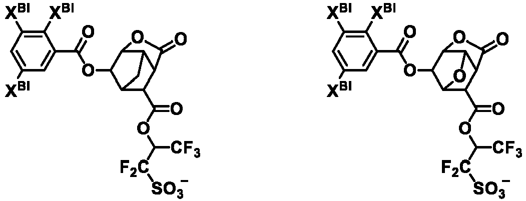

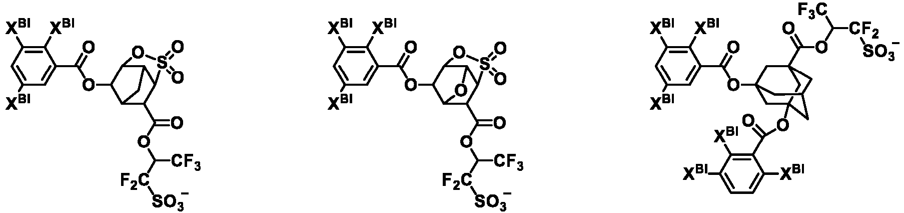

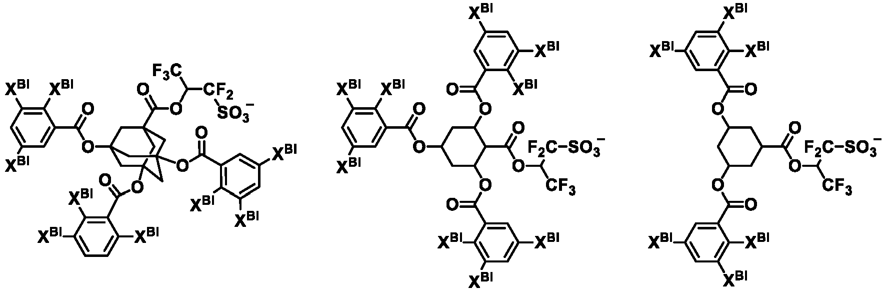

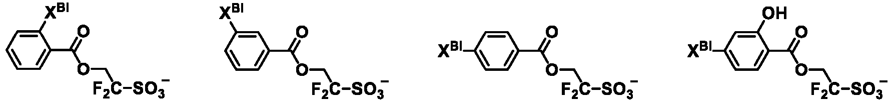

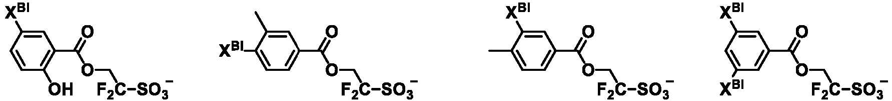

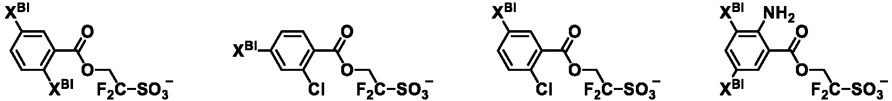

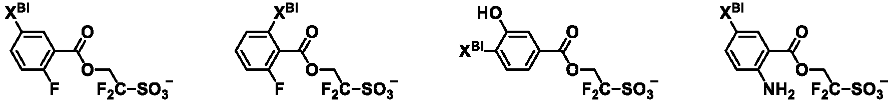

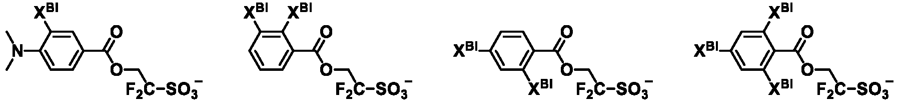

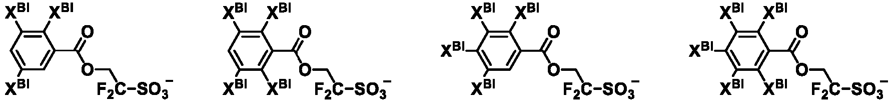

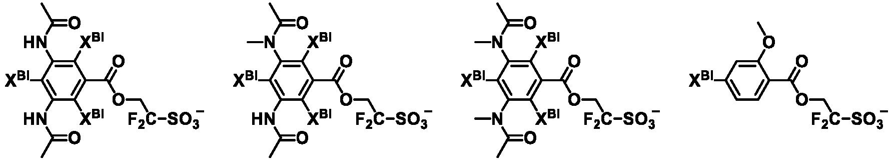

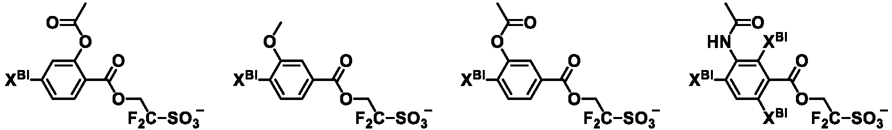

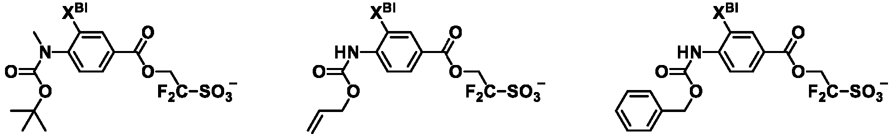

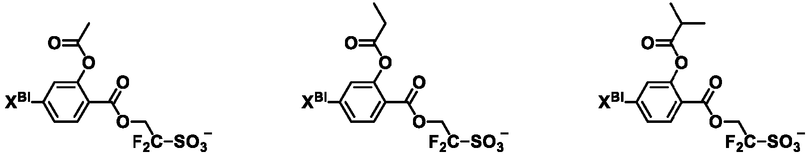

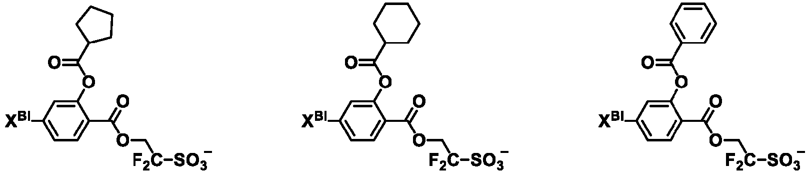

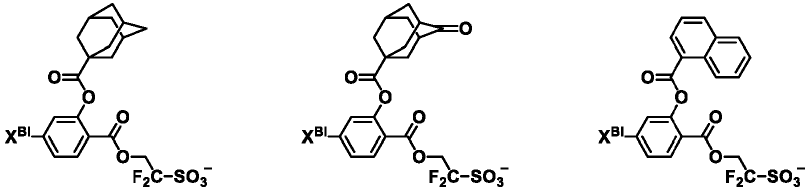

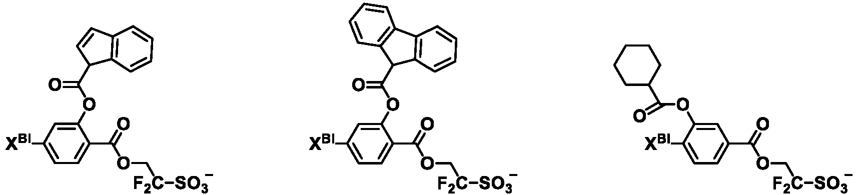

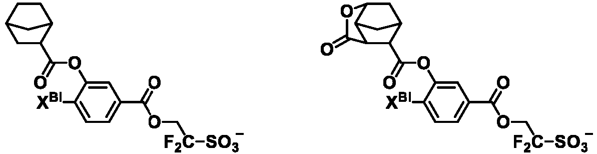

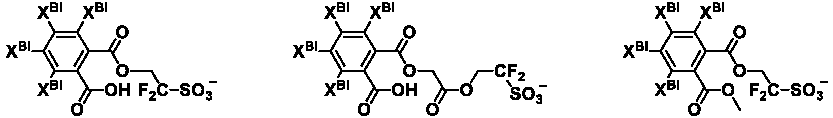

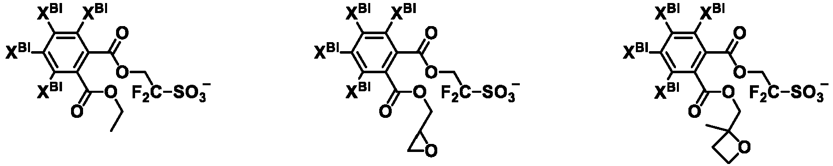

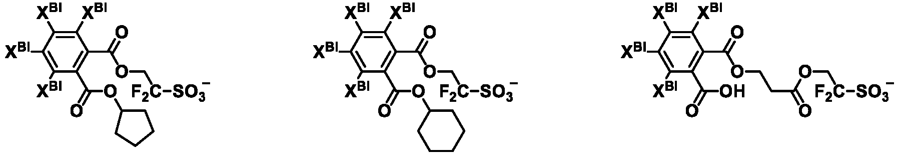

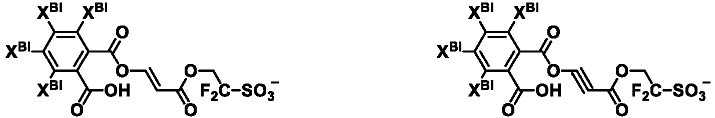

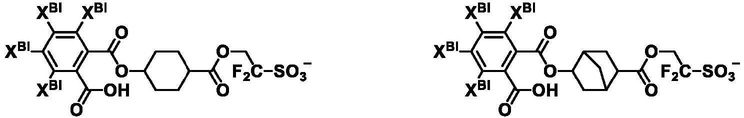

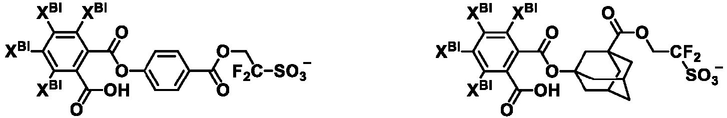

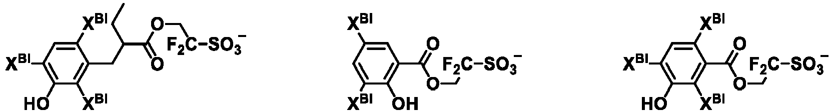

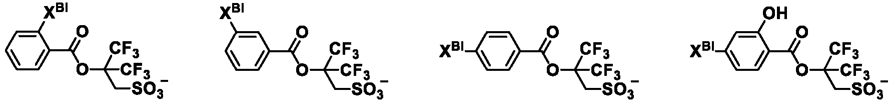

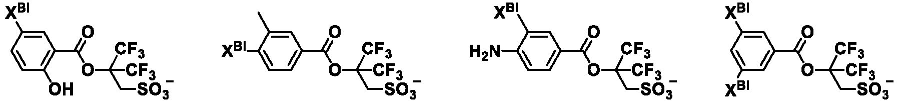

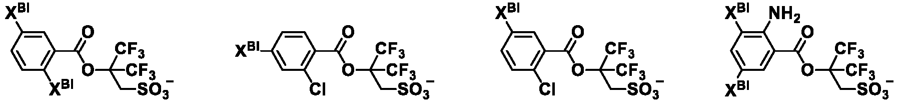

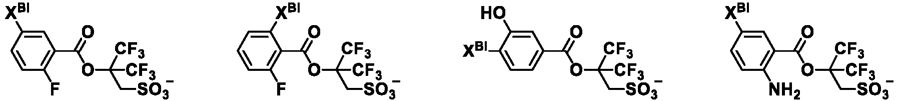

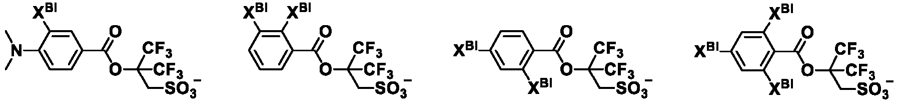

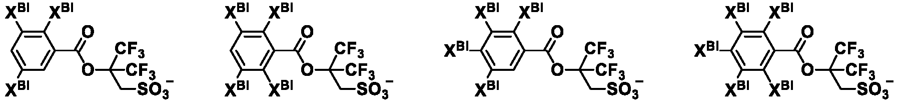

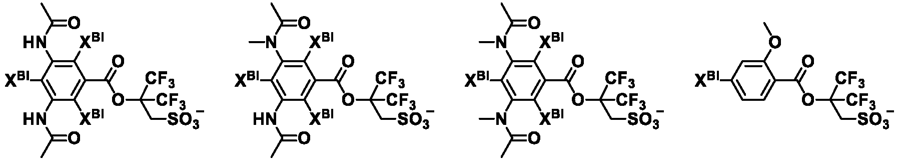

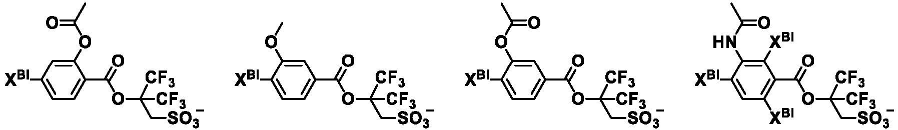

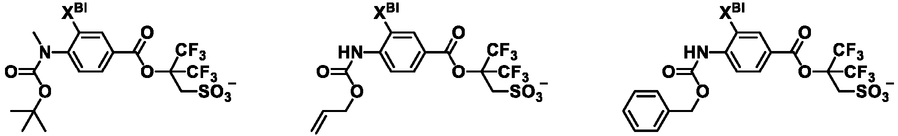

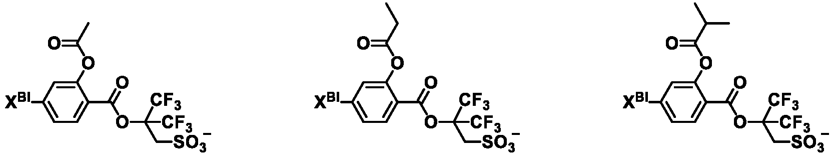

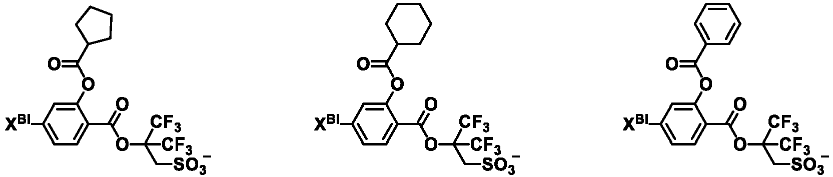

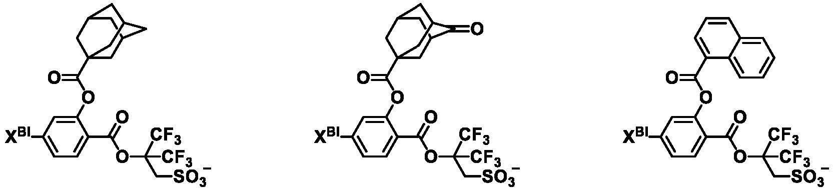

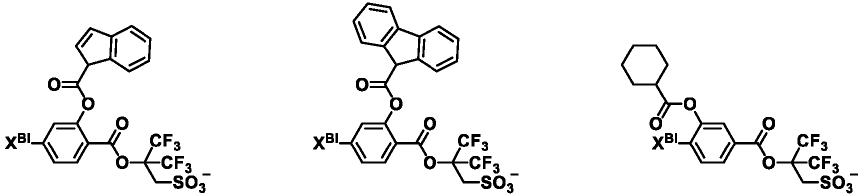

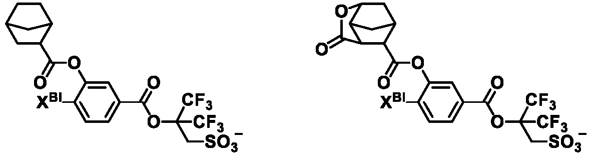

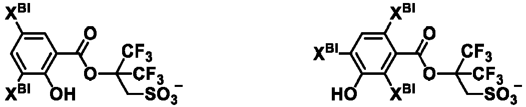

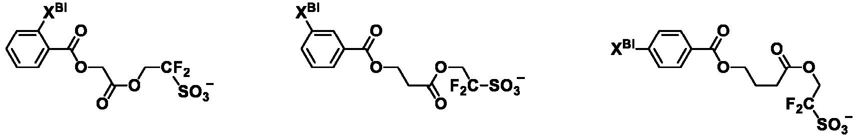

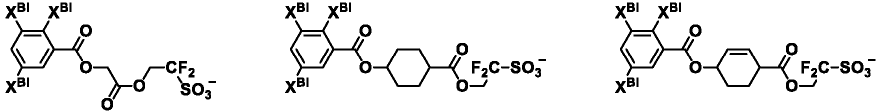

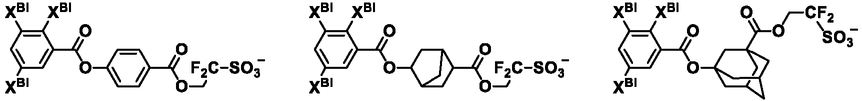

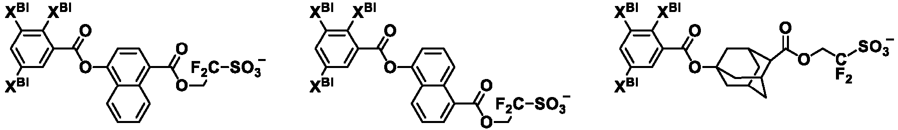

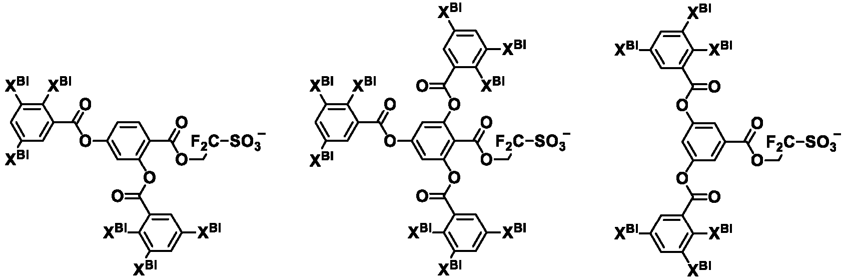

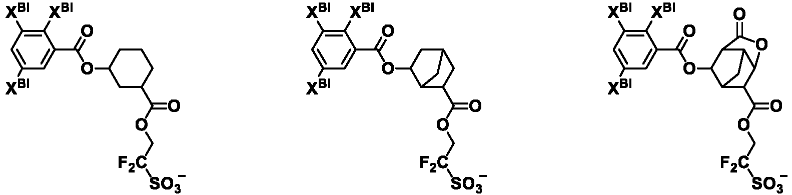

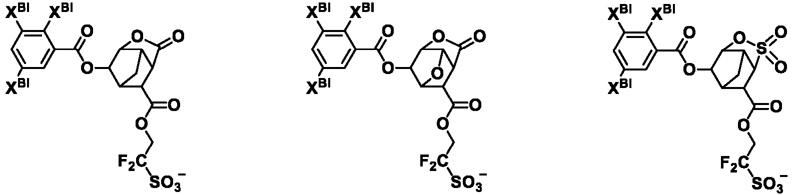

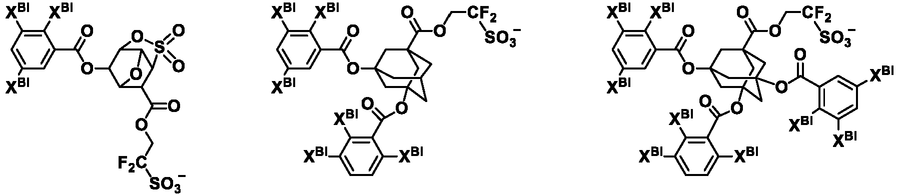

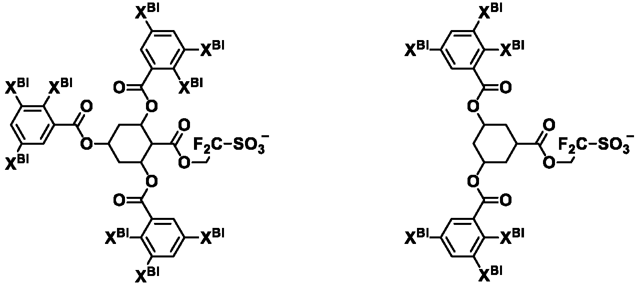

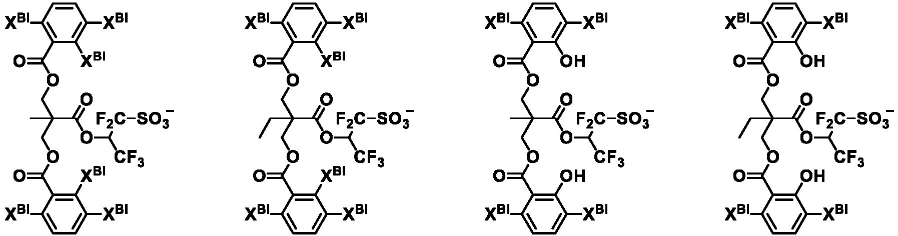

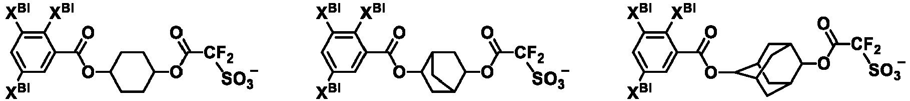

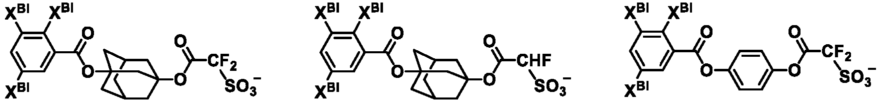

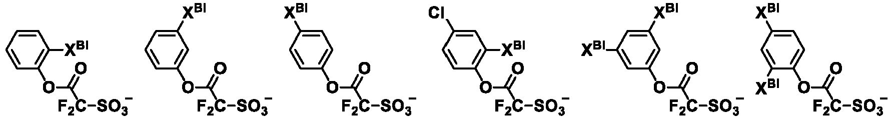

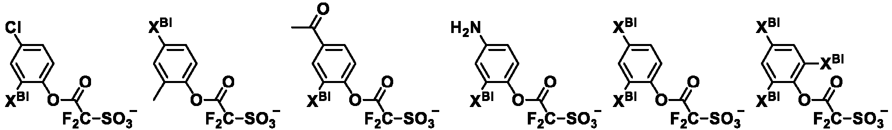

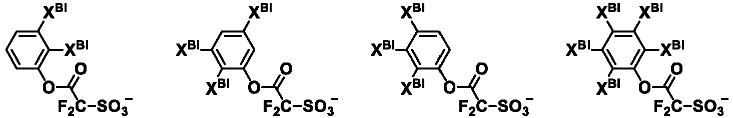

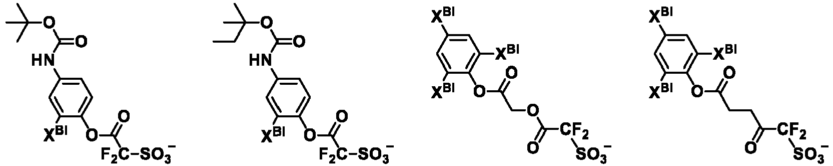

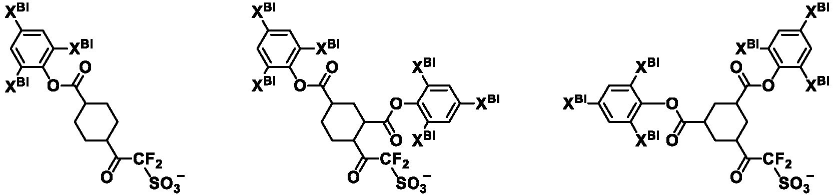

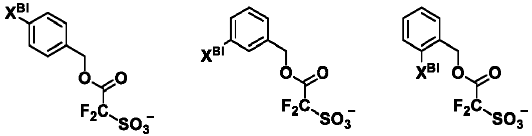

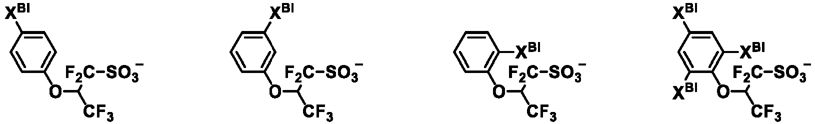

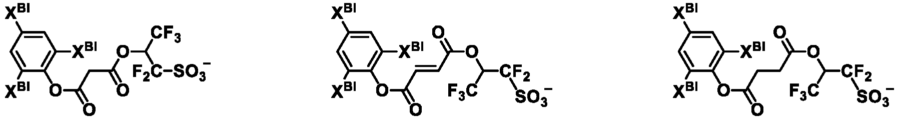

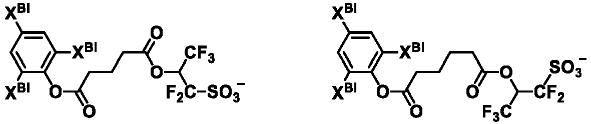

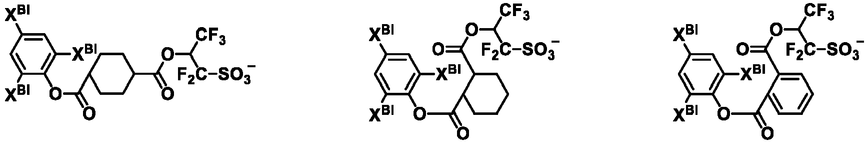

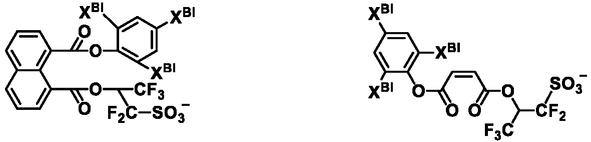

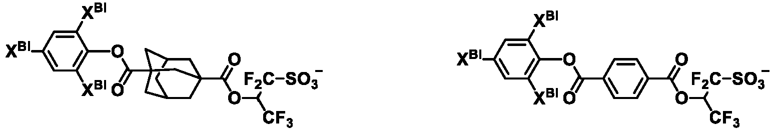

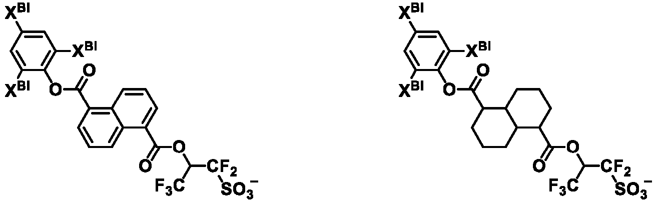

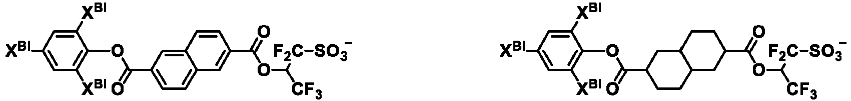

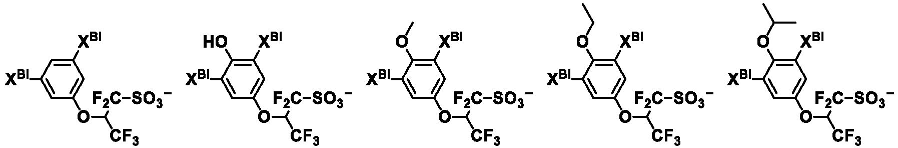

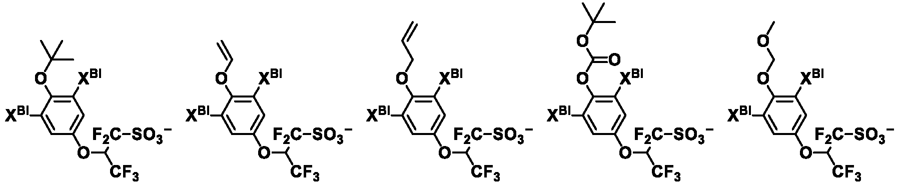

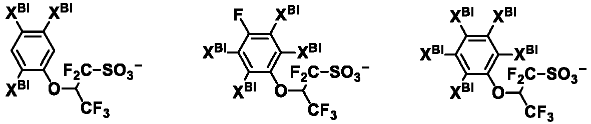

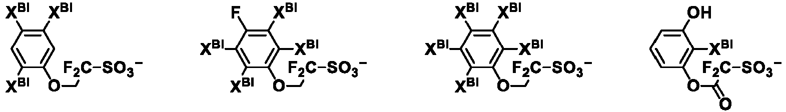

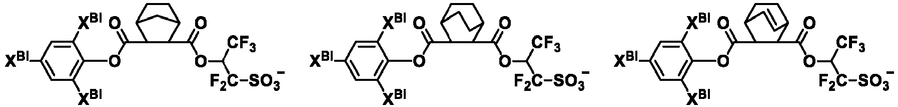

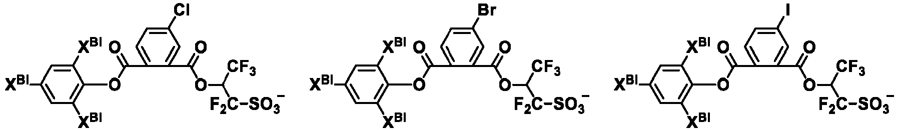

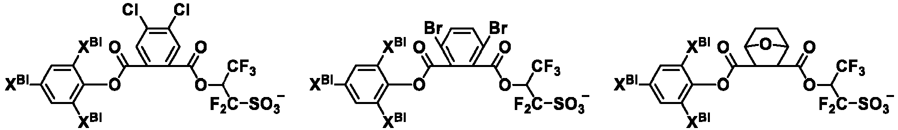

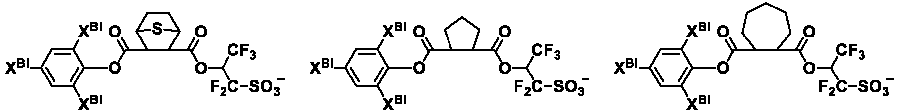

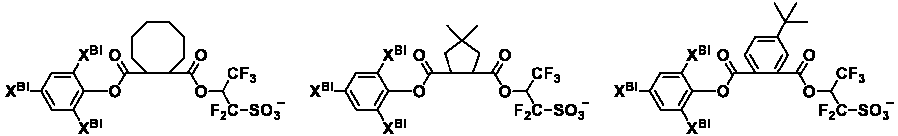

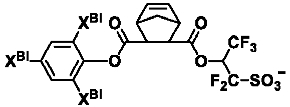

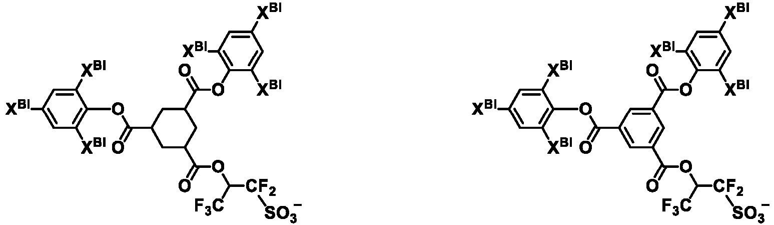

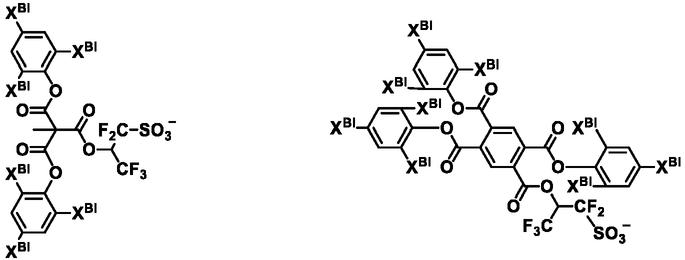

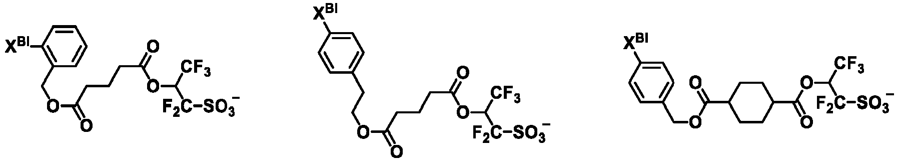

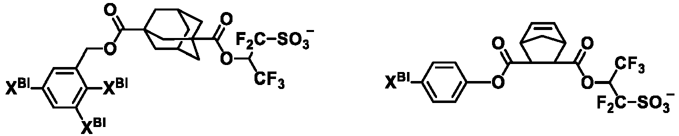

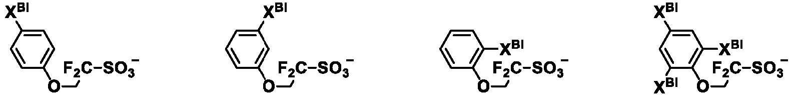

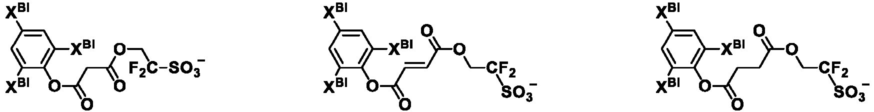

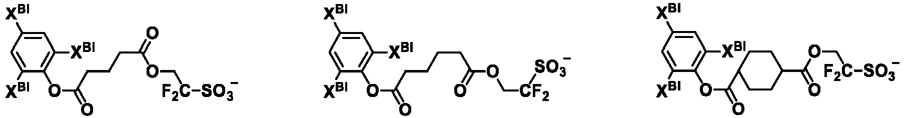

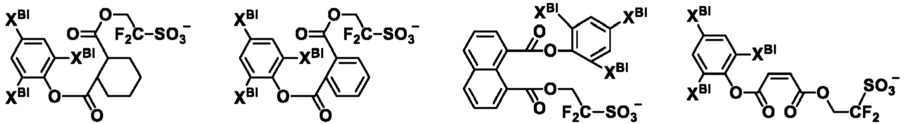

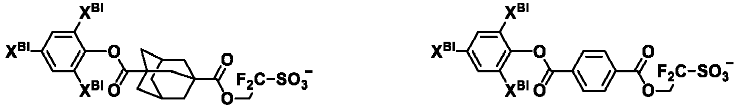

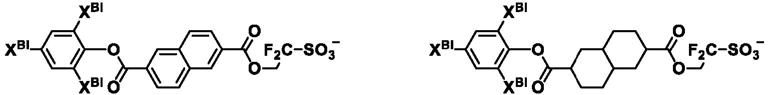

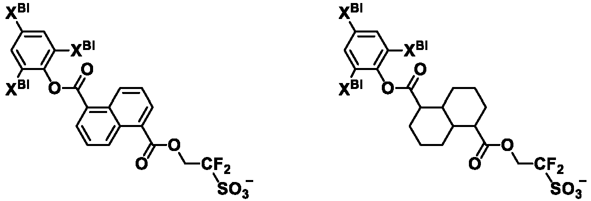

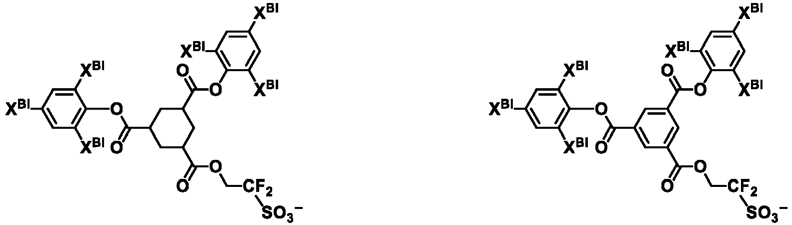

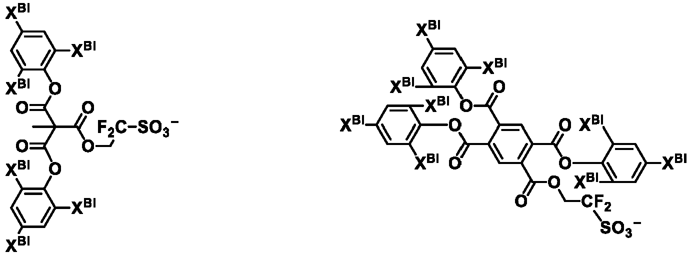

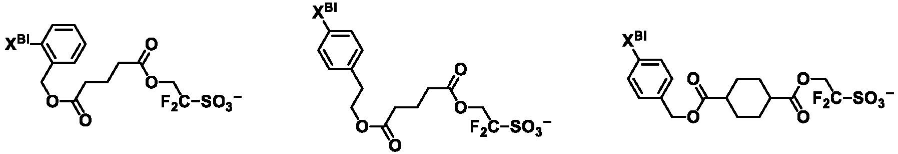

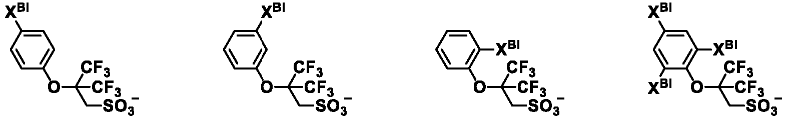

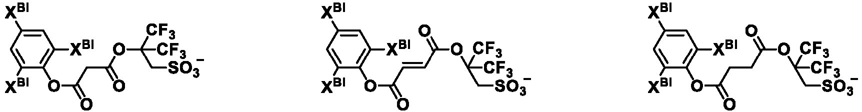

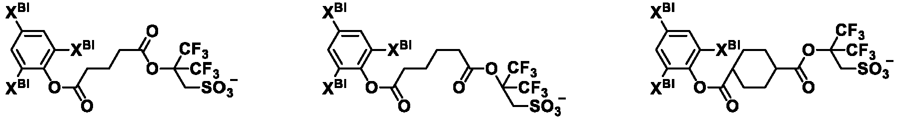

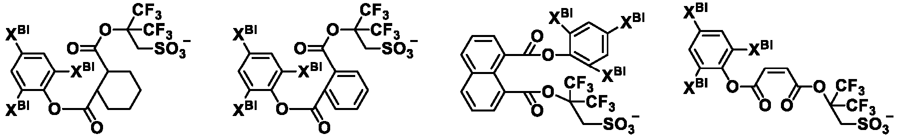

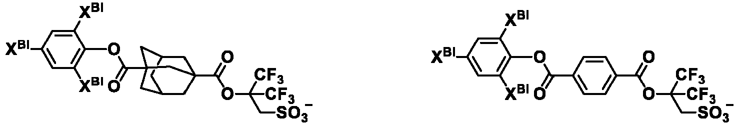

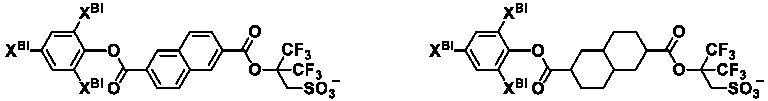

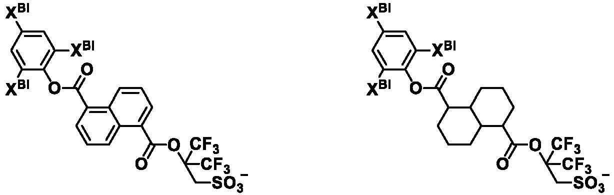

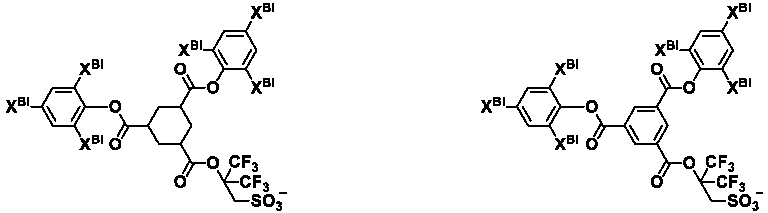

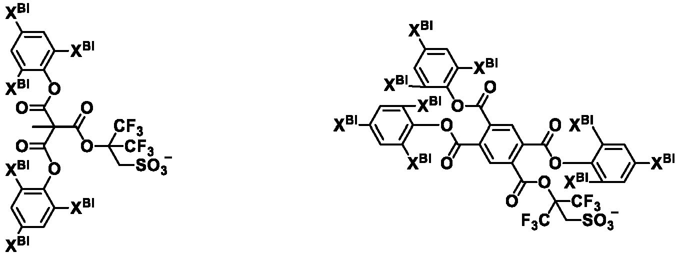

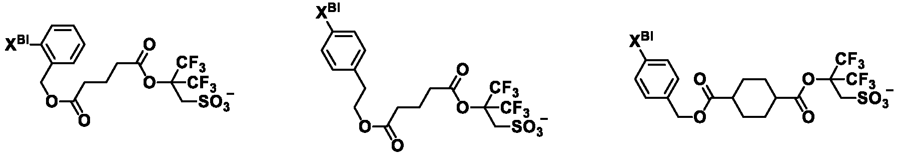

Other useful examples of the non-nucleophilic counter ion include fluorobenzenesulfonic acid anions having an iodized aromatic ring bonded thereto as described in JP 6648726, anions having an acid-catalyzed decomposition mechanism as described in WO 2021/200056 and JP-A 2021-070692, anions having a cyclic ether group as described in JP-A 2018-180525 and JP-A 2021-035935, and anions as described in JP-A 2018-092159.

Further useful examples of the non-nucleophilic counter ion include fluorine-free bulky benzenesulfonic acid anions as described in JP-A 2006-276759, JP-A 2015-117200, JP-A 2016-065016, and JP-A 2019-202974; fluorine-free benzenesulfonic acid or alkylsulfonic acid anions having an iodized aromatic group bonded thereto as described in JP 6645464.

Also useful are bissulfonic acid anions as described in JP-A 2015-206932, sulfonamide or sulfonimide anions having sulfonic acid side and different side as described in WO 2020/158366, and anions having a sulfonic acid side and a carboxylic acid side as described in JP-A 2015-024989.

In formulae (B7) and (B8), L¹ is a single bond, ether bond, ester bond, carbonyl, sulfonate ester bond, carbonate bond or carbamate bond. From the aspect of synthesis, an ether bond, ester bond or carbonyl is preferred, with the ester bond or carbonyl being more preferred.

In formula (B7), Rf¹ and Rf² are each independently fluorine or a C₁-C₆ fluorinated saturated hydrocarbyl group. It is preferred that both Rf¹ and Rf² be fluorine because the generated acid has a higher acid strength. Rf³ and Rf⁴ are each independently hydrogen, fluorine or a C₁-C₆ fluorinated saturated hydrocarbyl group. It is preferred for solvent solubility that at least one of Rf³ and Rf⁴ be trifluoromethyl.

In formula (B8), Rf⁵ and Rf⁶ are each independently hydrogen, fluorine or a C₁-C₆ fluorinated saturated hydrocarbyl group. It is excluded that all Rf⁵ and Rf⁶ are hydrogen at the same time. It is preferred for solvent solubility that at least one of Rf⁵ and Rf⁶ be trifluoromethyl.

In formulae (B7) and (B8), g1 and g2 each are an integer of 0 to 3, preferably 1.

Examples of the anion in repeat unit B7 are shown below, but not limited thereto. R^{A} is as defined above.

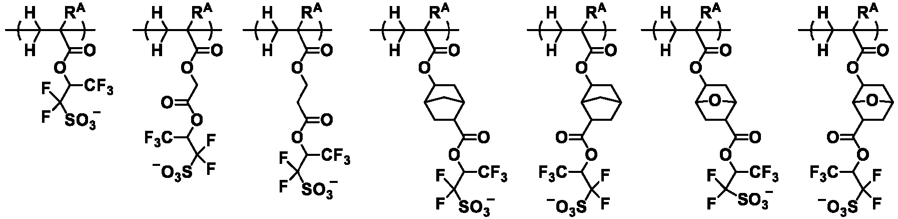

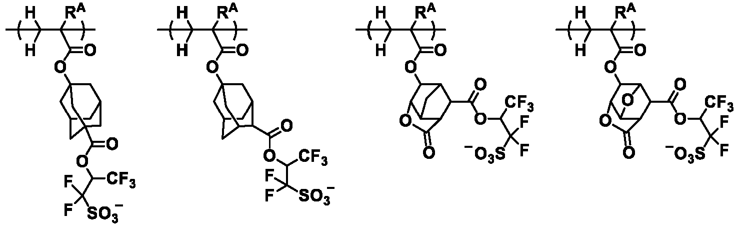

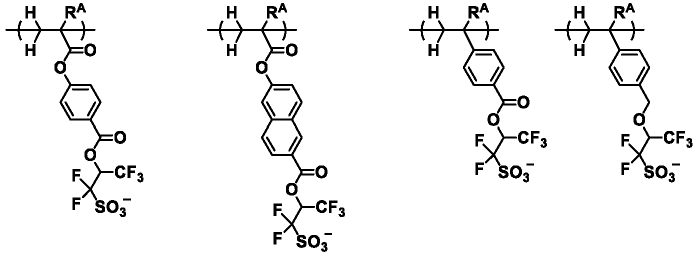

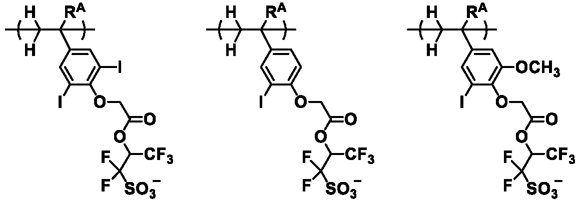

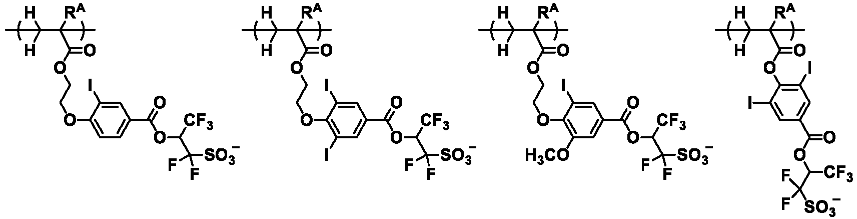

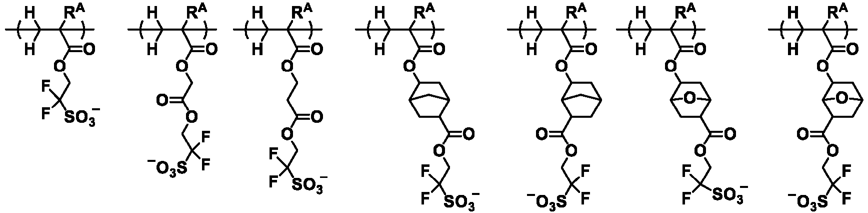

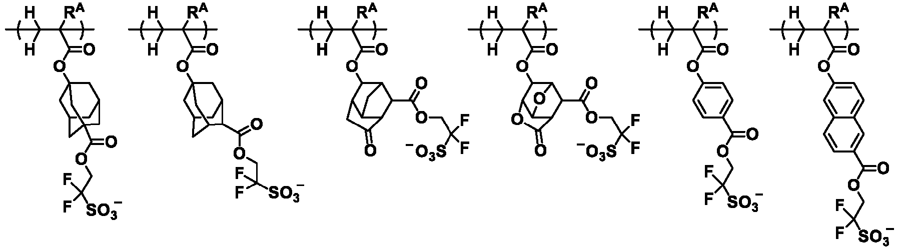

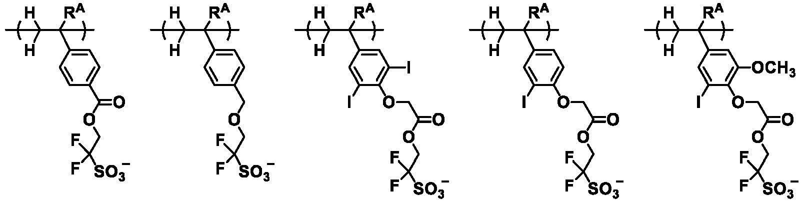

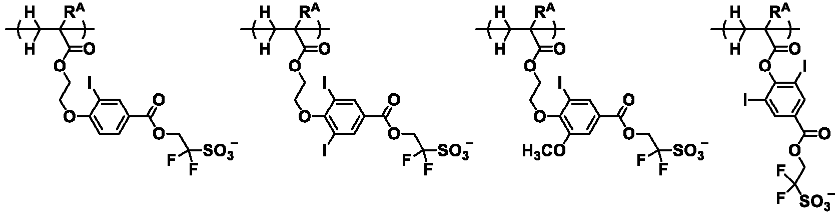

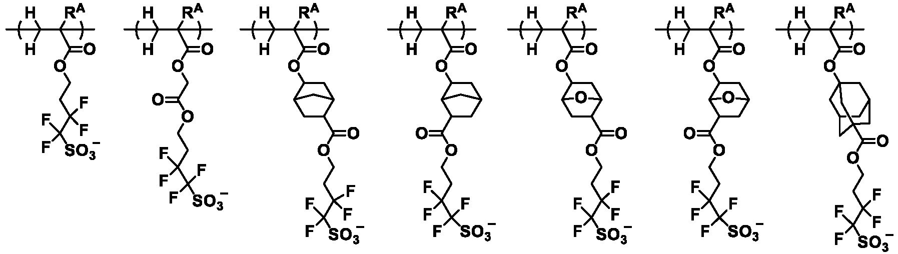

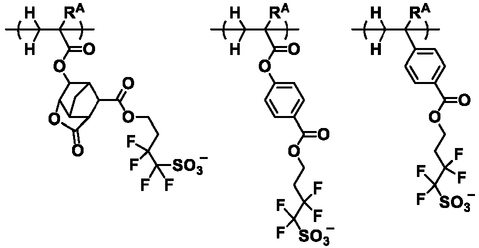

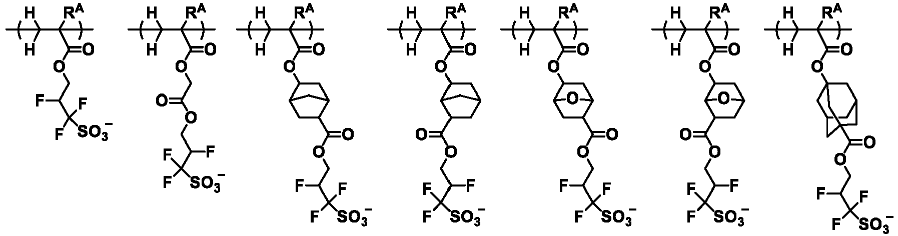

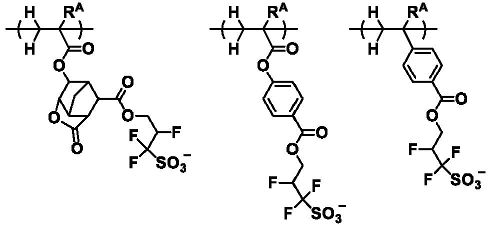

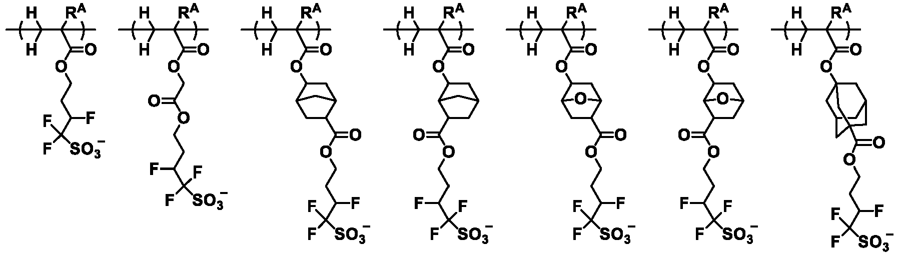

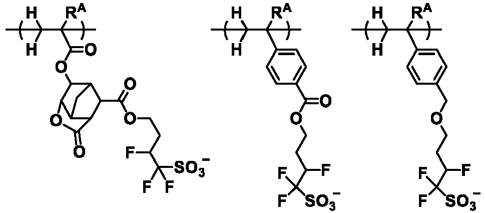

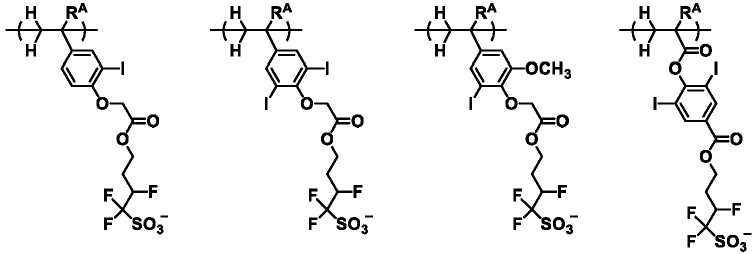

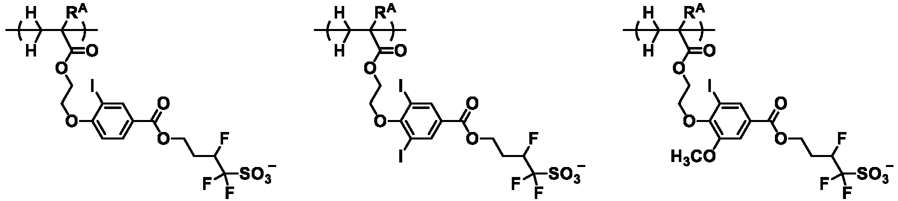

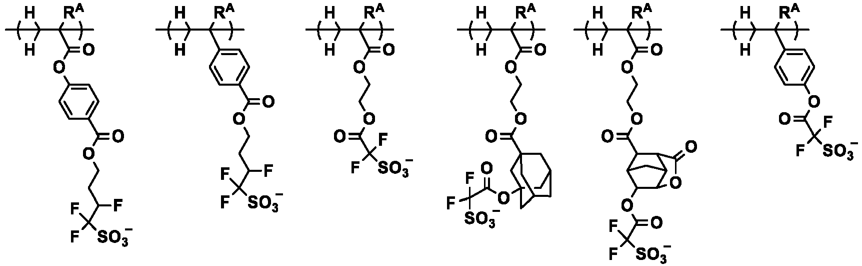

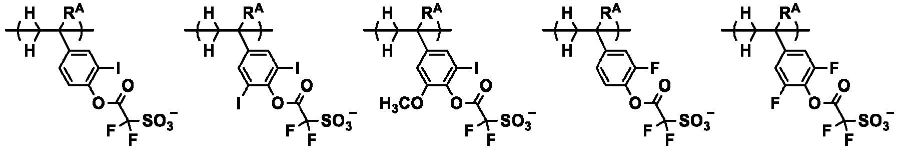

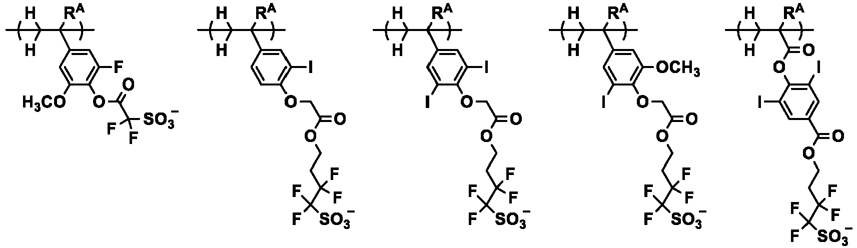

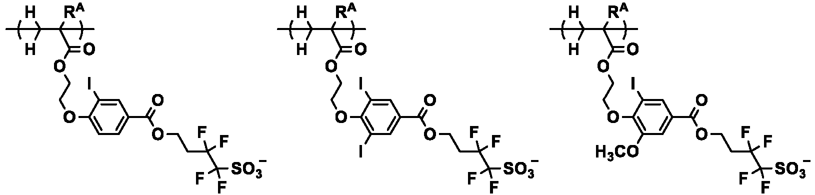

Examples of the anion in repeat unit B8 are shown below, but not limited thereto. R^{A} is as defined above.

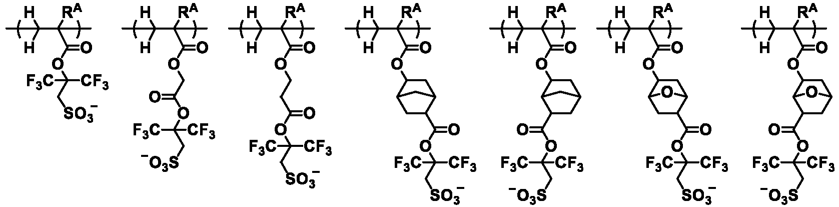

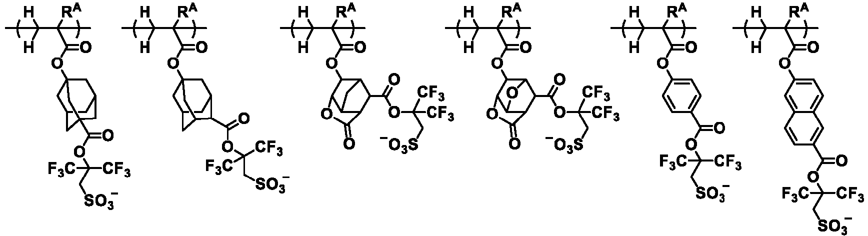

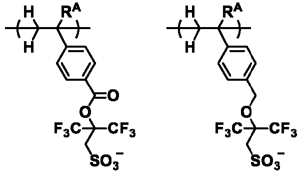

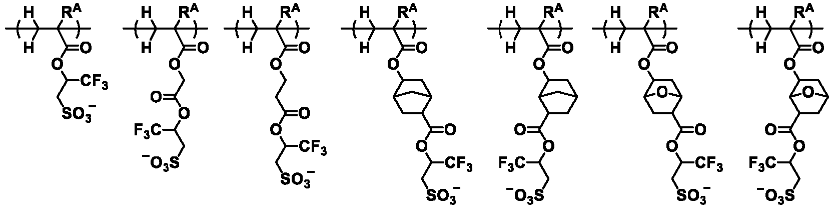

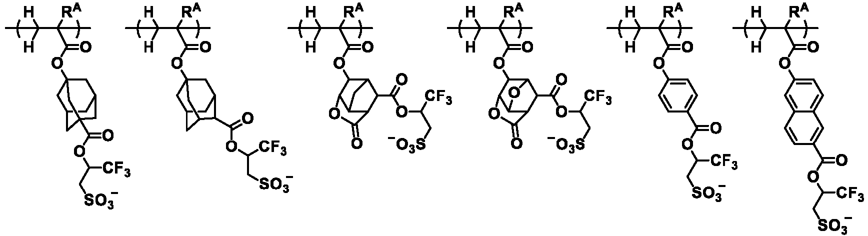

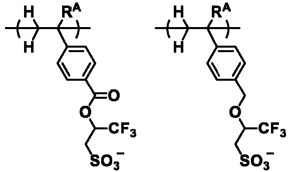

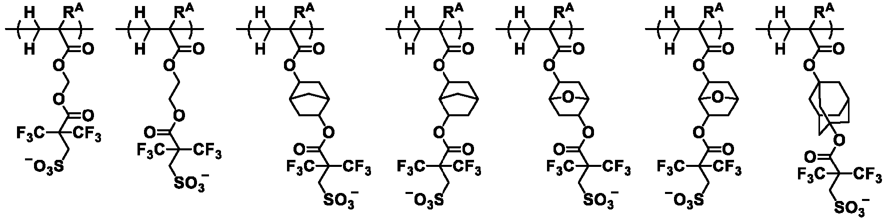

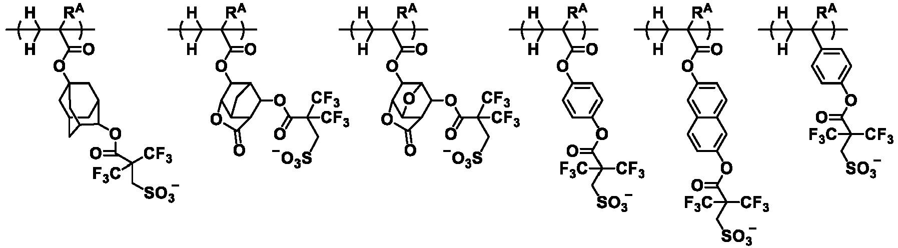

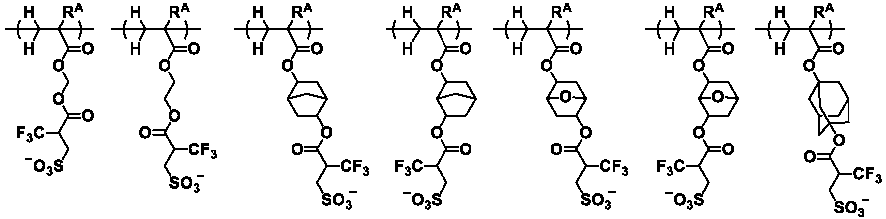

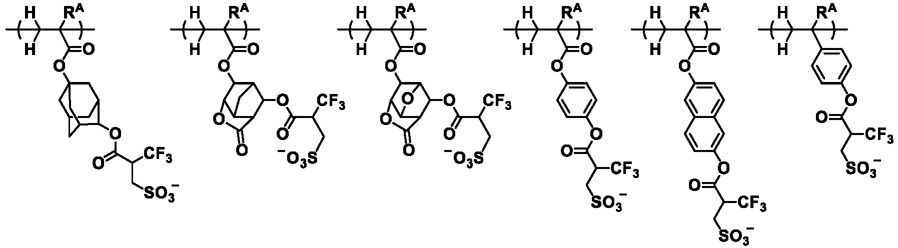

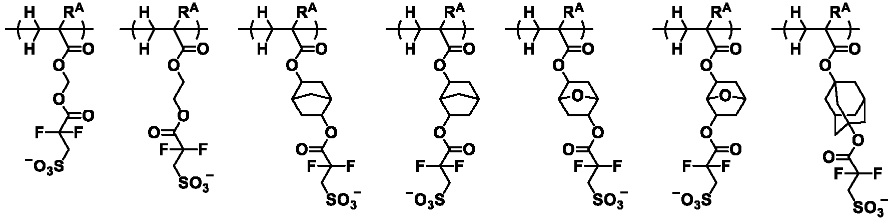

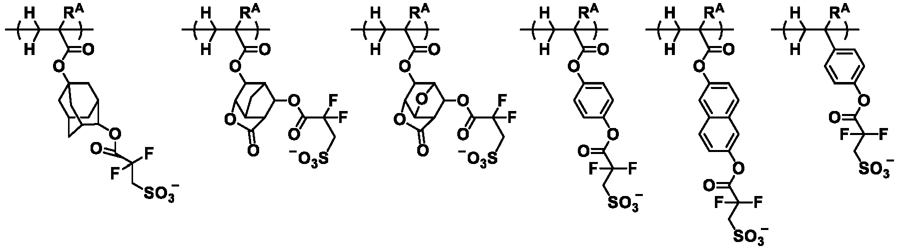

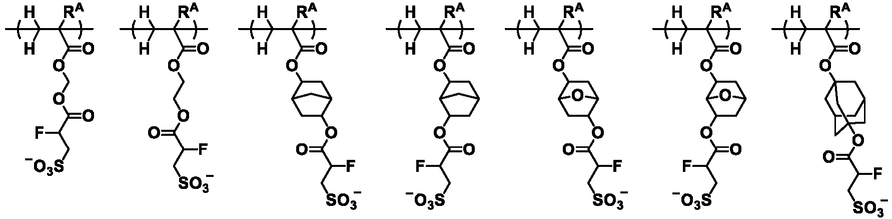

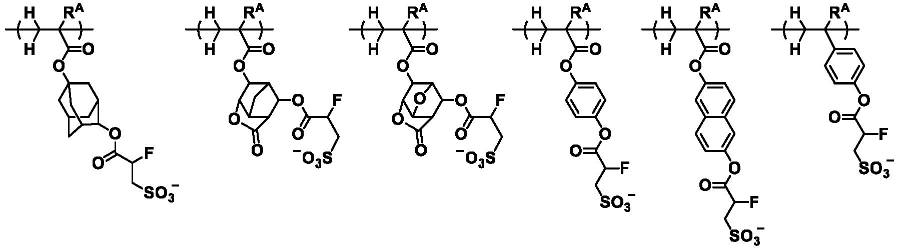

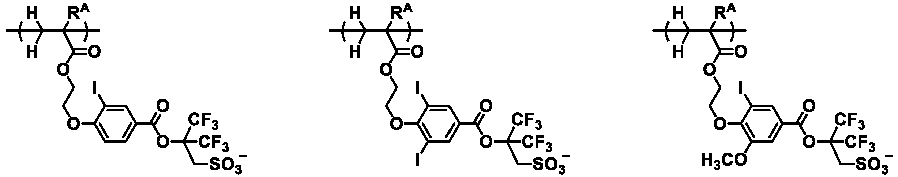

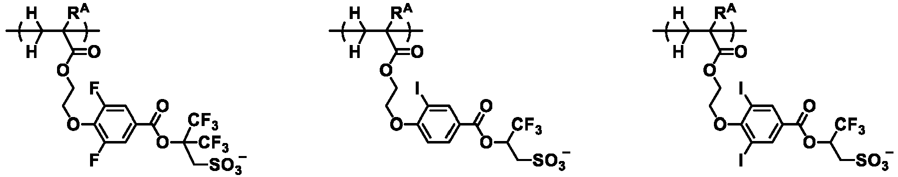

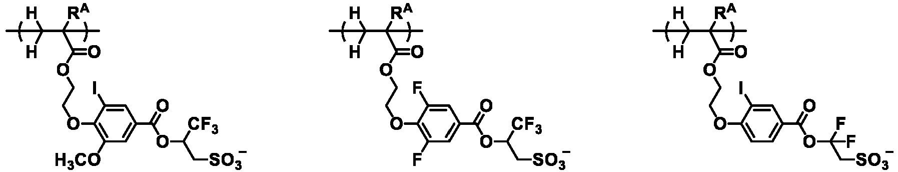

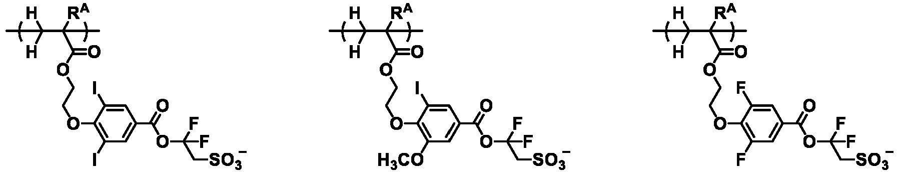

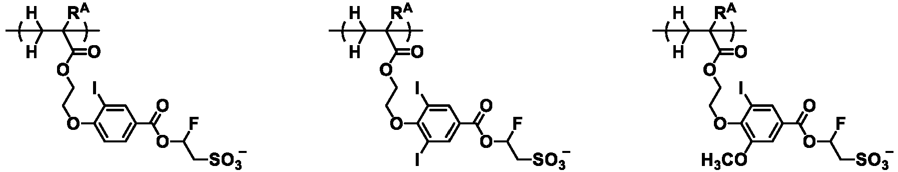

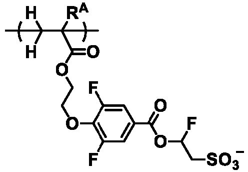

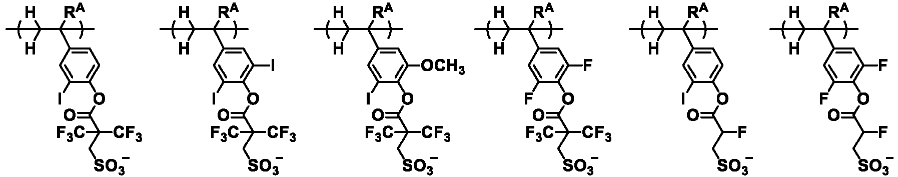

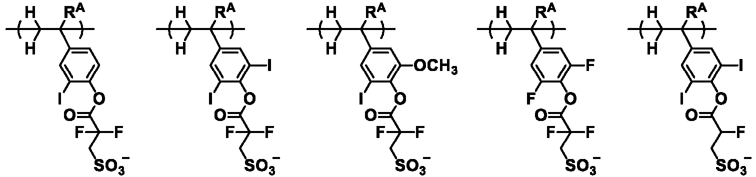

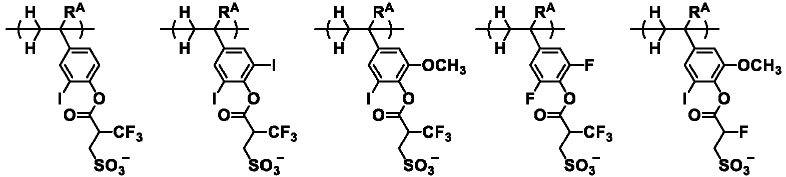

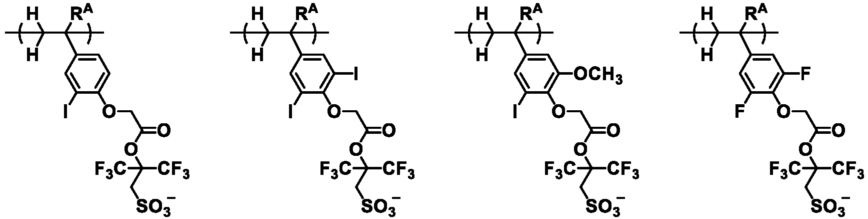

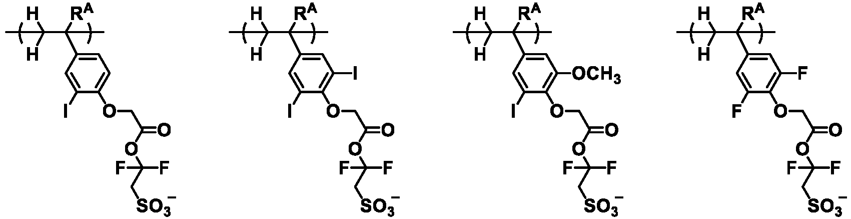

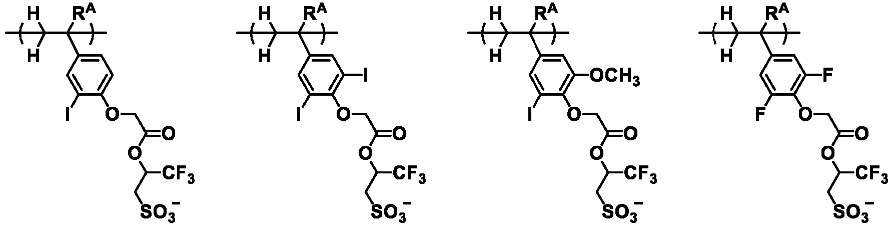

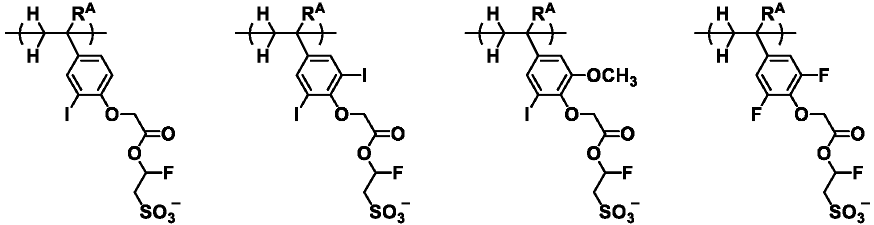

Examples of the anion in repeat unit B9 are shown below, but not limited thereto. R^{A} is as defined above.

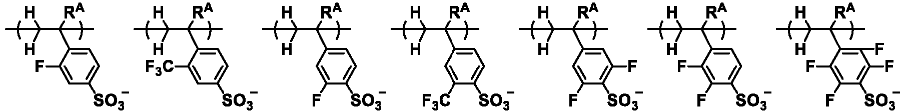

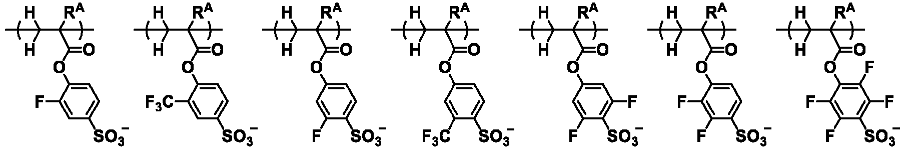

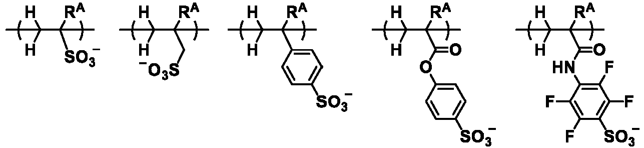

In formulae (B6) to (B9), A⁺ is an onium cation. Suitable onium cations include ammonium, sulfonium and iodonium cations, with the sulfonium and iodonium cations being preferred. Examples of the sulfonium cation are as exemplified above for the sulfonium cation having formula (cation-1). Examples of the iodonium cation are as exemplified above for the iodonium cation having formula (cation-2).

The repeat units B6 to B9 are capable of generating an acid upon exposure to high-energy radiation. It is believed that binding of the relevant units to a polymer enables to appropriately control acid diffusion and to form a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing profile degradation due to unwanted film thickness loss in the unexposed region.

Of repeat units B6 to B9, repeat units B7 to B9 are preferred for the processing of photomask blanks because an optimum acid strength is available for the suppression of acid diffusion and the design of an acid labile group on the polymer. The repeat units B7 and B8 are more preferred

When repeat units B6 to B9 are included, their content is preferably 0.1 to 30 mol%, more preferably 0.5 to 20 mol% based on the overall repeat units of the polymer. Each of repeat units B6 to B9 may be of one type or a combination of plural types.

The content of repeat units having an aromatic ring structure is preferably at least 65 mol%, more preferably at least 75 mol%, even more preferably at least 85 mol% based on the overall repeat units of the polymer. When the polymer does not contain repeat units B6 to B9, it is preferred that all units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the following formulae (B 10) to (B 12), which are also referred to as repeat units B10 to B12. While these units do not exhibit acidity, they may be used as auxiliary units for providing adhesion to substrates or adjusting solubility.

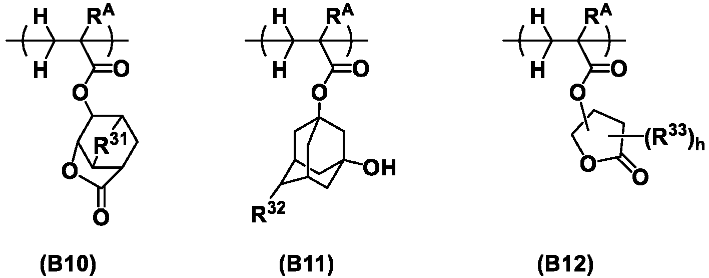

In formulae (B 10) to (B12), R^{A} is each independently hydrogen, fluorine, methyl or trifluoromethyl. R³¹ is -O- or methylene. R³² is hydrogen or hydroxy. R³³ is a C₁-C₄ saturated hydrocarbyl group, and h is an integer of 0 to 3.

When repeat units B10 to B12 are included, their content is preferably 0 to 20 mol%, more preferably 0 to 10 mol% based on the overall repeat units of the polymer. Each of repeat units B10 to B12 may be of one type or a combination of plural types.

The polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630, for example.

The polymer should preferably have a Mw of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER is degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.0, more preferably 1.0 to 1.9. A polymer with such a narrow dispersity eliminates the risk that foreign particles are left on the pattern after development and the pattern profile is aggravated.

The base polymer is designed such that the dissolution rate in alkaline developer is preferably up to 10 nm/min, more preferably up to 7 nm/min, even more preferably up to 5 nm/min. In the advanced generation of lithography wherein the coating film on the substrate is in a thin film range of up to 100 nm, the influence of pattern film thickness loss during alkaline development becomes strong. When the polymer has an alkaline dissolution rate of greater than 10 nm/min, pattern collapse occurs, i.e., a small-size pattern cannot be formed. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto a 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

In addition to the polymer defined above, the base polymer (B) may contain another polymer. The other polymer may be any of prior art well-known base polymers used in resist compositions. The content of the other polymer is not particularly limited as long as the benefits of the invention are not impaired.

### (C) Organic solvent

The chemically amplified positive resist composition may comprise an organic solvent as component (C). The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone, cyclopentanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone (GBL), and mixtures thereof. Where an acid labile group of acetal form is used, a high boiling alcohol solvent such as diethylene glycol, propylene glycol, glycerol, 1,4-butanediol or 1,3-butanediol may be added to accelerate deprotection reaction of acetal.

Of the above organic solvents, it is recommended to use 1-ethoxy-2-propanol, PGMEA, PGME, cyclohexanone, EL, GBL, and mixtures thereof.

In the resist composition, the organic solvent (C) is preferably used in an amount of 200 to 10,000 parts, more preferably 400 to 5,000 parts by weight per 80 parts by weight of the base polymer (B). The organic solvent may be used alone or in admixture.

### (D) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (D1), repeat units having the formula (D2), repeat units having the formula (D3), and repeat units having the formula (D4), and may contain repeat units of at least one type selected from repeat units having the formula (D5) and repeat units having the formula (D6). It is noted that repeat units having formulae (D1), (D2), (D3), (D4), (D5), and (D6) are also referred to as repeat units D1, D2, D3, D4, D5, and D6, respectively, hereinafter. Since the fluorinated polymer also has a surface-active function, it can prevent insoluble residues from re-depositing onto the substrate during the development step and is thus effective for preventing development defects.

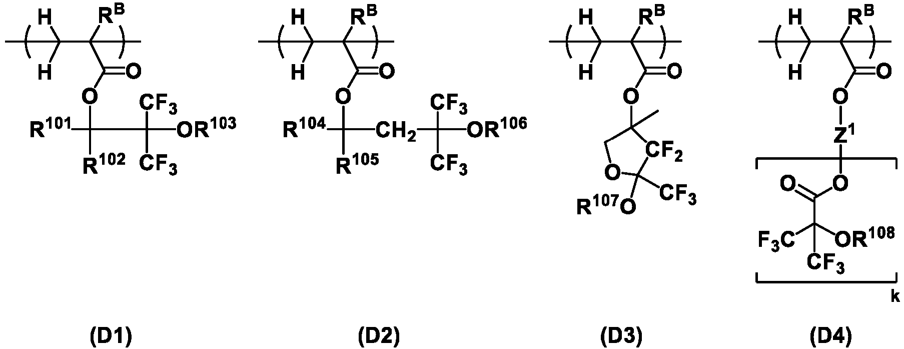

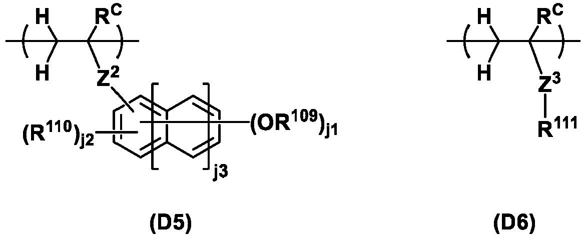

In formulae (D1) to (D6), j 1 is an integer of 1 to 3, j2 is an integer satisfying: 0 ≤ j2 ≤ 5+2(j3)-j1, j3 is 0 or 1, and k is an integer of 1 to 3. R^{B} is each independently hydrogen, fluorine, methyl or trifluoromethyl. R^{C} is each independently hydrogen or methyl. R¹⁰¹, R¹⁰², R¹⁰⁴ and R¹⁰⁵ are each independently hydrogen or a C₁-C₁₀ saturated hydrocarbyl group. R¹⁰³, R¹⁰⁶, R¹⁰⁷ and R¹⁰⁸ are each independently hydrogen, a C₁-C₁₅ hydrocarbyl group or fluorinated hydrocarbyl group, or an acid labile group. An ether bond or carbonyl moiety may intervene in a carbon-carbon bond in the hydrocarbyl groups or fluorinated hydrocarbyl groups represented by R¹⁰³, R¹⁰⁶, R¹⁰⁷ and R¹⁰⁸. R¹⁰⁹ is hydrogen or a C₁-C₅ straight or branched hydrocarbyl group in which a heteroatom-containing moiety may intervene in a carbon-carbon bond. R¹¹⁰ is a C₁-C₅ straight or branched hydrocarbyl group in which a heteroatom-containing moiety may intervene in a carbon-carbon bond. R¹¹¹ is a C₁-C₂₀ saturated hydrocarbyl group in which at least one hydrogen is substituted by fluorine and some constituent -CH₂- may be replaced by an ester bond or ether bond. Z¹ is a C₁-C₂₀ (k+1)-valent hydrocarbon group or C₁-C₂₀ (k+1)-valent fluorinated hydrocarbon group. Z² is a single bond, *-C(=O)-O- or *-C(=O)-NH- wherein * designates a point of attachment to the carbon atom in the backbone. Z³ is a single bond, -O-, *-C(=O)-O-Z³¹-Z^{32_} or *-C(=O)-NH-Z³¹-Z³²-, wherein Z³¹ is a single bond or a C₁-C₁₀ saturated hydrocarbylene group, Z³² is a single bond, ester bond, ether bond or sulfonamide bond, and * designates a point of attachment to the carbon atom in the backbone.

In formulae (D1) and (D2), the C₁-C₁₀ saturated hydrocarbyl group represented by R¹⁰¹, R¹⁰², R¹⁰⁴ and R¹⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (D1) to (D4), the C₁-C₁₅ hydrocarbyl group represented by R¹⁰³, R¹⁰⁶, R¹⁰⁷ and R¹⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

In formula (D4), examples of the C₁-C₂₀ (k+1)-valent hydrocarbon group Z¹ include the foregoing C₁-C₂₀ alkyl groups and C₃-C₂₀ cyclic saturated hydrocarbyl groups, with k number of hydrogen atoms being eliminated. Examples of the C₁-C₂₀ (k+1)-valent fluorinated hydrocarbon group Z¹ include the foregoing (k+1)-valent hydrocarbon groups in which at least one hydrogen atom is substituted by fluorine.

Examples of the repeat units D1 to D4 are given below, but not limited thereto. Herein R^{B} is as defined above.

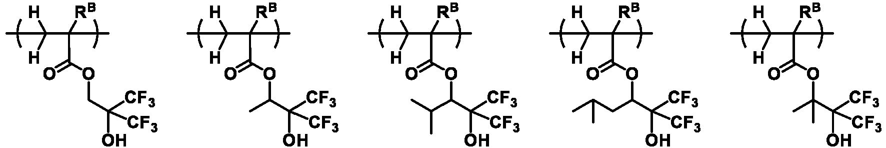

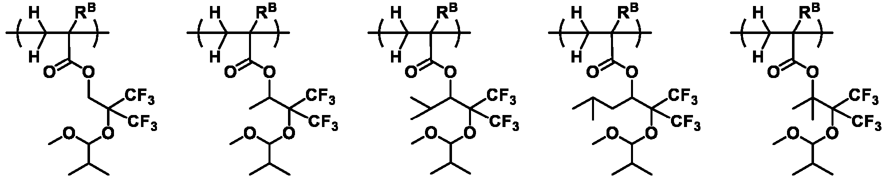

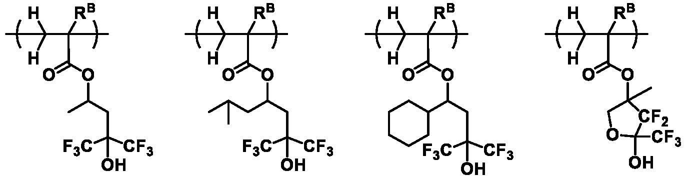

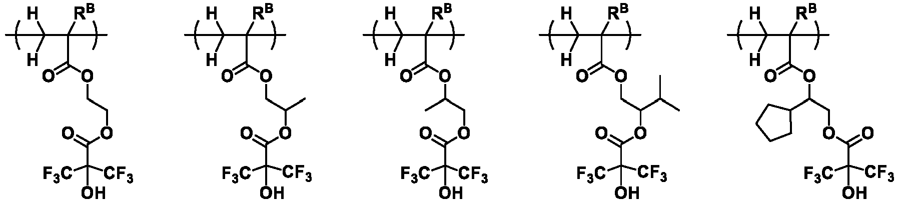

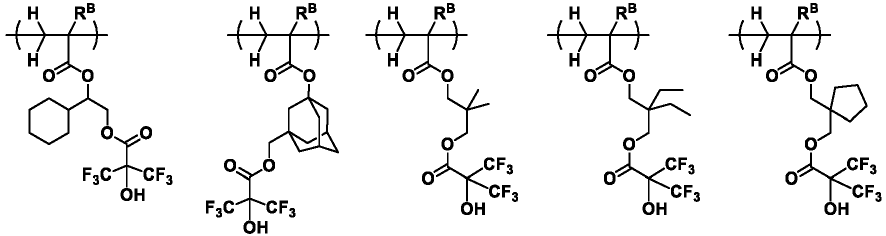

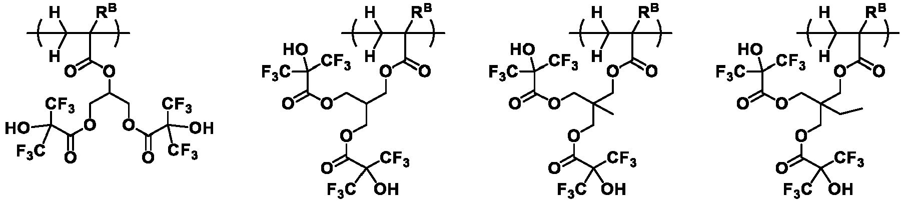

In formula (D5), examples of the C₁-C₅ hydrocarbyl groups R¹⁰⁹ and R¹¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (D5), -OR¹⁰⁹ is preferably a hydrophilic group. In this case, R¹⁰⁹ is preferably hydrogen or a C₁-C₅ alkyl group in which oxygen intervenes in a carbon-carbon bond.

In formula (D5), Z² is preferably *-C(=O)-O- or *-C(=O)-NH-. Also preferably R^{C} is methyl. The inclusion of carbonyl in Z² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

Examples of the repeat unit D5 are given below, but not limited thereto. Herein R^{C} is as defined above.

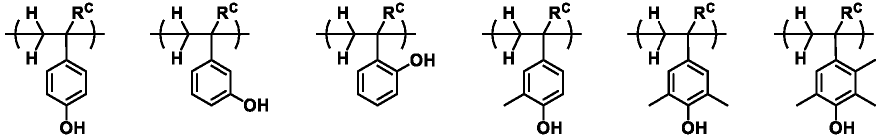

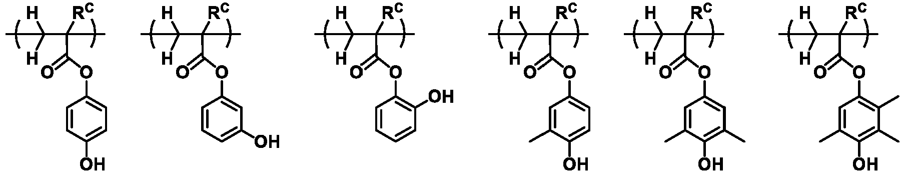

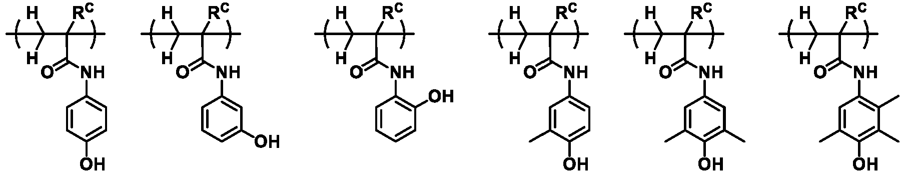

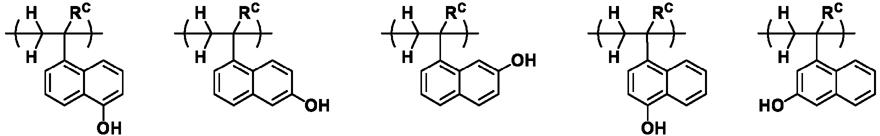

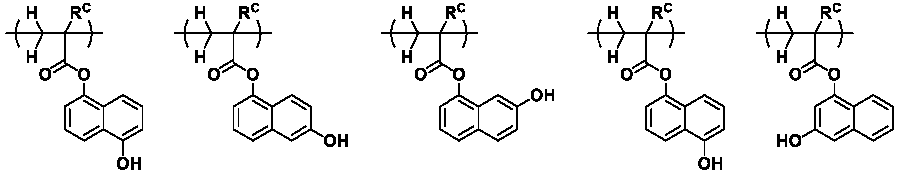

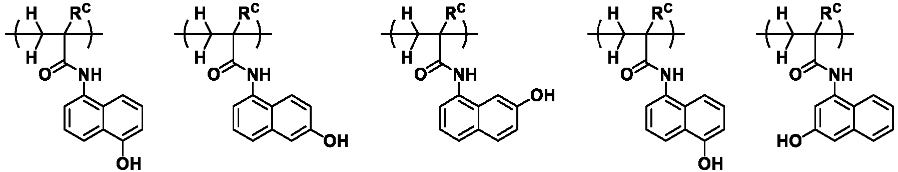

In formula (D6), the C₁-C₁₀ saturated hydrocarbylene group Z³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R¹¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit D6 are given below, but not limited thereto. Herein R^{C} is as defined above.

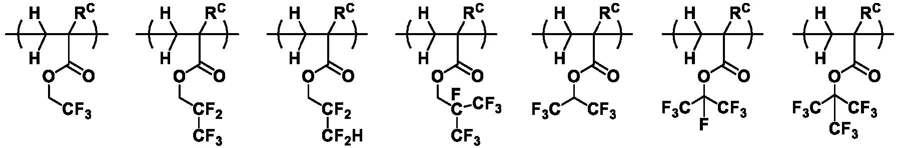

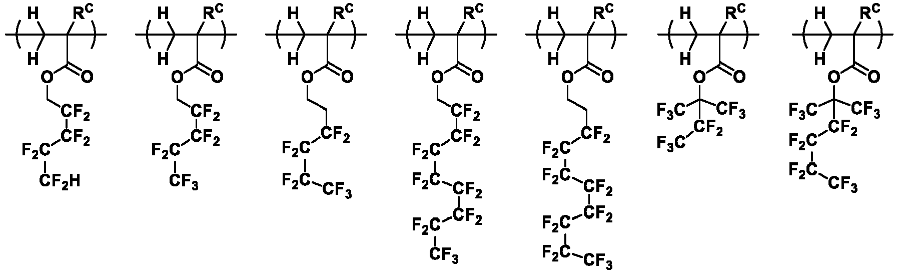

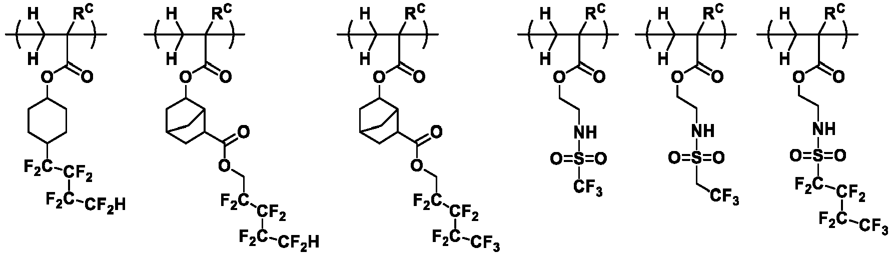

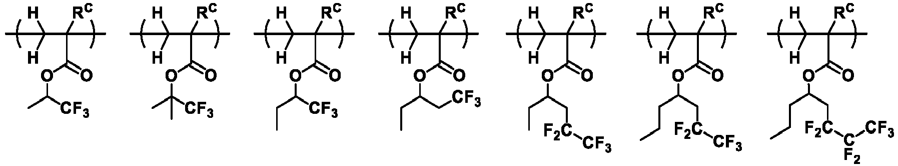

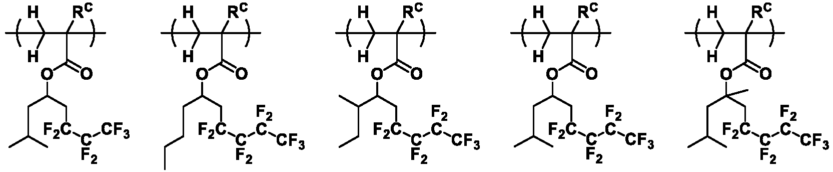

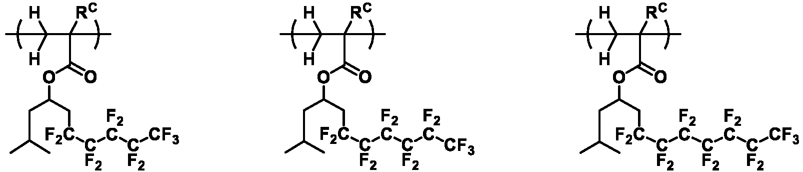

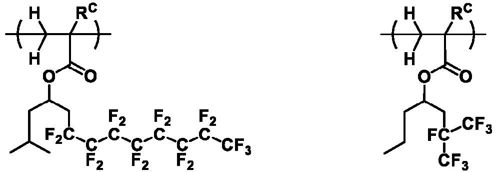

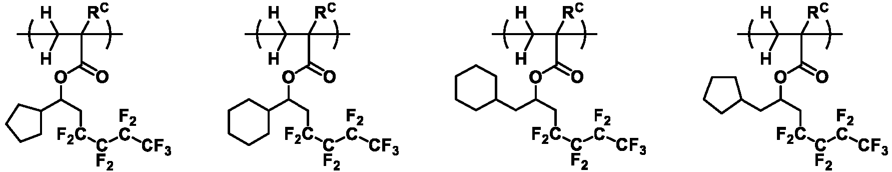

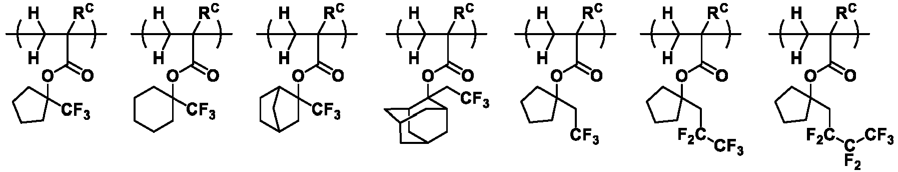

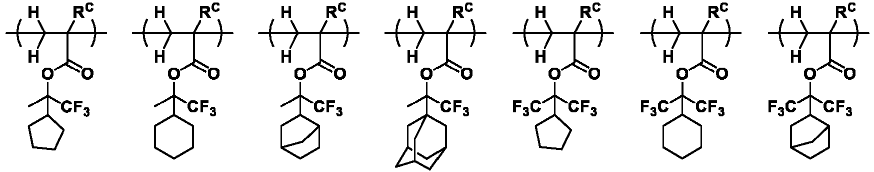

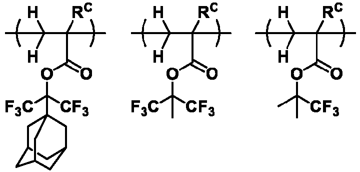

The content of repeat units D1 to D4 is preferably 15 to 95 mol%, more preferably 20 to 85 mol% based on the overall repeat units of the fluorinated polymer. The content of repeat unit D5 and/or D6 is preferably 5 to 85 mol%, more preferably 15 to 80 mol% based on the overall repeat units of the fluorinated polymer. Each of repeat units D1 to D6 may be used alone or in admixture.

The fluorinated polymer may comprise additional repeat units as well as the repeat units D1 to D6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of less than 2,000 helps acid diffusion, degrading resolution and detracting from age stability. A polymer with too high Mw has a reduced solubility in solvent, with a risk of leaving coating defects. The fluorinated polymer preferably has a dispersity (Mw/Mn) of 1.0 to 2.2, more preferably 1.0 to 1.7.

In the resist composition, the fluorinated polymer (D) is preferably used in an amount of 0.01 to 30 parts by weight, more preferably 0.1 to 20 parts by weight per 80 parts by weight of the base polymer (B). The fluorinated polymer may be used alone or in admixture.

### (E) Quencher

The chemically amplified positive resist composition preferably comprises a quencher as component (E). As used herein, the quencher refers to a compound capable of trapping an acid generated from the acid generator upon exposure. The quencher is effective for holding down the rate of diffusion of the acid (generated by the acid generator) in the resist film. Even when a substrate whose outermost surface is made of a chromium-containing material is used, the quencher is effective for suppressing the influence of the acid (generated in the resist film) on the chromium-containing material.

The quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy, ether bond, ester bond, lactone ring, cyano, or sulfonate ester group as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. The α-non-fluorinated carboxylic acid functions as a quencher because it does not induce substantial deprotection reaction.

Examples of the onium salt of α-non-fluorinated carboxylic acid include compounds having the formula (E1).

**R²⁰¹-CO₂⁻Mq_{A}⁺** **(E1)**

In formula (E1), R²⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R²⁰¹ may be saturated or unsaturated and straight, branched or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or trialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy moiety, cyano moiety, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (E1), Mq_{A}⁺ is an onium cation. The onium cation is preferably selected from sulfonium, iodonium and ammonium cations, more preferably sulfonium and iodonium cations. Exemplary sulfonium cations are as exemplified above for the sulfonium cation having formula (cation-1). Exemplary iodonium cations are as exemplified above for the iodonium cation having formula (cation-2).

Examples of the anion in the onium salt having formula (E1) are shown below, but not limited thereto.

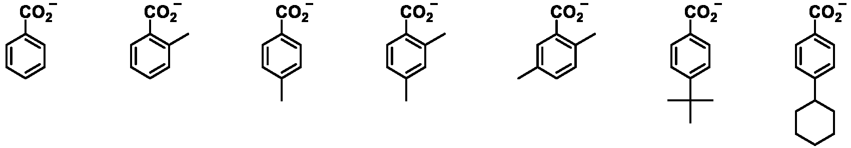

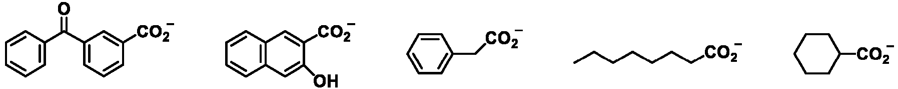

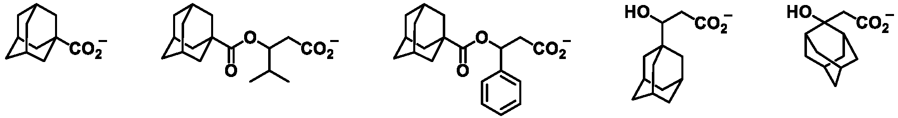

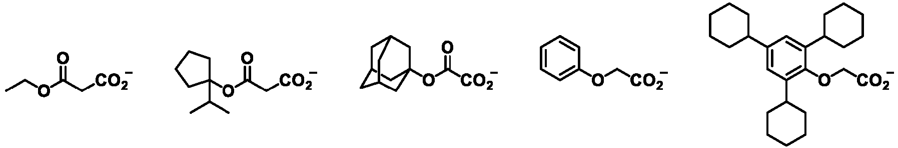

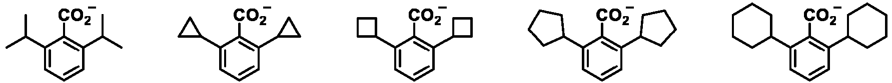

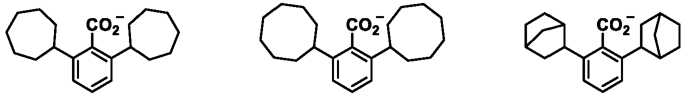

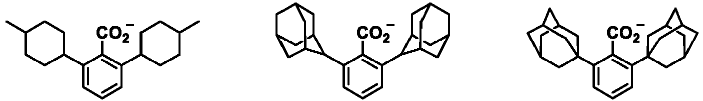

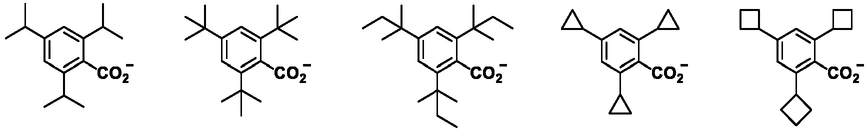

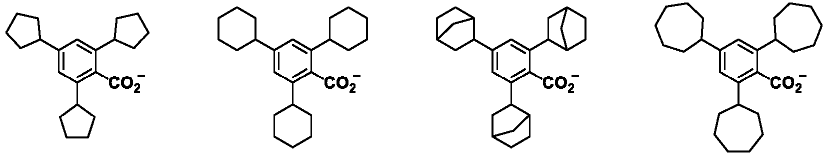

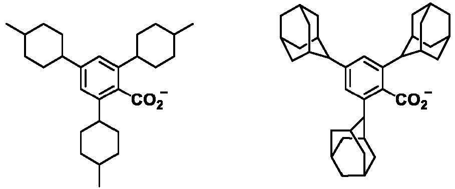

A sulfonium salt of iodized benzene ring-containing carboxylic acid having the formula (E2) is also useful as the quencher.

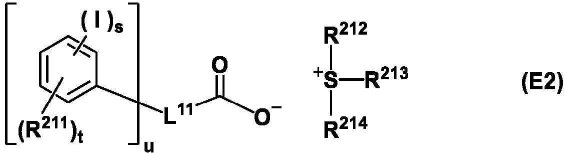

In formula (E2), s is an integer of 1 to 5, t is an integer of 0 to 3, s+t is from 1 to 5, and u is an integer of 1 to 3.

In formula (E2), R²¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy or C₁-C₄ saturated hydrocarbylsulfonyloxy group, in which some or all hydrogen may be substituted by halogen, or -N(R^{211A})-C(=O)-R^{211B}, or -N(R^{211A})-C(=O)-O-R^{211B}. R^{211A} is hydrogen or a C₁-C₆ saturated hydrocarbyl group. R^{211B} is a C₁-C₆ saturated hydrocarbyl or C₂-C₈ unsaturated aliphatic hydrocarbyl group. A plurality of R²¹¹ may be identical or different when t and/or u is 2 or 3.

In formula (E2), L¹¹ is a single bond or a C₁-C₂₀ (u+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (E2), R²¹², R²¹³ and R²¹⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also, R²¹² and R²¹³ may bond together to form a ring with the sulfur atom to which they are attached.

Examples of the compound having formula (E2) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (E3) is also useful as the quencher.

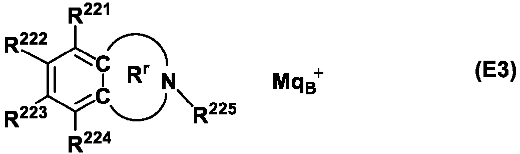

In formula (E3), R²²¹ to R²²⁴ are each independently hydrogen, -L¹²-CO₂⁻, or a C₁-C₂₀ hydrocarbyl group which may contain a heteroatom. R²²¹ and R²²², R²²² and R²²³, or R²²³ and R²²⁴ may bond together to form a ring with the carbon atom to which they are attached. L¹² is a single bond or a C₁-C₂₀ hydrocarbylene group which may contain a heteroatom. R²²⁵ is hydrogen or a C₁-C₂₀ hydrocarbyl group which may contain a heteroatom.

In formula (E3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L¹²-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (E3) has at least one -L¹²-CO₂⁻. That is, at least one of R²²¹ to R²²⁴ is -L¹²-CO₂⁻, and/or at least one of hydrogen atoms bonded to carbon atoms in the ring R^{r} is substituted by -L¹²-CO₂⁻.

In formula (E3), Mq_{B}⁺ is a sulfonium, iodonium or ammonium cation, with the sulfonium cation being preferred. Examples of the sulfonium cation are as exemplified above for the sulfonium cation having formula (cation-1).

Examples of the anion in the compound having formula (E3) are shown below, but not limited thereto.

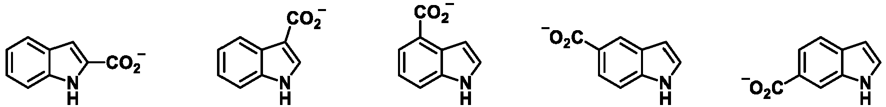

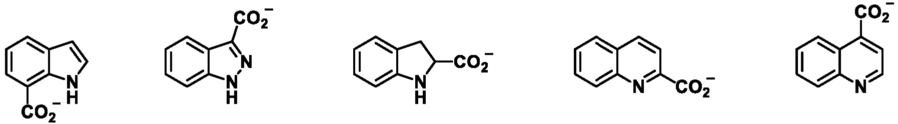

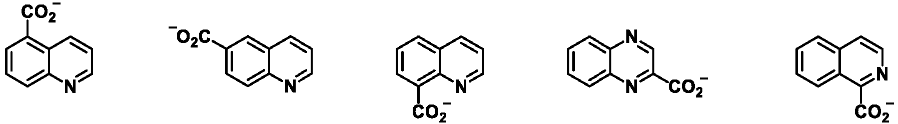

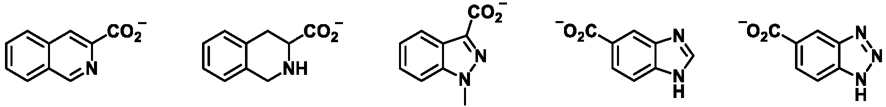

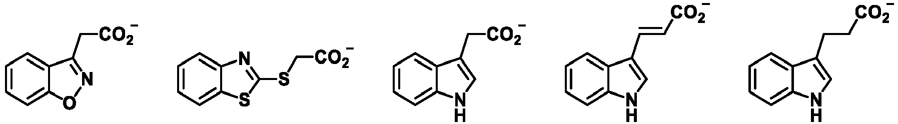

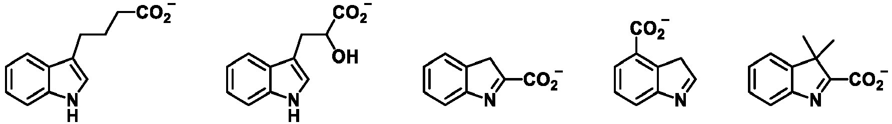

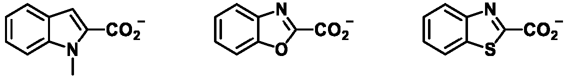

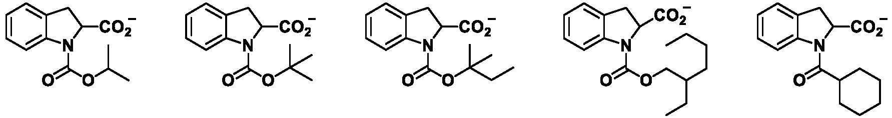

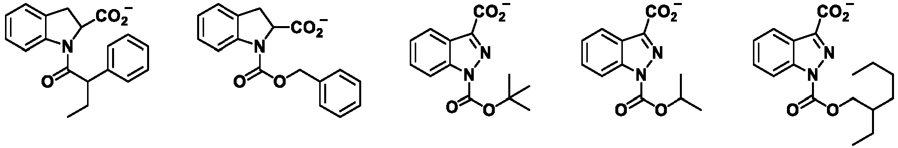

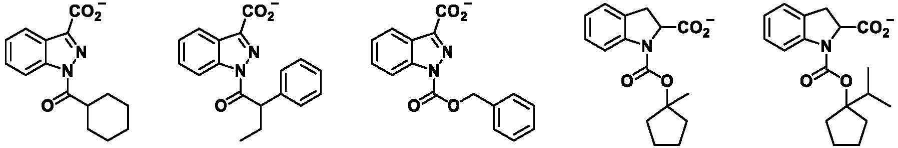

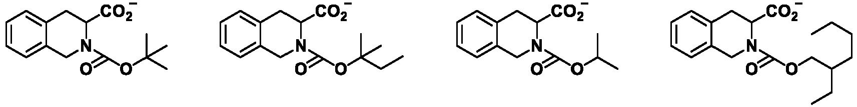

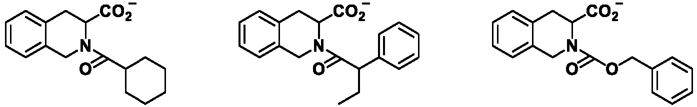

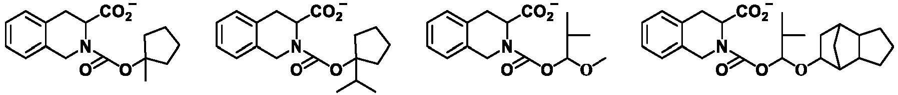

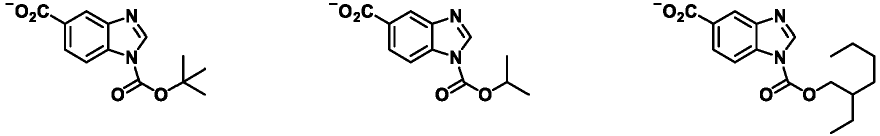

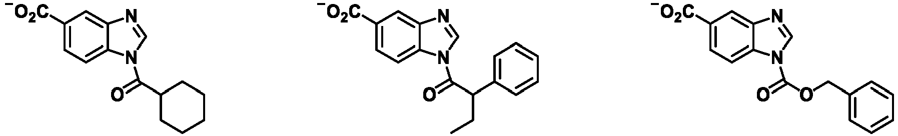

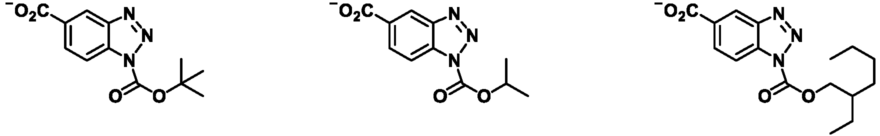

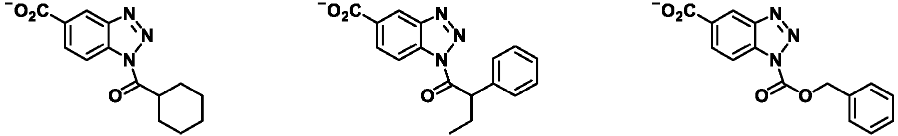

Weak acid betaine compounds are also useful as the quencher. Non-limiting examples thereof are shown below.

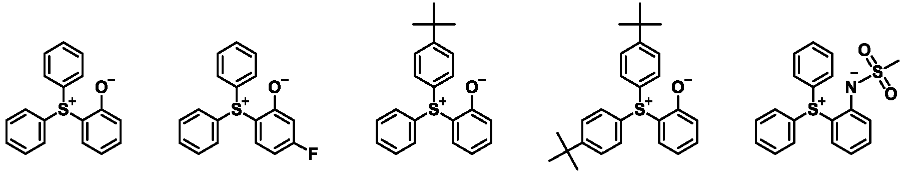

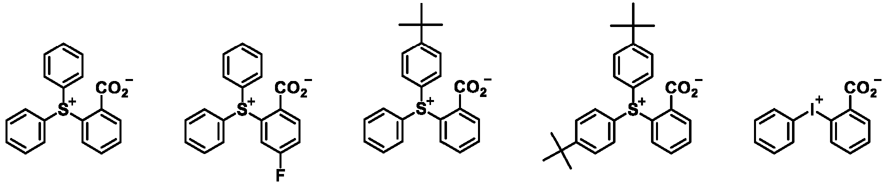

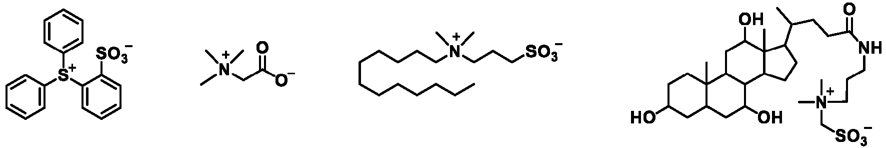

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When used, the quencher (E) is preferably added in an amount of 0 to 50 parts, more preferably 0.1 to 40 parts by weight per 80 parts by weight of the base polymer (B). The quencher may be used alone or in admixture.

When the chemically amplified positive resist composition contains both the PAG (A) and the quencher (E), the weight ratio of the PAG to the quencher, (A)/(E) is preferably less than 3/1, more preferably less than 2.5/1, even more preferably less than 2/1. As long as the weight ratio of the PAG to the quencher is in the range, the resist composition is able to fully suppress acid diffusion, leading to improved resolution and dimensional uniformity.

### (F) Other photoacid generator

In addition to the PAG in the form of the onium salt having formula (A), the chemically amplified positive resist composition may further comprise another photoacid generator (PAG). The other PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable other PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arylsulfonate and alkanesulfonate type PAGs are preferred because they generate acids having an appropriate strength to deprotect the acid labile group in repeat unit B2.

The preferred PAGs are salt compounds having a sulfonium anion of the structure shown below.

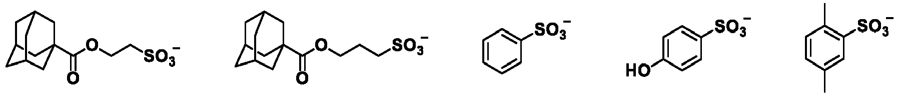

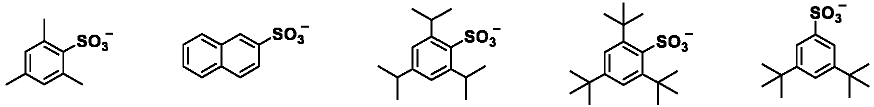

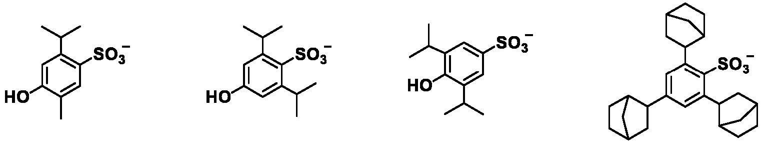

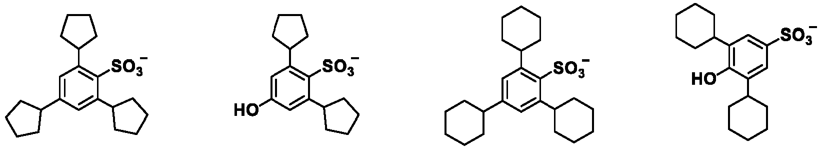

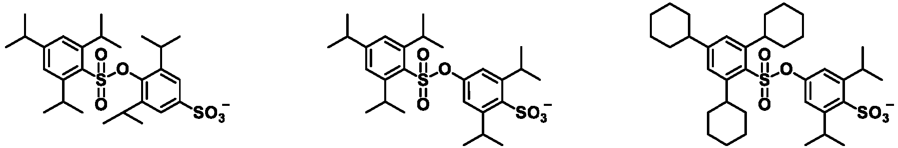

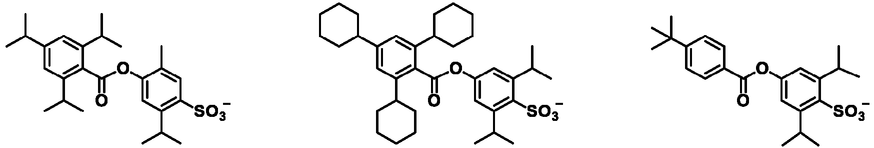

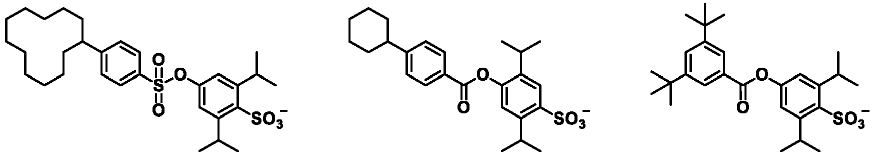

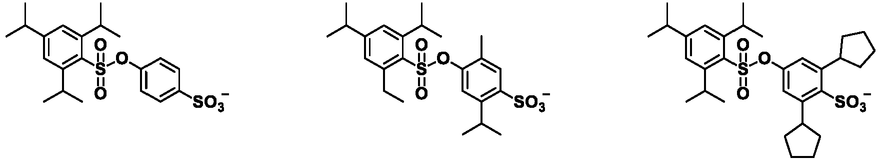

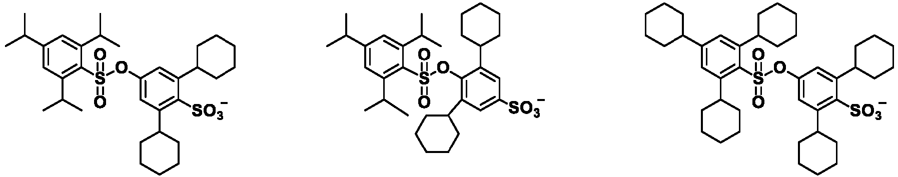

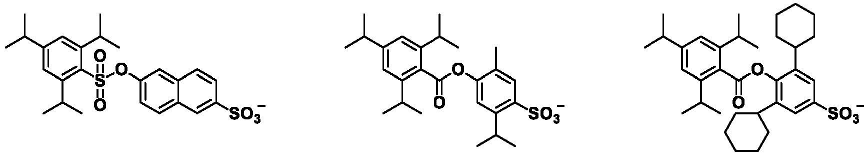

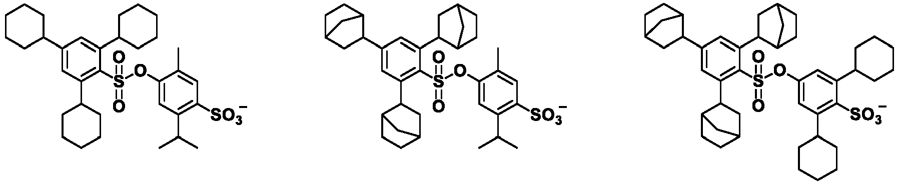

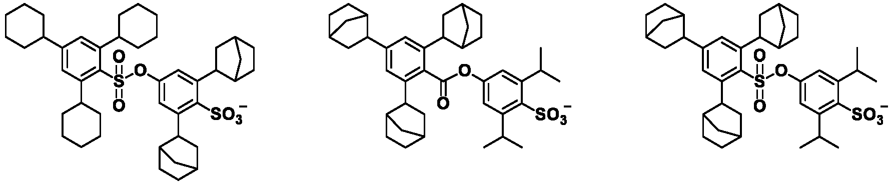

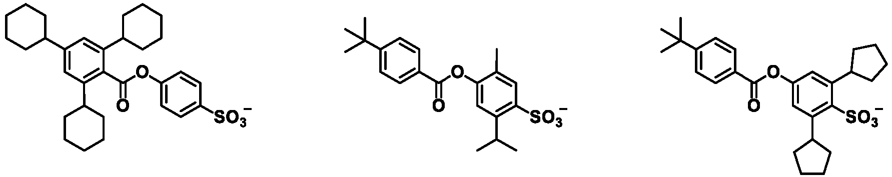

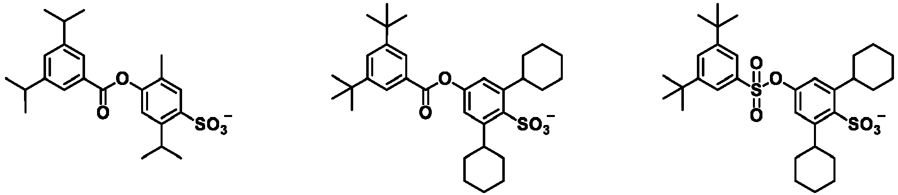

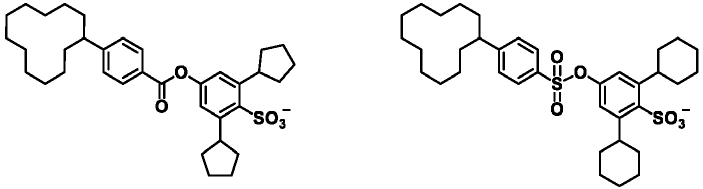

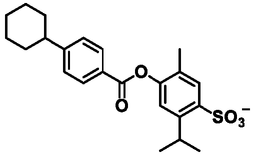

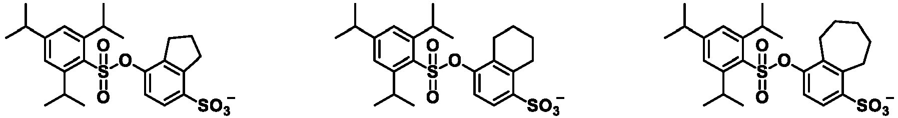

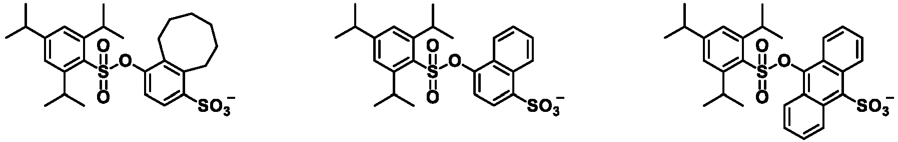

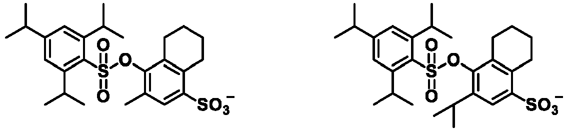

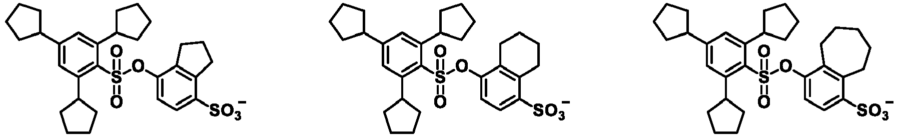

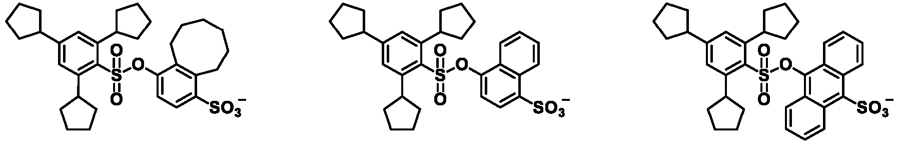

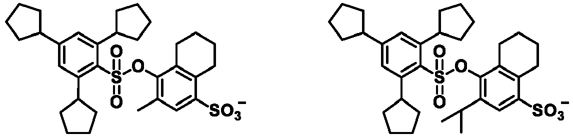

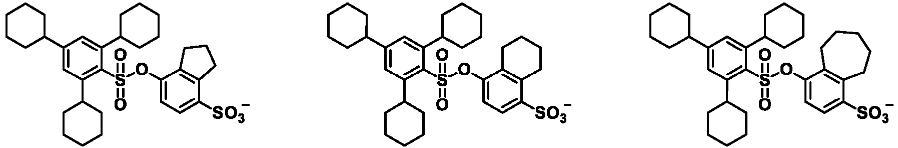

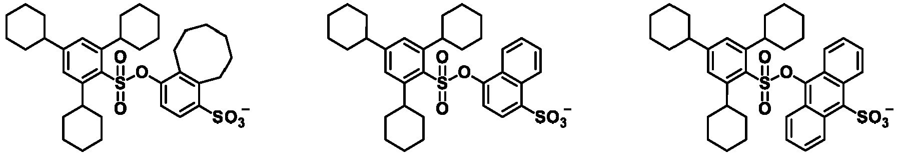

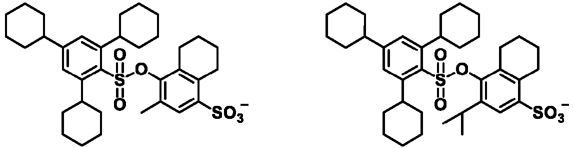

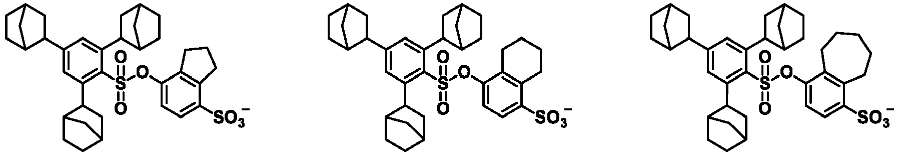

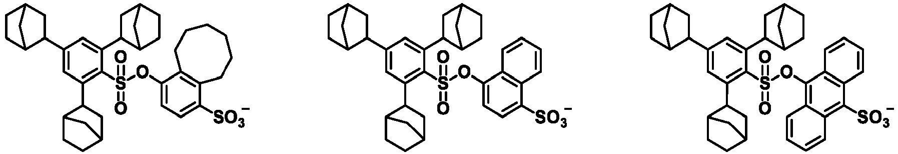

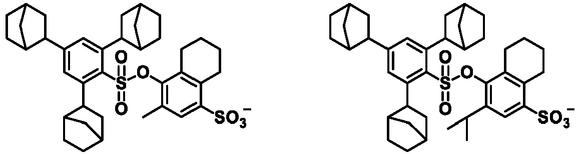

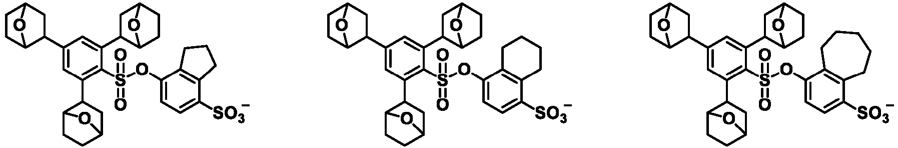

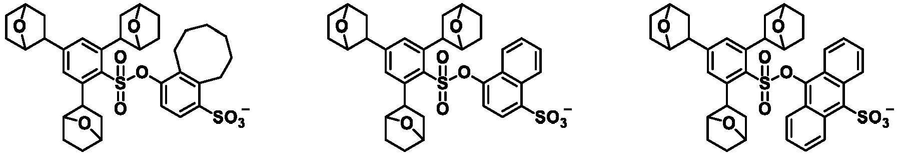

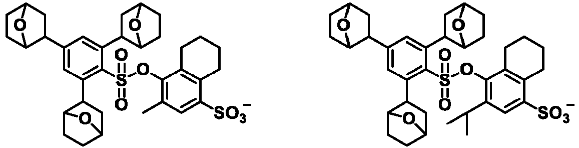

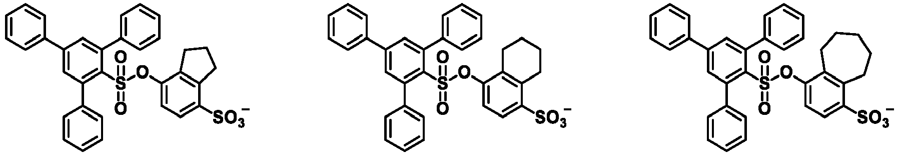

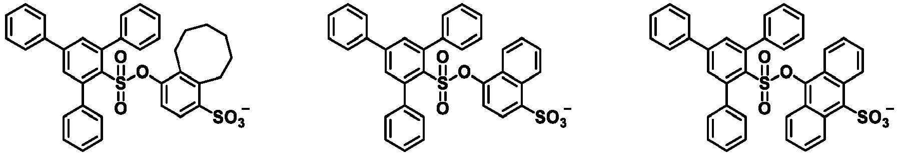

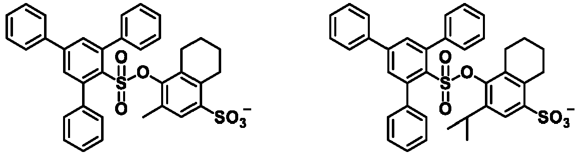

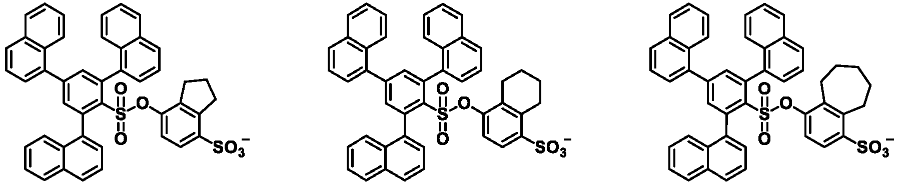

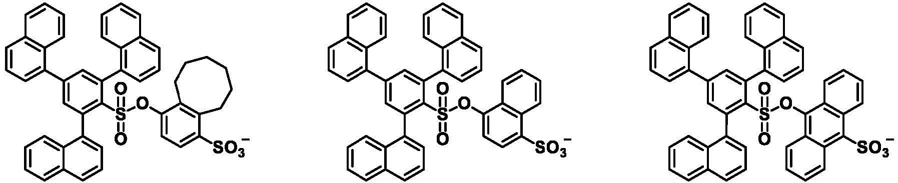

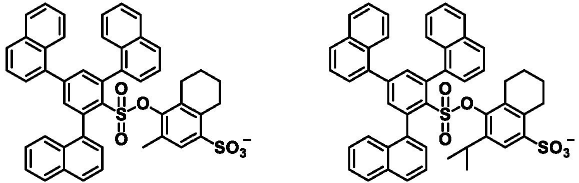

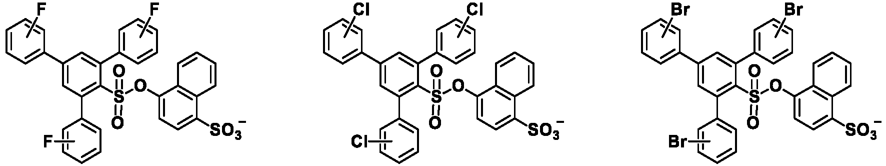

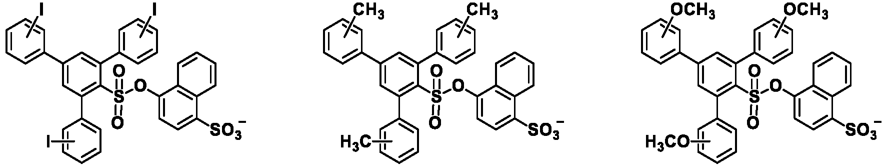

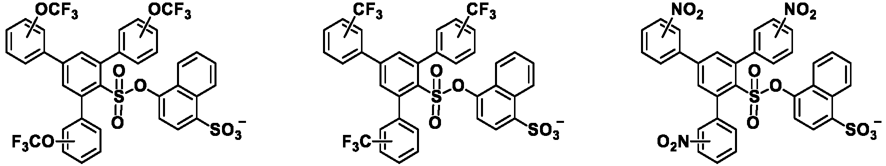

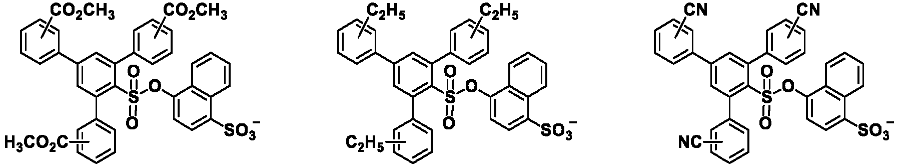

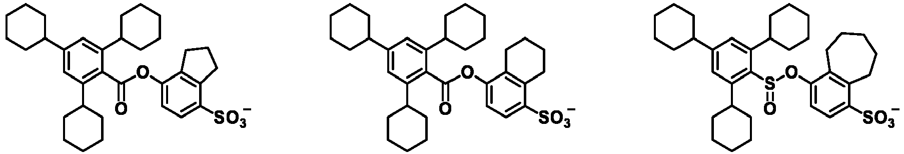

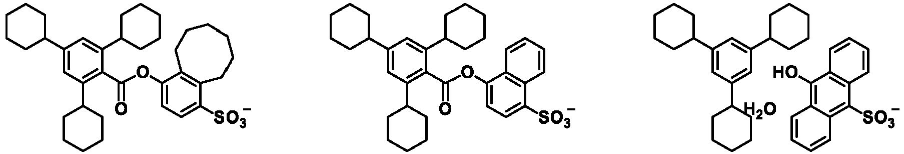

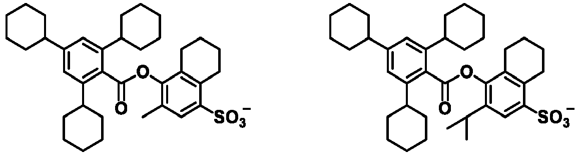

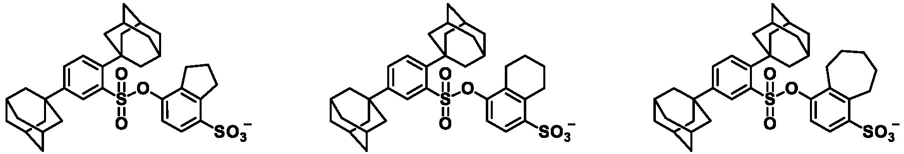

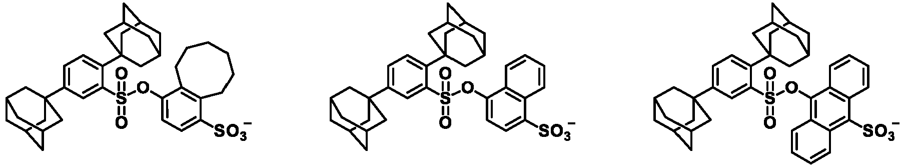

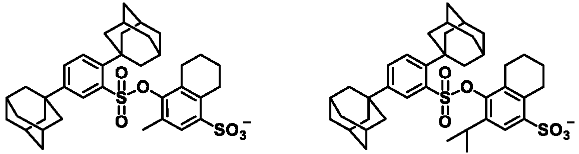

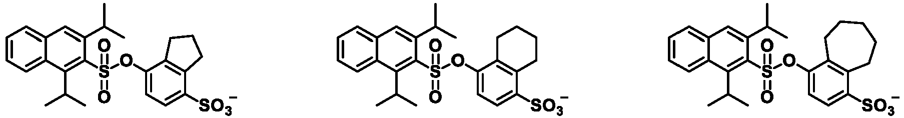

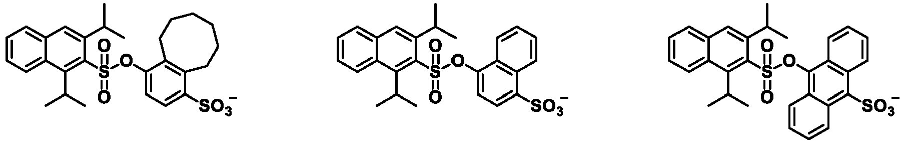

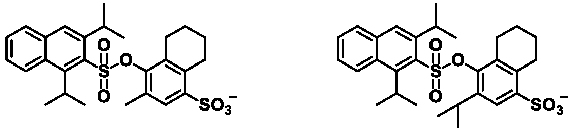

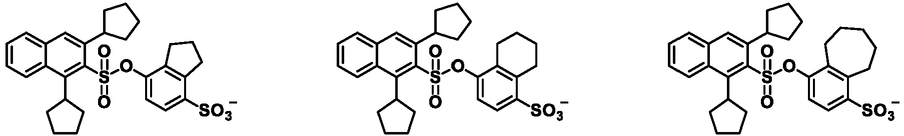

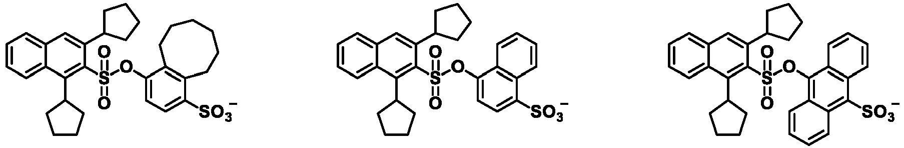

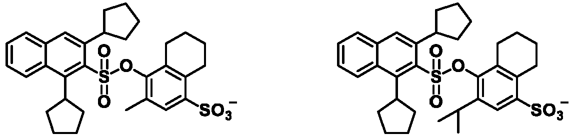

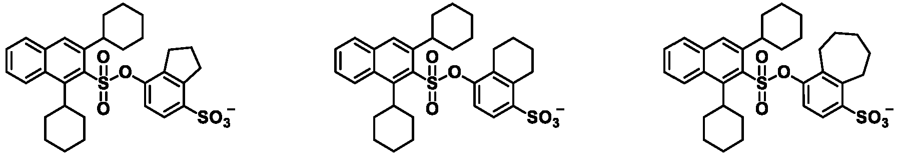

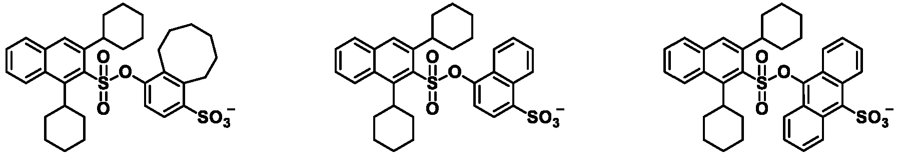

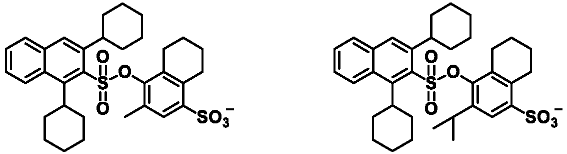

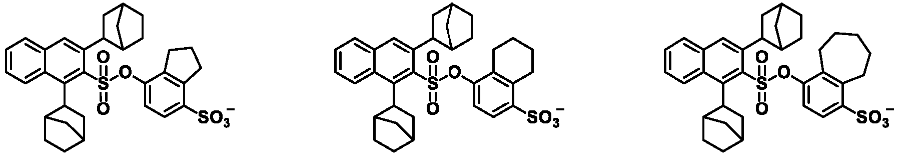

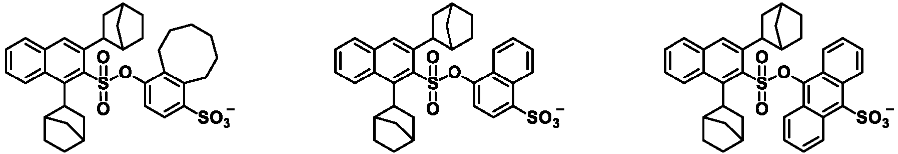

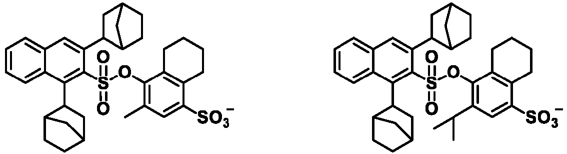

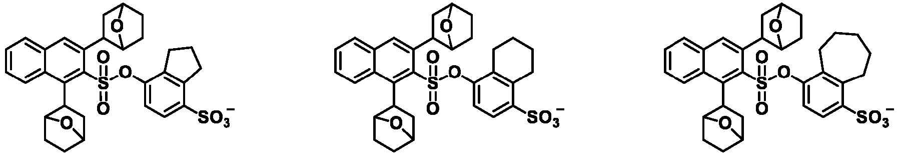

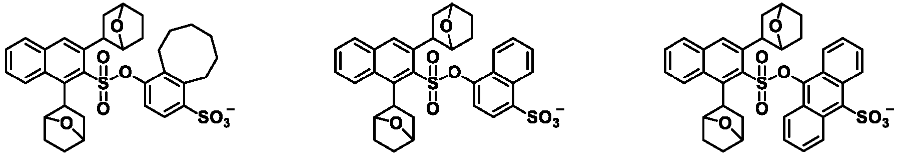

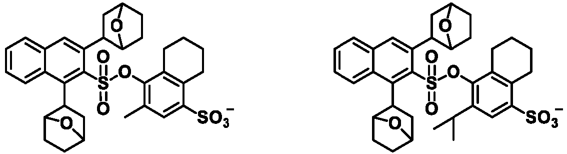

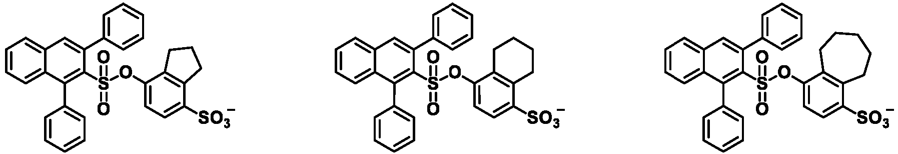

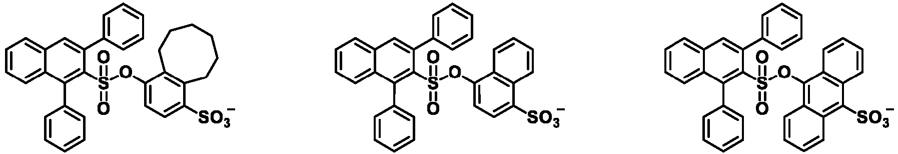

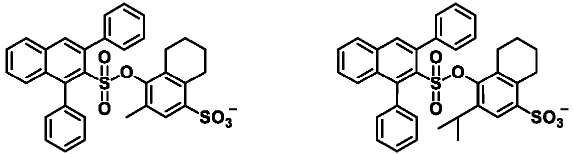

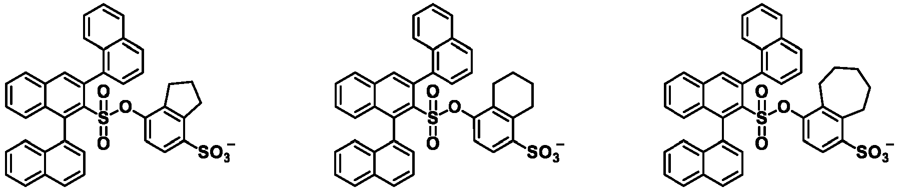

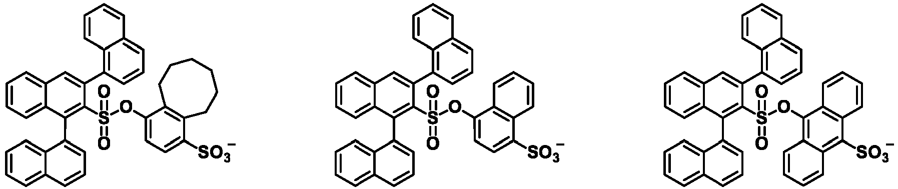

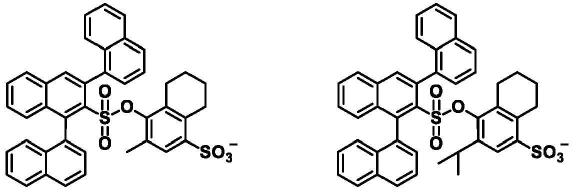

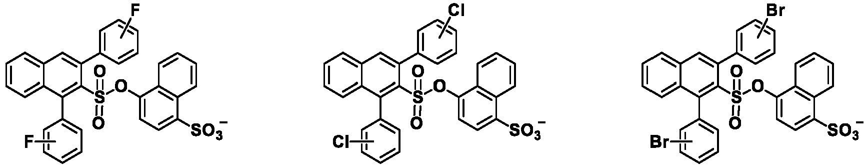

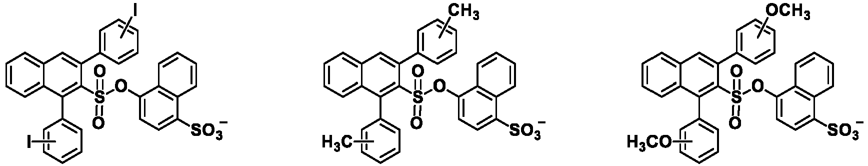

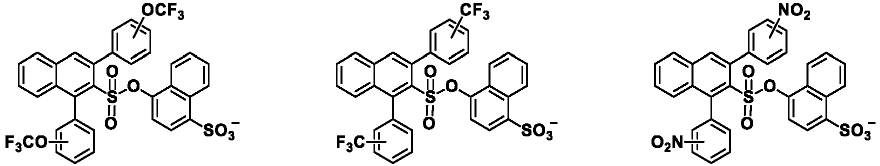

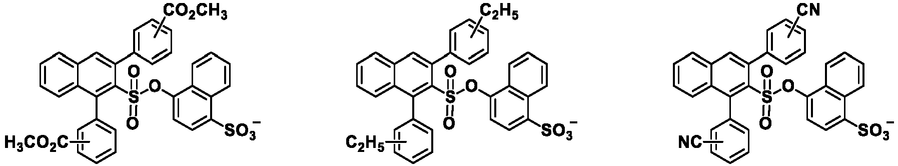

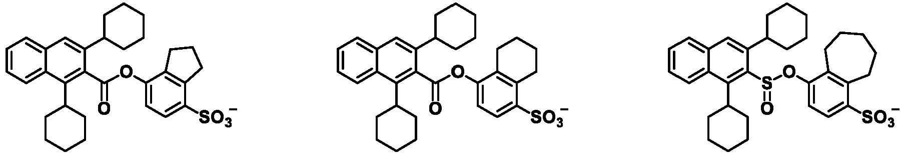

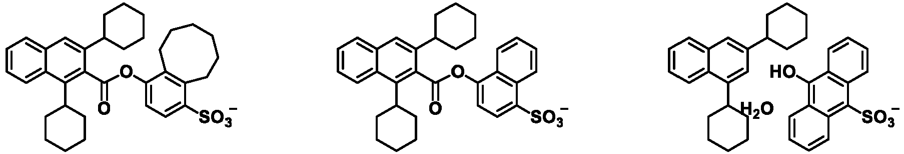

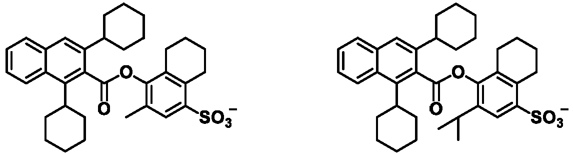

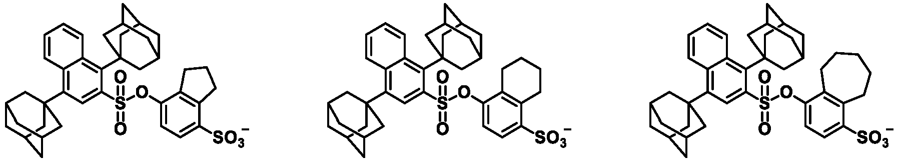

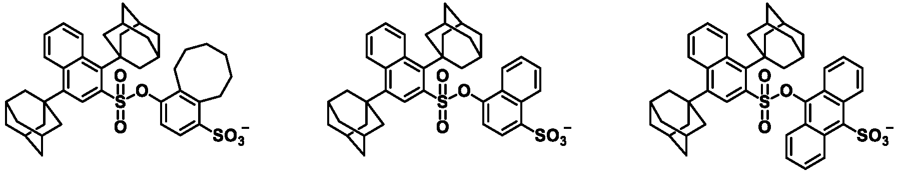

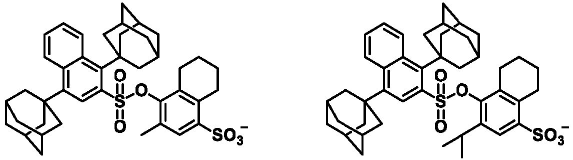

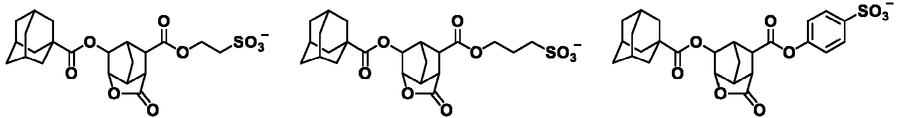

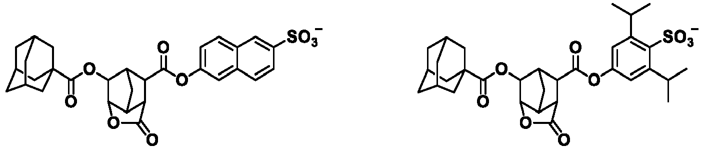

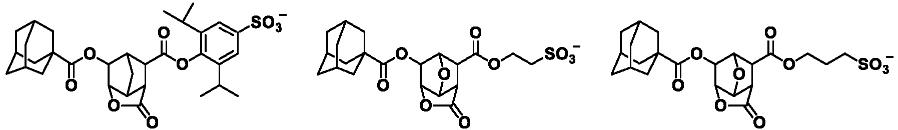

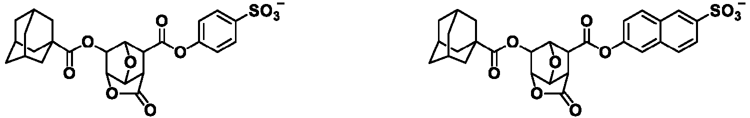

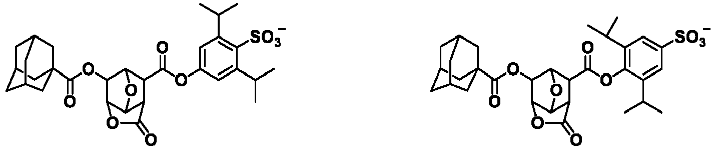

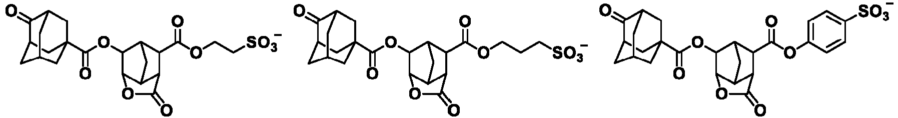

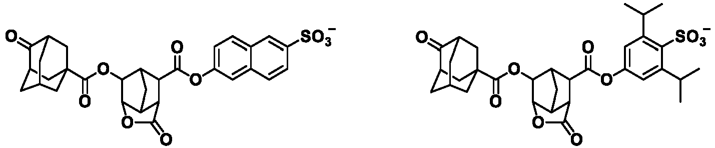

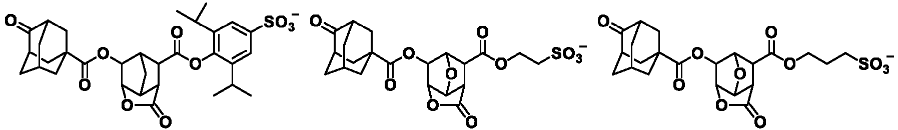

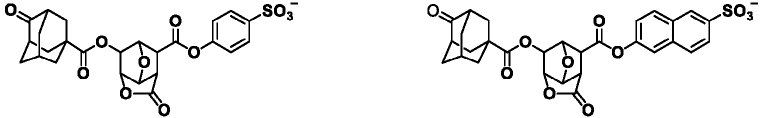

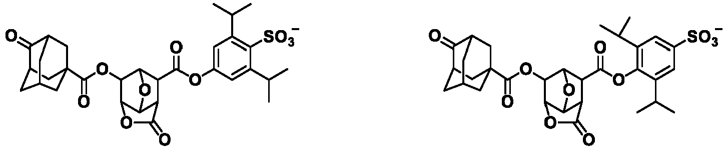

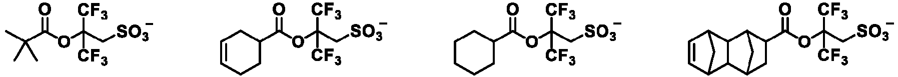

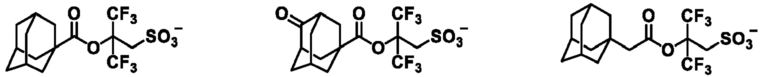

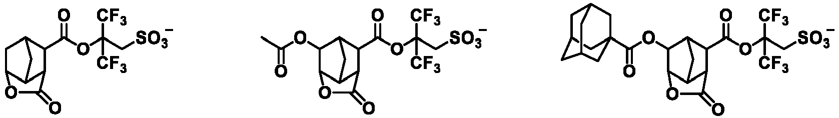

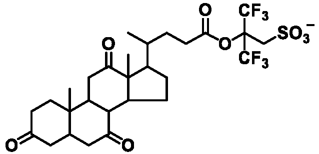

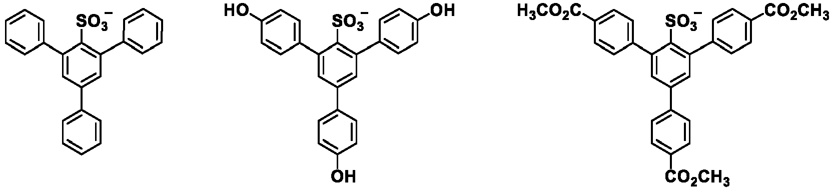

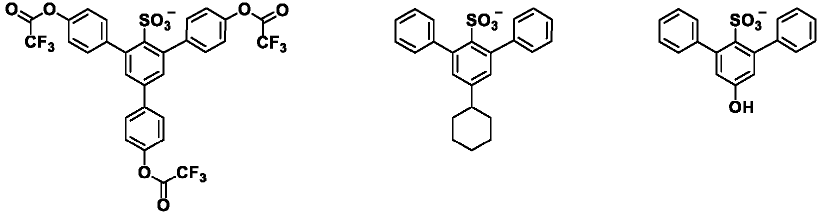

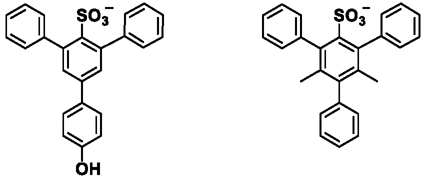

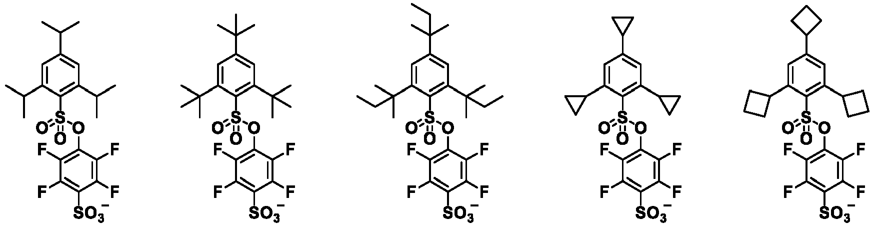

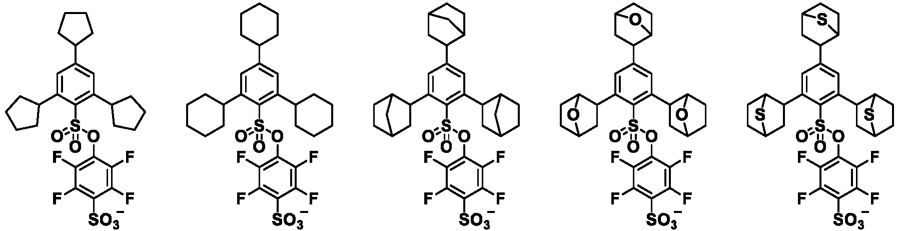

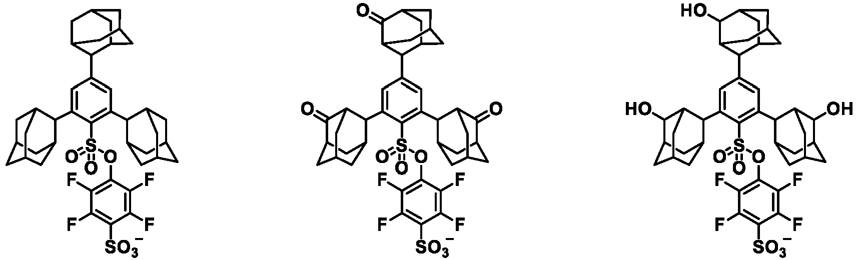

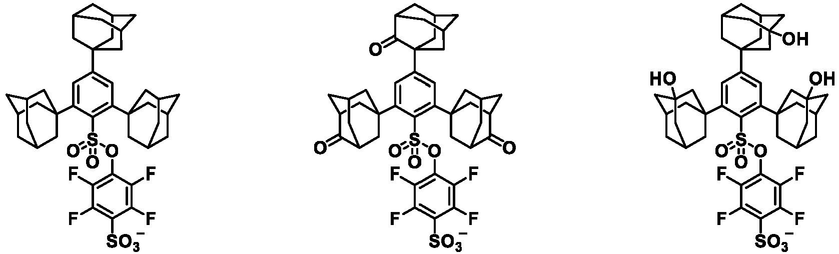

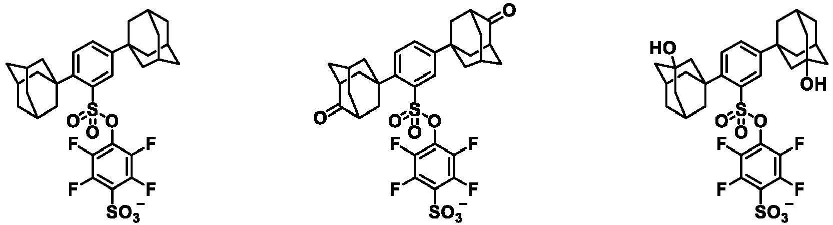

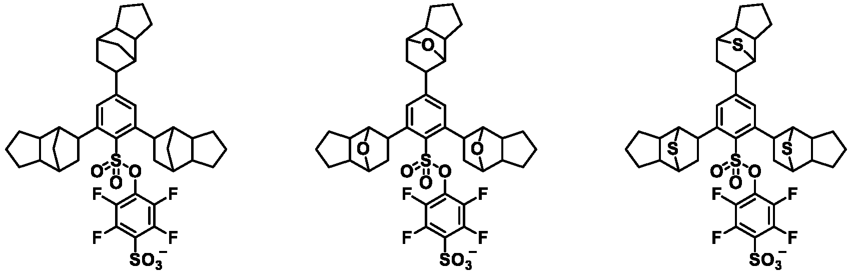

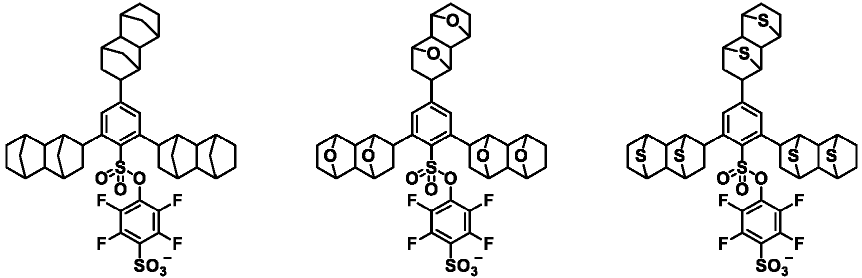

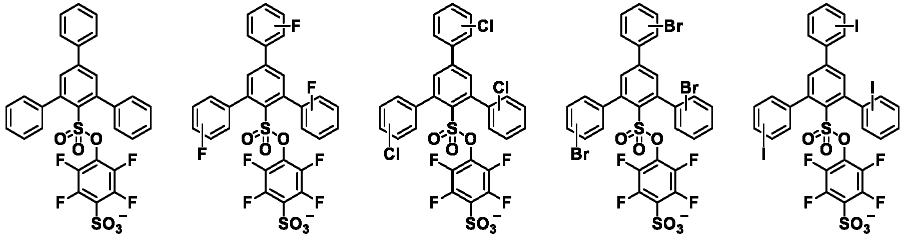

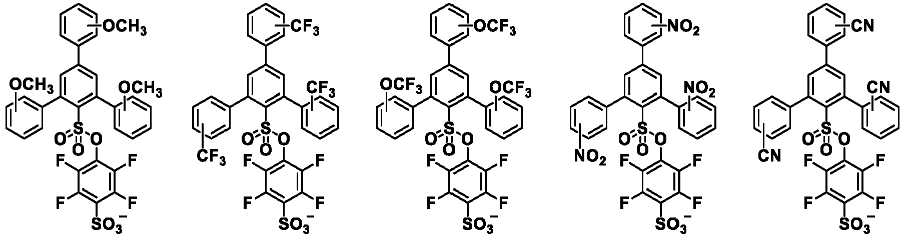

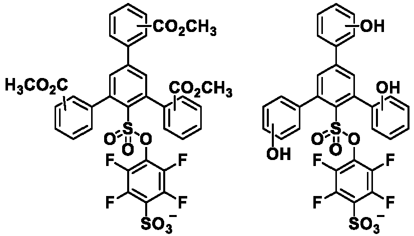

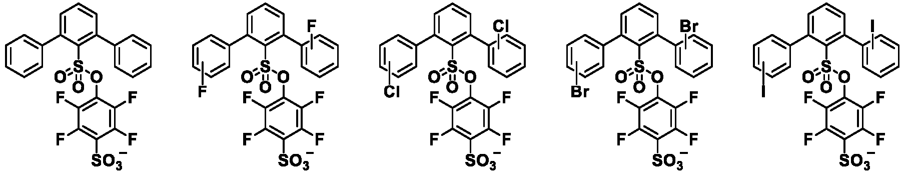

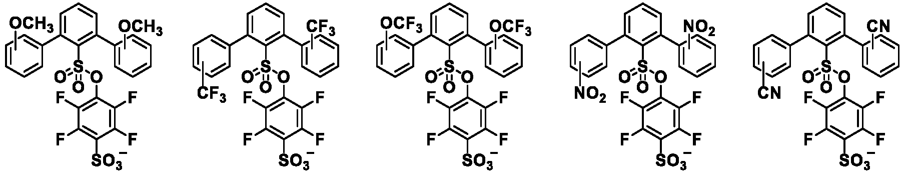

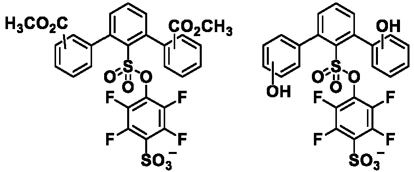

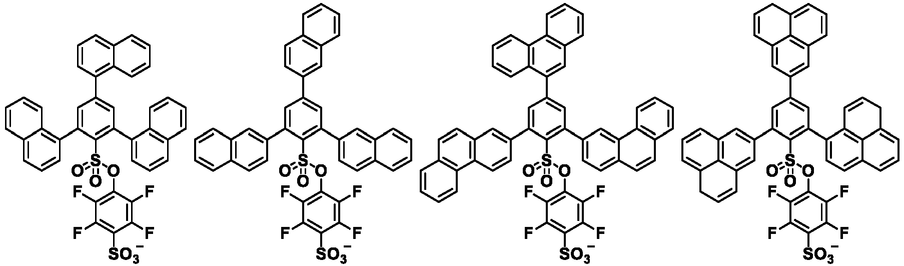

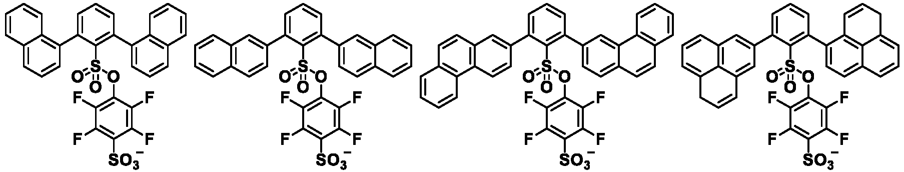

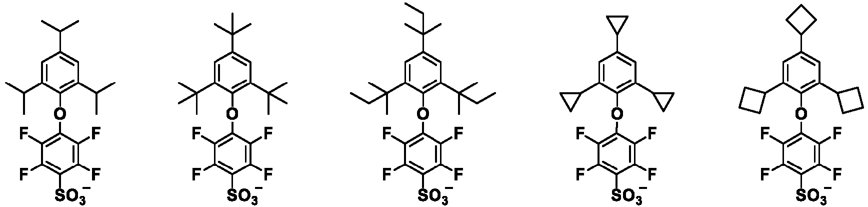

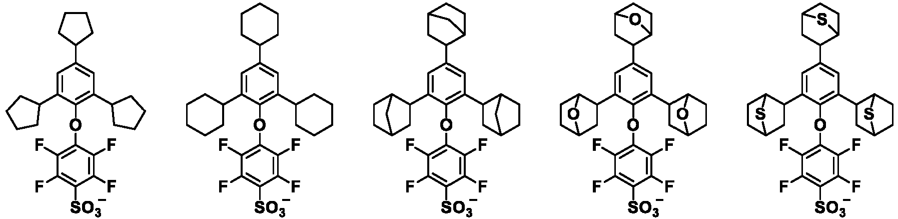

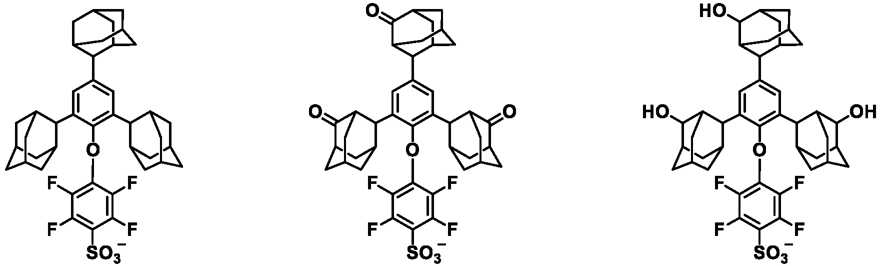

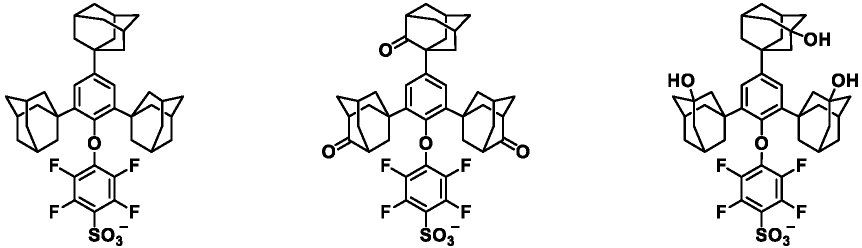

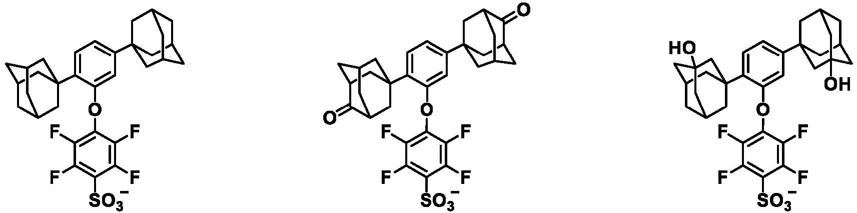

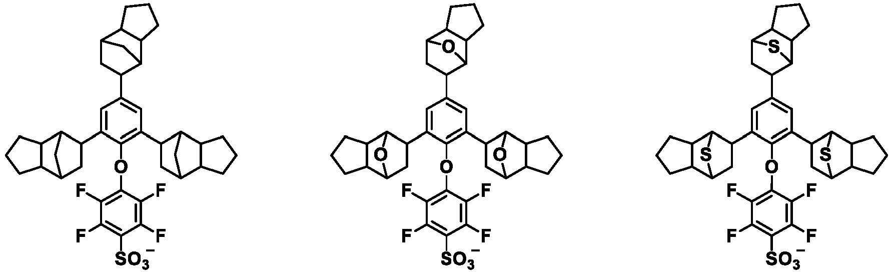

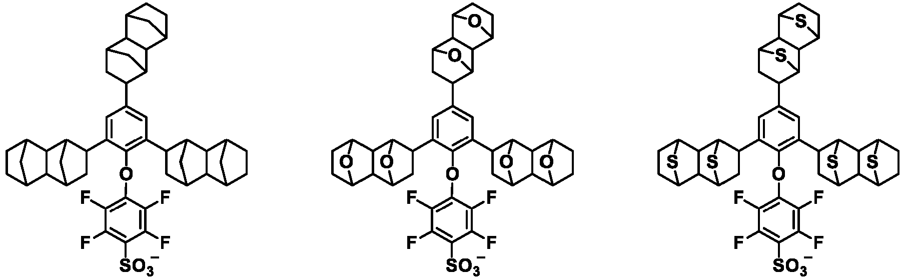

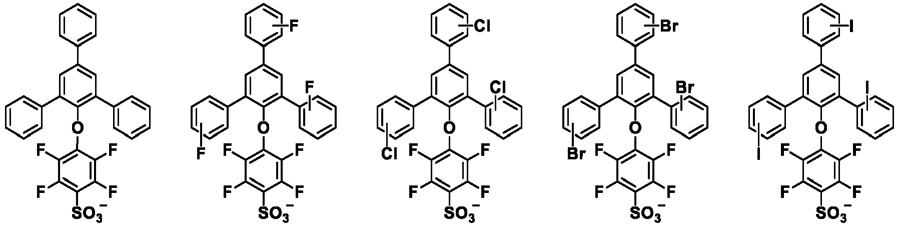

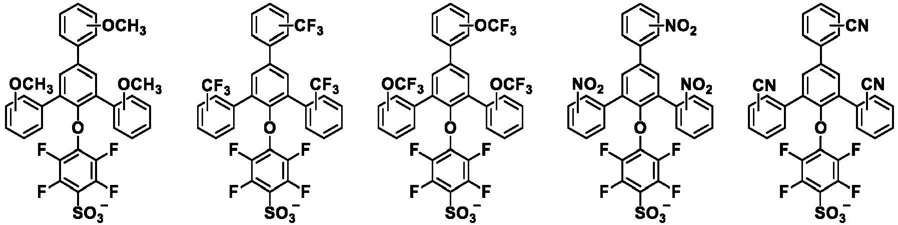

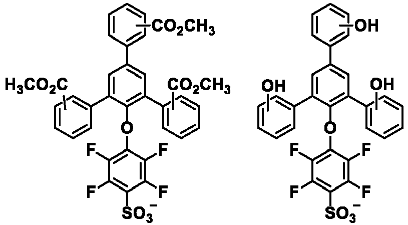

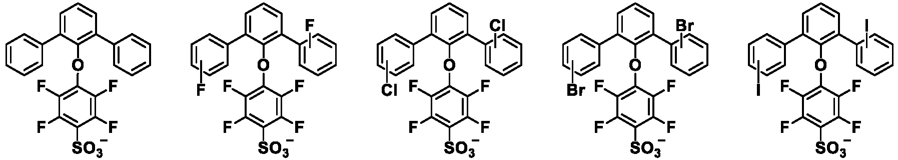

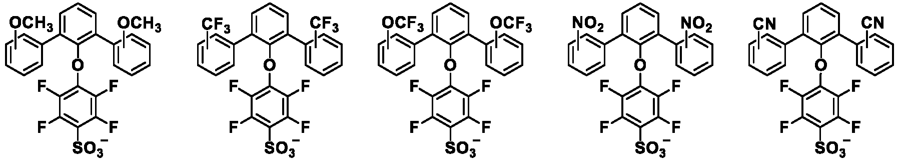

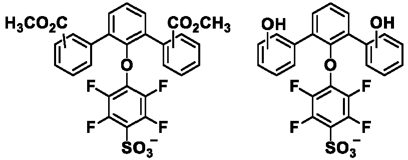

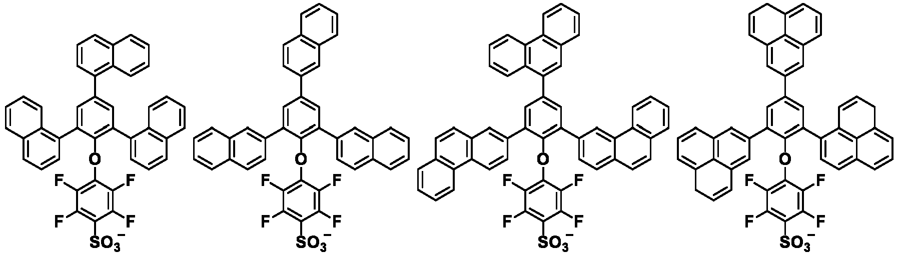

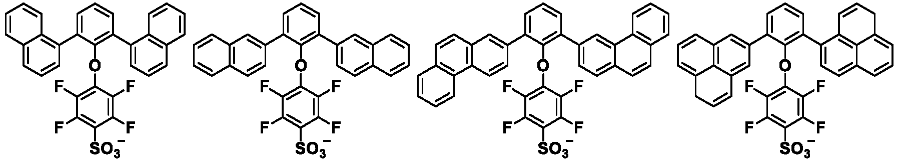

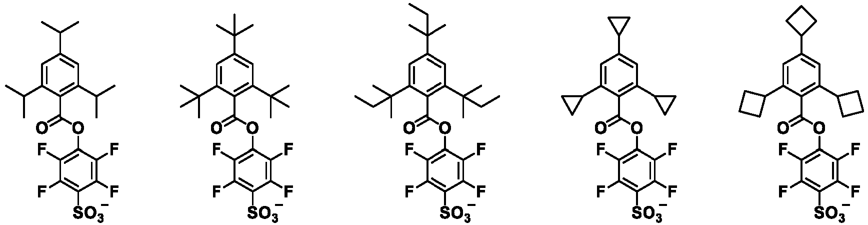

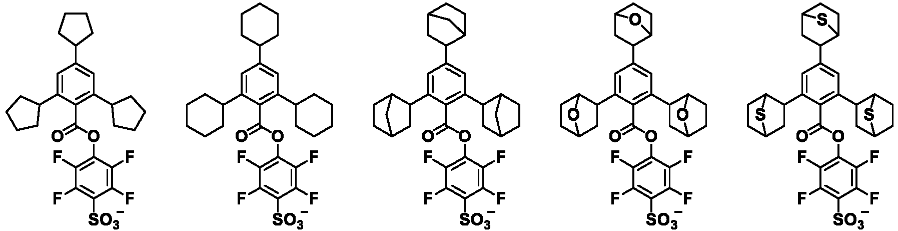

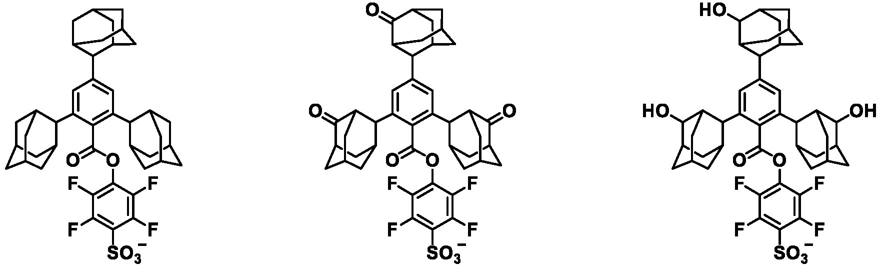

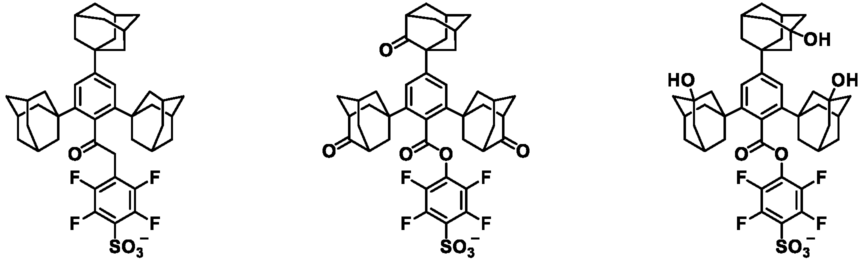

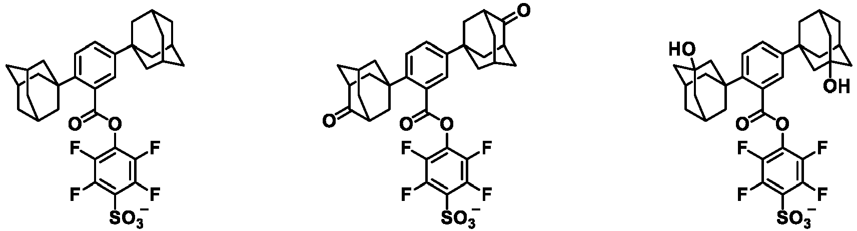

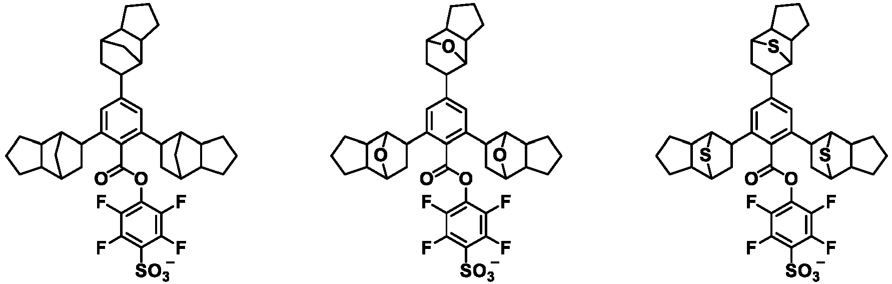

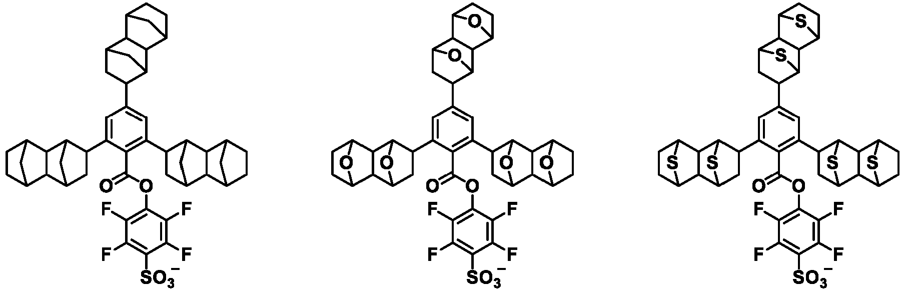

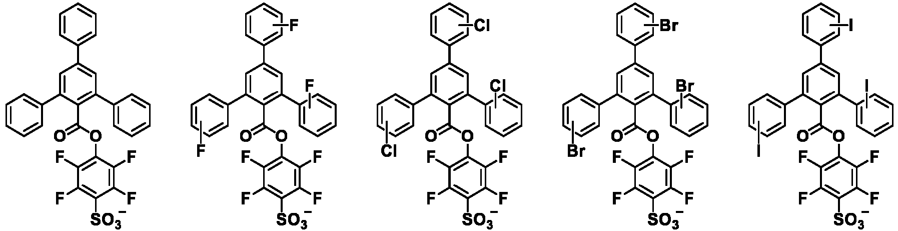

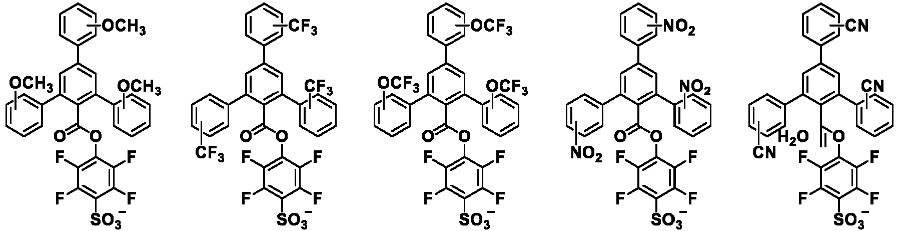

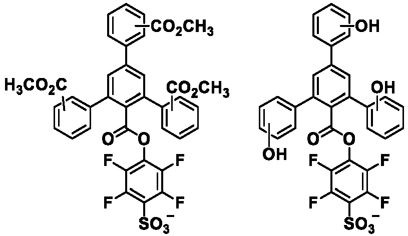

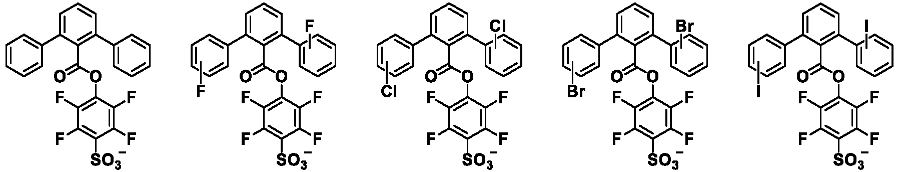

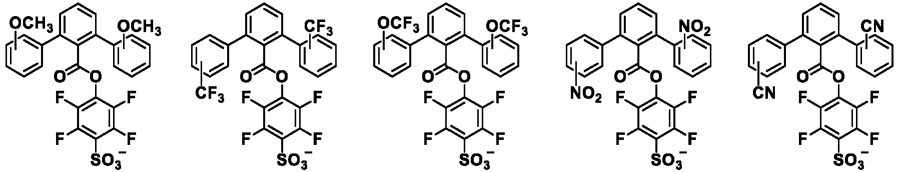

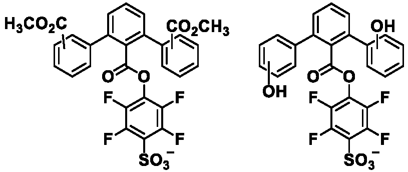

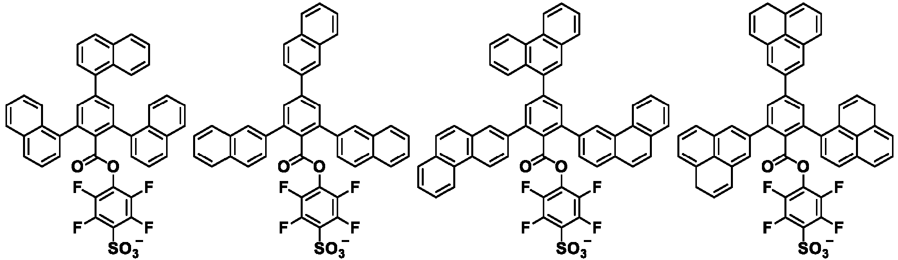

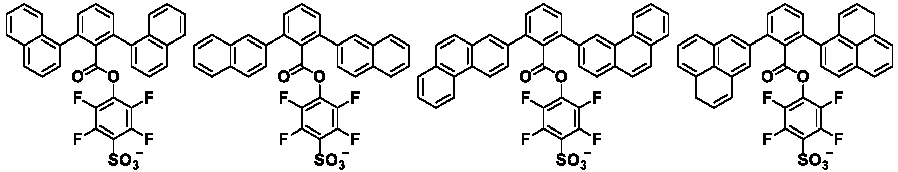

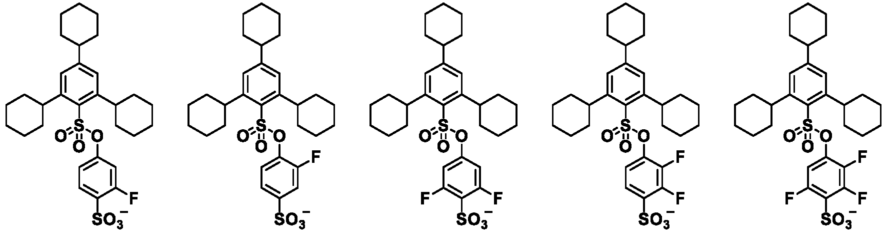

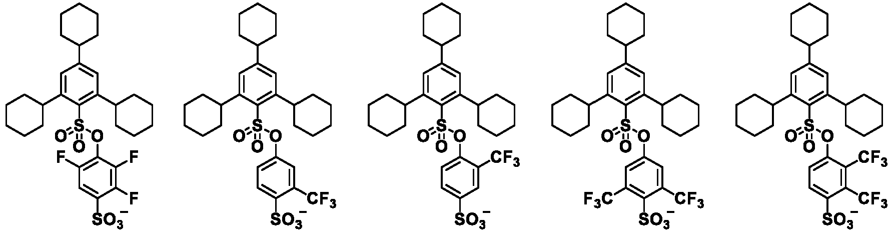

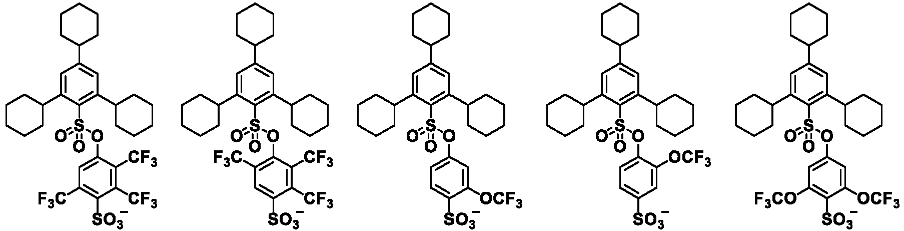

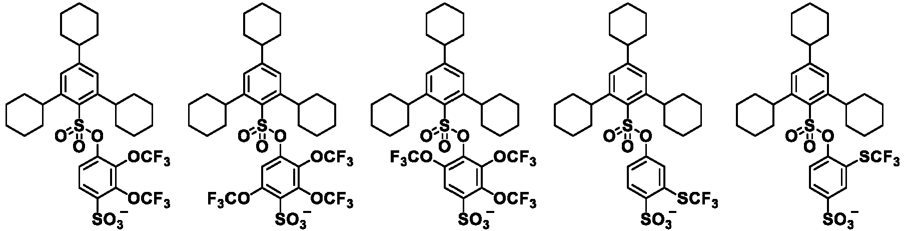

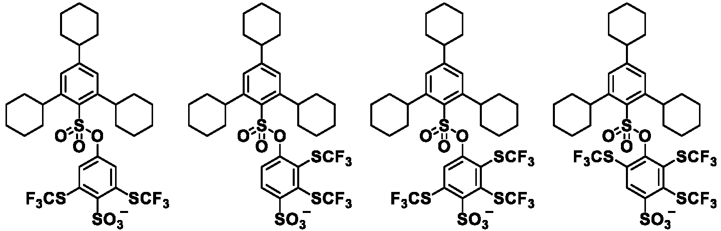

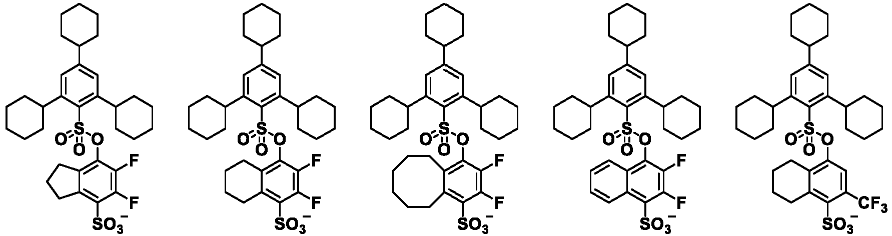

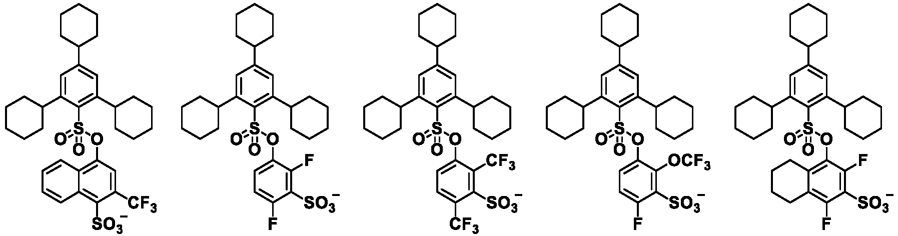

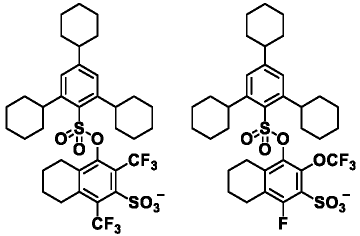

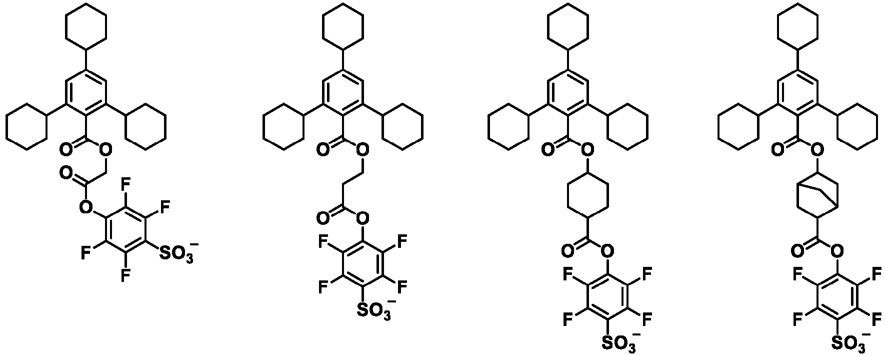

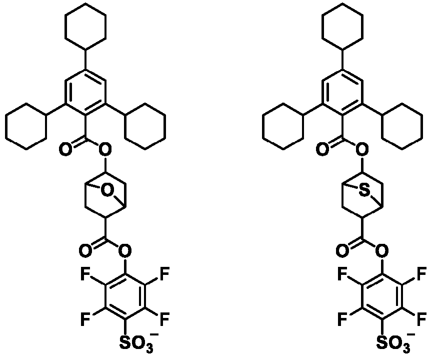

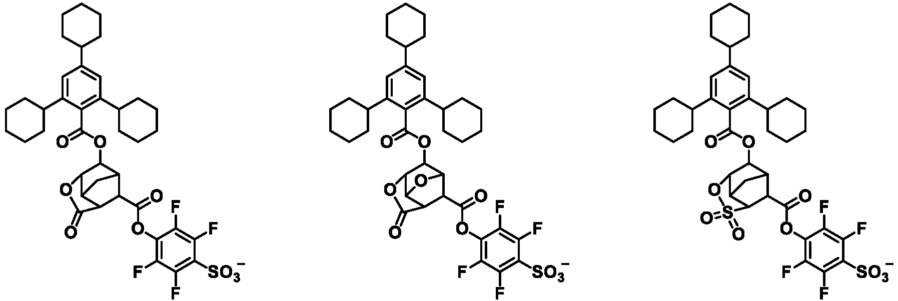

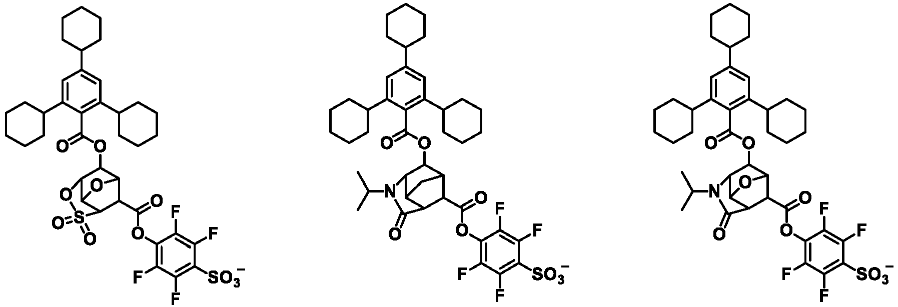

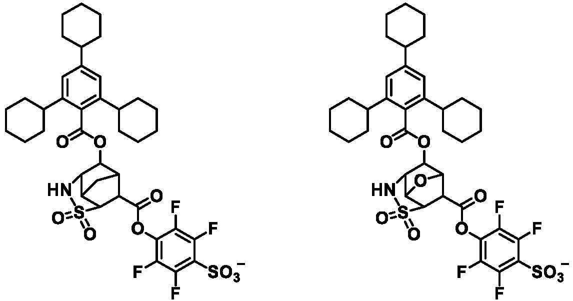

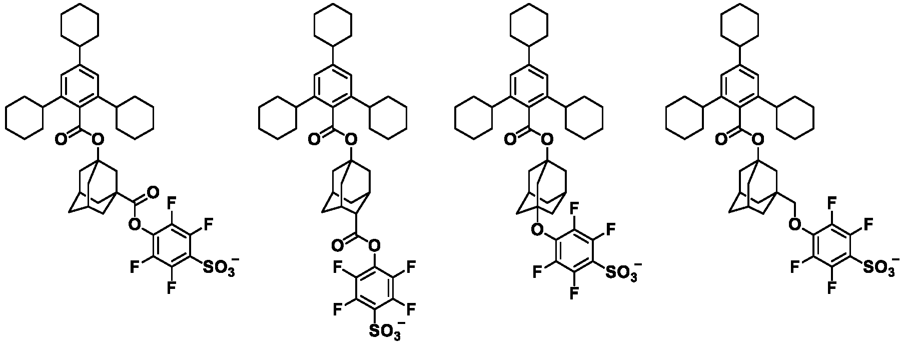

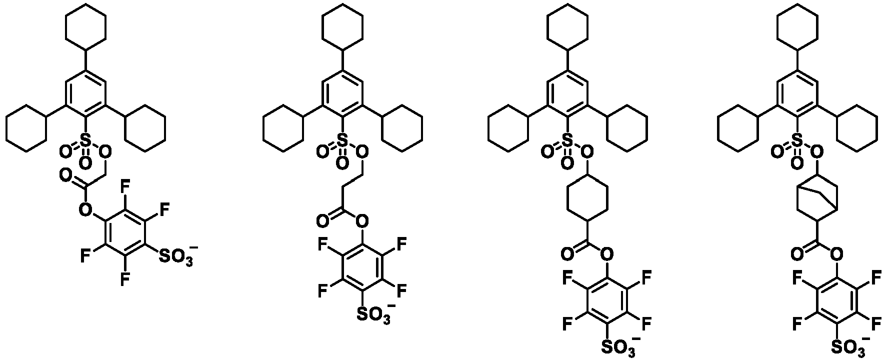

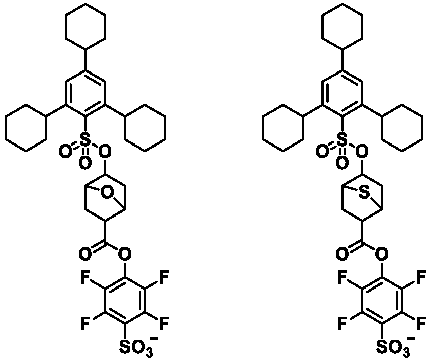

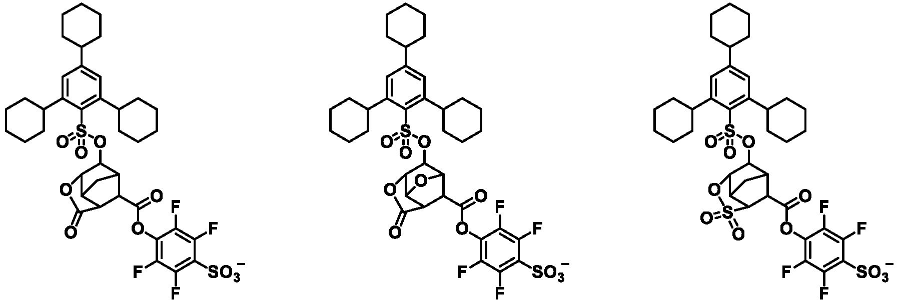

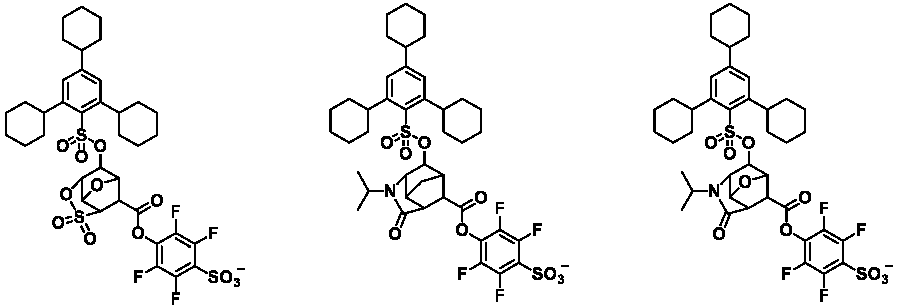

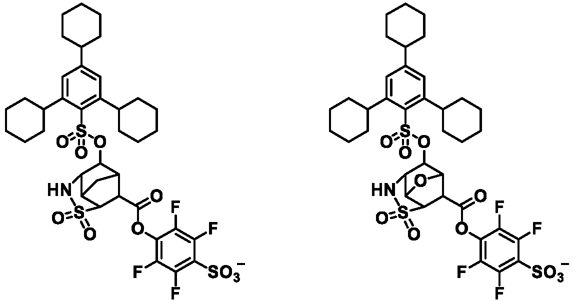

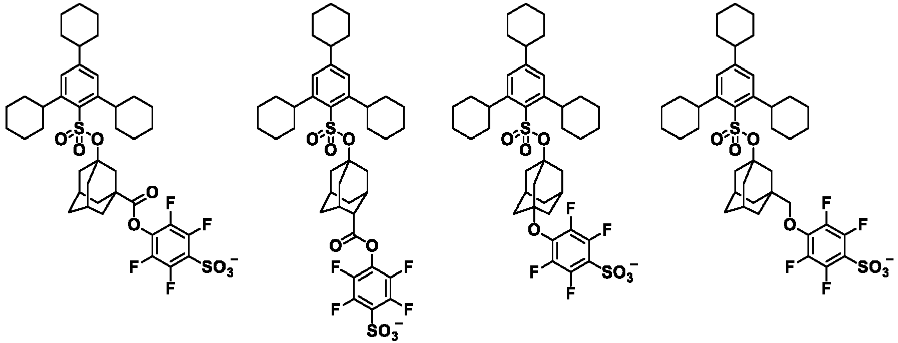

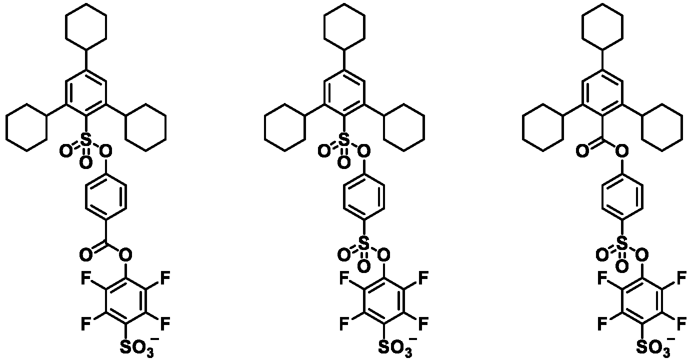

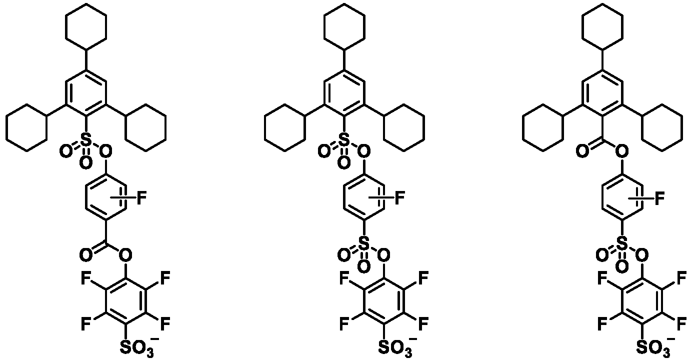

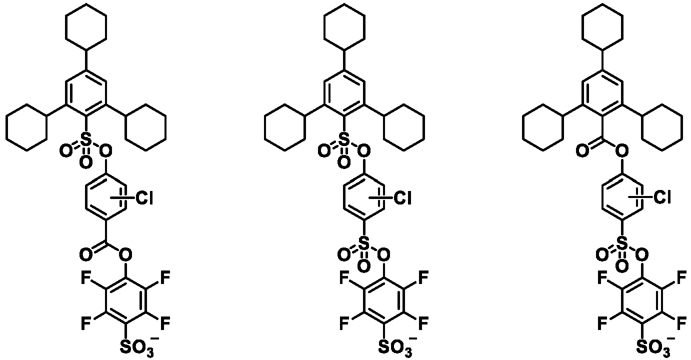

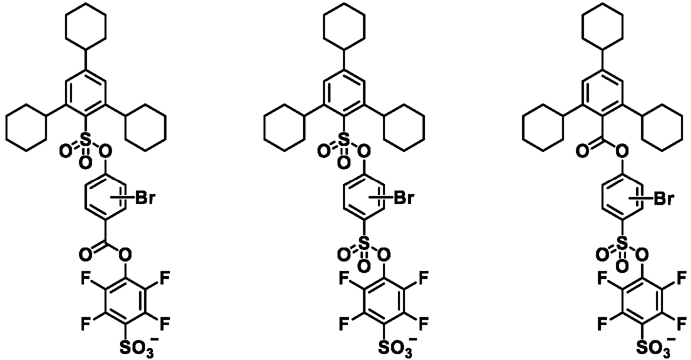

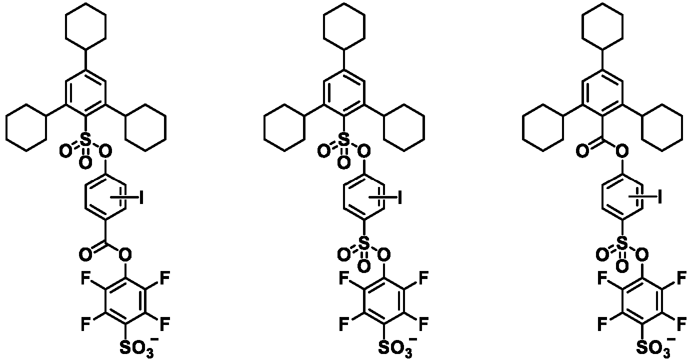

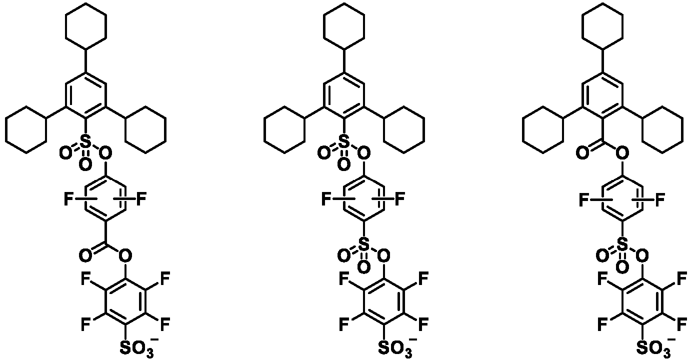

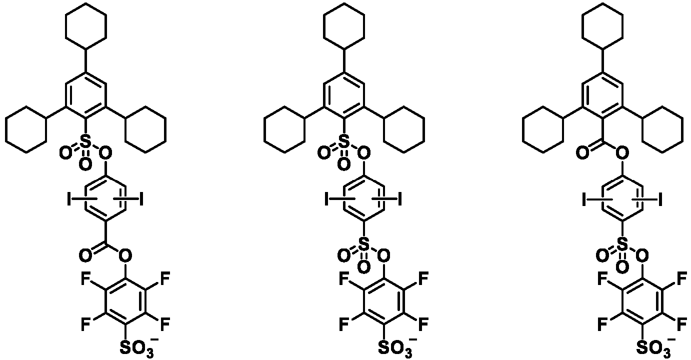

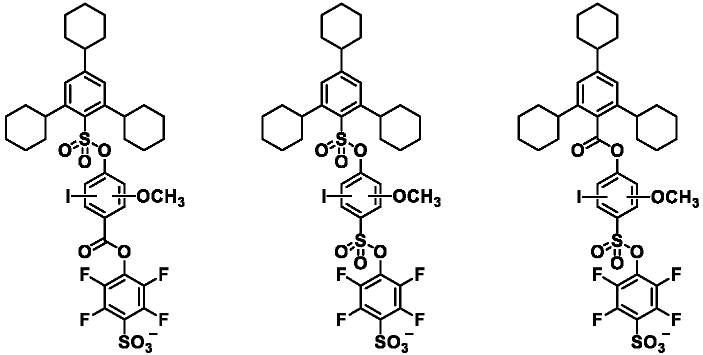

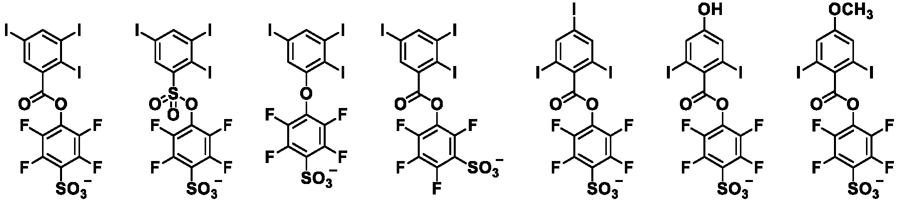

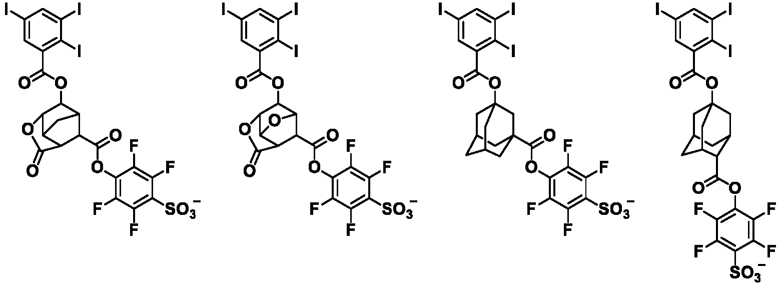

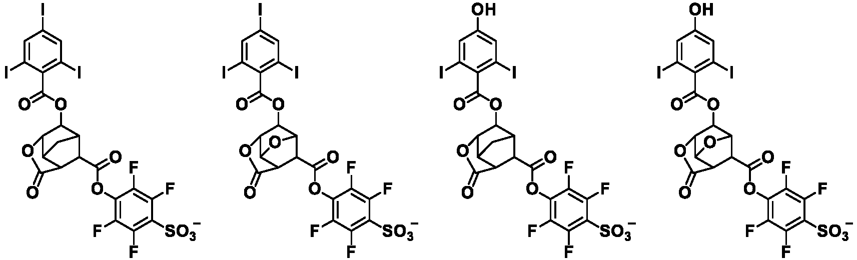

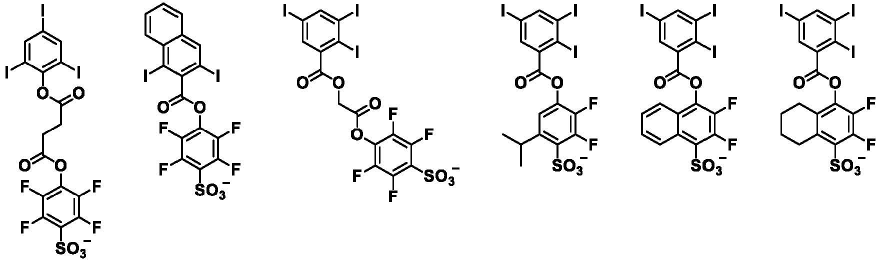

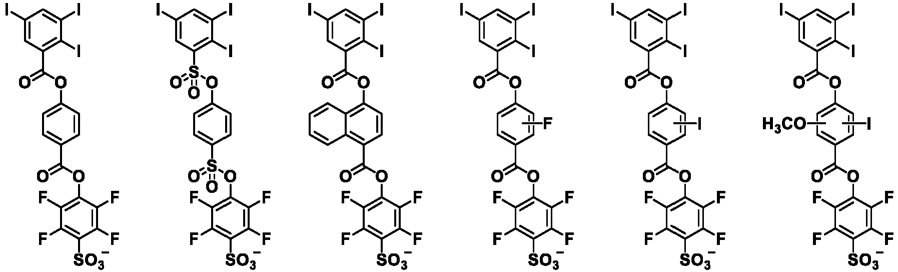

Also preferred as the PAG is a salt compound containing an anion having the formula (F1).

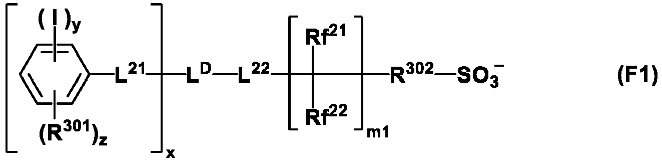

In formula (F1), m1 is 0 or 1, x is an integer of 1 to 3, y is an integer of 1 to 5, z is an integer of 0 to 3, and y+z is from 1 to 5.

In formula (F1), L²¹ is a single bond, ether bond, ester bond, sulfonate ester bond, carbonate bond or carbamate bond.

In formula (F1), L²² is an ether bond, ester bond, sulfonate ester bond, carbonate bond or carbamate bond.

In formula (F1), L^{D} is a single bond or a C₁-C₂₀ hydrocarbylene group when x is 1. L^{B} is a C₁-C₂₀ (x+1)-valent hydrocarbon group when x is 2 or 3. The hydrocarbylene group and (x+1)-valent hydrocarbon group may contain at least one moiety selected from ether bond, carbonyl moiety, ester bond, amide bond, sultone ring, lactam ring, carbonate bond, halogen, hydroxy moiety and carboxy moiety.

The C₁-C₂₀ hydrocarbylene group L^{D} may be saturated or unsaturated and straight, branched or cyclic. Examples thereof include C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and combinations thereof. The C₁-C₂₀ (x+1)-valent hydrocarbon group L^{D} may be saturated or unsaturated and straight, branched or cyclic. Examples thereof include those exemplified above for the C₁-C₂₀ hydrocarbylene group, with one or two hydrogen atoms being eliminated.

In formula (F 1), Rf²¹ and Rf²² are each independently hydrogen, fluorine or trifluoromethyl, at least one being fluorine or trifluoromethyl.

In formula (F1), R³⁰¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{301A})(R^{301B}), -N(R^{301C})-C(=O)-R^{301D} or -N(R^{301C})-C(=O)-O-R^{301D}. R^{301A} and R^{301B} are each independently hydrogen or a C₁-C₆ saturated hydrocarbyl group. R^{301C} is hydrogen or a C₁-C₆ saturated hydrocarbyl group. R^{301D} is a C₁-C₆ saturated hydrocarbyl group or C₂-C₈ unsaturated aliphatic hydrocarbyl group.

The C₁-C₆ saturated hydrocarbyl group represented by R³⁰¹, R^{301A}, R^{301B} and R^{301C} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R³⁰¹ are as exemplified above for the saturated hydrocarbyl group. Examples of the saturated hydrocarbyl moiety in the C₂-C₆ saturated hydrocarbylcarbonyloxy group represented by R³⁰¹ are as exemplified above for the C₁-C₆ saturated hydrocarbyl group, but of 1 to 5 carbon atoms.

The C₂-C₈ unsaturated aliphatic hydrocarbyl group represented by R^{301D} may be straight, branched or cyclic and examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (F 1), R³⁰² is a C₁-C₂₀ saturated hydrocarbylene group or C₆-C₁₄ arylene group. Some or all of the hydrogen atoms in the saturated hydrocarbylene group may be substituted by halogen other than fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₁₄ aryl groups, halogen, and hydroxy.

The C₁-C₂₀ hydrocarbylene group represented by R³⁰² may be saturated or unsaturated and straight, branched or cyclic. Examples thereof are as exemplified above for the C₁-C₂₀ hydrocarbylene group L^{D}.

Examples of the C₆-C₂₀ arylene group represented by R³⁰² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl. Examples of the C₆-C₁₄ arylene moiety which is a substituent on the arylene group include phenylene, naphthylene, phenanthrenediyl and anthracenediyl.

More preferably, the anion has the formula (F2).

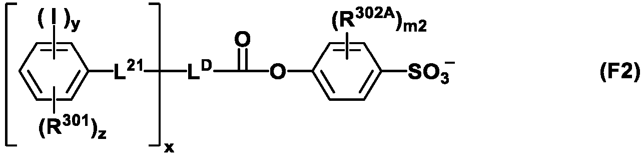

In formula (F2), x, y, z, L²¹, L^{D}, and R³⁰¹ are as defined above. The subscript m2 is an integer of 1 to 4. R^{302A} is a C₁-C₂₀ saturated hydrocarbyl group, C₁-C₂₀ saturated hydrocarbyloxy group, C₆-C₁₄ aryl group, halogen or hydroxy group. When m2 is 2, 3 or 4, a plurality of R^{302A} may be identical or different.

Examples of the anion having formula (F1) are shown below, but not limited thereto.

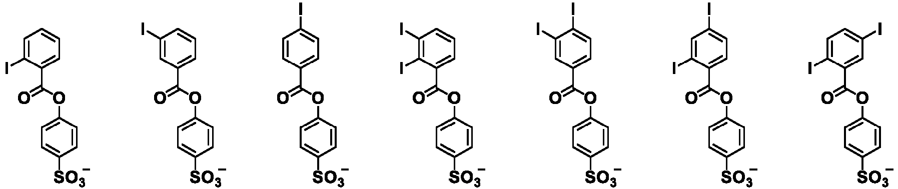

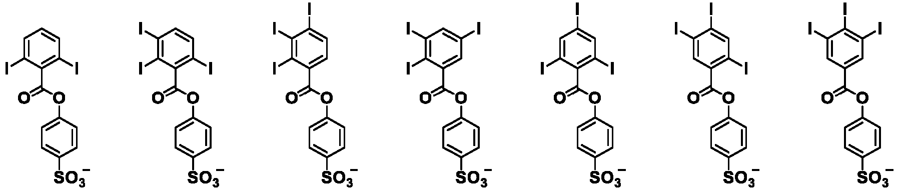

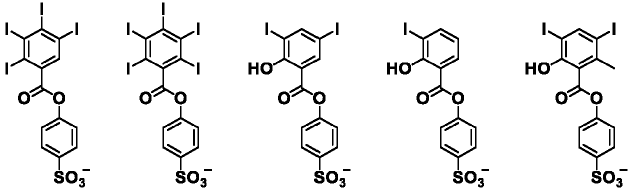

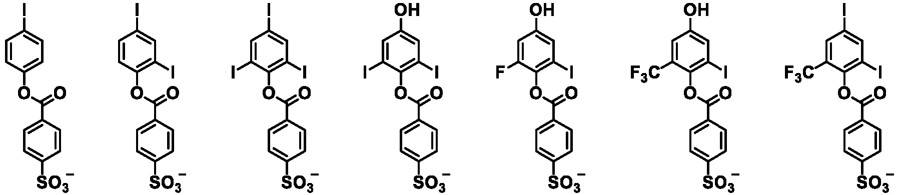

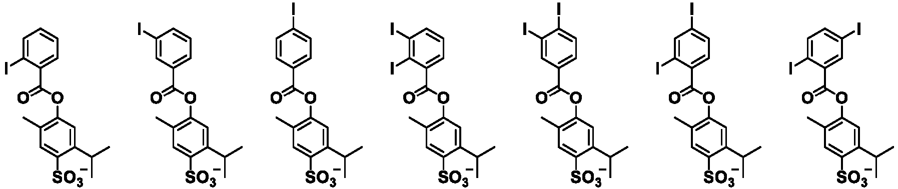

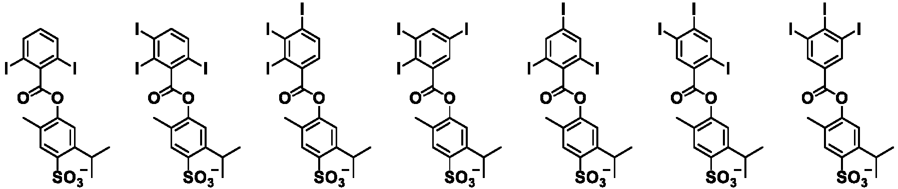

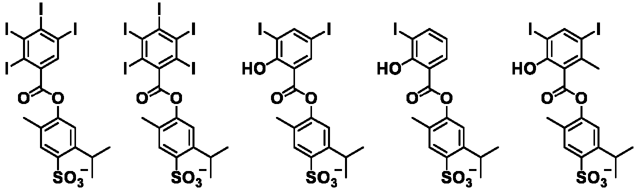

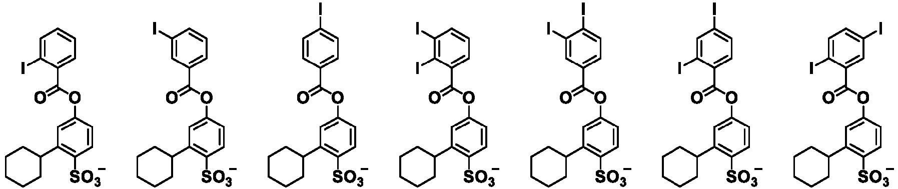

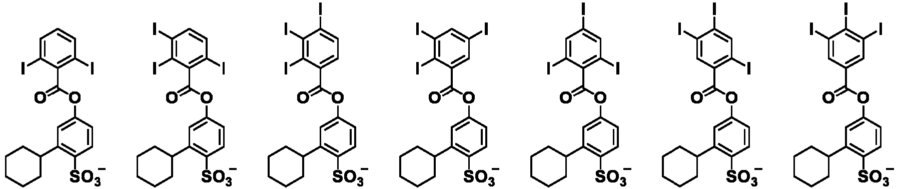

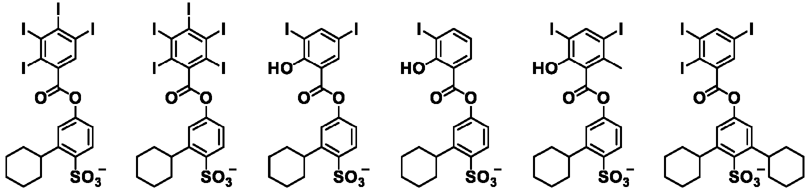

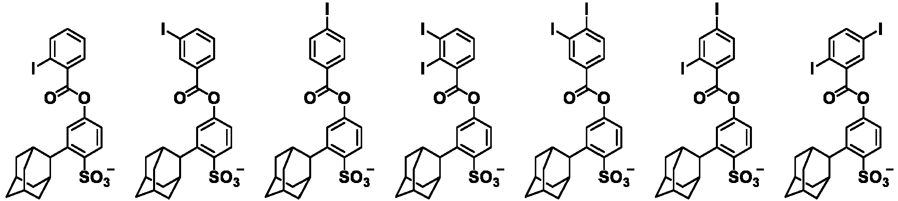

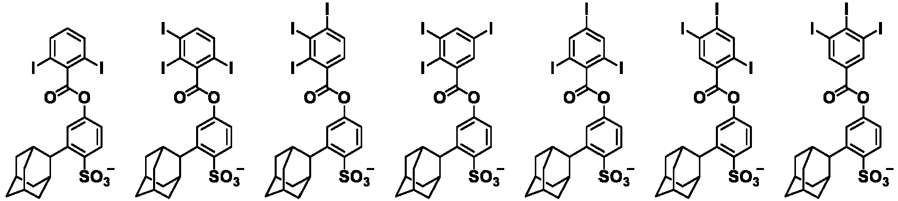

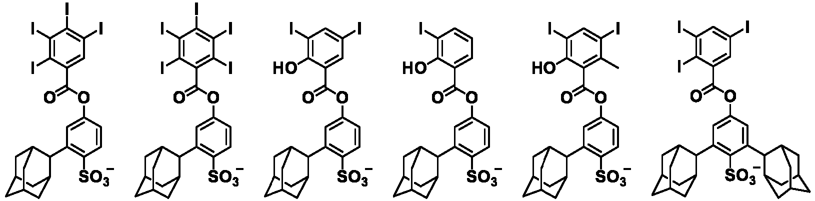

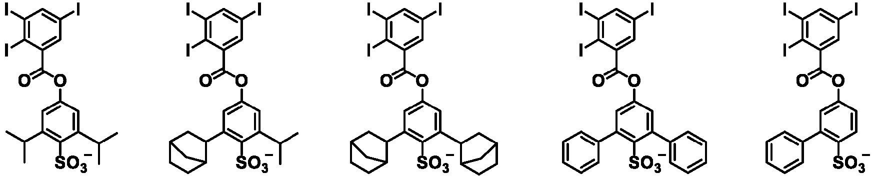

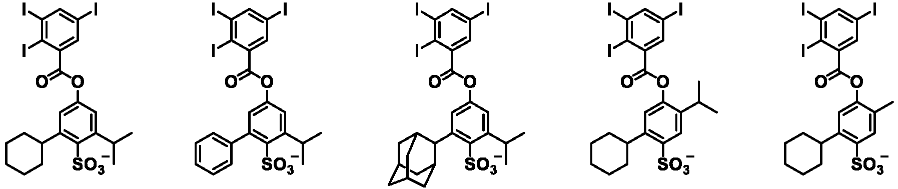

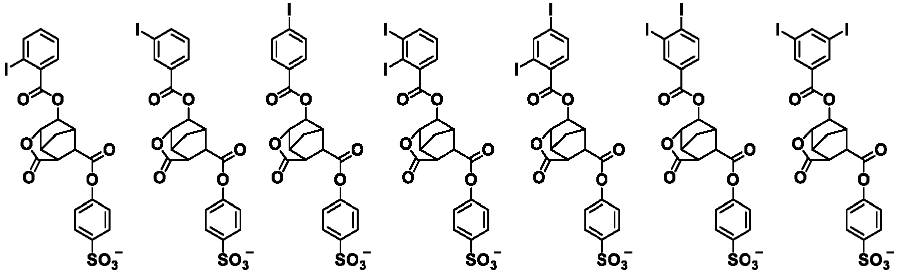

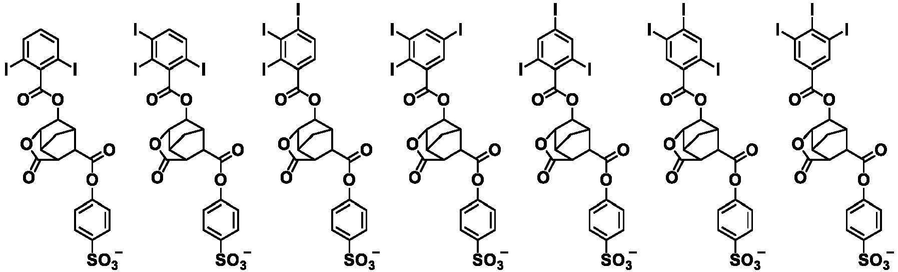

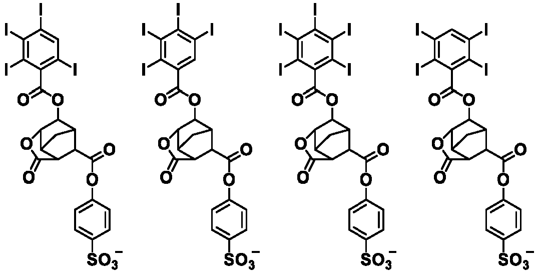

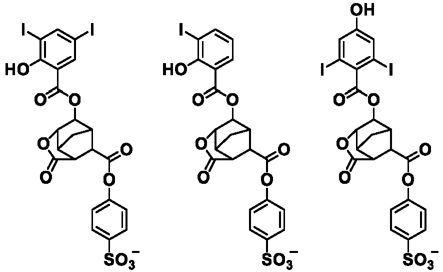

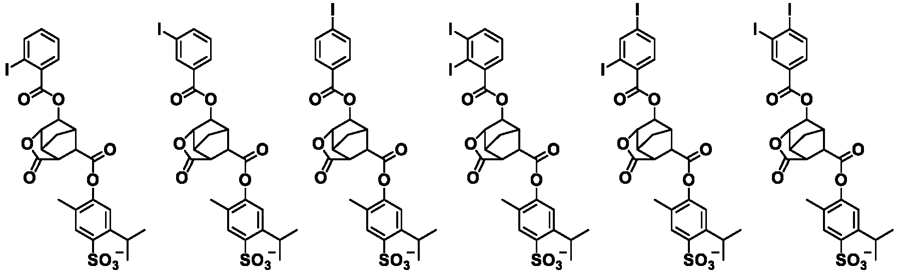

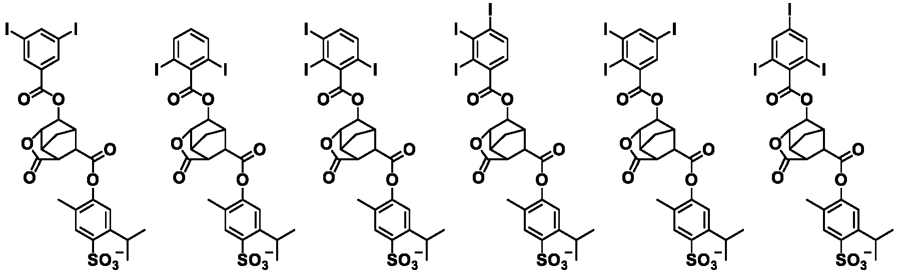

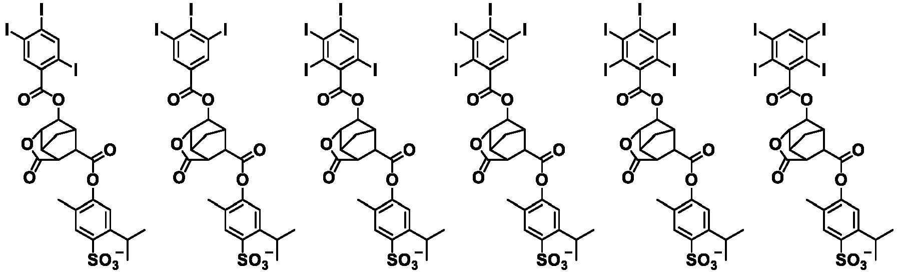

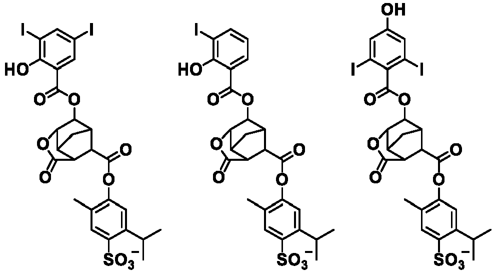

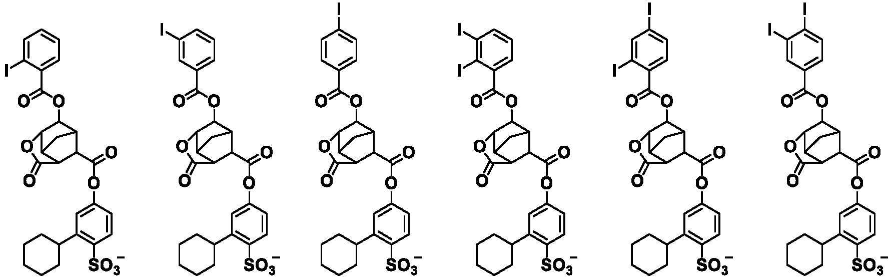

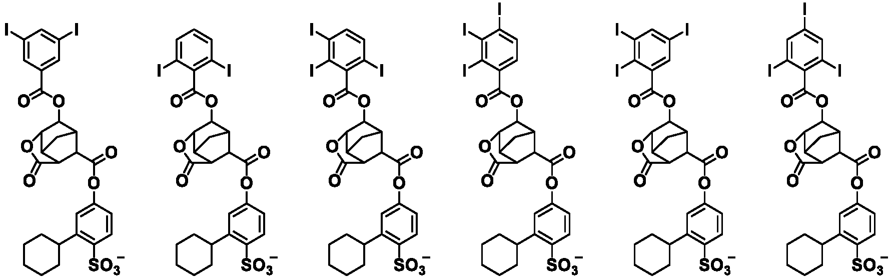

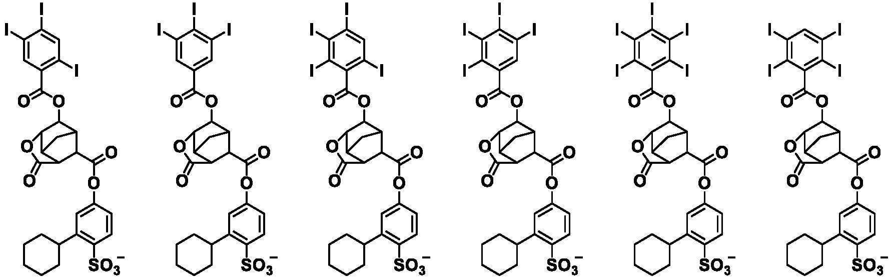

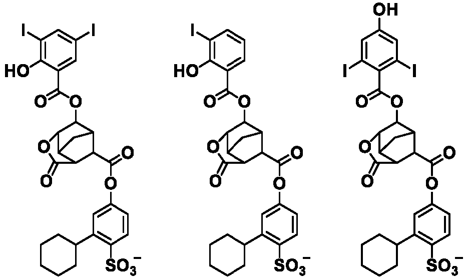

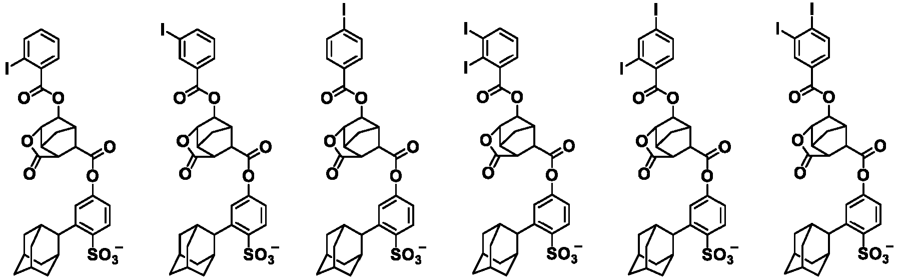

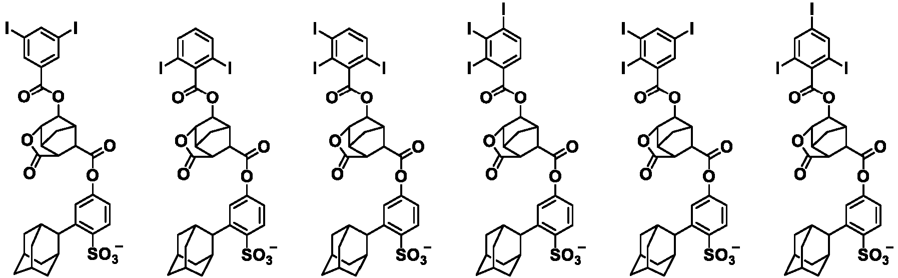

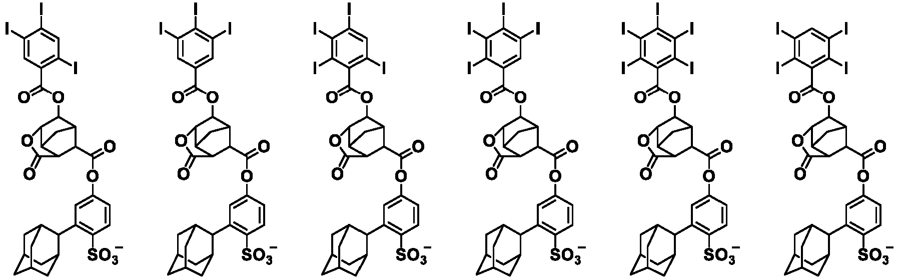

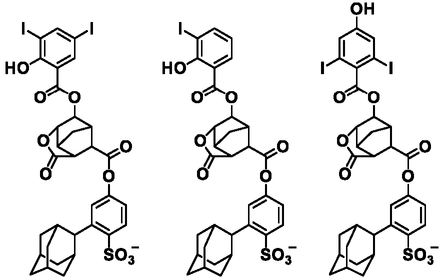

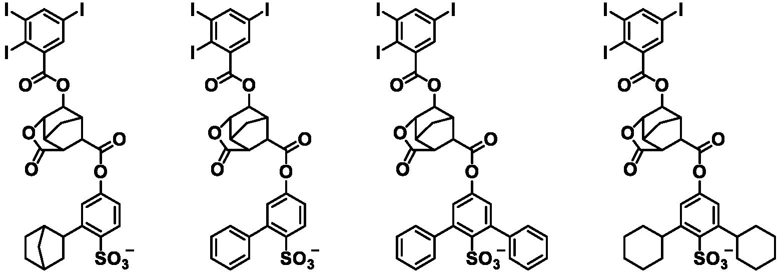

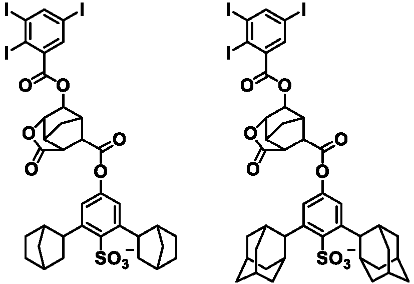

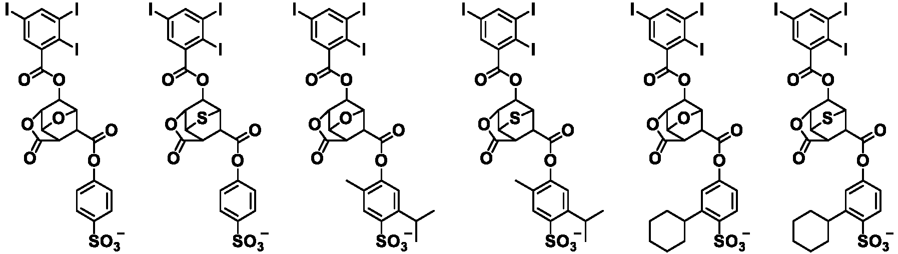

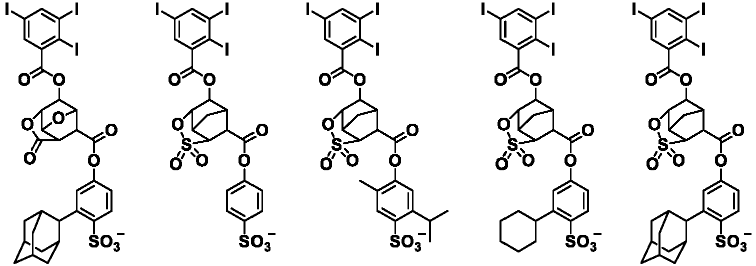

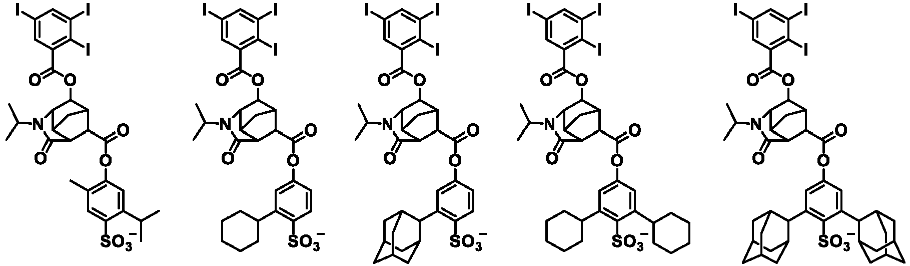

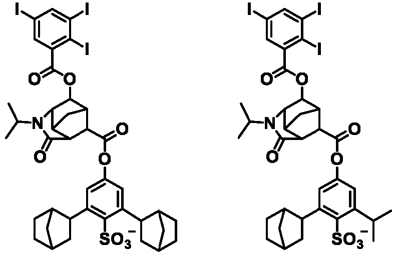

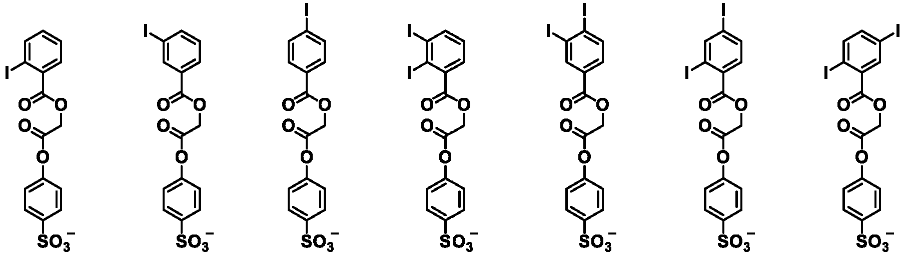

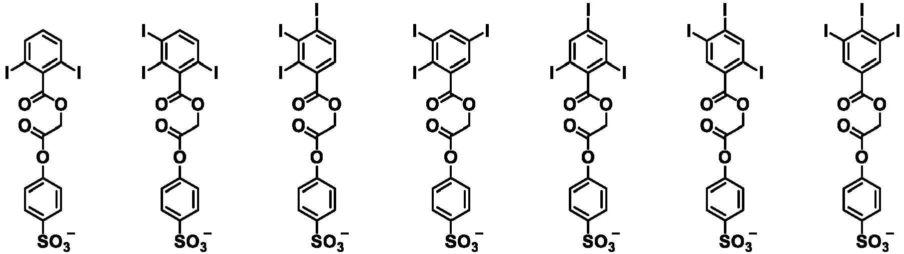

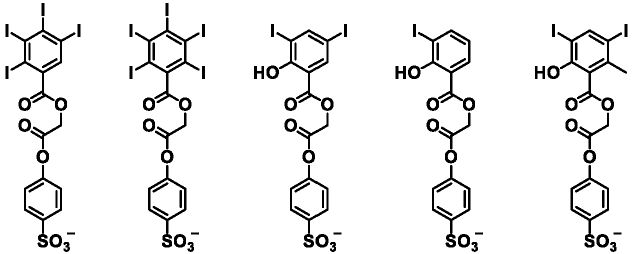

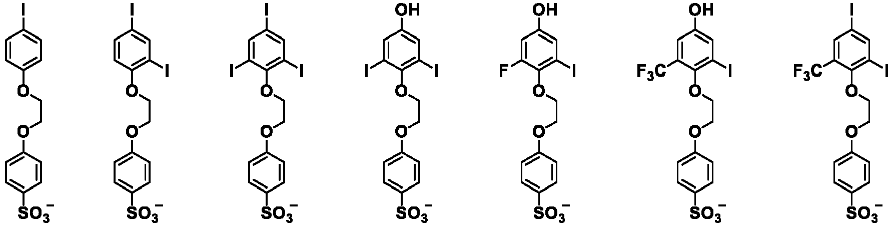

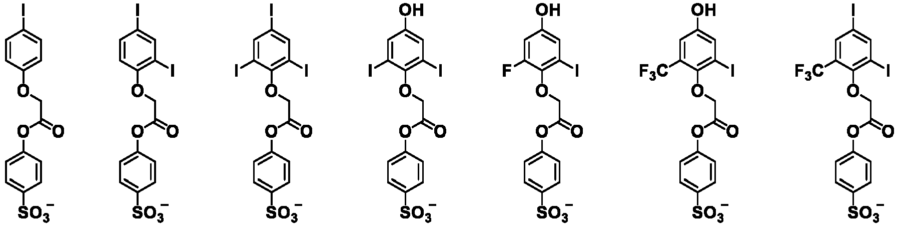

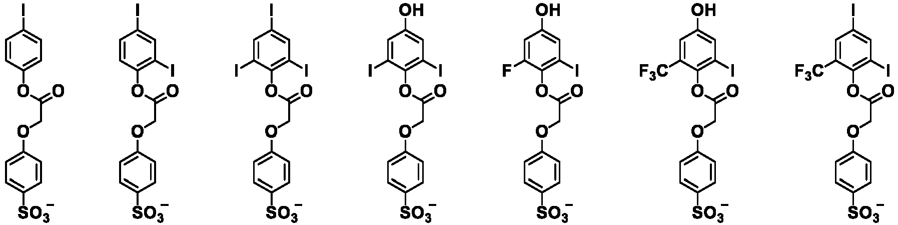

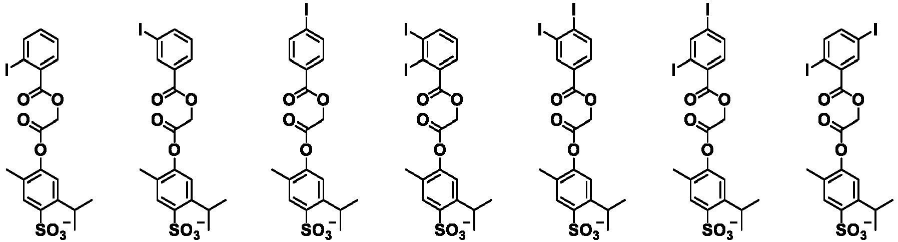

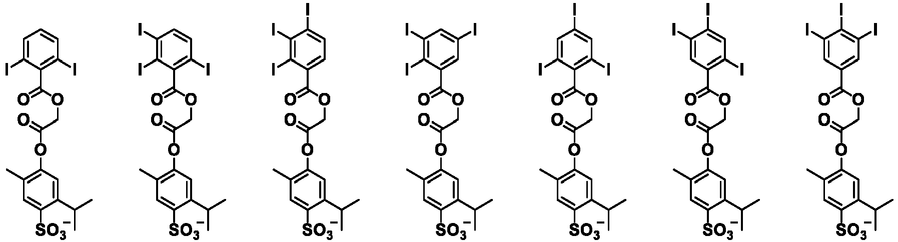

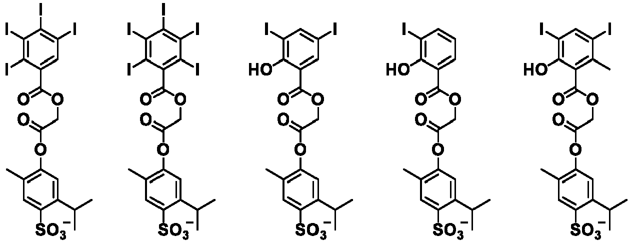

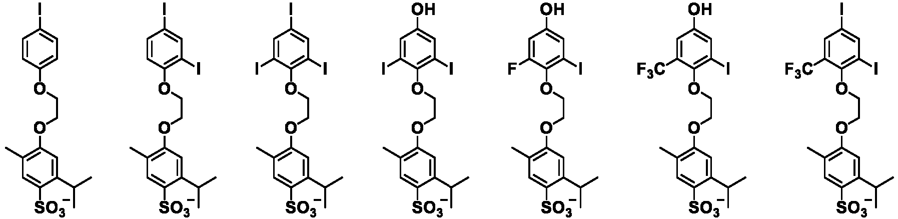

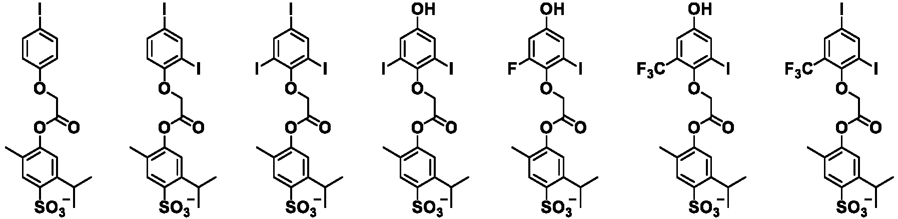

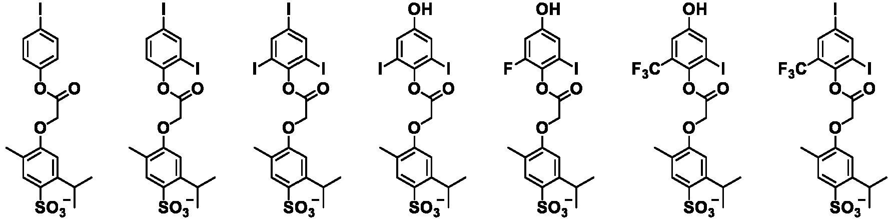

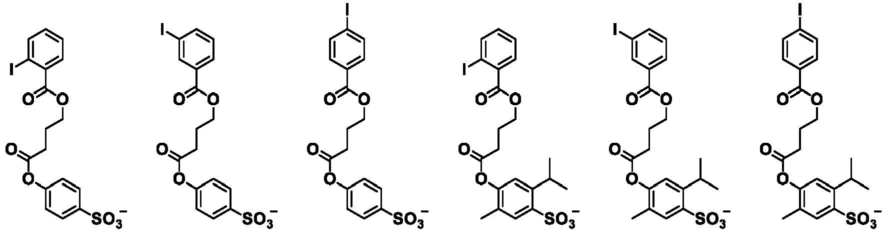

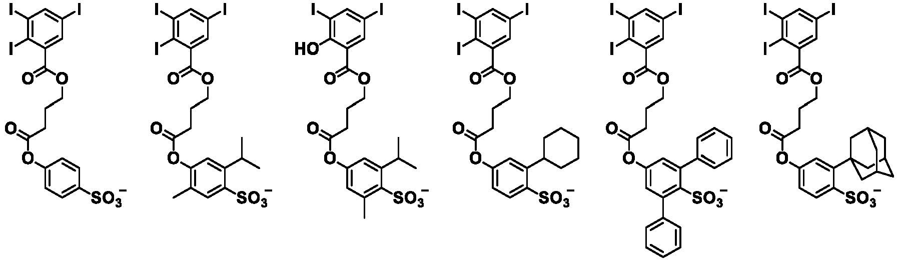

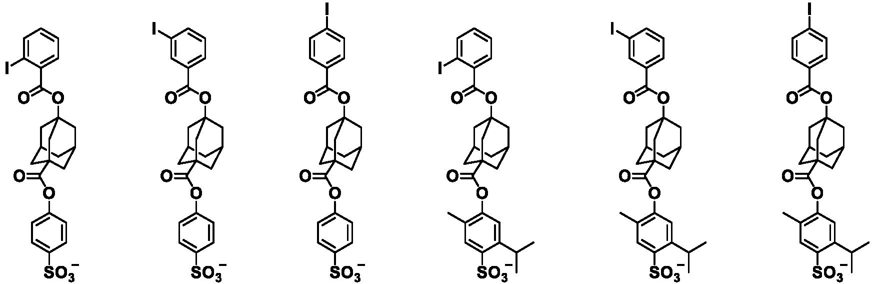

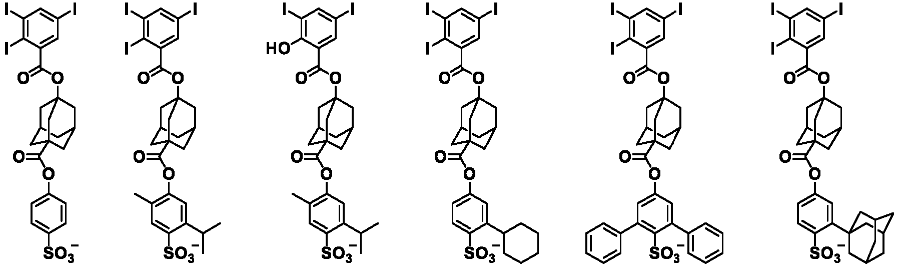

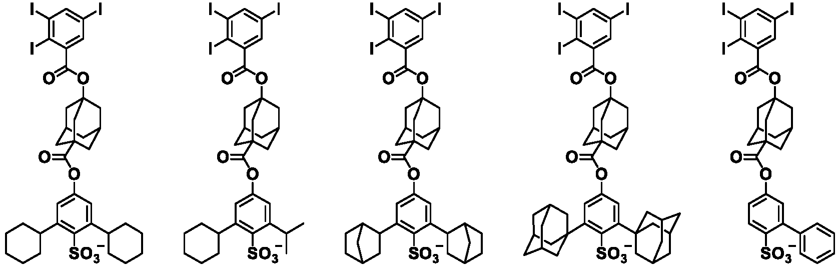

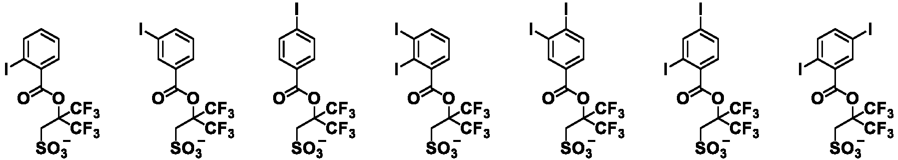

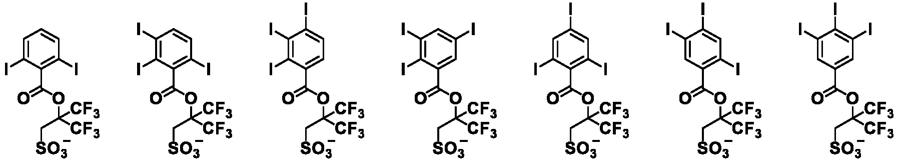

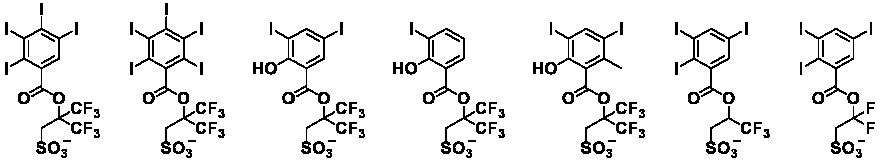

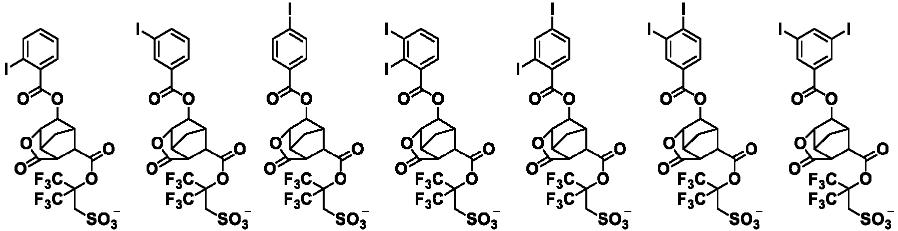

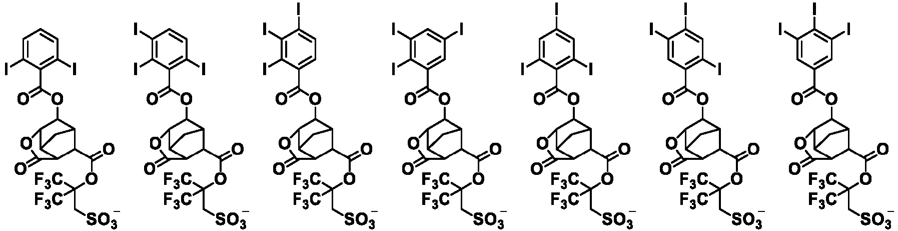

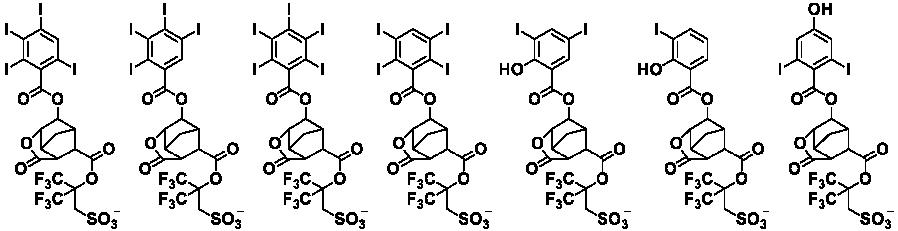

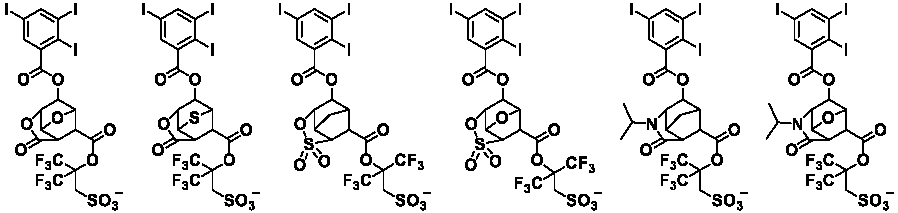

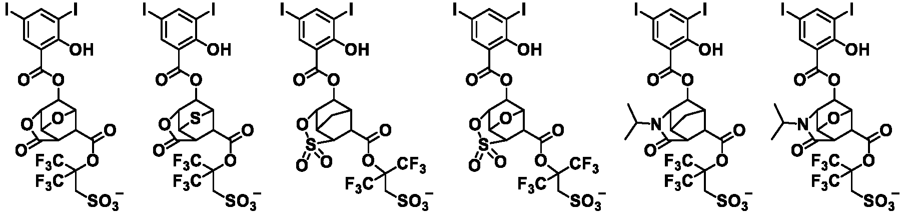

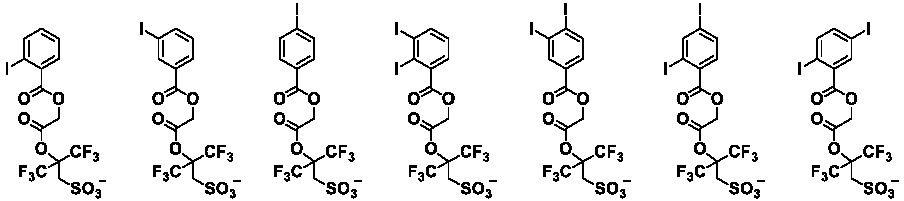

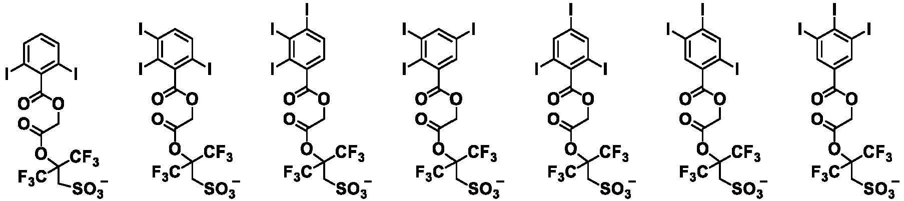

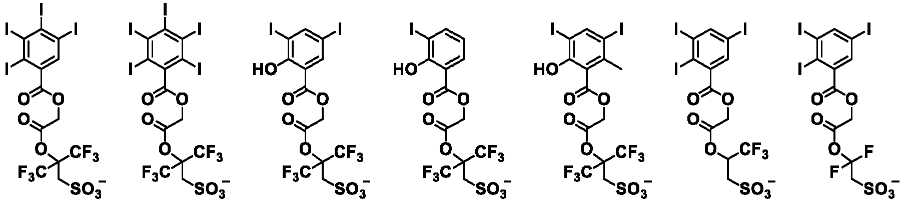

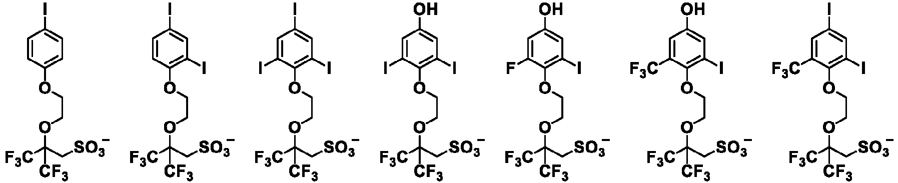

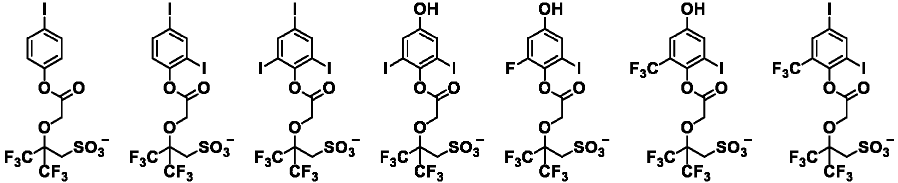

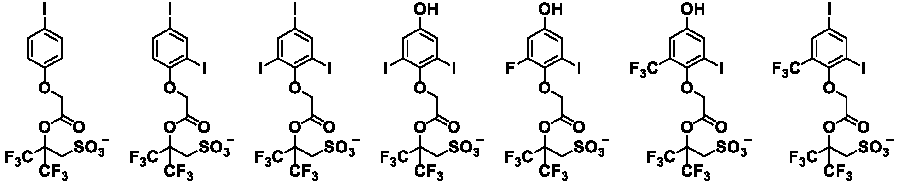

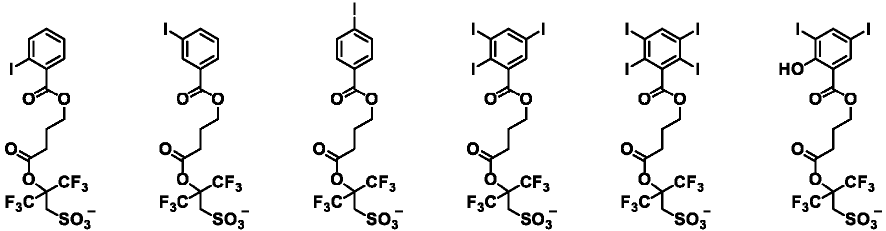

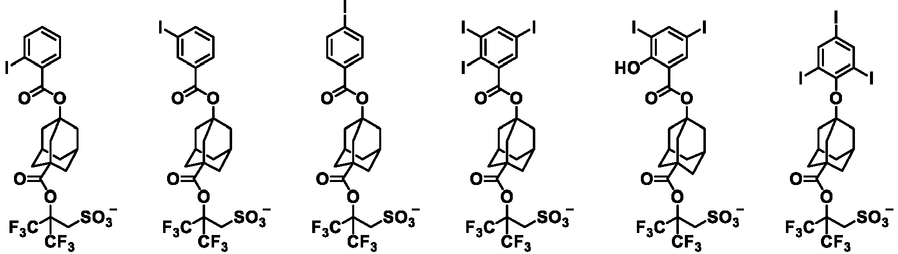

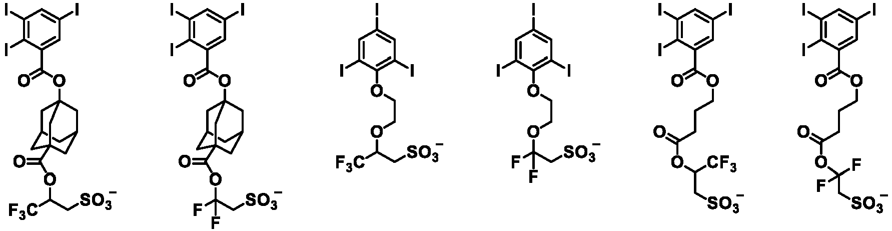

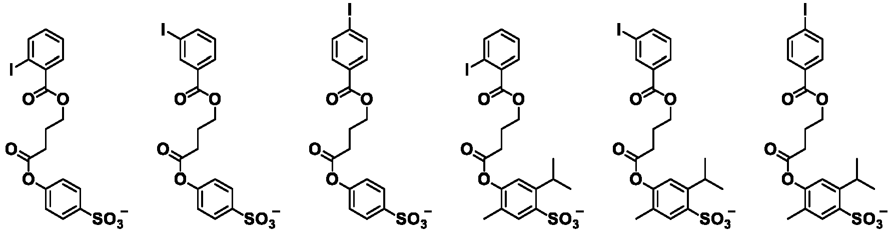

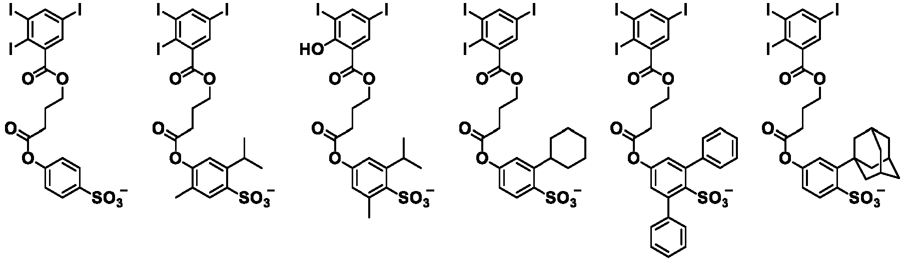

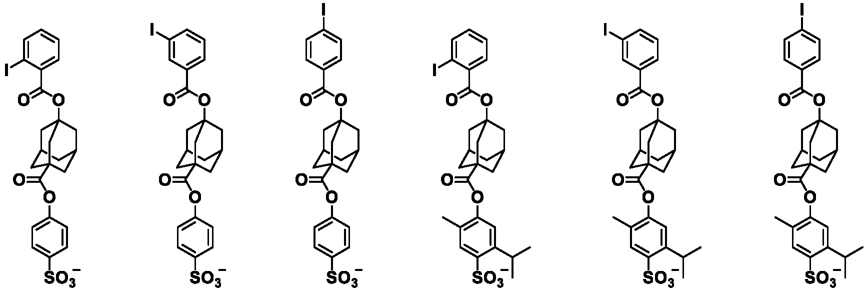

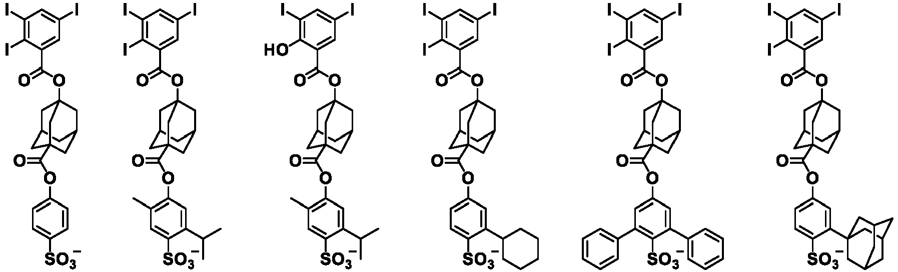

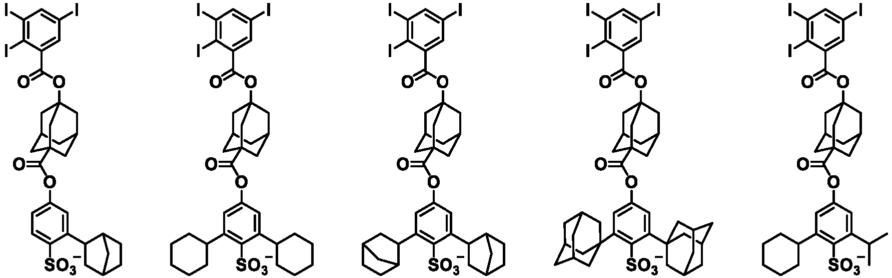

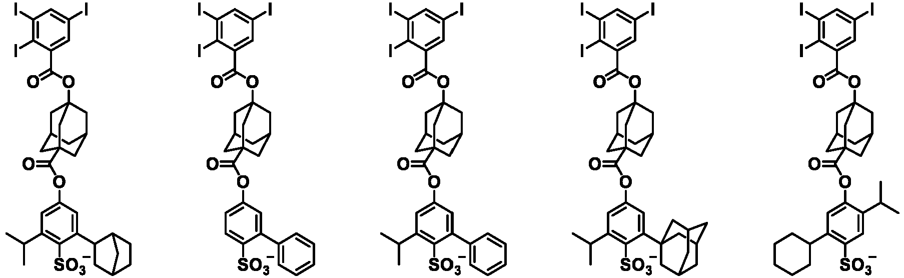

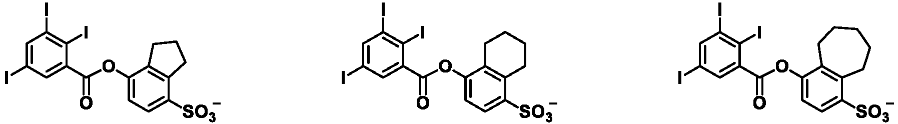

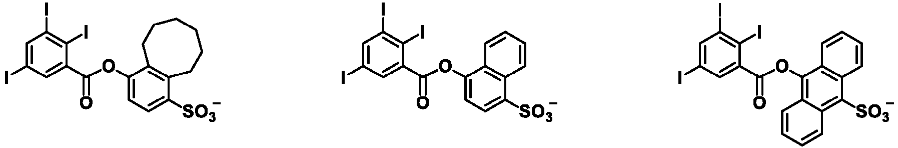

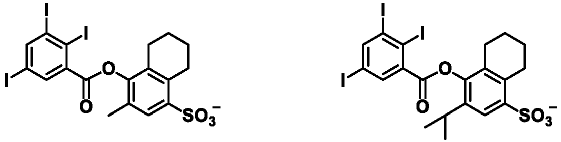

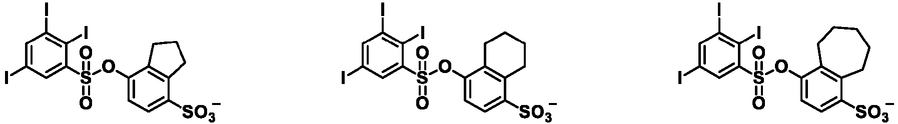

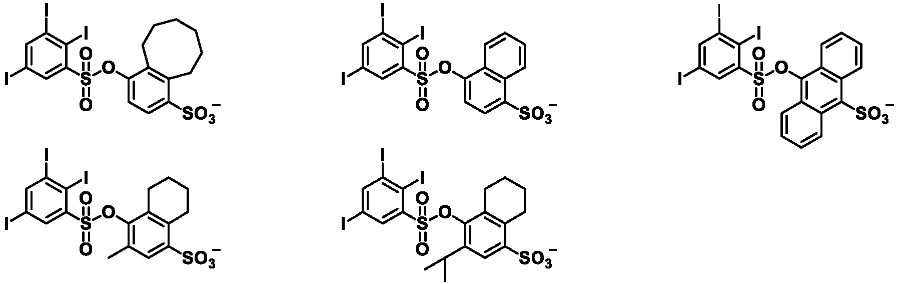

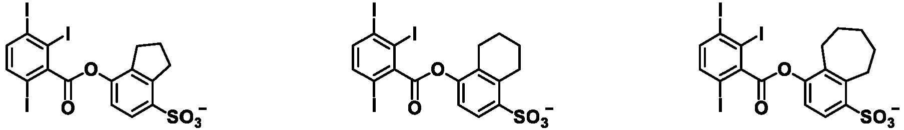

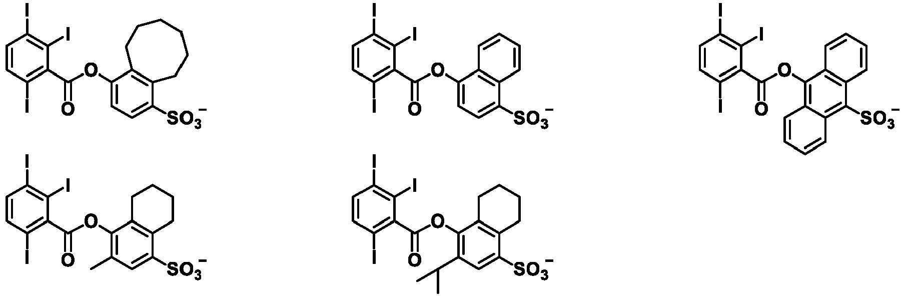

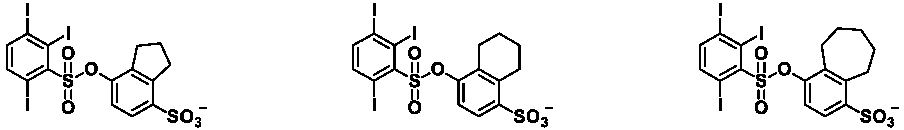

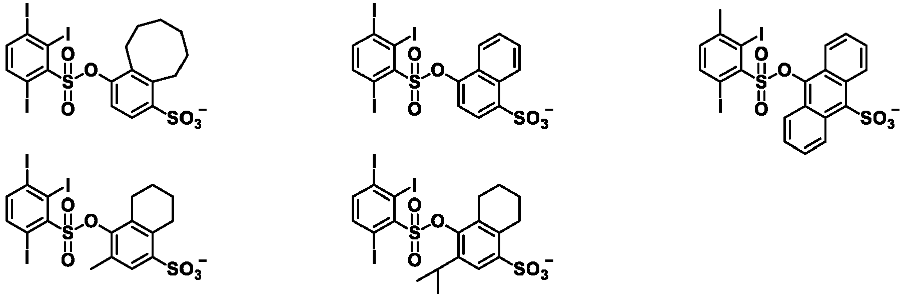

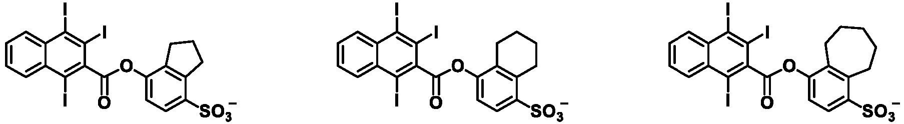

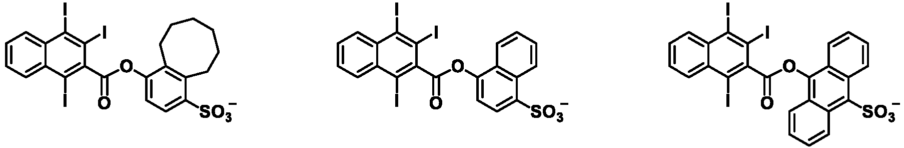

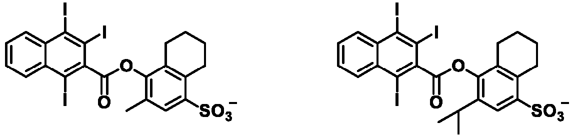

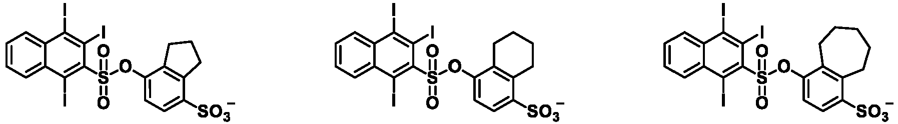

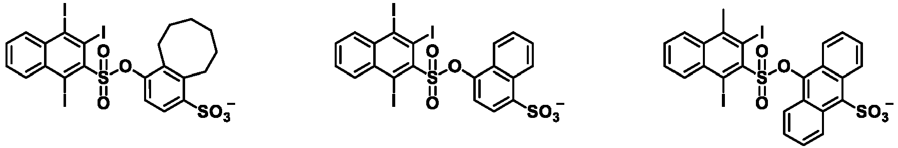

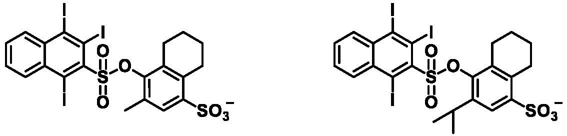

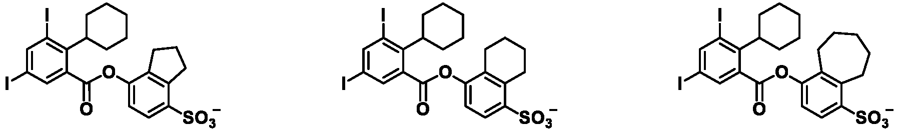

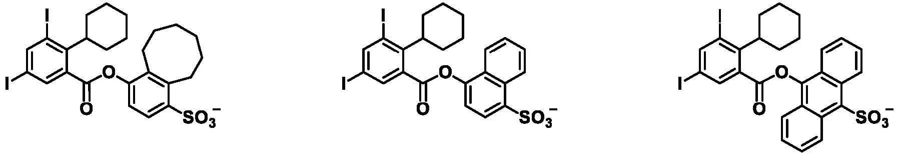

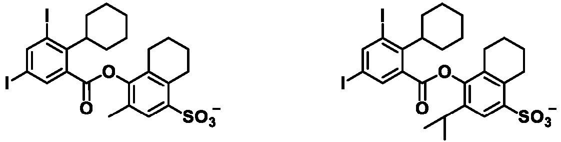

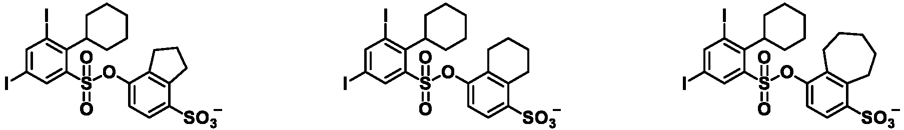

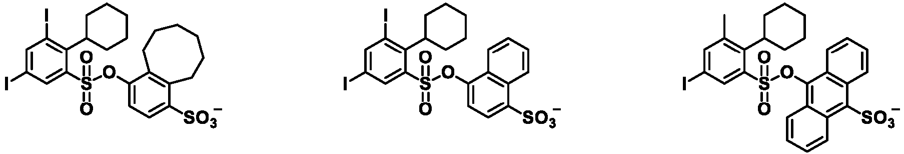

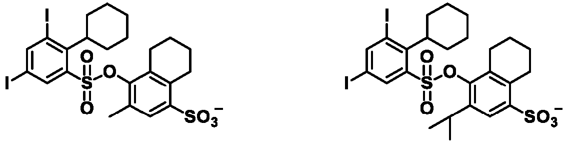

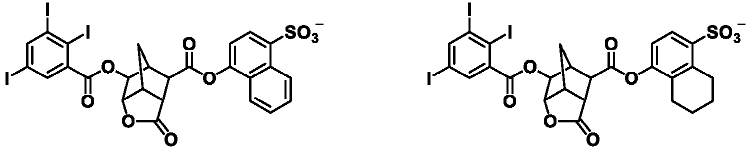

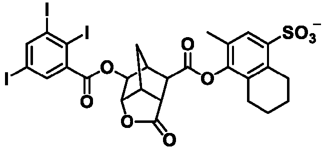

Preferred examples of the cation that pairs with the anion include sulfonium and iodonium cations. Examples of the sulfonium cation are as exemplified above for the sulfonium cation having formula (cation-1), but not limited thereto. Examples of the iodonium cation are as exemplified above for the iodonium cation having formula (cation-2), but not limited thereto.

The other PAG generates an acid having a pKa value of preferably -2.0 or larger, more preferably -1.0 or larger. The upper limit of pKa is preferably 2.0. Notably, the pKa value is computed using pKa DB in software ACD/Chemsketch ver: 9.04 of Advanced Chemistry Development Inc.

When the resist composition contains the other PAG (F), the amount of the PAG (F) used is preferably 1 to 10 parts, more preferably 1 to 5 parts by weight per 80 parts by weight of the base polymer (B). The inclusion of the other PAG provides for appropriate adjustment of the amount of acid generated in the exposed region and the degree of dissolution inhibition in the unexposed region. The other PAG may be used alone or in admixture.

In addition to the PAG (A), the preferred resist composition comprises the polymer-bound PAG containing repeat units having formulae (B6) to (B9) or the other PAG (F). This embodiment allows the base polymer in the resist film to undergo deprotection reaction to a full extent and is successful in forming a pattern of satisfactory profile at an improved resolution.

### (G) Surfactant

The resist composition may contain any conventional surfactants for facilitating to coat the composition to the substrate. A number of surfactants are known in the art as described in WO 2006/121096, JP-A 2008-102383, JP-A 2008-304590, JP-A 2004-115630, and JP-A 2005-008766, and any suitable one may be chosen therefrom.

When the resist composition contains the surfactant (G), the amount of the surfactant (G) added is preferably up to 2 parts by weight, more preferably up to 1 part by weight and preferably at least 0.01 part by weight per 80 parts by weight of the base polymer (B).

### Process

A further embodiment of the invention is a pattern forming process comprising the steps of applying the chemically amplified positive resist composition defined above onto a substrate to form a resist film thereon, exposing the resist film to a pattern of high-energy radiation, and developing the exposed resist film in an alkaline developer.

The substrate used herein may be selected from, for example, substrates for IC fabrication, e.g., Si, SiO, SiO₂, SiN, SiON, TiN, WSi, BPSG, SOG, and organic antireflective coating, and substrates for mask circuit fabrication, e.g., Cr, CrO, CrON, MoSi₂, Si, SiO, and SiO₂.

The resist composition is first applied onto a substrate by a suitable coating technique such as spin coating. The coating is prebaked on a hotplate preferably at a temperature of 60 to 150°C for 1 to 20 minutes, more preferably at 80 to 140°C for 1 to 10 minutes to form a resist film of 0.03 to 2 µm thick.

Then the resist film is exposed patternwise to high-energy radiation. Examples of the high-energy radiation include UV, deep UV, excimer laser radiation (typically, KrF and ArF), EB, EUV, X-ray, γ-ray, and synchrotron radiation.

On use of UV, deep UV, excimer laser radiation, EUV, X-ray, γ-ray, and synchrotron radiation, the resist film is exposed through a mask having the desired pattern, preferably in a dose of 1 to 300 mJ/cm², more preferably 10 to 200 mJ/cm². On use of EB, a pattern may be directly written preferably in a dose of 1 to 300 µC/cm2, more preferably 10 to 200 µC/cm². The resist composition of the invention is particularly useful in the EUV and EB lithography processes.

The exposure may be performed by conventional lithography whereas the immersion lithography of holding a liquid, typically water between the resist film and the mask may be employed if desired. In the case of immersion lithography, a protective film which is insoluble in water may be formed on the resist film.

After the exposure, the resist film may be baked (PEB), for example, on a hotplate preferably at 60 to 150°C for 1 to 20 minutes, and more preferably at 80 to 140°C for 1 to 10 minutes.

Finally, development is carried out using as the developer an aqueous alkaline solution, such as a 0.1 to 5 wt%, preferably 2 to 3 wt%, aqueous solution of tetramethylammonium hydroxide (TMAH), this being done by a conventional method such as dip, puddle, or spray development for a period of 0.1 to 3 minutes, and preferably 0.5 to 2 minutes. In this way the exposed region of resist film is dissolved away, forming the desired pattern on the substrate.

The resist composition of the invention is advantageous particularly on use under the situation that requires high etching resistance, and a minimal change of pattern line width and minimal LER even when the time duration from exposure to PEB is prolonged. It is also advantageous for pattern formation on a substrate having a surface layer of material to which the resist pattern is less adherent with a likelihood of pattern stripping or pattern collapse, specifically a substrate having sputter deposited thereon a layer of metallic chromium or a chromium compound containing one or more light elements such as oxygen, nitrogen and carbon. The resist composition is particularly useful in forming a pattern on a photomask blank as the substrate.

### EXAMPLES

Examples of the invention are given below by way of illustration and not by way of limitation. The abbreviation "pbw" is parts by weight. Analysis is made by IR and proton-NMR spectroscopy and time-of-flight mass spectrometry (TOF-MS). The analyzers used are shown below.

| | |
|---|---|
| IR: | NICOLET 6700 by Thermo Fisher Scientific Inc. |
| ¹H-NMR: | ECA-500 by JEOL Ltd. |
| MALDI TOF-MS: | S3000 by JEOL Ltd. |

### [1] Synthesis of onium salts

### Example 1-1

### Synthesis of PAG-1

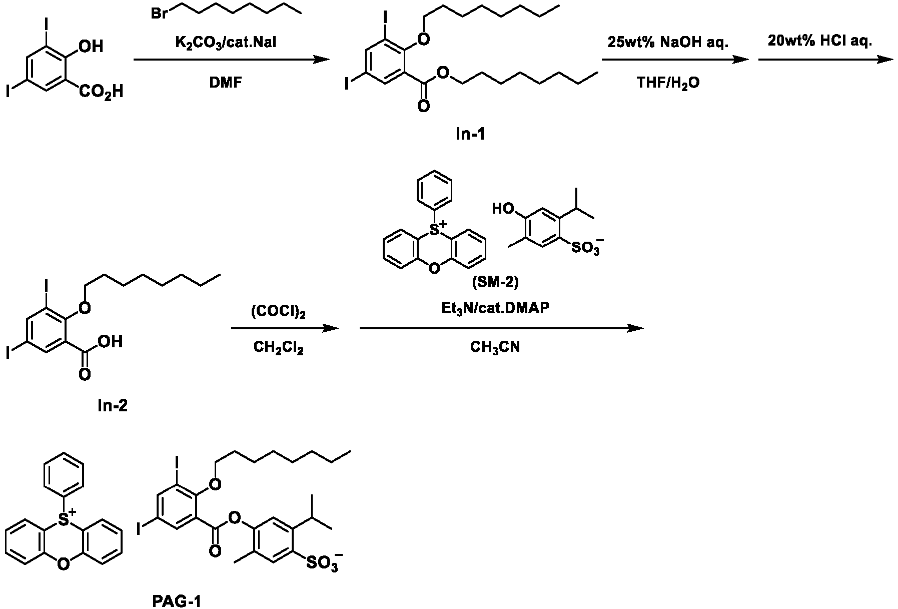

### (1) Synthesis of Intermediate In-1

In a reactor under nitrogen atmosphere, 195.0 g of 3,5-diiodosalicylic acid, 105.9 g of potassium carbonate and 7.5 g of sodium iodide were suspended in 1,000 g of dimethylformamide (DMF). After the reactor was heated at a temperature of 50°C, 212.4 g of 1-bromooctane was added dropwise to the suspension, which was aged in the reactor at a temperature of 75°C for 12 hours. After aging, the reaction solution was cooled down and 1,000 g of water was added to quench the reaction. The desired compound was extracted with 1,000 g of hexane and 250 g of toluene and subjected to ordinary aqueous workup. The solvent was distilled off, obtaining 306.3 g of Intermediate In-1 as oily matter (yield 100%). Intermediate In-1 was used in the subsequent step without further purification.

### (2) Synthesis of Intermediate In-2

In a reactor under nitrogen atmosphere, 306.3 g of Intermediate In-1 was dissolved in 700 g of THF. After the reactor was heated at a temperature of 50°C, 120.0 g of 25 wt% aqueous solution of sodium hydroxide was added dropwise. At the end of addition, the solution was heated under reflux and aged at a temperature of 70°C for 12 hours. After aging, the reaction solution was cooled down and 1,000 g of hexane was added. The desired sodium salt was extracted twice with 1,000 g of water. To the water layer after extraction, 145.5 g of 20 wt% hydrochloric acid was added to turn the pH of the solution acidic. The desired compound was extracted with 2,000 g of ethyl acetate, and subjected to ordinary aqueous workup. Through the subsequent solvent distillation and recrystallization from hexane, 162.3 g of Intermediate In-2 was obtained as white crystals (yield 65%).

### (3) Synthesis of onium salt PAG-1

In a reactor under nitrogen atmosphere, 10.1 g of Intermediate In-2 and 0.02 g of DMF were dissolved in 40 g of methylene chloride. At room temperature, 3.1 g of oxalyl chloride was added dropwise to the solution. At the end of addition, the reactor was heated at a temperature of 40°C, at which the solution was aged for 4 hours to form an acid chloride. After aging, the reaction solution was distilled, obtaining 12.6 g of the acid chloride. Then 12.6 g of the acid chloride, 10.6 g of reactant SM-2, and 0.3 g of 4-dimethylaminopyridine were suspended in 60 g of acetonitrile. The reactor was heated to a temperature of 50°C, after which a mixture of 2.6 g of triethylamine and 10 g of acetonitrile was added dropwise. At the end of addition, the reactor was heated at a temperature of 70°C, at which the solution was aged for 12 hours. After aging, the reaction solution was cooled down and 50 g of water was added to quench the reaction. The target compound was then extracted with methylene chloride. Through the subsequent ordinary aqueous workup and solvent distillation, 17.0 g of onium salt PAG-1 was obtained as oily matter (yield 86%).

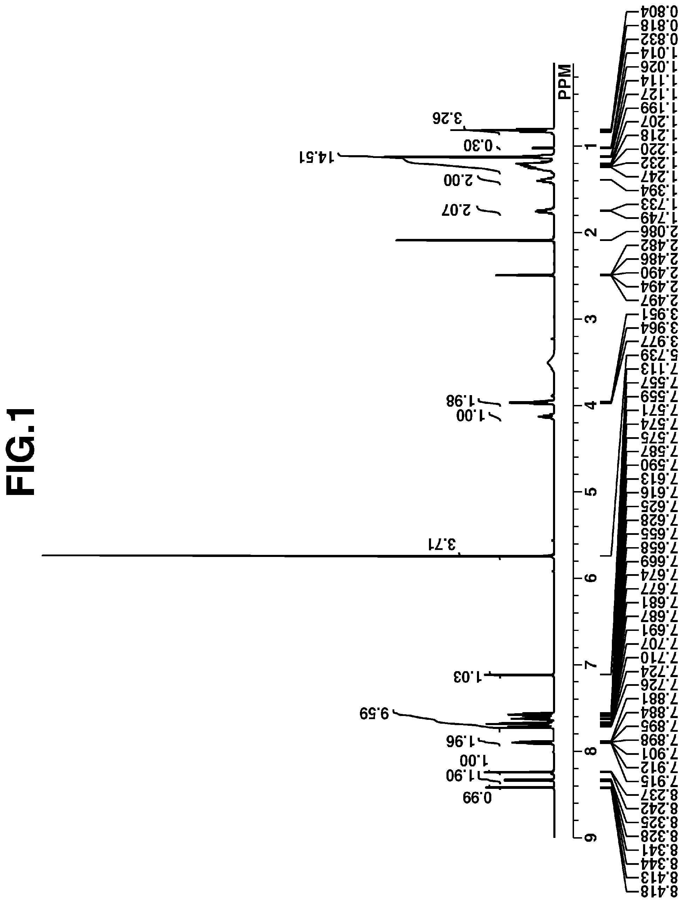

PAG-1 was analyzed by IR and TOF-MS, with the results shown below. The spectrum of PAG-1 analyzed by ¹H-NMR/DMSO-d₆ spectroscopy is shown in FIG. 1.

| | |
|---|---|
| IR (D-ATR): | v = 3058, 3021, 2926, 2855, 1749, 1593, 1583, 1565, 1537, 1465, 1440, 1383, 1322, 1271, 1253, 1230, 1185, 1145, 1085, 1061, 1040, 997, 912, 885, 815, 765, 733, 714, 664, 639, 622, 593, 562, 526, 487, 439 cm⁻¹ |
| MALDI TOF-MS: | POSITIVE M⁺ 277 (corresponding to C₁₈H₁₃OS⁺) |
| | NEGATIVE M⁻ 713 (corresponding to C₂₅H₃₁I₂O₆S⁻) |

### Examples 1-2 to 1-10

### Synthesis of PAG-2 to PAG-10

Onium salts PAG-2 to PAG-10 shown below were synthesized by well-known organic synthesis reaction using corresponding reactants.

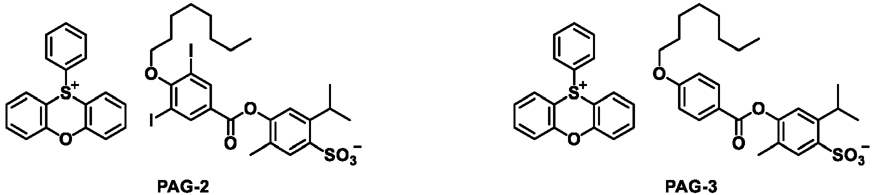

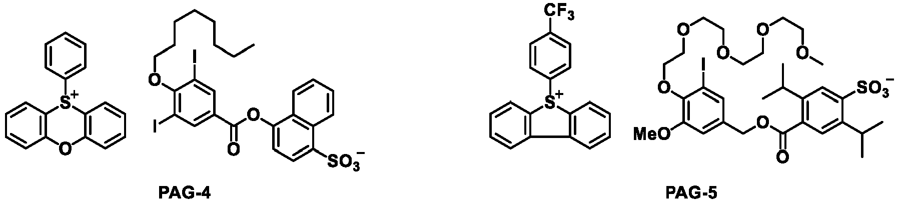

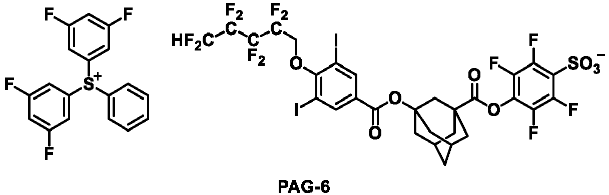

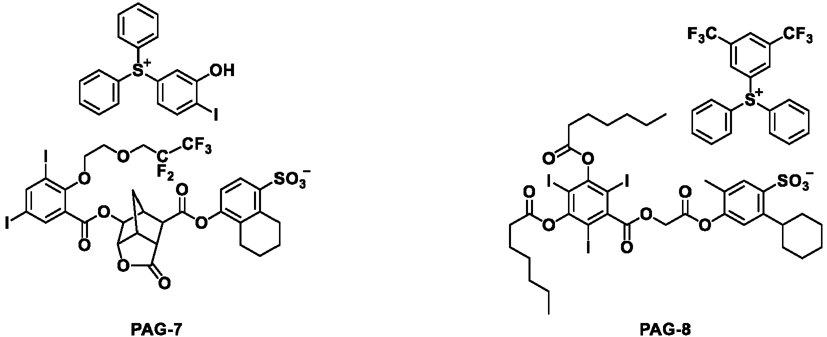

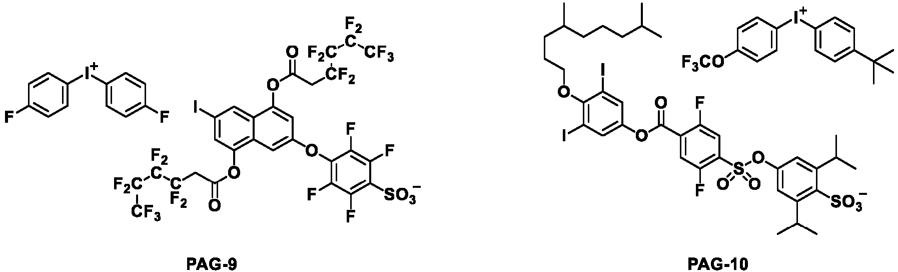

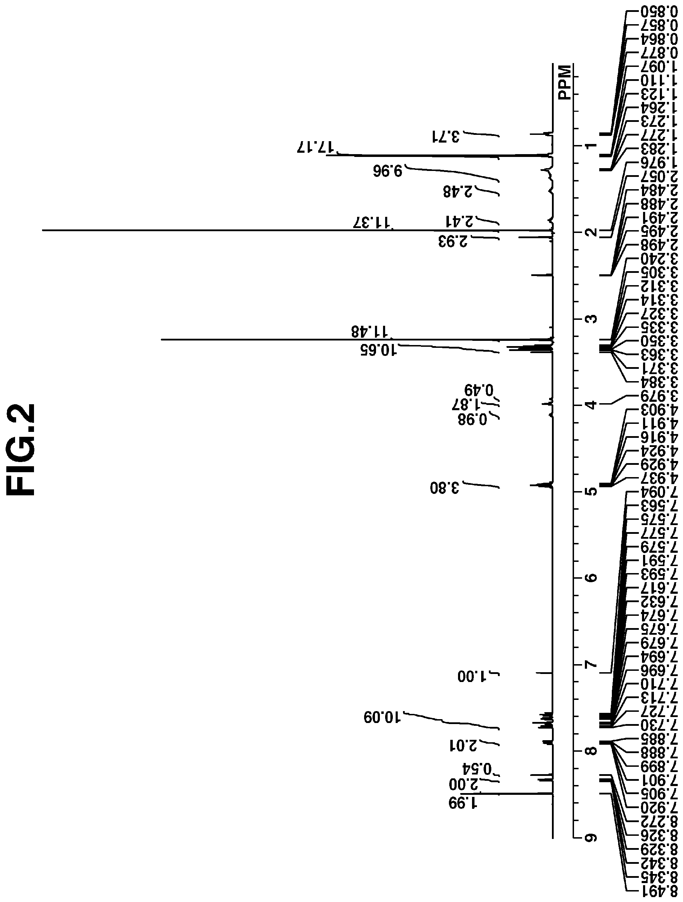

PAG-2 was analyzed by IR and TOF-MS, with the results shown below. The ¹H-NMR/DMSO-d₆ spectrum of PAG-2 is shown in FIG. 2.

| | |
|---|---|
| IR (D-ATR): | v = 3063, 2927, 2856, 1737, 1593, 1574, 1534, 1466, 1440, 1371, 1322, 1269, 1251, 1148, 1100, 1041, 968, 911, 886, 761, 706, 682, 658, 636, 617, 562, 526, 488 cm⁻¹ |
| MALDI TOF-MS: | POSITIVE M⁺ 277 (corresponding to C₁₈H₁₃OS⁺) |
| | NEGATIVE M⁻ 713 (corresponding to C₂₅H₃₁I₂O₆S⁻) |

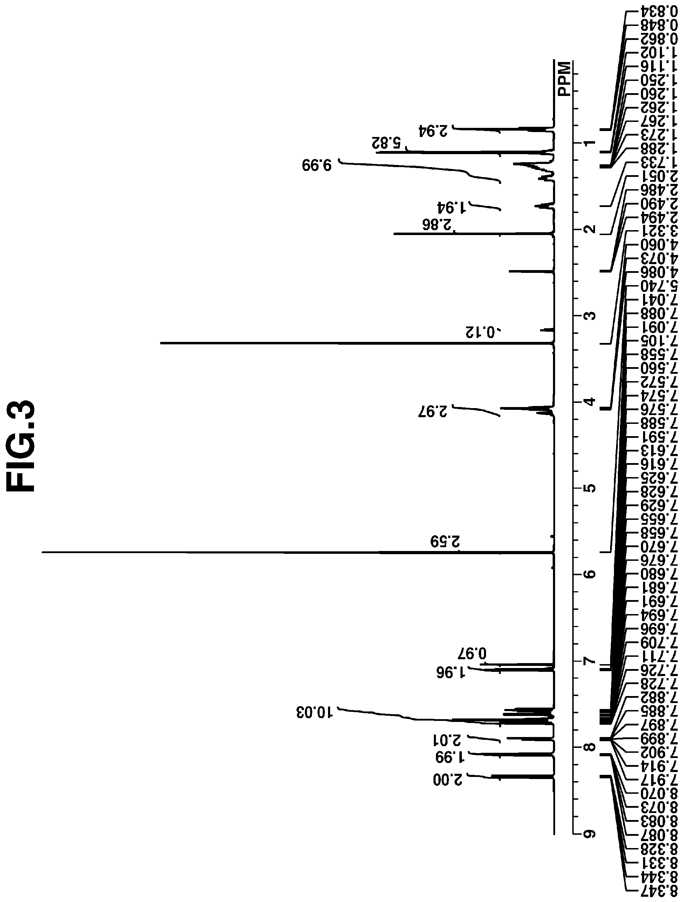

PAG-3 was analyzed by IR and TOF-MS, with the results shown below. The ¹H-NMR/DMSO-d₆ spectrum of PAG-3 is shown in FIG. 3.

| | |
|---|---|
| IR (D-ATR): | v = 3059, 3020, 2927, 2857, 1729, 1605, 1581, 1510, 1465, 1441, 1392, 1318, 1271, 1250, 1216, 1168, 1150, 1088, 1066, 1040, 966, 910, 886, 846, 816, 765, 733, 716, 682, 655, 632, 587, 561, 524, 487 cm⁻¹ |
| MALDI TOF-MS: | POSITIVE M⁺ 277 (corresponding to C₁₈H₁₃OS⁺) |
| | NEGATIVE M⁻ 461 (corresponding to C₂₅H₃₃O₆S⁻) |

### [2] Synthesis of base polymers

### Synthesis Example

### Synthesis of Base Polymers P-1 to P-6

Base polymers P-1 to P-6 were synthesized by combining monomers, performing copolymerization reaction in a solvent, pouring the reaction solution to hexane for precipitation, washing the solid precipitate with hexane, isolation and drying. The polymer was analyzed for composition by ¹H-NMR and ¹³C-NMR spectroscopy and for Mw and Mw/Mn by GPC versus polystyrene standards using THF solvent.

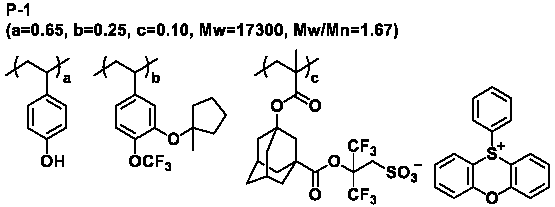

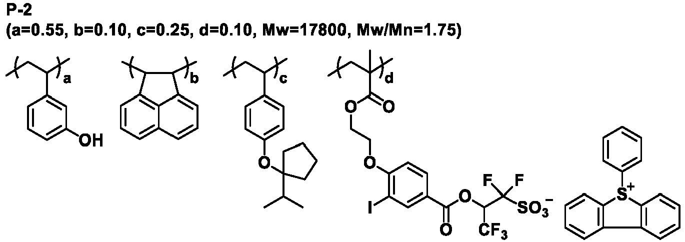

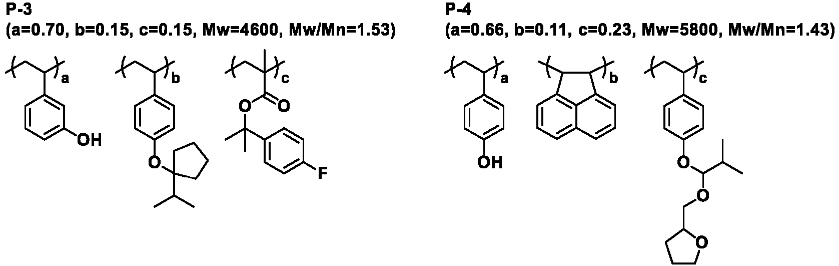

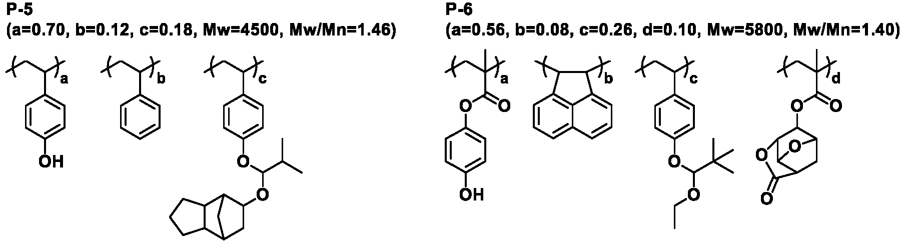

### [3] Preparation of chemically amplified positive resist compositions

### Examples 2-1 to 2-50 and Comparative Examples 1-1 to 1-31

A chemically amplified positive resist composition was prepared by dissolving selected components in an organic solvent in accordance with the formulation shown in Tables 1 to 3, and filtering the solution through a nylon filter with a pore size of 5 nm and a UPE filter with a pore size of 1 nm. The organic solvent was a mixture of 940 pbw of PGMEA, 1,870 pbw of EL and 1,870 pbw of PGME.

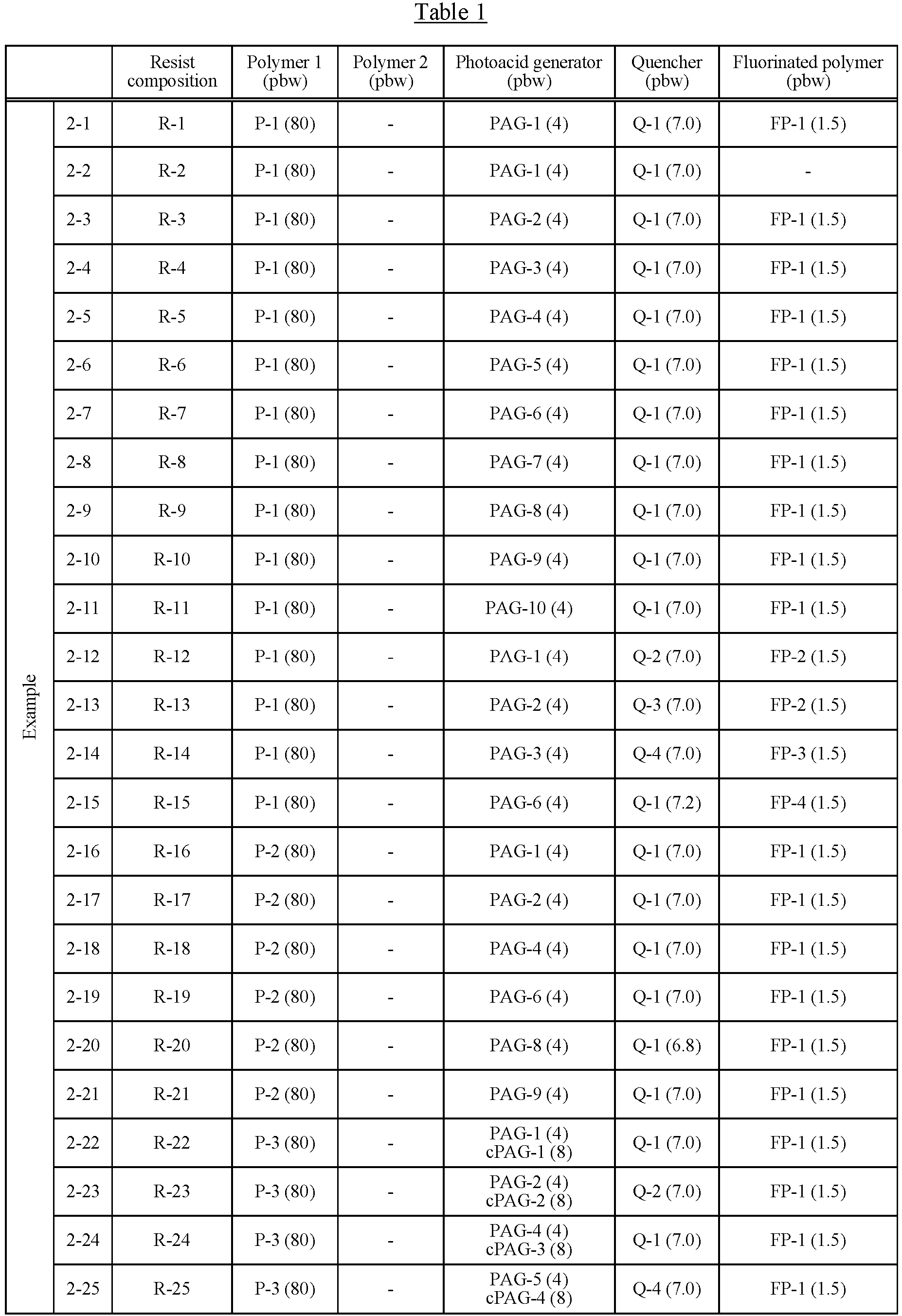

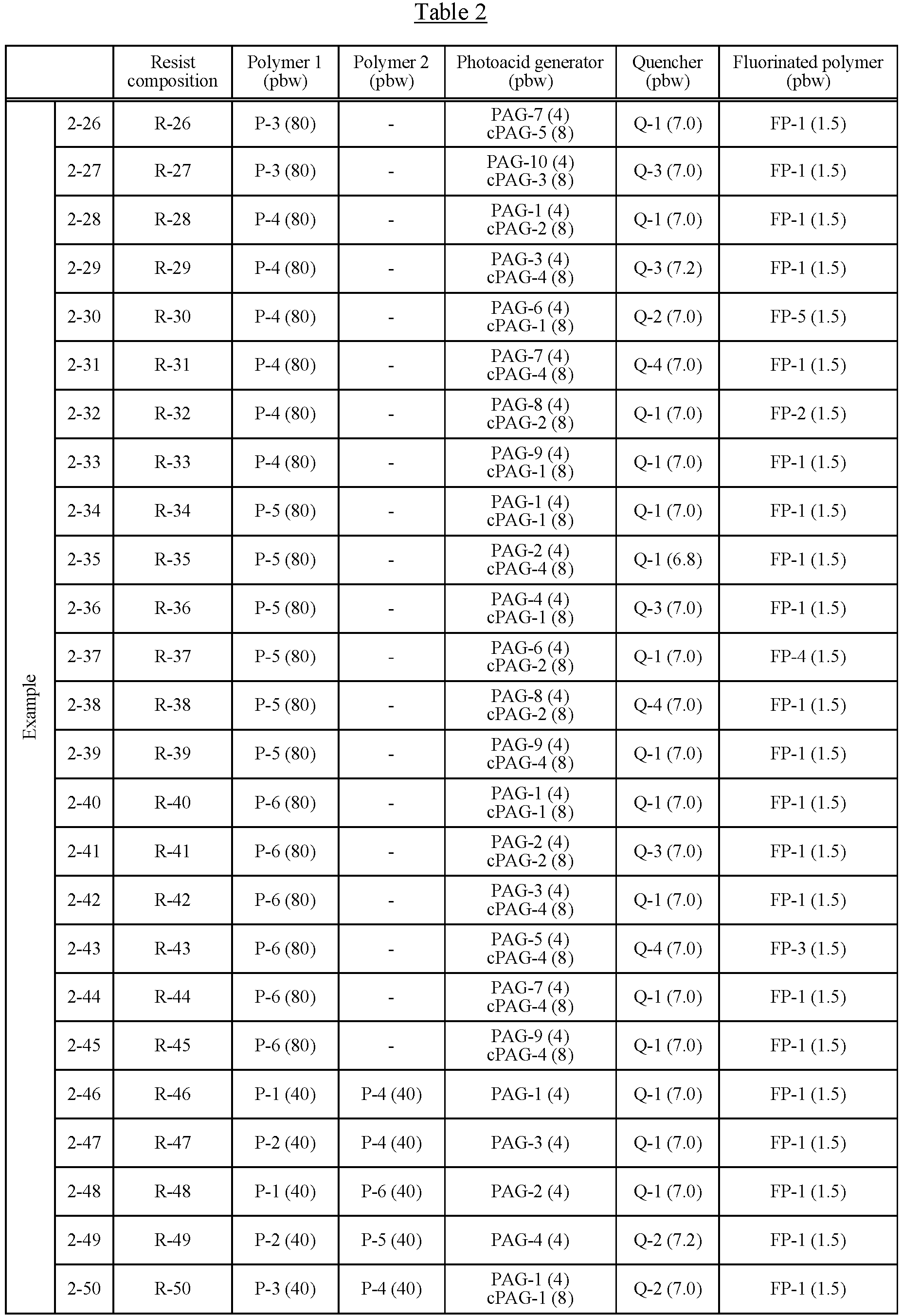

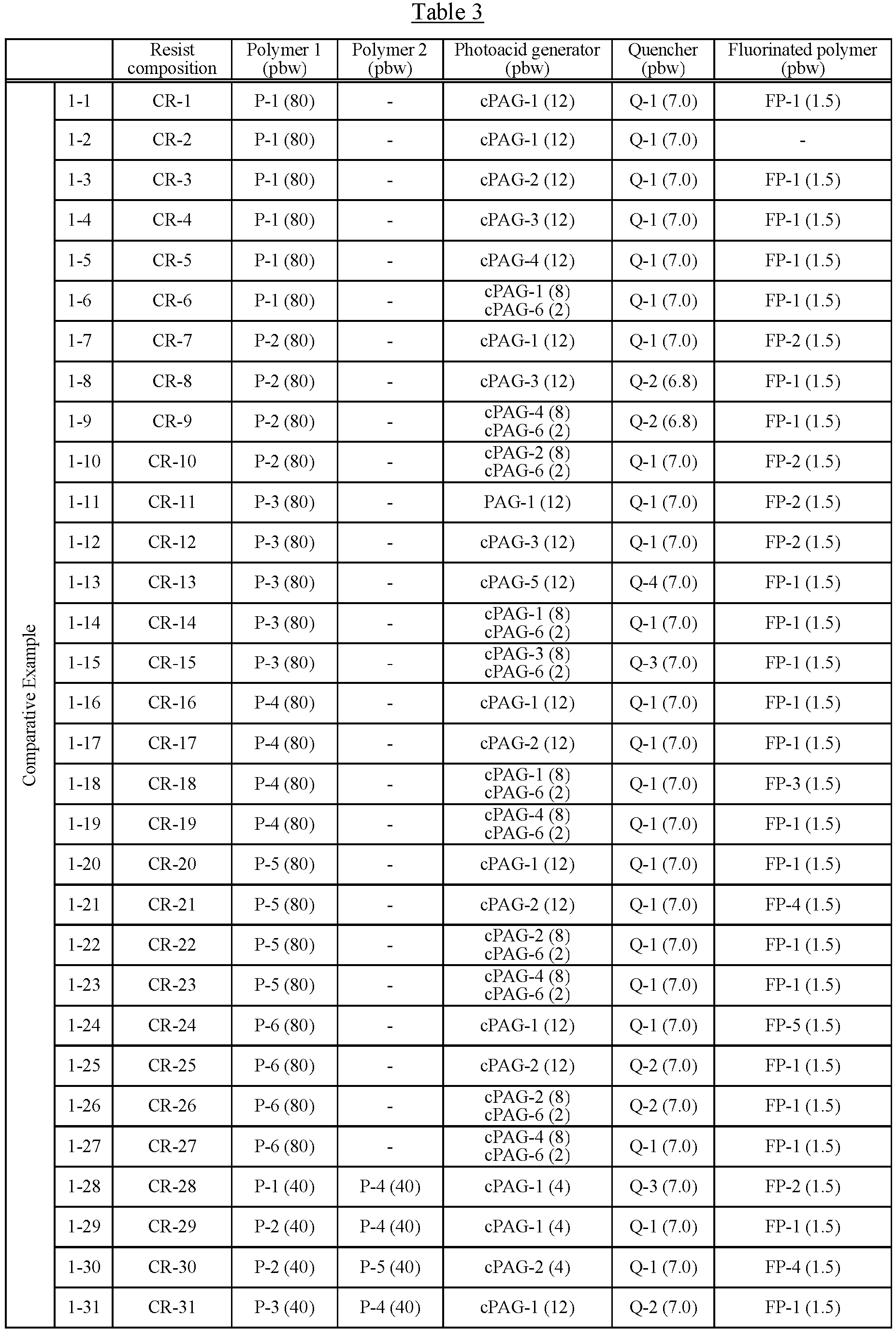

Comparative photoacid generators cPAG-1 to cPAG-6, Quenchers Q-1 to Q-4, and Fluorinated Polymers FP-1 to FP-5 in Tables 1 to 3 are identified below.

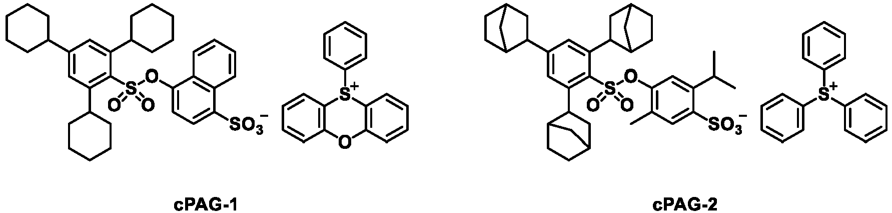

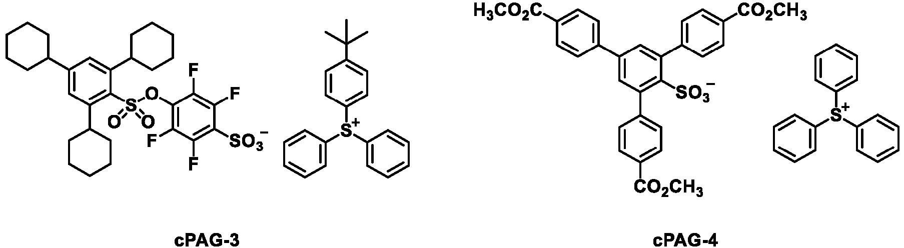

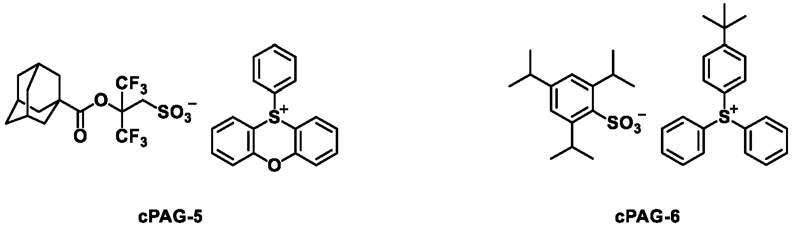

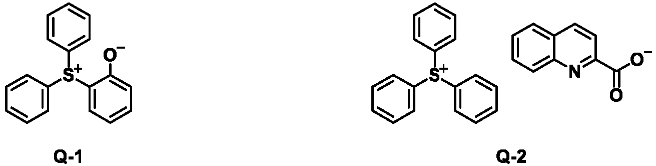

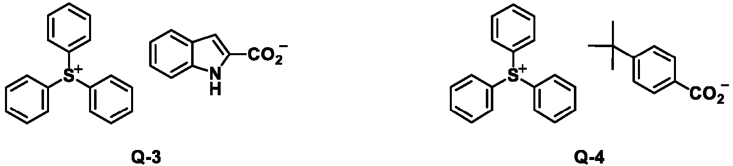

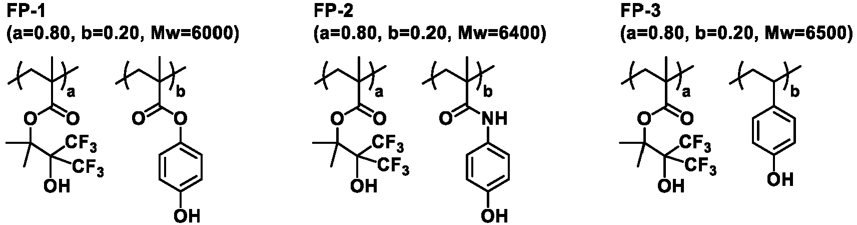

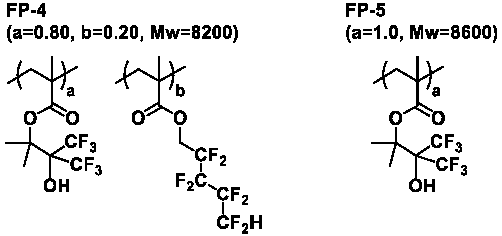

### [4] EB lithography test

### Examples 3-1 to 3-50 and Comparative Examples 2-1 to 2-31

A photomask blank of reflection type for an EUV lithography mask was furnished by starting with a low-coefficient-of-thermal-expansion glass substrate of 6 inches squares and depositing thereon a multilayer reflective film of 40 Mo/Si layers with a thickness of 284 nm, a Ru film of 3.5 nm thick as protective film, a TaN film of 70 nm thick as absorbing layer, and a CrN film of 6 nm thick as hard mask. Using a coater/developer system ACT-M (Tokyo Electron Ltd.), each of the resist compositions (R-1 to R-50, CR-1 to CR-31) was spin coated onto the photomask blank, and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum IS resolution) was defined as the minimum size at the dose which provided a 9:1 resolution for an isolated space (IS) of 200 nm. The edge roughness (LER) of a 200-nm LS pattern was measured under SEM. The pattern was visually observed to judge whether or not the profile was rectangular. The results are shown in Tables 4 to 6.

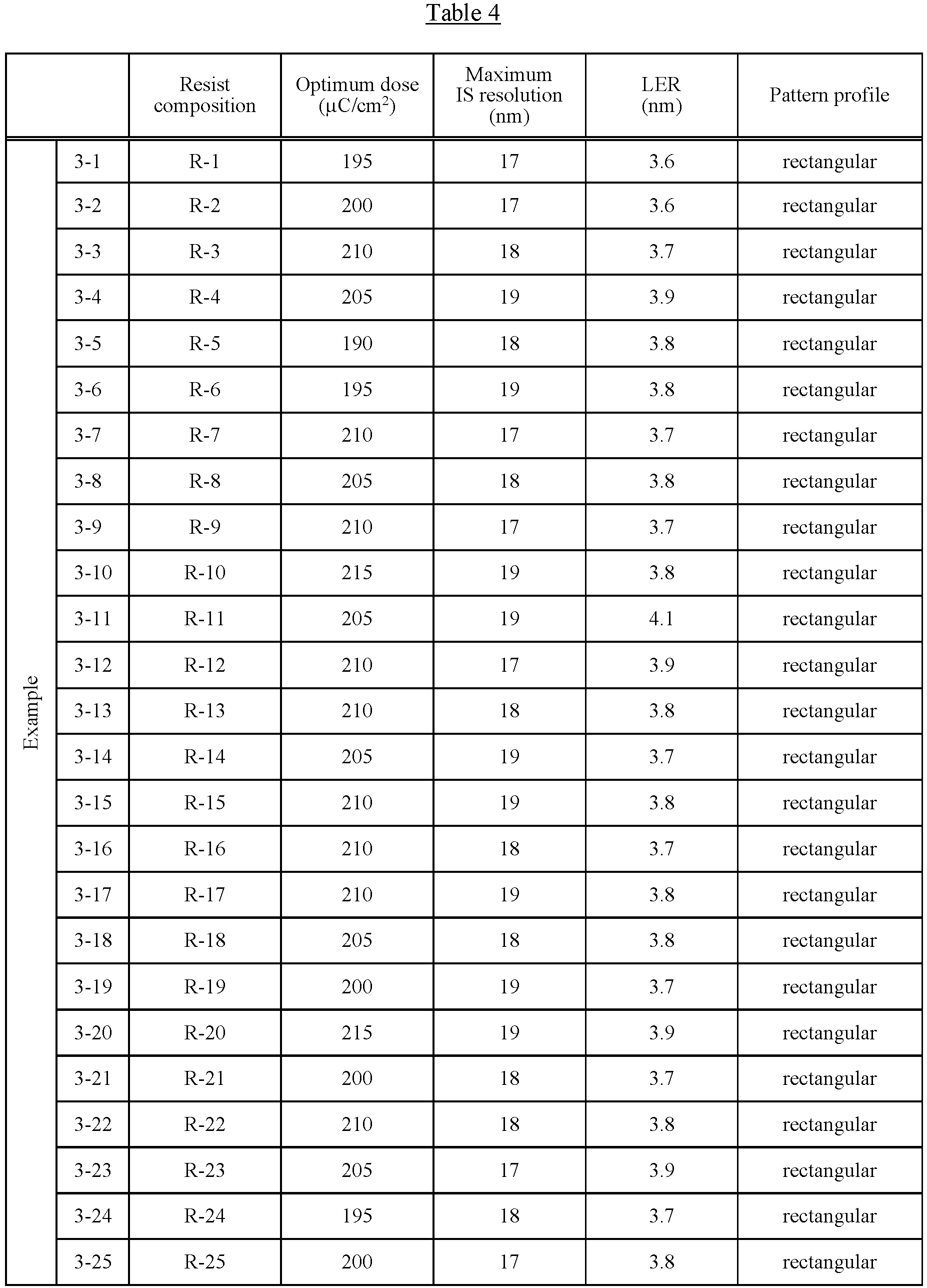

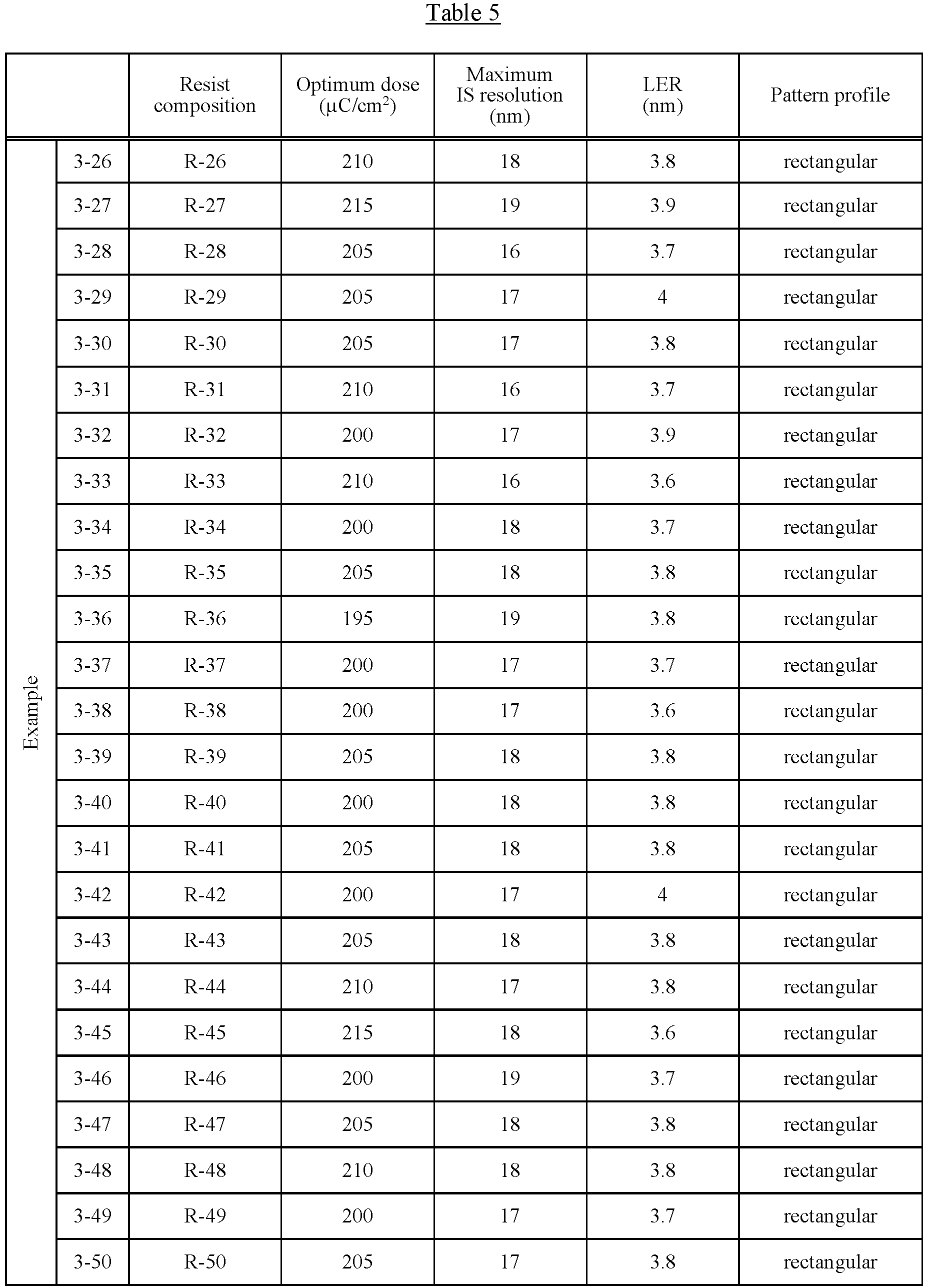

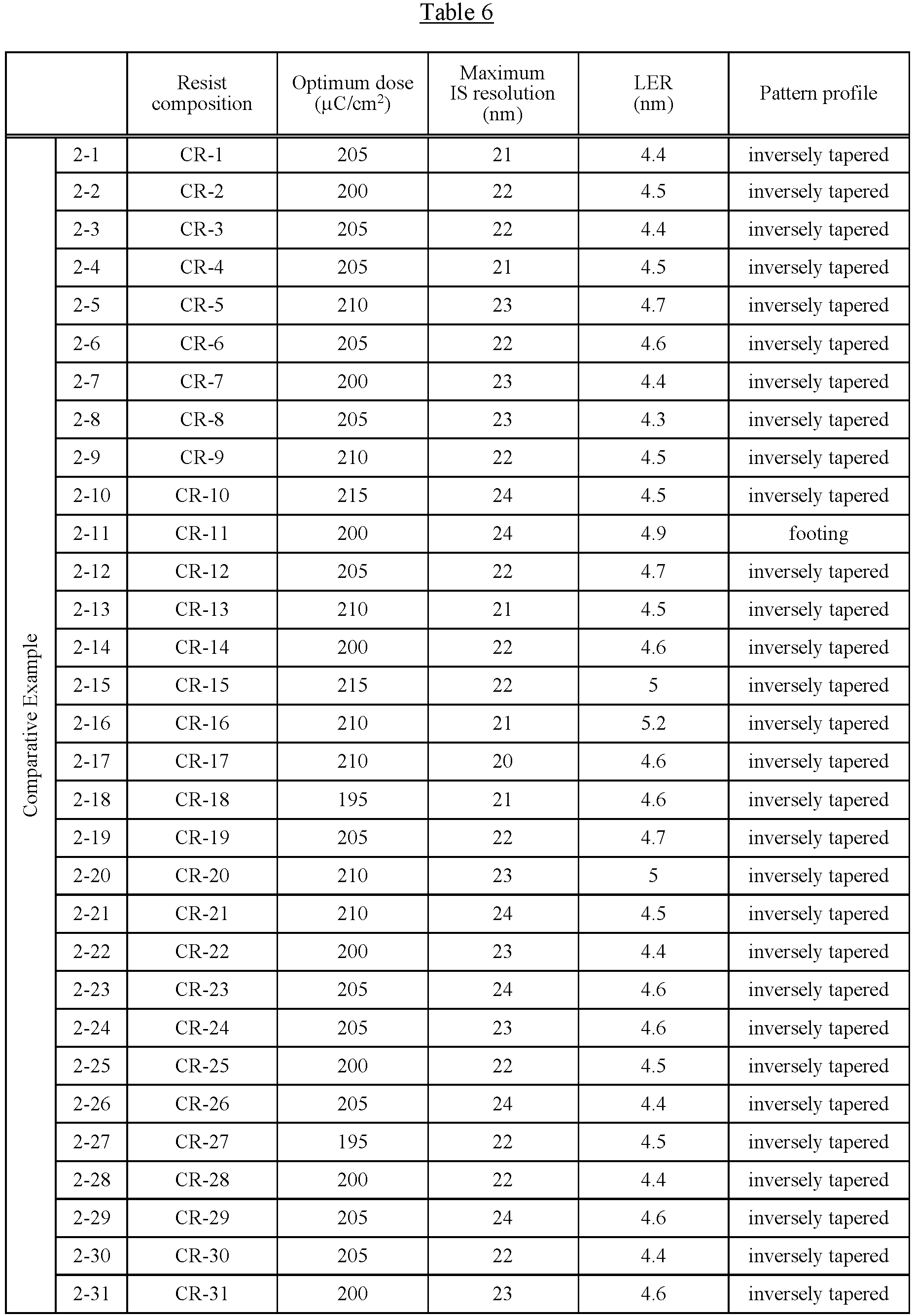

As is evident from Tables 4 to 6, the onium salt or photoacid generator, the chemically amplified positive resist composition and the resist pattern forming process within the scope of the invention are effective in photolithography for the fabrication of semiconductor devices and the processing of photomask blanks of transmission and reflection types.

## Claims

1. An onium salt having the formula (A): 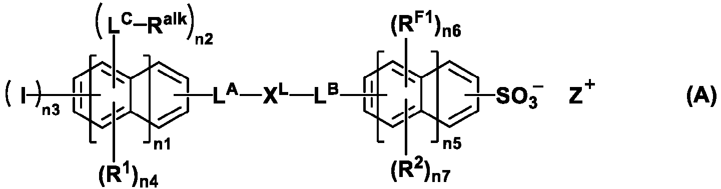 wherein n1 is 0 or 1, n2 is 1, 2 or 3, n3 is an integer of 0 to 4, n4 is an integer of 0 to 4, with the proviso that n2+n3+n4 is from 1 to 5 in case of n1=0, and n2+n3+n4 is from 1 to 7 in case of n1=1,
n5 is 0, 1 or 2, n6 is an integer of 0 to 4 in case of n5=0, an integer of 0 to 6 in case of n5=1, and an integer of 0 to 8 in case of n5=2, n7 is an integer of 0 to 4 in case of n5=0, an integer of 0 to 6 in case of n5=1, and an integer of 0 to 8 in case of n5=2, with the proviso that n6+n7 is from 0 to 4 in case of n5=0, from 0 to 6 in case of n5=1, and from 0 to 8 in case of n5=2,
R^{alk} is a C₆-C₁₈ alkyl group or C₄-C₁₈ fluorinated alkyl group, with the proviso that when R^{alk} is a C₆-C₁₈ alkyl group, the alkyl group has at least one straight chain structure of 6 or more carbon atoms, and when R^{alk} is a C₄-C₁₈ fluorinated alkyl group, the fluorinated alkyl group has at least two groups selected from -CF₂- and -CF₃, some -CH₂- in the alkyl or fluorinated alkyl group may be replaced by an ether bond or carbonyl moiety, and the alkyl or fluorinated alkyl group may contain a cyclic structure selected from cyclopentane, cyclohexane, adamantane, norbornane and benzene rings at the end or in a carbon-carbon bond thereof,
R¹ is a halogen exclusive of iodine or a C₁-C₂₀ hydrocarbyl group which may contain a heteroatom,
R² is a C₁-C₂₀ hydrocarbyl group which may contain a heteroatom, a plurality of R² may bond together to form a ring with the carbon atom to which they are attached when n7 is 2 or more,
R^{F1} is fluorine, a C₁-C₆ fluorinated alkyl group, C₁-C₆ fluorinated alkoxy group, or C₁-C₆ fluorinated thioalkoxy group, a plurality of R^{F1} may be identical or different when n6 is 2 or more,
L^{A}, L^{B}, and L^{C} are each independently a single bond, ether bond, ester bond, sulfonate ester bond, carbonate bond, or carbamate bond,
X^{L} is a single bond or a C₁-C₄₀ hydrocarbylene group which may contain a heteroatom, and
Z⁺ is an onium cation.

2. The onium salt of claim 1 wherein Z⁺ is a sulfonium cation having the formula (cation-1) or iodonium cation having the formula (cation-2): 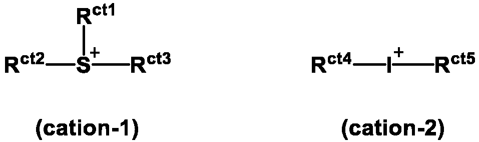 wherein R^{ct1} to R^{ct5} are each independently halogen or a C₁-C₃₀ hydrocarbyl group which may contain a heteroatom, and R^{ct1} and R^{ct2} may bond together to form a ring with the sulfur atom to which they are attached.

3. A photoacid generator comprising the onium salt of claim 1 or 2.

4. A chemically amplified positive resist composition comprising the photoacid generator of claim 3.

5. The chemically amplified positive resist composition of claim 4, further comprising a base polymer containing a polymer which is decomposed under the action of acid to increase its solubility in alkaline developer.

6. The chemically amplified positive resist composition of claim 5 wherein the polymer comprises repeat units having the formula (B1): 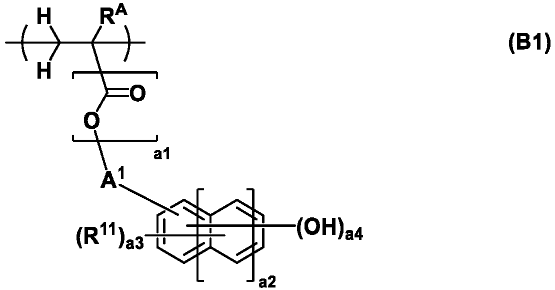 wherein a1 is 0 or 1, a2 is an integer of 0 to 2, a3 is an integer satisfying 0 ≤ a3 ≤ 5+2(a2)-a4, a4 is an integer of 1 to 3,
R^{A} is hydrogen, fluorine, methyl or trifluoromethyl,
R¹¹ is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group, and
A¹ is a single bond or C₁-C₁₀ saturated hydrocarbylene group in which some -CH₂- may be replaced by -O-.

7. The chemically amplified positive resist composition of claim 5 or 6 wherein the polymer further comprises repeat units having the formula (B2-1) or (B2-2): 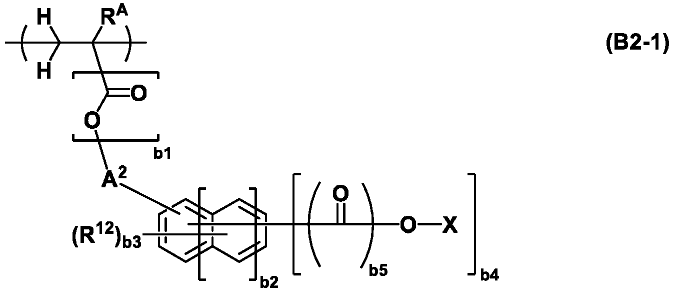 wherein b1 is 0 or 1, b2 is an integer of 0 to 2, b3 is an integer satisfying 0 ≤ b3 ≤ 5+2(b2)-b4, b4 is an integer of 1 to 3, b5 is 0 or 1,
R^{A} is hydrogen, fluorine, methyl or trifluoromethyl,
R¹² is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group,
A² is a single bond or C₁-C₁₀ saturated hydrocarbylene group in which some -CH₂- may be replaced by -O-,
X is an acid labile group when b4 is 1, and X is each independently hydrogen or an acid labile group, at least one being an acid labile group, when b4 is 2 or 3. 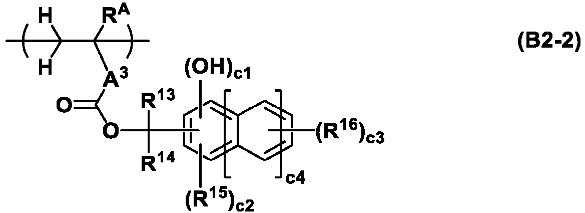 wherein c1 is an integer of 0 to 2, c2 is an integer of 0 to 2, c3 is an integer of 0 to 5, c4 is an integer of 0 to 2,
R^{A} is hydrogen, fluorine, methyl or trifluoromethyl,
R¹³ and R¹⁴ are each independently a C₁-C₁₀ hydrocarbyl group which may contain a heteroatom, R¹³ and R¹⁴ may bond together to form a ring with the carbon atom to which they are attached,
R¹⁵ is each independently fluorine, C₁-C₅ fluorinated alkyl group or C₁-C₅ fluorinated alkoxy group,
R¹⁶ is each independently a C₁-C₁₀ hydrocarbyl group which may contain a heteroatom, and
A³ is a single bond, phenylene group, naphthylene group, or *-C(=O)-O-A³¹-, wherein A³¹ is a C₁-C₂₀ aliphatic hydrocarbylene group which may contain hydroxy, ether bond, ester bond or lactone ring, or a phenylene or naphthylene group, * designates a point of attachment to the carbon atom in the backbone.

8. The chemically amplified positive resist composition of any one of claims 5 to 7 wherein the polymer further comprises repeat units of at least one type selected from repeat units having the formula (B3), repeat units having the formula (B4), and repeat units having the formula (B5): 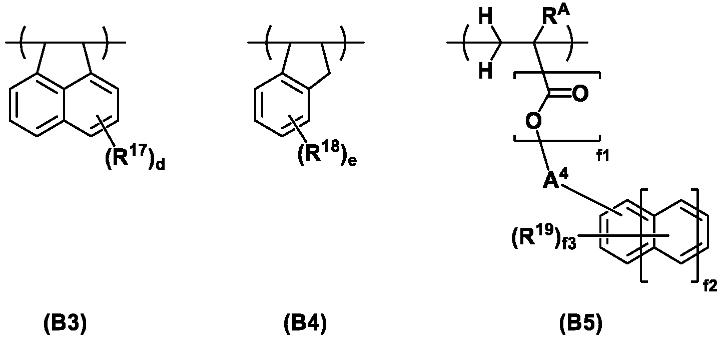 wherein d is an integer of 0 to 6, e is an integer of 0 to 4, f1 is 0 or 1, f2 is an integer of 0 to 2, and f3 is an integer of 0 to 5,
R^{A} is hydrogen, fluorine, methyl or trifluoromethyl,
R¹⁷ and R¹⁸ are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R¹⁹ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group, or cyano group, R¹⁹ may be a hydroxy group when f2 is 1 or 2, and
A⁴ is a single bond or C₁-C₁₀ saturated hydrocarbylene group in which some -CH₂- may be replaced by -O-.

9. The chemically amplified positive resist composition of any one of claims 5 to 8 wherein the polymer further comprises repeat units of at least one type selected from repeat units having the formulae (B6) to (B9): 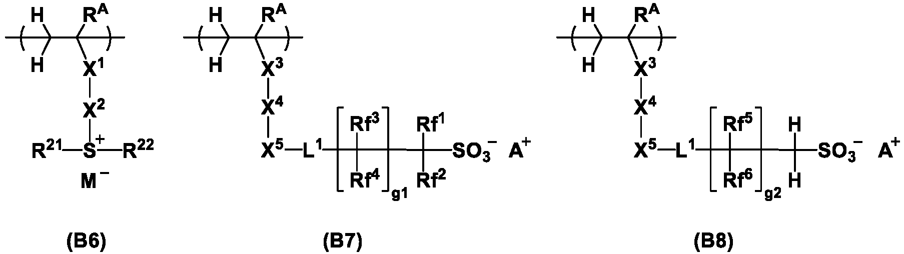 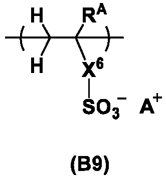 wherein R^{A} is each independently hydrogen, fluorine, methyl or trifluoromethyl,
X¹ is a single bond or phenylene group,
X² is *¹-C(=O)-O-X²¹-, *¹-C(=O)-NH-X²¹- or *¹-O-X²¹-, X²¹ is a C₁-C₆ aliphatic hydrocarbylene group, phenylene group, or divalent group obtained by combining the foregoing, which may contain a carbonyl moiety, ester bond, ether bond or hydroxy moiety, *¹ designates a point of attachment to X¹,
X³ is each independently a single bond, phenylene group, naphthylene group or *²-C(=O)-O-X³¹-, X³¹ is a C₁-C₁₀ aliphatic hydrocarbylene group, phenylene group, or naphthylene group, the aliphatic hydrocarbylene group may contain a hydroxy moiety, ether bond, ester bond or lactone ring, *² designates a point of attachment to the carbon atom in the backbone,
X⁴ is each independently a single bond, *³-X⁴¹-C(=O)-O-, *³-C(=O)-NH-X⁴¹- or *³-O-X⁴¹-, X⁴¹ is a C₁-C₂₀ hydrocarbylene group which may contain a heteroatom, *³ designates a point of attachment to X³,
X⁵ is each independently a single bond, *⁴-X⁵¹-C(=O)-O-, *⁴-C(=O)-NH-X⁵¹- or *⁴-O-X⁵¹-, X⁵¹ is a C₁-C₂₀ hydrocarbylene group which may contain a heteroatom, *⁴ designates a point of attachment to X⁴,
X⁶ is a single bond, methylene, ethylene, phenylene, fluorinated phenylene, trifluoromethyl-substituted phenylene, *²-C(=O)-O-X⁶¹-, *²-C(=O)-N(H)-X⁶¹-, or *²-O-X⁶¹-, X⁶¹ is a C₁-C₆ aliphatic hydrocarbylene group, phenylene group, fluorinated phenylene group, or trifluoromethyl-substituted phenylene group, which may contain a carbonyl moiety, ester bond, ether bond or hydroxy moiety, *² designates a point of attachment to the carbon atom in the backbone,
R²¹ and R²² are each independently a C₁-C₂₀ hydrocarbyl group which may contain a heteroatom, R²¹ and R²² may bond together to form a ring with the sulfur atom to which they are attached,
L¹ is a single bond, ether bond, ester bond, carbonyl group, sulfonate ester bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine or a C₁-C₆ fluorinated saturated hydrocarbyl group,
Rf⁵ and Rf⁶ are each independently hydrogen, fluorine or a C₁-C₆ fluorinated saturated hydrocarbyl group, excluding that all Rf⁵ and Rf⁶ are hydrogen at the same time,
M⁻ is a non-nucleophilic counter ion,
A⁺ is an onium cation,
g1 and g2 are each independently an integer of 0 to 3.

10. The chemically amplified positive resist composition of any one of claims 4 to 9, further comprising an organic solvent.

11. The chemically amplified positive resist composition of any one of claims 4 to 10, further comprising a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 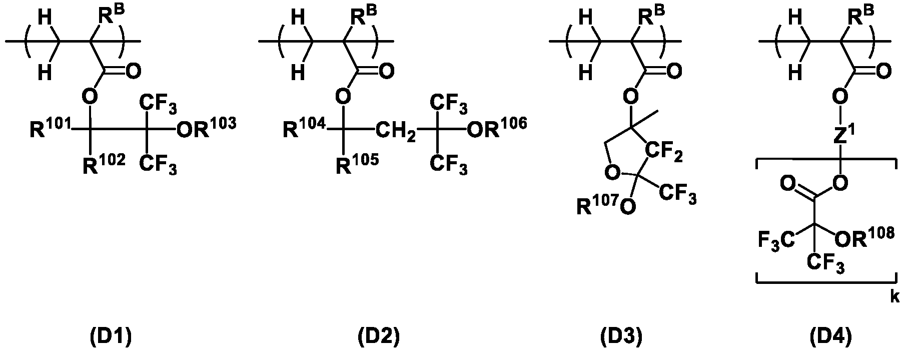 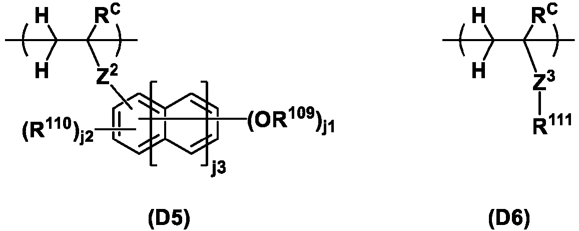 wherein j 1 is an integer of 1 to 3, j2 is an integer satisfying 0 ≤ j2 ≤ 5+2(j3)-j1, j3 is 0 or 1, k is an integer of 1 to 3,
R^{B} is each independently hydrogen, fluorine, methyl or trifluoromethyl,
R^{C} is each independently hydrogen or methyl,
R¹⁰¹, R¹⁰², R¹⁰⁴ and R¹⁰⁵ are each independently hydrogen or a C₁-C₁₀ saturated hydrocarbyl group,
R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
R¹⁰⁹ is hydrogen or a C₁-C₅ straight or branched hydrocarbyl group in which a heteroatom-containing moiety may intervene in a carbon-carbon bond,
R¹¹⁰ is a C₁-C₅ straight or branched hydrocarbyl group in which a heteroatom-containing moiety may intervene in a carbon-carbon bond,
R¹¹¹ is a C₁-C₂₀ saturated hydrocarbyl group in which at least one hydrogen is substituted by fluorine, and in which some -CH₂- may be replaced by an ester bond or ether bond,
Z¹ is a C₁-C₂₀ (k+1)-valent hydrocarbon group or C₁-C₂₀ (k+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

12. The chemically amplified positive resist composition of any one of claims 4 to 11, further comprising a quencher.

13. The chemically amplified positive resist composition of any one of claims 4 to 12, further comprising a photoacid generator other than the photoacid generator of claim 3.

14. A resist pattern forming process comprising the steps of:
applying the chemically amplified positive resist composition of any one of claims 4 to 13 onto a substrate to form a resist film thereon,
exposing the resist film to high-energy radiation, and
developing the exposed resist film in an alkaline developer.

15. The process of claim 14 wherein the substrate is a photomask blank.
